# EUROPEAN PATENT APPLICATION

(11) **EP 4 438 040 A1**
(43) Date of publication of application: **02.10.2024**
(21) Application number: 22864522.2
(22) Date of filing: 30.08.2022
(51) Int. Cl.: A61K 31/424, A61K 31/437, A61K 31/4985, A61K 31/5377, A61K 31/5386, A61P 3/00, A61P 3/06, A61P 9/00, A61P 9/04, A61P 9/10, A61P 9/12, A61P 21/00, A61P 25/00, A61P 25/02, A61P 25/28, A61P 35/00, A61P 43/00, C07D 498/04, C07D 519/00

(54) **NITROGEN-CONTAINING TRICYCLIC COMPOUND AND PHARMACEUTICAL USE THEREOF**

(30) Priority: 01.09.2021 JP 2021142742
(71) Applicant: Japan Tobacco Inc., Tokyo 105-6927 (JP)
(72) Inventor: YAMAKAWA, Maki, Takatsuki-shi, Osaka 569-1125 (JP); SUZAWA, Koichi, Takatsuki-shi, Osaka 569-1125 (JP); YAMASHITA, Tomoya, Takatsuki-shi, Osaka 569-1125 (JP); UENO, Hiroshi, Takatsuki-shi, Osaka 569-1125 (JP); MANABE, Tomoyuki, Takatsuki-shi, Osaka 569-1125 (JP)
(74) Representative: Vossius & Partner Patentanwälte Rechtsanwälte mbB
(86) International application number: PCT/JP2022/032483
(87) International publication number: WO 2023/032940

(57) **Abstract**

The present invention provides a compound having a PDHK inhibitory activity and useful for the treatment or prophylaxis of diabetes (type 1 diabetes, type 2 diabetes etc.), insulin resistance syndrome, metabolic syndrome, hyperglycemia, hyperlactacidemia, diabetic complications (diabetic neuropathy, diabetic retinopathy, diabetic nephropathy, cataract etc.), cardiac failure (acute cardiac failure, chronic cardiac failure), cardiomyopathy, myocardial ischemia, myocardial infarction, angina pectoris, dyslipidemia, atherosclerosis, peripheral arterial disease, intermittent claudication, chronic obstructive pulmonary diseases, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy or chronic kidney disease. The present invention relates to a compound of the formula [I], the formula [II] or the formula [III], or a pharmaceutically acceptable salt thereof: 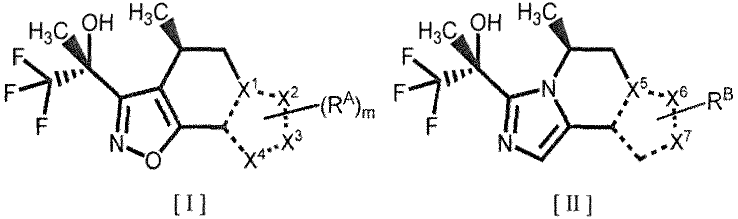 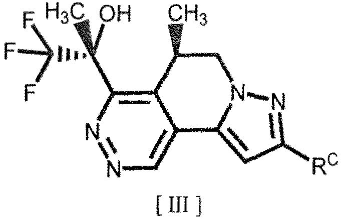 wherein each symbol is as defined in the DESCRIPTION.

## Description

### [Technical Field]

The present invention relates to a nitrogen-containing tricyclic compound and a pharmaceutical use thereof. More particularly, the present invention relates to a nitrogen-containing tricyclic compound or a pharmaceutically acceptable salt thereof having a pyruvate dehydrogenase kinase (hereinafter to be abbreviated as PDHK) inhibitory activity, a pharmaceutical composition containing the same, a therapeutic or prophylactic agent containing the same for diabetes (type 1 diabetes, type 2 diabetes etc.), insulin resistance syndrome, metabolic syndrome, hyperglycemia, hyperlactacidemia, diabetic complications (diabetic neuropathy, diabetic retinopathy, diabetic nephropathy, cataract etc.), cardiac failure (acute cardiac failure, chronic cardiac failure), cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy, or chronic kidney disease, and the like.

### [Background Art]

In tissues, for reactions using energy such as biosynthesis, active transport, muscle contraction and the like, the energy is supplied by hydrolysis of adenosine triphosphate (ATP). ATP is produced by oxidation of metabolic fuel which yields much energy, such as glucose and free fatty acids. In oxidative tissues such as muscle, ATP is mostly produced from acetyl-CoA that enters citric acid cycle. Acetyl-CoA is produced by oxidation of glucose via glycolytic pathway or β oxidation of free fatty acid. An enzyme that plays a pivotal role in controlling acetyl-CoA production from glucose is pyruvate dehydrogenase (hereinafter to be abbreviated as PDH). PDH catalyzes reduction of nicotinamide adenine dinucleotide (NAD) to NADH, simultaneously with oxidation of pyruvic acid to acetyl-CoA and carbon dioxide (e.g., non-patent documents 1, 2).

PDH is a multienzyme complex consisting of three enzyme components (E1, E2 and E3) and some subunits localized in mitochondrial matrix. E1, E2 and E3 are responsible for decarboxylation from pyruvic acid, production of acetyl-CoA and reduction of NAD to NADH, respectively.

Two classes of enzyme having regulatory function bind to PDH. One is PDHK, which is a protein kinase having specificity to PDH. The role thereof is to inactivate E1α subunit of the PDH complex by phosphorylation. The other is PDH phosphatase, which is a specific protein phosphatase that activates PDH via dephosphorylation of E1α subunit. The proportion of PDH in its active (dephosphorylated) state is determined by the balance of kinase activity and phosphatase activity. The kinase activity is regulated by the relative concentration of metabolic substrates. For example, the kinase activity is activated by an increase in NADH/NAD, acetyl-CoA/CoA and ATP/adenosine diphosphate (ADP) ratios, and inhibited by pyruvic acid (e.g., non-patent document 3).

In the tissues of mammals, 4 kinds of PDHK isozymes are identified. Particularly, PDHK2 is expressed in a wide range of tissues including the liver, skeletal muscles and adipose tissues involved in glucose metabolism. Furthermore, since PDHK2 shows comparatively high sensitivity to activation by increased NADH/NAD or acetyl-CoA/CoA and inhibition by pyruvic acid, involvement in a short-term regulation of glucose metabolism is suggested (e.g., non-patent document 4).

In addition, PDHK1 is expressed in large amounts in cardiac muscle, skeletal muscle, pancreatic β cell and the like. Furthermore, since expression of PDHK1 is induced via activation of hypoxia inducible factor (HIF) 1 in ischemic state, its involvement in ischemic diseases and cancerous diseases is suggested (e.g., non-patent document 5).

In diseases such as insulin-dependent (type 1) diabetes, non-insulin-dependent (type 2) diabetes and the like, oxidation of lipids is promoted with simultaneous reduction in glucose utilization. This reduction in glucose utilization is one of the factors causing hyperglycemia. When the oxidative glucose metabolism decreases in type 1 and type 2 diabetes and obesity, PDH activity also decreases. It suggests involvement of reduced PDH activity in the reduced glucose utilization in type 1 and type 2 diabetes (e.g., non-patent documents 6, 7).

On the contrary, hepatic gluconeogenesis is enhanced in type 1 and type 2 diabetes, which also forms one factor causing hyperglycemia. The reduced PDH activity increases pyruvic acid concentration, which in turn increases availability of lactic acid as a substrate for hepatic gluconeogenesis. It suggests possible involvement of reduced PDH activity in the enhanced gluconeogenesis in type 1 and type 2 diabetes (e.g., non-patent documents 8, 9).

When PDH is activated by inhibition of PDHK, the rate of glucose oxidation is considered to rise. As a result, glucose utilization in the body is promoted and hepatic gluconeogenesis is suppressed, whereby hyperglycemia in type 1 and type 2 diabetes is expected to be improved (e.g., non-patent documents 10, 11, 12).

Another factor contributing to diabetes is impaired insulin secretion, which is known to be associated with reduced PDH activity in pancreatic β cells, and induction of PDHK1, 2 and 4 (e.g., non-patent documents 13, 14).

In addition, sustained hyperglycemia due to diabetes is known to cause complications such as diabetic neuropathy, diabetic retinopathy, diabetic nephropathy and the like. Thiamine and α-lipoic acid contribute to activation of PDH as coenzymes. Thiamine and α-lipoic acid, or thiamine derivatives and α-lipoic acid derivatives are shown to have a promising effect on the treatment of diabetic complications. Thus, activation of PDH is expected to improve diabetic complications (e.g., non-patent documents 15, 16).

Under ischemic conditions, limited oxygen supply reduces oxidation of both glucose and fatty acid and reduces the amount of ATP produced by oxidative phosphorylation in the tissues. In the absence of sufficient oxygen, ATP level is maintained by promoted anaerobic glycolysis. As a result, lactic acid increases and intracellular pH decreases. Even though the cells try to maintain homeostasis of ion by energy consumption, abnormally low ATP level and disrupted cellular osmolarity lead to cell death. In addition, adenosine monophosphate-activating kinase activated in an ischemic state inactivates acetyl-CoA carboxylase by phosphorylation. The levels of total malonyl-CoA in the tissue drop, carnitine palmitoyltransferase-I activity is therefore increased and fatty acid oxidation is favored over glucose oxidation by allowing the transport of acyl-CoA into mitochondria. Oxidation of glucose is capable of yielding more ATP per molecule of oxygen than is oxidation of fatty acids. Under ischemic conditions, therefore, when energy metabolism becomes glucose oxidation dominant by activation of PDH, the ability to maintain ATP level is considered to be enhanced (e.g., non-patent document 17).

In addition, since activation of PDH causes oxidation of pyruvic acid produced by glycolysis, and reducing production of lactic acid, the net proton burden is considered to be reduced in ischemic tissues. Therefore, PDH activation by inhibition of PDHK is expected to protectively act in ischemic diseases such as cardiac muscle ischemia (e.g., non-patent documents 18, 19).

A drug that activates PDH by inhibition of PDHK is considered to decrease lactate production since it promotes pyruvate metabolism. Hence, such drug is expected to be useful for the treatment of hyperlactacidemia such as mitochondrial disease, mitochondrial encephalomyopathy and sepsis (e.g., non-patent document 20).

In cancer cells, the expression of PDHK1 or 2 increases. In cancer cells, moreover, ATP production by oxidative phosphorylation in mitochondria decreases, and ATP production via the anaerobic glycolysis in cytoplasm increases. PDH activation by inhibition of PDHK is expected to promote oxidative phosphorylation in mitochondria, and increase production of active oxygen, which will induce apoptosis of cancer cells. Therefore, the PDH activation by PDHK inhibition is useful for the treatment of cancerous diseases (e.g., non-patent document 21).

Pulmonary hypertension is characterized by high blood pressure caused by partial narrowing of the pulmonary artery due to promoted cellular proliferation therein. In pulmonary hypertension, therefore, activation of PDH in the pulmonary artery cell is expected to promote oxidative phosphorylation in mitochondria, increase production of active oxygen, and induce apoptosis of the pulmonary artery cells. Therefore, the PDH activation by PDHK inhibition is considered to be useful for the treatment of pulmonary hypertension, for example, pulmonary arterial hypertension (e.g., non-patent document 22).

Energy production and glucose metabolism in the cerebrum decrease in Alzheimer disease, and also, PDH activity declines. When the PDH activity declines, production of acetyl CoA decreases. Acetyl CoA is utilized for ATP production in the electron transport system via the citric acid cycle. Acetyl CoA is also a starting material for synthesizing acetylcholine, which is one of the neurotransmitters. Therefore, reduced brain PDH activity in Alzheimer disease is considered to cause neuronal cell death due to the decreased ATP production. Moreover, it is considered that synthesis of acetylcholine, which is the transmitter for cholinergic nerve, is inhibited to induce deterioration of memory and the like. Activation of PDH in the brain is expected to enhance energy production and acetylcholine synthesis in Alzheimer disease. Therefore, activation of PDH by the inhibition of PDHK is considered to be useful for the treatment of Alzheimer disease (e.g., non-patent documents 23, 24).

Vascular dementia is a disease that is roughly classified into a large-vessel type and a small-vessel type. In the large-vessel type, cerebral infarction including ischemia reperfusion is a factor, and neuronal cell death is induced by an increase in pyruvic acid and lactic acid values due to a decrease in the intracerebral PDH activity, and a decrease in energy production. In the small-vessel type, white matter lesion due to cerebral hypoperfusion is a factor and is considered to cause cognitive dysfunction due to a chronic decrease in glucose metabolism. When PDH in the brain is activated in vascular dementia, a decrease in the lactic acid value and the like and an increase in the energy production are expected in the large-vessel type, and promoted glucose metabolism is expected in the small-vessel type. Therefore, activation of PDH by PDHK inhibitors is considered to be useful for the treatment of vascular dementia (e.g., non-patent documents 28, 29, 30).

It has been shown that dichloroacetic acid, which is a drug having a PDH activating action, provides promising effects for the treatment of diabetes, myocardial ischemia, myocardial infarction, angina pectoris, cardiac failure, hyperlactacidemia, brain ischemia, cerebral apoplexy, peripheral arterial disease, chronic obstructive pulmonary disease, cancerous disease, and pulmonary hypertension (e.g., non-patent documents 10, 18, 20, 22, 25, 26, 27).

It has been shown that a compound having a PDHK inhibitory action has a neuroprotective effect on retinal ischemia-reperfusion injury (non-patent document 31). Retinal ischemia injury is involved in diseases such as glaucoma, diabetic retinopathy, retinopathy of prematurity, retinal vein occlusion and the like.

It has also been shown that in disease model animals exhibiting chronic kidney disease-like renal disorder and decreased renal function, compounds having a PDHK inhibitory action reduce the severity of the diseases (patent document 1).

From the foregoing findings, a PDHK inhibitor is considered to be useful for the treatment or prophylaxis of diseases relating to glucose utilization disorder, for example, diabetes (type 1 diabetes, type 2 diabetes etc.), insulin resistance syndrome, metabolic syndrome, hyperglycemia, hyperlactacidemia, diabetic complications (diabetic neuropathy, diabetic retinopathy, diabetic nephropathy, cataract etc.). Furthermore, a PDHK inhibitor is considered to be useful for the treatment or prophylaxis of diseases caused by limited energy substrate supply to the tissues, for example, cardiac failure (acute cardiac failure, chronic cardiac failure), cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, Alzheimer disease, vascular dementia, glaucoma, diabetic retinopathy, retinopathy of prematurity, retinal vein occlusion, ischemic optic neuropathy and chronic kidney disease. Furthermore, a PDHK inhibitor is considered to be useful for the treatment or prophylaxis of mitochondrial disease, mitochondrial encephalomyopathy, cancer, pulmonary hypertension and the like.

Therefore, a PDHK inhibitor is considered to be useful for the treatment or prophylaxis of diabetes (type 1 diabetes, type 2 diabetes etc.), insulin resistance syndrome, metabolic syndrome, hyperglycemia, hyperlactacidemia, diabetic complications (diabetic neuropathy, diabetic retinopathy, diabetic nephropathy, cataract etc.), cardiac failure (acute cardiac failure, chronic cardiac failure), cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy, or chronic kidney disease.

### [Citation List]

### [Patent Literature]

[PTL 1]
WO 2020/054734

### [Non Patent Literature]

[NPL 1]
   Reed LJ, Hackert ML. Structure-function relationships in dihydrolipoamide acyltransferases. J Biol Chem. 1990 Jun 5; 265(16):8971-4.
[NPL 2]
   Patel MS, Roche TE. Molecular biology and biochemistry of pyruvate dehydrogenase complexes. FASEB J. 1990 Nov; 4 (14) :3224-33.
[NPL 3]
   Sugden MC, Holness MJ. Recent advances in mechanisms regulating glucose oxidation at the level of the pyruvate dehydrogenase complex by PDKs. Am J Physiol Endocrinol Metab. 2003 May; 284(5):E855-62.
[NPL 4]
   Bowker-Kinley MM, Davis WI, Wu P, Harris RA, Popov KM. Evidence for existence of tissue-specific regulation of the mammalian pyruvate dehydrogenase complex. Biochem J. 1998 Jan 1; 329 (Pt 1):191-6.
[NPL 5]
   Kim JW, Tchernyshyov I, Semenza GL, Dang CV. HIF-1-mediated expression of pyruvate dehydrogenase kinase: a metabolic switch required for cellular adaptation to hypoxia. Cell Metab. 2006 Mar; 3(3):177-85.
[NPL 6]
   Morino K, Petersen KF, Dufour S, Befroy D, Frattini J, Shatzkes N, et al. Reduced mitochondrial density and increased IRS-1 serine phosphorylation in muscle of insulin-resistant offspring of type 2 diabetic parents. J Clin Invest. 2005 Dec; 115(12):3587-93.
[NPL 7]
   Caterson ID, Fuller SJ, Randle PJ. Effect of the fatty acid oxidation inhibitor 2-tetradecylglycidic acid on pyruvate dehydrogenase complex activity in starved and alloxan-diabetic rats. Biochem J. 1982 Oct 15; 208(1):53-60.
[NPL 8]
   Boden G, Chen X, Stein TP. Gluconeogenesis in moderately and severely hyperglycemic patients with type 2 diabetes mellitus. Am J Physiol Endocrinol Metab. 2001 Jan; 280(1):E23-30.
[NPL 9]
   Shangraw RE, Fisher DM. Pharmacokinetics and pharmacodynamics of dichloroacetate in patients with cirrhosis. Clin Pharmacol Ther. 1999 Oct; 66(4):380-90.
[NPL 10]
   Stacpoole PW, Moore GW, Kornhauser DM. Metabolic effects of dichloroacetate in patients with diabetes mellitus and hyperlipoproteinemia. NEngl J Med. 1978 Mar 9; 298(10):526-30.
[NPL 11]
   Mayers RM, Leighton B, Kilgour E. PDH kinase inhibitors: a novel therapy for Type II diabetes? Biochem Soc Trans. 2005 Apr; 33(Pt 2):367-70.
[NPL 12]
   Jeoung NH, Rahimi Y, Wu P, Lee WN, Harris RA. Fasting induces ketoacidosis and hypothermia in PDHK2/PDHK4-double-knockout mice. Biochem J.2012 May 1; 443(3):829-39.
[NPL 13]
   Zhou YP, Berggren PO, Grill V. A fatty acid-induced decrease in pyruvate dehydrogenase activity is an important determinant of beta-cell dysfunction in the obese diabetic db/db mouse. Diabetes. 1996 May; 45(5):580-6.
[NPL 14]
   Xu J, Han J, Epstein PN, Liu YQ. Regulation of PDK mRNA by high fatty acid and glucose in pancreatic islets. Biochem Biophys Res Commun. 2006 Jun 9; 344(3):827-33.
[NPL 15]
   Benfotiamine. Monograph. Altern Med Rev. 2006 Sep; 11(3) :238-42.
[NPL 16]
   Vallianou N, Evangelopoulos A, Koutalas P. Alpha-lipoic Acid and diabetic neuropathy. Rev Diabet Stud. 2009 Winter; 6(4) :230-6.
[NPL 17]
   Ussher JR, Lopaschuk GD. The malonyl CoA axis as a potential target for treating ischaemic heart disease. Cardiovasc Res. 2008 Jul 15; 79(2) :259-68.
[NPL 18]
   Wargovich TJ, MacDonald RG, Hill JA, Feldman RL, StacpoolePW, Pepine CJ. Myocardial metabolic and hemodynamic effects of dichloroacetate in coronary arterial disease. Am J Cardiol. 1988 Jan 1; 61 (1):65-70.
[NPL 19]
   Taniguchi M, Wilson C, Hunter CA, Pehowich DJ, Clanachan AS, Lopaschuk GD. Dichloroacetate improves cardiac efficiency after ischemia independent of changes in mitochondrial proton leak. Am J Physiol Heart Circ Physiol. 2001 Apr; 280(4) :H1762-9.
[NPL 20]
   Stacpoole PW, Nagaraja NV, Hutson AD. Efficacy of dichloroacetate as a lactate-lowering drug. J Clin Pharmacol. 2003 Jul; 43 (7):683-91.
[NPL 21]
   Bonnet S, Archer SL, Allalunis-Turner J, Haromy A, Beaulieu C, Thompson R, et al. A mitochondria-K+ channel axis is suppressed in cancer and its normalization promotes apoptosis and inhibits cancer growth. Cancer Cell.2007 Jan; 11(1) :37-51.
[NPL 22]
   McMurtry MS, Bonnet S, Wu X, Dyck JR, Haromy A, Hashimoto K, et al. Dichloroacetate prevents and reverses pulmonary hypertension by inducing pulmonary artery smooth muscle cell apoptosis. Circ Res. 2004 Oct 15; 95(8):830-40.
[NPL 23]
   Saxena U. Bioenergetics breakdown in Alzheimer's disease: targets for new therapies. Int J Physiol Pathophysiol Pharmacol. 2011; 3(2):133-9.
[NPL 24]
   Stacpoole PW. The pyruvate dehydrogenase complex as a therapeutic target for age-related diseases. Aging Cell. 2012 Jun; 11(3):371-7.
[NPL 25]
   Marangos PJ, Turkel CC, Dziewanowska ZE, Fox AW. Dichloroacetate and cerebral ischaemia therapeutics. Expert Opin Investig Drugs. 1999 Apr; 8(4):373-82.
[NPL 26]
   Calvert LD, Shelley R, Singh SJ, Greenhaff PL, Bankart J, Morgan MD, et al. Dichloroacetate enhances performance and reduces blood lactate during maximal cycle exercise in chronic obstructive pulmonary disease. Am J Respir Crit Care Med. 2008 May 15; 177(10):1090-4.
[NPL 27]
   Flavin DF. Non-Hodgkin's Lymphoma Reversal with Dichloroacetate. J Oncol. Hindawi Publishing Corporation Journal of Oncology Volume 2010, Article ID 414726, 4 pages doi:10.1155/2010/414726.
[NPL 28]
   Froelich L, Goetz ME, Weinmueller M, Youdim MB, Barth N, Dirr A, Gsell W, Jellinger K, Beckmann H, Riederer P. (r)-, but not (s)-alpha lipoic acid stimulates deficient brain pyruvate dehydrogenase complex in vascular dementia, but not in Alzheimer dementia. J Neural Transm (Vienna). 2004 Mar; 111(3):295-310
[NPL 29]
   Parnetti L, Reboldi GP, Gallai V. Cerebrospinal fluid pyruvate levels in Alzheimer's disease and vascular dementia. Neurology. 2000 Feb 8; 54(3):735-7.
[NPL 30]
   Pascual B, Prieto E, Arbizu J, Marti-Climent J, Olier J, Masdeu JC. Brain glucose metabolism in vascular white matter disease with dementia: differentiation from Alzheimer disease. Stroke. 2010 Dec; 41(12):2889-93.
[NPL 31]
   Sato K, Mochida S, Tomimoto D, Konuma T, Kiyota N, Tsuda S, Shiga Y, Omodaka K, Nakazawa T. A pyruvate dehydrogenase kinase inhibitor prevents retinal cell death and improves energy metabolism in rat retinas after ischemia/reperfusion injury. Experimental eye research 2020 Apr; 193: 107997.

### [Summary of Invention]

The present invention is as follows.
[1] A compound of the formula [I], the formula [II], or the formula [III], or a pharmaceutically acceptable salt thereof: 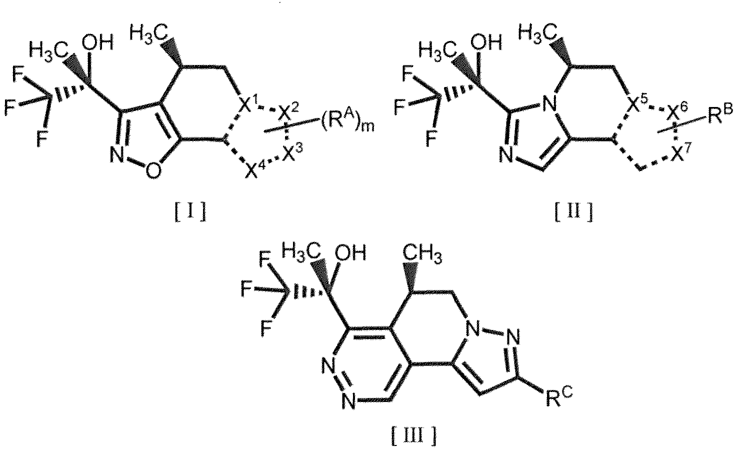 wherein
   -- is a single bond or a double bond,
   X¹, X², X³, X⁴, X⁵, X⁶ and X⁷ are each independently C or N, R^{A} is
      (1) C₁₋₆ alkyl wherein the C₁₋₆ alkyl is optionally substituted by one substituent selected from the group consisting of hydroxy and cyano,
      (2) halo C₁₋₄ alkyl,
      (3) -OR^{a} wherein R^{a} is
         (i) C₁₋₆ alkyl wherein the C₁₋₆ alkyl is optionally substituted by one substituent selected from the group consisting of
            (a) hydroxy,
            (b) cyano,
            (c) C₁₋₄ alkoxy,
            (d) C₃₋₆ cycloalkyl optionally substituted by one cyano,
            (e) phenyl, and
            (f) 4- to 6-membered saturated heterocyclyl having one oxygen atom,
         (ii) halo C₁₋₄ alkyl,
         (iii) 4- to 6-membered saturated heterocyclyl having one oxygen atom, or
         (iv) C₃₋₆ cycloalkyl wherein the C₃₋₆ cycloalkyl is optionally substituted by 1 or 2 substituents independently selected from the group consisting of C₁₋₆ alkyl, hydroxy, and cyano,
      (4) phenyl optionally substituted by one halogen,
      (5) C₃₋₆ cycloalkyl wherein the C₃₋₆ cycloalkyl is optionally substituted by 1 or 2 substituents independently selected from the group consisting of
         (i) halogen,
         (ii) C₁₋₄ alkyl,
         (iii) halo C₁₋₄ alkyl,
         (iv) hydroxy, and
         (v) cyano,
      (6) 4- to 6-membered saturated heterocyclyl having one oxygen atom, or
      (7) bridged C₅₋₁₀ cycloalkyl wherein the bridged C₅-₁₀ cycloalkyl is optionally substituted by one substituent selected from the group consisting of
         (i) C₁₋₄ alkoxycarbonyl,
         (ii) hydroxy C₁₋₄ alkyl,
         (iii) halo C₁₋₄ alkyl, and
         (iv) cyano,
   m is 0 or 1,
   R^{B} is
      (1) phenyl,
      (2) 4- to 6-membered saturated heterocyclyl having 1 or 2 hetero atoms independently selected from a nitrogen atom and an oxygen atom,
      (3) 6- to 10-membered saturated fused heterocyclyl having 1 or 2 hetero atoms independently selected from a nitrogen atom and an oxygen atom wherein the saturated fused heterocyclyl is optionally substituted by 1 or 2 halogens,
      (4) 6- to 10-membered spiro heterocyclyl having 1 or 2 hetero atoms independently selected from a nitrogen atom and an oxygen atom wherein the spiro heterocyclyl is optionally substituted by 1 or 2 halogens,
      (5) 5- to 10-membered bridged heterocyclyl having 1 or 2 hetero atoms independently selected from a nitrogen atom and an oxygen atom wherein the bridged heterocyclyl is optionally substituted by 1 or 2 substituents independently selected from the group consisting of halogen, cyano, and hydroxy C₁₋₄ alkyl,
      (6) bridged C₅₋₁₀ cycloalkyl wherein the bridged C₅₋₁₀ cycloalkyl is optionally substituted by one substituent selected from the group consisting of hydroxy C₁₋₄ alkyl and cyano, or
      (7) -OCH₂Cy¹ wherein Cy¹ is bridged C₅₋₁₀ cycloalkyl optionally substituted by one cyano, and
   R^{C} is
      (1) halo C₁₋₄ alkyl,
      (2) C₃₋₆ cycloalkyl optionally substituted by one halogen, or
      (3) bridged C₅₋₁₀ cycloalkyl wherein the bridged C₅₋₁₀ cycloalkyl is optionally substituted by one substituent selected from the group consisting of cyano and C₁₋₄ alkoxycarbonyl.
[2] The compound of [1] or a pharmaceutically acceptable salt thereof, which is a compound of the formula [I] or a pharmaceutically acceptable salt thereof.
[3] The compound of [1] or [2], wherein R^{A} is
   (1) halo C₁₋₄ alkyl,
   (2) -OR^{a} wherein R^{a} is
      (i) C₁₋₆ alkyl wherein the C₁₋₆ alkyl is optionally substituted by one substituent selected from the group consisting of
         (a) hydroxy,
         (b) C₃₋₆ cycloalkyl optionally substituted by one cyano, and
         (c) 4- to 6-membered saturated heterocyclyl having one oxygen atom,
      (ii) halo C₁₋₄ alkyl,
      (iii) 4- to 6-membered saturated heterocyclyl having one oxygen atom, or
      (iv) C₃₋₆ cycloalkyl wherein the C₃₋₆ cycloalkyl is optionally substituted by 1 or 2 substituents independently selected from the group consisting of C₁₋₆ alkyl, hydroxy, and cyano,
   (3) C₃₋₆ cycloalkyl wherein the C₃₋₆ cycloalkyl is optionally substituted by 1 or 2 substituents independently selected from the group consisting of
      (i) C₁₋₄ alkyl,
      (ii) hydroxy, and
      (iii) cyano, or
   (4) 4- to 6-membered saturated heterocyclyl having one oxygen atom, or a pharmaceutically acceptable salt thereof.
[4] The compound of [1] or [2], wherein m is 0, or a pharmaceutically acceptable salt thereof.
[5] The compound of [1], or a pharmaceutically acceptable salt thereof, which is a compound of the formula [II-a]: 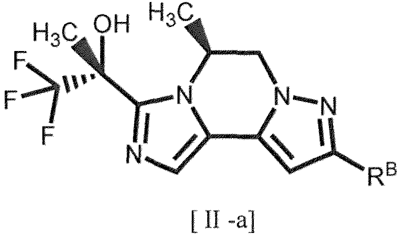 wherein R^{B} is
   (1) 6- to 10-membered spiro heterocyclyl having 1 or 2 hetero atoms independently selected from a nitrogen atom and an oxygen atom wherein the spiro heterocyclyl is optionally substituted by 1 or 2 halogens,
   (2) 5- to 10-membered bridged heterocyclyl having 1 or 2 hetero atoms independently selected from a nitrogen atom and an oxygen atom wherein the bridged heterocyclyl is optionally substituted by 1 or 2 substituents independently selected from the group consisting of halogen, cyano, and hydroxy C₁₋₄ alkyl,
   (3) bridged C₅₋₁₀ cycloalkyl wherein the bridged C₅₋₁₀ cycloalkyl is optionally substituted by one substituent selected from the group consisting of hydroxy C₁₋₄ alkyl and cyano, or
   (4) -OCH₂Cy¹ wherein Cy¹ is bridged C₅₋₁₀ cycloalkyl optionally substituted by one cyano,
   or a pharmaceutically acceptable salt thereof.
[6] A compound selected from the following formulas: 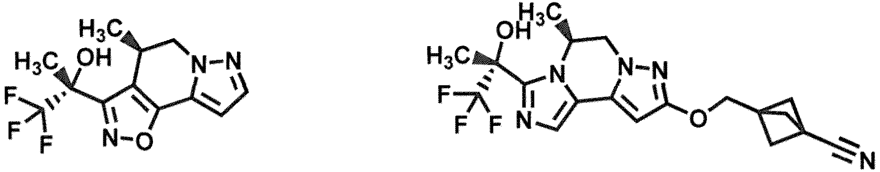 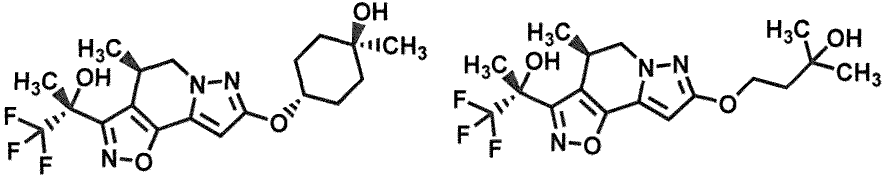 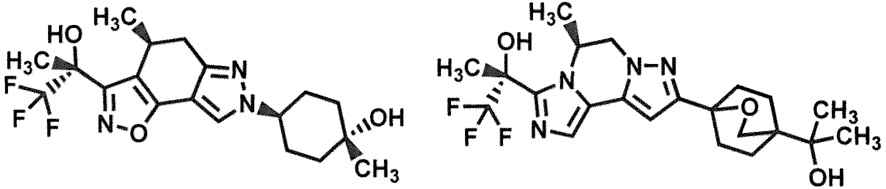 or a pharmaceutically acceptable salt thereof.
[7] A pharmaceutical composition comprising the compound of any one of [1] to [6] or a pharmaceutically acceptable salt thereof, and a pharmaceutically acceptable carrier.
[8] A PDHK inhibitor comprising the compound of any one of [1] to [6] or a pharmaceutically acceptable salt thereof.
[9] A PDHK2 inhibitor comprising the compound of any one of [1] to [6] or a pharmaceutically acceptable salt thereof.
[10] An agent for the treatment or prophylaxis of diabetes, insulin resistance syndrome, metabolic syndrome, hyperglycemia, hyperlactacidemia, diabetic complication, cardiac failure, cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy or chronic kidney disease, the agent comprising the compound of any one of [1] to [6] or a pharmaceutically acceptable salt thereof.
[11] The agent of [10], wherein the diabetes is type 1 diabetes or type 2 diabetes.
[12] The agent of [10], wherein the vascular dementia is a large-vessel type of vascular dementia or a small-vessel type of vascular dementia.
[13] The agent of [10], wherein the cardiac failure is acute cardiac failure or chronic cardiac failure.
[14] The agent of [10], wherein the pulmonary hypertension is pulmonary arterial hypertension.
[15] A method for inhibiting PDHK, comprising administering a therapeutically effective amount of the compound of any one of [1] to [6] or a pharmaceutically acceptable salt thereof to a mammal.
[16] A method for treating or preventing a disease selected from the group consisting of diabetes, insulin resistance syndrome, metabolic syndrome, hyperglycemia, hyperlactacidemia, diabetic complication, cardiac failure, cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy and chronic kidney disease, the method comprising administering a therapeutically effective amount of the compound of any one of [1] to [6] or a pharmaceutically acceptable salt thereof to a mammal.
[17] The method of [16], wherein the diabetes is type 1 diabetes or type 2 diabetes.
[18] The method of [16], wherein the vascular dementia is a large-vessel type of vascular dementia or a small-vessel type of vascular dementia.
[19] The method of [16], wherein the cardiac failure is acute cardiac failure or chronic cardiac failure.
[20] The method of [16], wherein the pulmonary hypertension is pulmonary arterial hypertension.
[21] Use of the compound of any one of [1] to [6] or a pharmaceutically acceptable salt thereof in the manufacture of a PDHK inhibitor.
[22] Use of the compound of any one of [1] to [6] or a pharmaceutically acceptable salt thereof in the manufacture of an agent for the treatment or prophylaxis of a disease selected from the group consisting of diabetes, insulin resistance syndrome, metabolic syndrome, hyperglycemia, hyperlactacidemia, diabetic complication, cardiac failure, cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy and chronic kidney disease.
[23] The use of [22], wherein the diabetes is type 1 diabetes or type 2 diabetes.
[24] The use of [22], wherein the vascular dementia is a large-vessel type of vascular dementia or a small-vessel type of vascular dementia.
[25] The use of [22] wherein the cardiac failure is acute cardiac failure or chronic cardiac failure.
[26] The use of [22] wherein the pulmonary hypertension is pulmonary arterial hypertension.
[27] The compound of any one of [1] to [6] or a pharmaceutically acceptable salt thereof for use in the treatment or prophylaxis of a disease selected from the group consisting of diabetes, insulin resistance syndrome, metabolic syndrome, hyperglycemia, hyperlactacidemia, diabetic complication, cardiac failure, cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy and chronic kidney disease.
[28] The compound of [27] or a pharmaceutically acceptable salt thereof, wherein the diabetes is type 1 diabetes or type 2 diabetes.
[29] The compound of [27] or a pharmaceutically acceptable salt thereof, wherein the vascular dementia is a large-vessel type of vascular dementia or a small-vessel type of vascular dementia.
[30] The compound of [27] or a pharmaceutically acceptable salt thereof, wherein the cardiac failure is acute cardiac failure or chronic cardiac failure.
[31] The compound of [27] or a pharmaceutically acceptable salt thereof, wherein the pulmonary hypertension is pulmonary arterial hypertension.
[32] A commercial package comprising the pharmaceutical composition of [7], and a written matter associated therewith, the written matter stating that the pharmaceutical composition can be used for the treatment or prophylaxis of a disease selected from the group consisting of diabetes, insulin resistance syndrome, metabolic syndrome, hyperglycemia, hyperlactacidemia, diabetic complication, cardiac failure, cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy and chronic kidney disease.
[33] A kit comprising the pharmaceutical composition of [7], and a written matter associated therewith, the written matter stating that the pharmaceutical composition can be used for the treatment or prophylaxis of a disease selected from the group consisting of diabetes, insulin resistance syndrome, metabolic syndrome, hyperglycemia, hyperlactacidemia, diabetic complication, cardiac failure, cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy and chronic kidney disease.

### [Description of Embodiments]

The definitions of the terms used in the present invention are as follows.

The "halogen" is fluoro, chloro, bromo, or iodo. As "halogen", fluoro or chloro is preferred.

The "C₁₋₄ alkyl" means a straight chain or branched chain alkyl having 1 to 4 carbon atoms. Examples thereof include methyl, ethyl, propyl, isopropyl, butyl, isobutyl, sec-butyl and tert-butyl. As "C₁₋₄ alkyl", methyl or ethyl is preferred.

The "C₁₋₆ alkyl" means a straight chain or branched chain alkyl having 1 to 6 carbon atoms. Examples thereof include methyl, ethyl, propyl, isopropyl, 1,1-dimethylpropyl, 1-ethylpropyl, 1-methyl-1-ethyl-propyl, butyl, isobutyl, sec-butyl, tert-butyl, pentyl, isopentyl, neopentyl, hexyl, isohexyl, 1,1-dimethylbutyl, 2,2-dimethylbutyl, 3,3-dimethylbutyl, 2-ethylbutyl and the like.

The "halo C₁₋₄ alkyl" means straight chain or branched chain alkyl having 1 to 4 carbon atoms and substituted by 1 to 5 "halogens" defined above. When alkyl is substituted by plural halogens, the halogens may be the same or different. Examples of the "halo C₁₋₄ alkyl" include fluoromethyl, difluoromethyl, trifluoromethyl, 1-fluoroethyl, 1-fluoro-1-methylethyl, 1,1-difluoroethyl, 2,2-difluoroethyl, 2-fluoro-2-methylethyl, 2,2,2-trifluoroethyl, pentafluoroethyl, 1,1-difluoropropyl, 1,1-difluoro-2-methylpropyl and the like. As "halo C₁₋₄ alkyl", C₁₋₄ alkyl substituted by 1 to 3 fluoros is preferred.

The "hydroxy C₁₋₄ alkyl" means "C₁₋₄ alkyl" defined above which is substituted by one hydroxy group. Examples thereof include hydroxymethyl, 2-hydroxyethyl, 1-hydroxy-1-methylethyl, 3-hydroxypropyl, 4-hydroxybutyl and the like. As "hydroxy C₁₋₄ alkyl", hydroxymethyl is preferred.

The "C₁₋₄ alkoxy" means alkyl-oxy in which the alkyl moiety is "C₁₋₄ alkyl" defined above and includes, for example, methoxy, ethoxy, propoxy, isopropoxy, butoxy, isobutoxy, sec-butoxy and tert-butoxy. As "C₁₋₄ alkoxy", methoxy is preferred.

The "C₁₋₄ alkoxycarbonyl" means alkyl-oxy-carbonyl in which the alkyl moiety is "C₁₋₄ alkyl" defined above and includes, for example, methoxycarbonyl, ethoxycarbonyl, propoxycarbonyl, isopropoxycarbonyl, butoxycarbonyl, isobutoxycarbonyl, sec-butoxycarbonyl and tert-butoxycarbonyl.

The "C₃₋₆ cycloalkyl" means a 3- to 6-membered monocyclic hydrocarbon ring group and includes, for example, cyclopropyl, cyclobutyl, cyclopentyl and cyclohexyl.

The "bridged C₅₋₁₀ cycloalkyl" means a bridged cyclic saturated hydrocarbon group having 5 to 10 carbon atoms. The bridged cyclic saturated hydrocarbon group means a ring group in which two cycloalkyls share three or more atoms. Examples of the "bridged C₅₋₁₀ cycloalkyl" include bicyclo [1.1.1]pentyl, bicyclo[2.1.1]hexyl, bicyclo[2.2.1]heptyl, and bicyclo[2.2.2]octyl.

The "4- to 6-membered saturated heterocyclyl having one oxygen atom" means a 4- to 6-membered monocyclic saturated heterocyclic group having one oxygen atom besides carbon atom. Examples of the saturated heterocyclyl include oxetanyl, tetrahydrofuranyl, and tetrahydropyranyl.

The "4- to 6-membered saturated heterocyclyl having 1 or 2 hetero atoms independently selected from a nitrogen atom and an oxygen atom" means a 4- to 6-membered monocyclic saturated heterocyclic group having, besides carbon atom, 1 or 2 hetero atoms independently selected from the group consisting of a nitrogen atom and an oxygen atom. Examples of the saturated heterocyclyl include oxetanyl, tetrahydrofuranyl, tetrahydropyranyl, azetidinyl, pyrrolidinyl, piperidinyl, piperazinyl, morpholinyl, and the like, and morpholinyl is preferred.

The "6- to 10-membered saturated fused heterocyclyl having 1 or 2 hetero atoms independently selected from a nitrogen atom and an oxygen atom" means a 6- to 10-membered fused bicyclic saturated heterocyclic group having, besides carbon atom, 1 or 2 hetero atoms independently selected from the group consisting of a nitrogen atom and an oxygen atom. The fused bicyclic saturated heterocycle means a heterocycle in which two saturated rings share two atoms. Examples of the saturated fused heterocyclyl include the following partial structures.

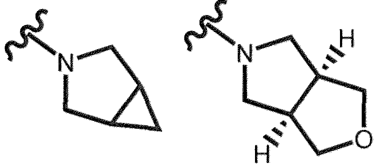

The "6- to 10-membered spiro heterocyclyl having 1 or 2 hetero atoms independently selected from a nitrogen atom and an oxygen atom" means a 6- to 10-membered spirocyclic saturated heterocyclic group having, besides carbon atom, 1 or 2 hetero atoms independently selected from the group consisting of a nitrogen atom and an oxygen atom. The spirocyclic saturated heterocycle means a heterocycle in which two saturated rings share one atom. Examples of the spiro heterocyclyl include the following partial structures.

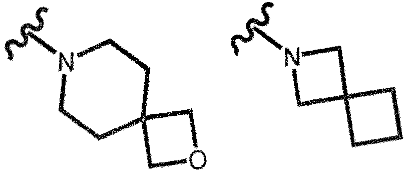

The "5- to 10-membered bridged heterocyclyl having 1 or 2 hetero atoms independently selected from a nitrogen atom and an oxygen atom" means a heterocyclyl in which one or two carbon atoms of the "bridged C₅₋₁₀ cycloalkyl" defined above are replaced with hetero atoms independently selected from the group consisting of a nitrogen atom and an oxygen atom. Examples of the bridged heterocyclyl include the following partial structures.

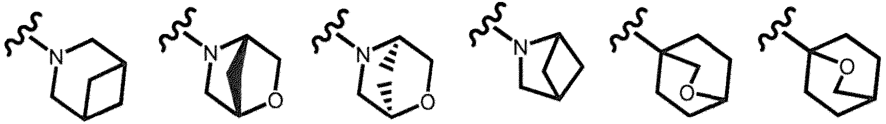

Preferred embodiments of the compound of the formula [I] or the formula [II] are explained below.

One of the preferred embodiments of the formula [I] is the formula [I-a]: 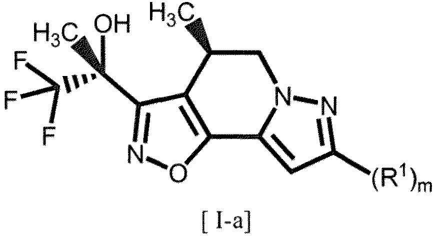 wherein R¹ is
(1) -OR^{a} wherein R^{a} is
   (i) C₁₋₆ alkyl wherein the C₁₋₆ alkyl is optionally substituted by one substituent selected from the group consisting of
      (a) hydroxy,
      (b) cyano,
      (c) C₁₋₄ alkoxy,
      (d) phenyl, and
      (e) 4- to 6-membered saturated heterocyclyl having one oxygen atom,
   (ii) halo C₁₋₄ alkyl, or
   (iii) C₃₋₆ cycloalkyl wherein the C₃₋₆ cycloalkyl is optionally substituted by 1 or 2 substituents independently selected from the group consisting of C₁₋₆ alkyl, hydroxy, and cyano, or
(2) 4- to 6-membered saturated heterocyclyl having one oxygen atom, and
   other symbol is as defined for the aforementioned formula [I].

Another preferred embodiment of the formula [I] is the formula [I-b]: 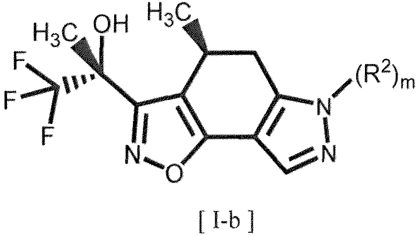 wherein R² is
(1) C₁₋₆ alkyl wherein the C₁₋₆ alkyl is optionally substituted by one substituent selected from the group consisting of hydroxy and cyano,
(2) halo C₁₋₄ alkyl,
(3) C₃₋₆ cycloalkyl wherein the C₃₋₆ cycloalkyl is optionally substituted by 1 or 2 substituents independently selected from the group consisting of
   (i) C₁₋₄ alkyl,
   (ii) cyano, and
   (iii) hydroxy, or
(4) bridged C₅₋₁₀ cycloalkyl wherein the bridged C₅₋₁₀ cycloalkyl is optionally substituted by one cyano, and
other symbol is as defined for the aforementioned formula [I].

Another preferred embodiment of the formula [I] is the formula [I-c]: 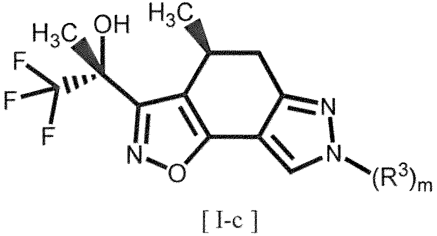 wherein R³ is
(1) C₁₋₆ alkyl wherein the C₁₋₆ alkyl is optionally substituted by one substituent selected from the group consisting of hydroxy and cyano,
(2) halo C₁₋₄ alkyl,
(3) C₃₋₆ cycloalkyl wherein the C₃₋₆ cycloalkyl is optionally substituted by 1 or 2 substituents independently selected from the group consisting of
   (i) C₁₋₄ alkyl,
   (ii) cyano, and
   (iii) hydroxy, or
(4) bridged C₅₋₁₀ cycloalkyl wherein the bridged C₅₋₁₀ cycloalkyl is optionally substituted by one cyano, and
other symbol is as defined for the aforementioned formula [I].

A compound of the formula [I-b] or the formula [I-c], wherein m is 0 is a compound represented by the formula [I-d].

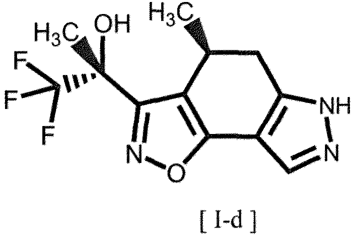

A compound of the formula [I-a], wherein m is 0 is a compound represented by the formula [I-e].

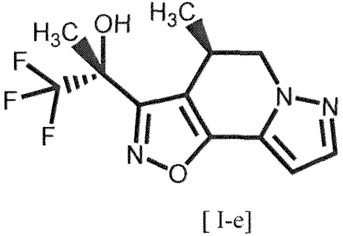

Another preferred embodiment of the formula [I] is the formula [I-f]: 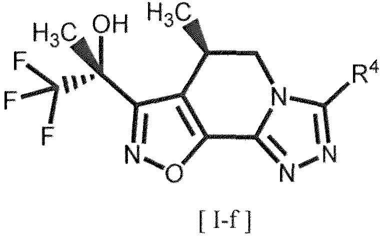 wherein R⁴ is
(1) C₁₋₆ alkyl,
(2) halo C₁₋₄ alkyl,
(3) C₃₋₆ cycloalkyl optionally substituted by one halogen,
(4) phenyl optionally substituted by one halogen, or
(5) bridged C₅₋₁₀ cycloalkyl wherein the bridged C₅₋₁₀ cycloalkyl is optionally substituted by one substituent selected from the group consisting of
   (i) C₁₋₄ alkoxycarbonyl,
   (ii) hydroxy C₁₋₄ alkyl, and
   (iii) halo C₁₋₄ alkyl.

One of the preferred embodiments of the formula [II] is the formula [II-b]: 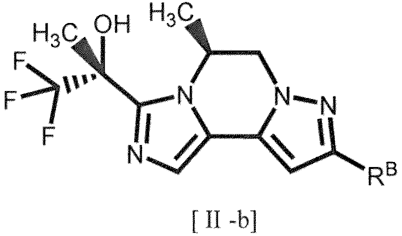 wherein the symbol is as defined for the aforementioned formula [II].

The "pharmaceutically acceptable salt" may be any salt without excessive toxicity known in the art. Specifically, salts with inorganic acids, salts with organic acids, salts with inorganic bases, salts with organic bases and the like can be mentioned. Various forms of pharmaceutically acceptable salts are well known in the art and for example, they are described in the following reference documents:
(a) Berge et al., J. Pharm. Sci., 66, p1-19(1977),
(b) Stahl et al., "Handbook of Pharmaceutical Salts: Properties, Selection, and Use" (Wiley-VCH, Weinheim, Germany, 2002),
(c) Paulekuhn et al., J. Med. Chem., 50, p6665-6672 (2007).

A pharmaceutically acceptable salt of a compound of the formula [I], the formula [II] or the formula [III] can be obtained by reacting the compound with an inorganic acid, organic acid, inorganic base or organic base according to a method known per se. The compound of the formula [I], the formula [II] or the formula [III] may be formed with one half molecule, one molecule or two or more molecules of an acid or base per molecule of the compound of the formula [I], the formula [II] or the formula [III],

Examples of the salt with inorganic acid include salts with hydrofluoric acid, hydrochloric acid, hydrobromic acid, hydroiodic acid, nitric acid, phosphoric acid and sulfuric acid.

Examples of the salt with organic acid include salts with acetic acid, adipic acid, alginic acid, 4-aminosalicylic acid, anhydromethylenecitric acid, benzoic acid, benzenesulfonic acid, calcium edetate, camphoric acid, camphor-10-sulfonic acid, carbonic acid, citric acid, edetic acid, ethane-1,2-disulfonic acid, dodecylsulfuric acid, ethanesulfonic acid, fumaric acid, glucoheptonic acid, gluconic acid, glucuronic acid, glycolylarsanilic acid, hexylresorcylic acid, hydroxy-naphthoic acid, 2-hydroxy-1-ethanesulfonic acid, lactic acid, lactobionic acid, malic acid, maleic acid, mandelic acid, methanesulfonic acid, methylsulfuric acid, methylnitric acid, methylenebis(salicylic acid), galactaric acid, naphthalene-2-sulfonic acid, 2-naphthoic acid, 1,5-naphthalenedisulfonic acid, oleic acid, oxalic acid, pamoic acid, pantothenic acid, pectic acid, picric acid, propionic acid, polygalacturonic acid, salicylic acid, stearic acid, succinic acid, tannic acid, tartaric acid, teoclic acid, thiocyanic acid, trifluoroacetic acid, p-toluenesulfonic acid, undecanoic acid, aspartic acid and glutamic acid.

Examples of the salt with inorganic base include a salt with lithium, sodium, potassium, magnesium, calcium, barium, aluminum, zinc, bismuth or ammonium.

Examples of the salt with organic base include a salt with arecoline, betaine, choline, clemizole, ethylenediamine, N-methylglucamine, N-benzylphenethylamine, tris(hydroxymethyl)methylamine, arginine or lysine.

A preferred embodiment of the "pharmaceutically acceptable salt" is as described below.

Examples of the salt with inorganic acid include salts with hydrochloric acid, nitric acid, sulfuric acid, phosphoric acid and hydrobromic acid.

Examples of the salt with organic acid include salts with oxalic acid, maleic acid, citric acid, fumaric acid, lactic acid, malic acid, succinic acid, tartaric acid, acetic acid, trifluoroacetic acid, benzoic acid, glucuronic acid, oleic acid, pamoic acid, methanesulfonic acid, benzenesulfonic acid, p-toluenesulfonic acid and 2-hydroxy-1-ethanesulfonic acid.

Examples of the salt with inorganic base include salts with sodium, potassium, calcium, magnesium and zinc.

Examples of the salt with organic base include salts with tris(hydroxymethyl)methylamine, N-methylglucamine and lysine.

The compound of the formula [I], the formula [II] or the formula [III] or a pharmaceutically acceptable salt thereof may exist as a solvate. The term "solvate" refers to the compound of the formula [I], the formula [II] or the formula [III] or a pharmaceutically acceptable salt thereof with which a solvent molecule is associated, and also includes hydrates. Such solvates are preferably pharmaceutically acceptable solvates and include, for example, hydrate, ethanol solvate, dimethyl sulfoxide-solvate and the like of the compound of the formula [I], the formula [II] or the formula [III] or a pharmaceutically acceptable salt thereof.

Specific examples include hemihydrate, monohydrate, dihydrate or mono(ethanol)solvate of the compound of the formula [I], the formula [II] or the formula [III] or a monohydrate of hydrochloride of the compound of the formula [I], the formula [II] or the formula [III], dihydrate of hydrochloride of the same and the like. Such solvates can be produced according to conventional methods.

The compound of the formula [I], the formula [II] or the formula [III] may exist as a stereoisomer that should be recognized as a cis/trans isomer. In this case, these compounds may exist as a cis isomer, a trans isomer, or a mixture of a cis isomer and a trans isomer.

The compound of the formula [I], the formula [II] or the formula [III] may exist as a tautomer. In this case, these compounds may exist as an individual tautomer or a mixture of tautomers.

The compound of the formula [I], the formula [II] or the formula [III] may contain one or more asymmetric carbons. In this case, these compounds may exist as a single enantiomer, a single diastereomer, a mixture of enantiomers or a mixture of diastereomers.

The compound of the formula [I], the formula [II] or the formula [III] may exist as an atropisomer. In this case, these compounds may exist as an individual atropisomer or a mixture of atropisomers.

The compound of the formula [I], the formula [II] or the formula [III] may simultaneously contain plural structural characteristics that produce the above-mentioned isomers. Moreover, these compounds may contain the above-mentioned isomers at any ratio.

In the absence of other reference such as annotation and the like, the formulas, chemical structures and compound names indicated in the present specification without specifying the stereochemistry thereof encompass all the above-mentioned isomers that may exist.

A diastereomeric mixture can be separated into each diastereomer by conventional methods such as chromatography, crystallization and the like. In addition, each diastereomer can also be formed by using a stereochemically single starting material, or by a synthesis method using a stereoselective reaction.

An enantiomeric mixture can be separated into each single enantiomer by a method well known in the art.

For example, a mixture of enantiomers may be reacted with a substantially pure enantiomer which is known as a chiral auxiliary to form a mixture of diastereomers, which may be then isolated into a diastereomer with an enhanced isomeric ratio or a substantially pure single diastereomer by a common method such as fractionated crystallization or chromatography. The added chiral auxiliary may be removed from the isolated diastereomer by a cleavage reaction to give a desirable enantiomer.

In addition, a mixture of enantiomers of a compound can also be directly separated by a chromatography method using a chiral solid phase well known in the art. Alternatively, one of the enantiomers can also be obtained by using a substantially pure optically active starting material or stereoselective synthesis (asymmetric induction) of a prochiral intermediate using a chiral auxiliary or an asymmetric catalyst.

The absolute steric configuration can be determined based on the X-ray crystal analysis of the resultant crystalline product or intermediate. In this case, a resultant crystalline product or intermediate derivatized with a reagent having an asymmetric center with a known steric configuration may be used where necessary.

The compound of the formula [I], the formula [II] or the formula [III] may be labeled with an isotope (²H, ³H, ¹⁴C, ³⁵S and the like).

A compound of the formula [I], the formula [II] or the formula [III] or a pharmaceutically acceptable salt thereof is preferably a substantially purified compound thereof or a pharmaceutically acceptable salt thereof. Further preferably, it is a compound thereof or a pharmaceutically acceptable salt thereof that is purified to a purity of not less than 80%.

The pharmaceutical composition of the present invention may be produced by appropriately admixing a suitable amount of a compound of the formula [I], the formula [II] or the formula [III] or a pharmaceutically acceptable salt thereof with at least one kind of a pharmaceutically acceptable carrier according to a method known in the art of pharmaceutical preparations. The content of the compound or a pharmaceutically acceptable salt thereof in the pharmaceutical composition varies depending on the dosage form, the dose, and the like. It is, for example, 0.1 to 100 wt% of the whole composition.

A dosage form of the compound of the formula [I], the formula [II] or the formula [III] or a pharmaceutically acceptable salt thereof includes an oral preparation such as a tablet, a capsule, a granule, a powder, a lozenge, a syrup, an emulsion, and a suspension or a parenteral preparation such as an external preparation, a suppository, an injection, an eye drop, a nasal preparation, and a pulmonary preparation.

Examples of the "pharmaceutically acceptable carrier" include various organic or inorganic carrier substances conventionally used as preparation materials, and include excipient, disintegrant, binder, glidant, lubricant, and the like for solid preparations, and solvent, solubilizing agent, suspending agent, isotonic agent, buffering agent, soothing agent, and the like for liquid preparations and base, emulsifier, wetting agent, stabilizer, stabilizing agent, dispersing agent, plasticizer, pH adjuster, absorption promoter, gelation agent, preservative, filler, dissolving agent, solubilizing agent, suspending agent, and the like for semisolid preparations. Where necessary, moreover, additives such as preservative, antioxidant, colorant, sweetening agent, and the like may also be used.

Examples of the "excipient" include lactose, sucrose, D-mannitol, D-sorbitol, cornstarch, dextrin, crystalline cellulose, crystalline cellulose, carmellose, carmellose calcium, sodium carboxymethyl starch, low-substituted hydroxypropylcellulose, gum arabic, and the like.

Examples of the "disintegrant" include carmellose, carmellose calcium, carmellose sodium, sodium carboxymethyl starch, croscarmellose sodium, crospovidone, low-substituted hydroxypropylcellulose, hydroxypropylmethylcellulose, crystalline cellulose, and the like.

Examples of the "binder" include hydroxypropylcellulose, hydroxypropylmethylcellulose, povidone, crystalline cellulose, sucrose, dextrin, starch, gelatin, carmellose sodium, gum arabic, and the like.

Examples of the "glidant" include light anhydrous silicic acid, magnesium stearate, and the like.

Examples of the "lubricant" include magnesium stearate, calcium stearate, talc, and the like.

Examples of the "solvent" include purified water, ethanol, propylene glycol, macrogol, sesame oil, corn oil, olive oil, and the like.

Examples of the "solubilizing agents" include propylene glycol, D-mannitol, benzyl benzoate, ethanol, triethanolamine, sodium carbonate, sodium citrate, and the like.

Examples of the "suspending agent" include benzalkonium chloride, carmellose, hydroxypropylcellulose, propylene glycol, povidone, methylcellulose, glycerol monostearate, and the like.

Examples of the "isotonic agent" include glucose, D-sorbitol, sodium chloride, D-mannitol, and the like.

Examples of the "buffering agent" include sodium hydrogenphosphate, sodium acetate, sodium carbonate, sodium citrate, and the like.

Examples of the "soothing agent" include benzyl alcohol and the like.

Examples of the "base" include water, animal and vegetable oils (olive oil, corn oil, peanut oil, sesame oil, castor oil, and the like), lower alcohols (ethanol, propanol, propylene glycol, 1,3-butyleneglycol, phenol, and the like), higher fatty acid and ester thereof, waxes, higher alcohol, polyhydric alcohol, hydrocarbons (white petrolatum, liquid paraffin, paraffin, and the like), hydrophilic petrolatum, purified lanolin, water absorption ointment, hydrous lanolin, hydrophilic ointment, starch, pullulan, gum arabic, gum tragacanth, gelatin, dextran, cellulose derivative (methylcellulose, carboxymethylcellulose, hydroxyethylcellulose, hydroxypropylcellulose, and the like), synthetic polymer (carboxyvinyl polymer, sodium polyacrylate, poly(vinyl alcohol), polyvinylpyrrolidone, and the like), propylene glycol, macrogol (macrogol 200 - 600 and the like), and a combination of two or more kinds thereof.

Examples of the "preservative" include ethyl paraoxybenzoate, chlorobutanol, benzyl alcohol, sodium dehydroacetate, sorbic acid, and the like.

Examples of the "antioxidant" include sodium sulfite, ascorbic acid and the like.

Examples of the "colorant" include food colors (e.g., Food Color Red No. 2 or 3, Food Color yellow No. 4 or 5 etc.), β-carotene, and the like.

Examples of the "sweetening agent" include saccharin sodium, dipotassium glycyrrhizinate, aspartame, and the like.

The pharmaceutical composition of the present invention can be administered orally or parenterally (topical, rectal, intravenous administration, intramuscular, subcutaneous, and the like) to mammals other than human (e.g., mouse, rat, hamster, guinea pig, rabbit, cat, dog, swine, bovine, horse, sheep, monkey and the like) and human. The dose varies depending on the subject of administration, disease, symptom, dosage form, administration route and the like. For example, the daily dose for oral administration to an adult patient is generally within the range of about 0.01 mg to 1 g, based on the compound of the formula [I], the formula [II] or the formula [III] as the active ingredient. This amount can be administered in one to several portions.

The compound of the formula [I], the formula [II] or the formula [III], or a pharmaceutically acceptable salt thereof has a PDHK inhibitory action, and is useful for the treatment and/or prophylaxis of various diseases or conditions expected to be improved by controlling PDHK activity. Examples of various diseases or conditions expected to be improved by controlling PDHK activity include diseases such as diabetes (type 1 diabetes, type 2 diabetes), insulin resistance syndrome, metabolic syndrome, hyperglycemia, hyperlactacidemia, diabetic complications (diabetic neuropathy, diabetic retinopathy, diabetic nephropathy, cataract), cardiac failure (acute cardiac failure, chronic cardiac failure), cardiomyopathy, myocardial ischemia, myocardial infarction, angina pectoris, dyslipidemia, atherosclerosis, peripheral arterial disease, intermittent claudication, chronic obstructive pulmonary diseases, brain ischemia, cerebral apoplexy, mitochondrial disease, mitochondrial encephalomyopathy, cancer, pulmonary hypertension (pulmonary arterial hypertension), Alzheimer disease, vascular dementia (large-vessel type or small-vessel type vascular dementia), glaucoma, diabetic retinopathy, retinopathy of prematurity, retinal vein occlusion, ischemic optic neuropathy, chronic kidney disease, and the like.

The symptoms of Alzheimer disease include a decline in cognitive function, mental symptoms and behavioral disorders, and the like.

To "inhibit PDHK" means to eliminate or attenuate the activity of PDHK by inhibit the function thereof. For example, it means to inhibit the function as PDHK based on the conditions in the below-mentioned Experimental Example 1. To "inhibit PDHK", human PDHK is preferably inhibited. To "inhibit PDHK", preferably, "PDHK2 is inhibited".

The "PDHK inhibitor" means a substance that binds to PDHK and inhibits the function of PDHK. As the "PDHK inhibitor", a "human PDHK inhibitor" is preferred. As the "PDHK inhibitor", an "inhibitor of PDHK2" is preferred.

In the present specification, the "treatment" also includes improvement of symptoms, prevention of severity, maintenance of remission, prevention of exacerbation, and further, prevention of recurrence.

In the present specification, the "prevention" or "prophylaxis" means to suppress the onset of symptoms.

In the present specification, presentation of preferred embodiments and options of the compound, method, use and composition of the present invention also includes combinations of preferred embodiments and options as long as they can be combined and are free of inconsistency.

The production methods of the compound of the formula [I], the formula [II] or the formula [III] or a pharmaceutically acceptable salt thereof are explained in the following. However, the production method of the compound or a pharmaceutically acceptable salt thereof is not limited to such production methods.

The compound obtained in each step can be isolated or purified as necessary by conventional methods such as distillation, recrystallization, column chromatography and the like. In some cases, the next step may be performed without isolation or purification. When the reaction to be performed in each step is an anhydrous reaction, it is preferably performed in an inert gas atmosphere of argon, nitrogen and the like.

### [Production Method 1]

The compound of the formula [I-1] can be obtained by Production Method 1 shown by the following scheme. 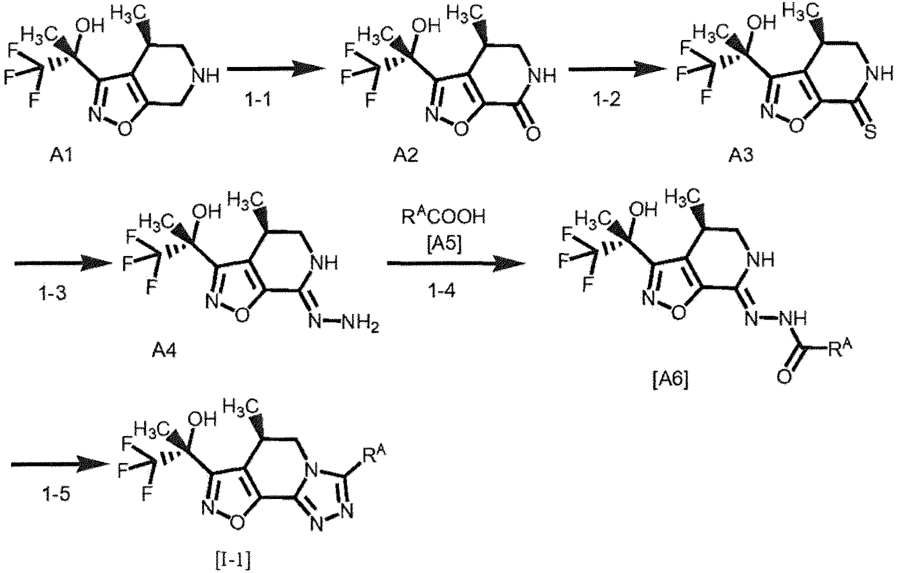 wherein each symbol is as defined for the aforementioned formula [I].

### Step 1-1

Compound A2 can be obtained by an oxidation reaction of compound A1. For example, compound A2 can be obtained by reacting compound A1 with iodosobenzene in a solvent in the presence of tetra-n-butylammonium iodide.

Examples of the solvent include a mixed solvent of acetonitrile and water.

Compound A1 can be obtained by Production Example 12 described in WO 2019/151274.

### Step 1-2

Compound A3 can be obtained by reacting compound A2 with a sulfur reagent. For example, compound A3 can be obtained by reacting compound A2 with a sulfur reagent in a solvent at room temperature to 120°C.

Examples of the sulfur reagent include Lawesson's reagent (2,4-bis(4-methoxyphenyl)-1,3,2,4-dithiadiphosphetane-2,4-disulfide).

Examples of the solvent include toluene and pyridine.

### Steps 1-3 and 1-4

Compound [A6] can be obtained by reacting compound A3 with hydrazine to give compound A4, and then reacting compound A4 with compound [A5]. The reaction of compound A4 with hydrazine can be performed by, for example, reacting compound A4 with hydrazine in a solvent at room temperature to 120°C.

Examples of the solvent include ethanol and isopropanol.

The reaction of compound A4 with compound [A5] can be performed by, for example, reacting compound A4 with compound [A5] in a solvent in the presence of a condensing agent under ice-cooling to room temperature.

Examples of the condensing agent include 1-(3-dimethylaminopropyl)-3-ethylcarbodiimide hydrochloride and 1-hydroxybenzotriazole monohydrate.

Examples of the solvent include acetonitrile and N,N-dimethylformamide.

Compound [A5] may be a commercially available product, or may be obtained by appropriately converting a commercially available product by a method well known to those of ordinary skill in the art.

Compound [A6] can also be obtained by reacting acid chloride of compound [A5] (R^{A}COCl) with compound A4. For example, compound [A6] can be obtained by reacting R^{A}COCl with compound A4 in a solvent in the presence of a base under ice-cooling to room temperature.

Examples of the base include diisopropylethylamine and sodium hydrogen carbonate.

Examples of the solvent include tetrahydrofuran and chloroform.

### Step 1-5

Compound [I-1] can be obtained by a dehydration reaction of compound [A6]. For example, compound [I-1] can be obtained by reacting compound [A6] with a dehydrating reagent in a solvent.

Examples of the dehydrating reagent include (methoxycarbonylsulfamoyl)triethylammonium hydroxide (Burgess reagent), acetic anhydride and acetic acid, trifluoroacetic anhydride and trifluoroacetic acid.

Examples of the solvent include N-methylpyrrolidone, tetrahydrofuran, dichloromethane and toluene.

### [Production Method 2]

A compound of the formula B15 can be obtained by Production Method 2 shown by the following scheme. 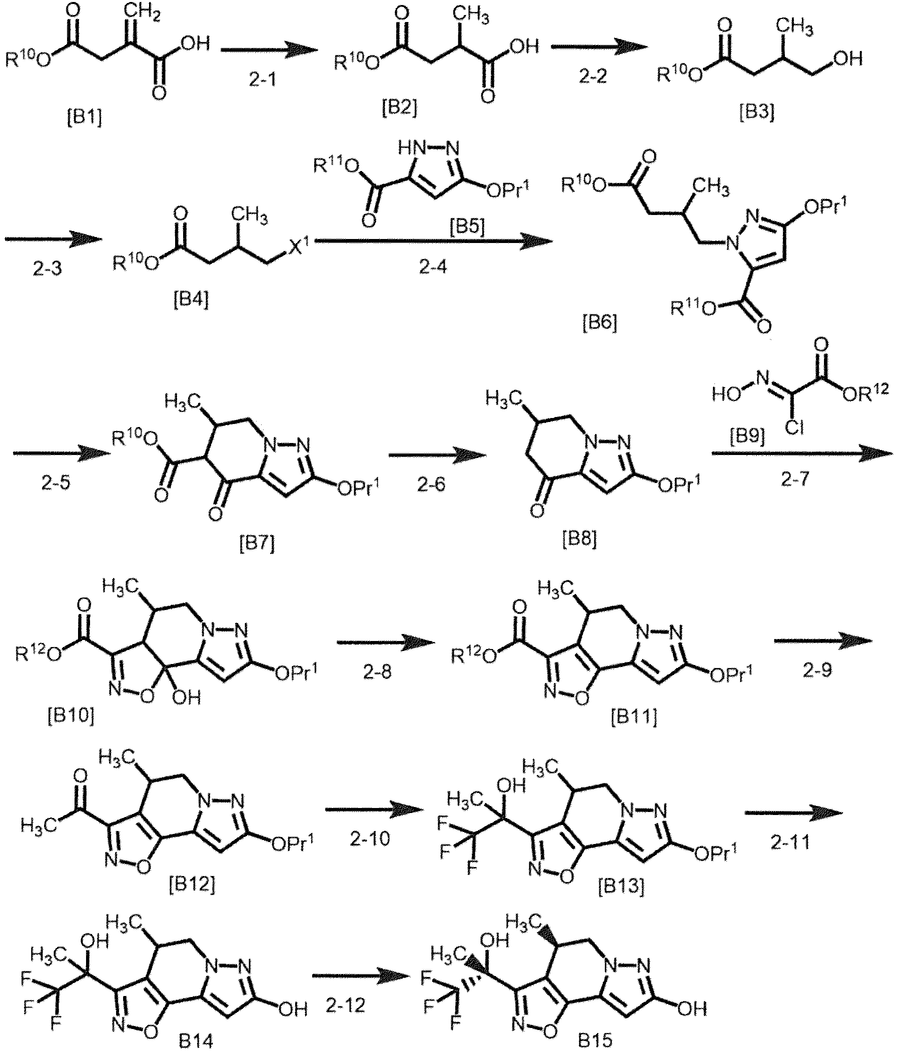
wherein R¹⁰, R¹¹ and R¹² are each independently C₁₋₄ alkyl;
X¹ is a leaving group such as methanesulfonyloxy and the like; and
Pr¹ is a hydroxy-protecting group such as benzyl and the like.

### Step 2-1

Compound [B2] can be obtained by reducing the double bond of compound [B1]. For example, compound [B2] can be obtained by catalytic reduction of compound [B1] in a solvent under a hydrogen atmosphere in the presence of a palladium catalyst at room temperature.

Examples of the palladium catalyst include palladium carbon.

Examples of the solvent include methanol, ethanol, and ethyl acetate.

Compound [B1] may be a commercially available product, or may be obtained by appropriately converting a commercially available product by a method well known to those of ordinary skill in the art.

### Step 2-2

Compound [B3] can be obtained by reducing the carboxy group of compound [B2]. For example, compound [B3] can be obtained by reacting compound [B2] with a reducing agent in a solvent under ice-cooling to room temperature.

Examples of the reducing agent include borane.

Examples of the solvent include tetrahydrofuran.

### Step 2-3

Compound [B4] can be obtained by converting the hydroxy group of compound [B3] to a leaving group. For example, when X¹ is methanesulfonyloxy, compound [B4] can be obtained by reacting compound [B3] with methanesulfonic anhydride in a solvent in the presence of a base.

Examples of the base include triethylamine.

Examples of the solvent include chloroform, dichloromethane, and tetrahydrofuran.

### Step 2-4

Compound [B6] can be obtained by reacting compound [B4] and compound [B5]. For example, compound [B6] can be obtained by reacting compound [B4] with compound [B5] in a solvent in the presence of a base.

Examples of the base include potassium carbonate and cesium carbonate.

Examples of the solvent include N,N-dimethylformamide.

### Step 2-5

Compound [B7] can be obtained by intramolecular Claisen condensation of compound [B6]. For example, compound [B7] can be obtained by treating compound [B6] with a base in a solvent at room temperature to 120°C.

Examples of the base include potassium tert-butoxide.

Examples of the solvent include toluene and tetrahydrofuran.

### Step 2-6

Compound [B8] can be obtained by treating compound [B7] with sodium chloride or lithium chloride in a solvent at 100°C to 160°C.

Examples of the solvent include water and dimethyl sulfoxide.

### Step 2-7

Compound [B10] can be obtained by reacting compound [B8] and compound [B9]. For example, compound [B10] can be obtained by reacting compound [B8] treated with a base with compound [B9] treated with a base in a solvent at -78°C to room temperature.

Examples of the base include lithium bis(trimethylsilyl)amide, sodium bis(trimethylsilyl)amide, and potassium bis(trimethylsilyl)amide.

Examples of the solvent include tetrahydrofuran.

Compound [B9] may be a commercially available product, or may be obtained by appropriately converting a commercially available product by a method well known to those of ordinary skill in the art.

### Step 2-8

Compound [B11] can be obtained by converting the hydroxy group of compound [B10] to a leaving group, followed by an elimination reaction. When the leaving group is methanesulfonyloxy, compound [B11] can be obtained by reacting compound [B10] with methanesulfonic anhydride in a solvent in the presence of a base under ice-cooling, followed by heating at 40°C to 70°C.

Examples of the base include triethylamine.

Examples of the solvent include tetrahydrofuran and toluene.

### Step 2-9

Compound [B12] can be obtained by reacting compound [B11] with methylmagnesium halide. For example, compound [B12] can be obtained by reacting compound [B11] with methylmagnesium halide in a solvent in the presence of a base at -78°C to 0°C.

Examples of the methylmagnesium halide include methylmagnesium bromide.

Examples of the base include triethylamine.

Examples of the solvent include tetrahydrofuran, 2-methyltetrahydrofuran, toluene, and diethyl ether.

### Step 2-10

Compound [B13] can be obtained by reacting compound [B12] with (trifluoromethyl)trimethylsilane. For example, compound [B13] can be obtained by reacting compound [B12] with (trifluoromethyl)trimethylsilane in a solvent in the presence of an additive under ice-cooling to room temperature.

Examples of the additive include tetra-n-butylammonium fluoride, lithium acetate, potassium carbonate, and cesium fluoride.

Examples of the solvent include tetrahydrofuran, N,N-dimethylformamide, and N,N-dimethylacetamide.

The methyl group of compound [B12] becomes a steric hindrance and the reaction proceeds diastereoselectively.

### Step 2-11

Compound B14 can be obtained by deprotecting compound [B13]. For example, when Pr¹ is benzyl, compound B14 can be obtained by catalytic reduction of compound [B13] in a solvent under a hydrogen atmosphere in the presence of a palladium catalyst at room temperature.

Examples of the palladium catalyst include palladium carbon.

Examples of the solvent include tetrahydrofuran and methanol.

### Step 2-12

Compound B15 can be obtained by purifying compound B14 by chiral column chromatography. The steric configuration of compound B15 can be determined by, for example, X-ray crystal structure analysis.

Compound B15 can also be obtained by purifying compound [B11] by chiral column chromatography, and then performing reactions similar to those in Steps 2-9 to 2-11.

### [Production Method 3]

A compound of the formula B15 can also be obtained by Production Method 3 shown by the following scheme. 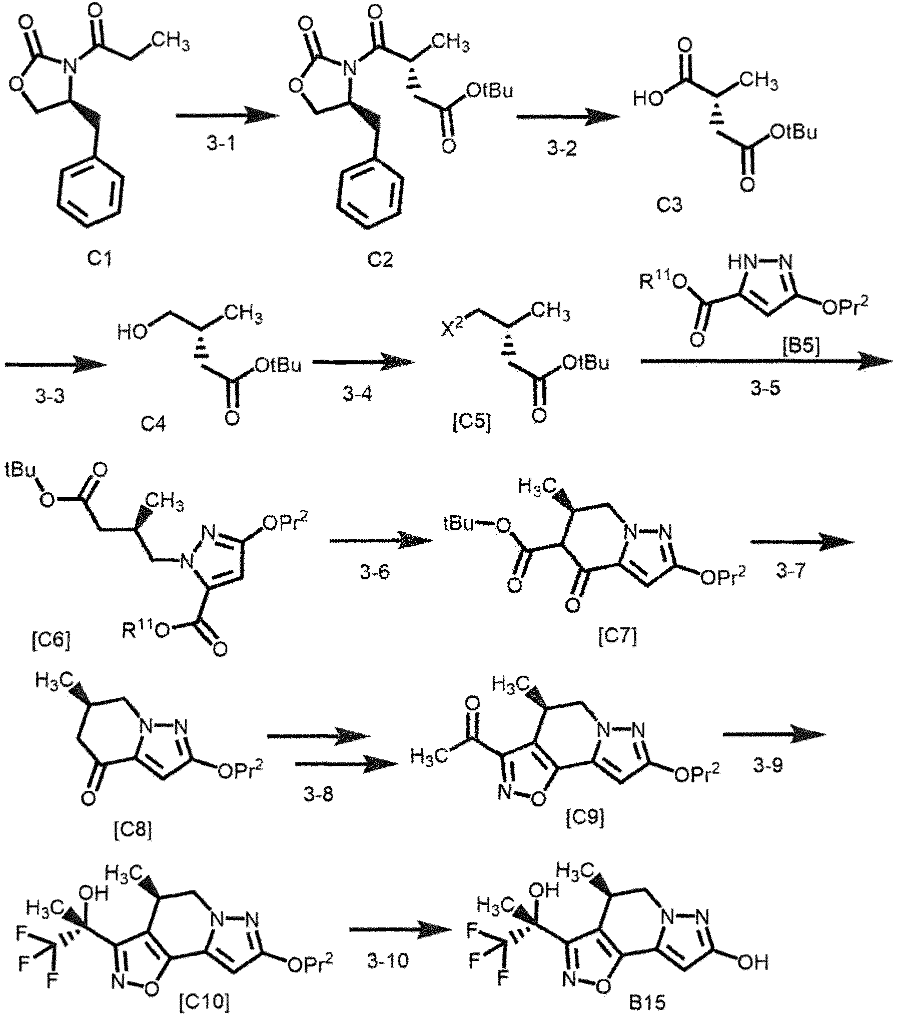
wherein X² is a leaving group such as ethanesulfonyloxy and the like;
Pr² is a hydroxy-protecting group such as benzyl and the like; and
other each symbol is as defined above.

### Step 3-1

Compound C2 can be obtained by reacting compound C1 with tert-butyl bromoacetate. For example, compound C2 can be obtained by reacting compound C1 with tert-butyl bromoacetate in a solvent at -78°C to room temperature in the presence of a base.

Examples of the base include lithium bis(trimethylsilyl)amide, sodium bis(trimethylsilyl)amide and potassium bis(trimethylsilyl)amide.

Examples of the solvent include tetrahydrofuran.

Compound C1 may be a commercially available product, or may be obtained by appropriately converting a commercially available product by a method well known to those of ordinary skill in the art.

### Step 3-2

Compound C3 can be obtained by removing the chiral auxiliary group of compound C2. For example, C3 can be obtained by reacting compound C2 with alkali and an oxidizing agent in a solvent under ice-cooling to room temperature.

Examples of the alkali include lithium hydroxide.

Examples of the oxidizing agent include hydrogen peroxide water.

Examples of the solvent include tetrahydrofuran and water.

### Step 3-3

Compound C4 can be obtained by reducing the carboxy group of compound C3. For example, compound C4 can be obtained by reacting compound C3 with a reducing agent in a solvent under ice-cooling to room temperature.

Examples of the reducing agent include borane.

Examples of the solvent include tetrahydrofuran.

### Step 3-4

Compound [C5] can be obtained by converting the hydroxy group of compound C4 to a leaving group. For example, when X² is ethanesulfonyloxy, compound [C5] can be obtained by reacting compound C4 with ethanesulfonyl chloride in a solvent in the presence of a base.

Examples of the base include triethylamine.

Examples of the solvent include chloroform, dichloromethane, and tetrahydrofuran.

### Step 3-5

Compound [C6] can be obtained by reacting compound [C5] with compound [B5]. For example, compound [C6] can be obtained by an operation similar to that in Step 2-4.

### Step 3-6

Compound [C7] can be obtained by intramolecular Claisen condensation of compound [C6]. For example, compound [C7] can be obtained by an operation similar to that in Step 2-5.

### Step 3-7

Compound [C8] can be obtained by treating compound [C7] with an acid in a solvent at 80°C to 110°C.

Examples of the acid include trifluoroacetic acid.

Examples of the solvent include toluene.

### Step 3-8

Compound [C9] can be obtained using compound [C8] by reactions similar to those in Steps 2-7, 2-8 and 2-9.

### Step 3-9

Compound [C10] can be obtained by reacting compound [C9] with (trifluoromethyl)trimethylsilane. For example, compound [C10] can be obtained by an operation similar to that in Step 2-10.

The methyl group of compound [C9] becomes a steric hindrance and the reaction proceeds diastereoselectively, whereby compound [C10] is obtained as an optically active form.

### Step 3-10

Compound B15 can be obtained by deprotection of compound [C10]. For example, when Pr² is benzyl, compound B15 can be obtained by an operation similar to that in Step 2-11.

### [Production Method 4]

Compounds of the formulas [1-2] to [1-5] can be obtained by converting compound B15, which is obtained by Production Method 2 or 3, by Production Method 4 shown by the following scheme. 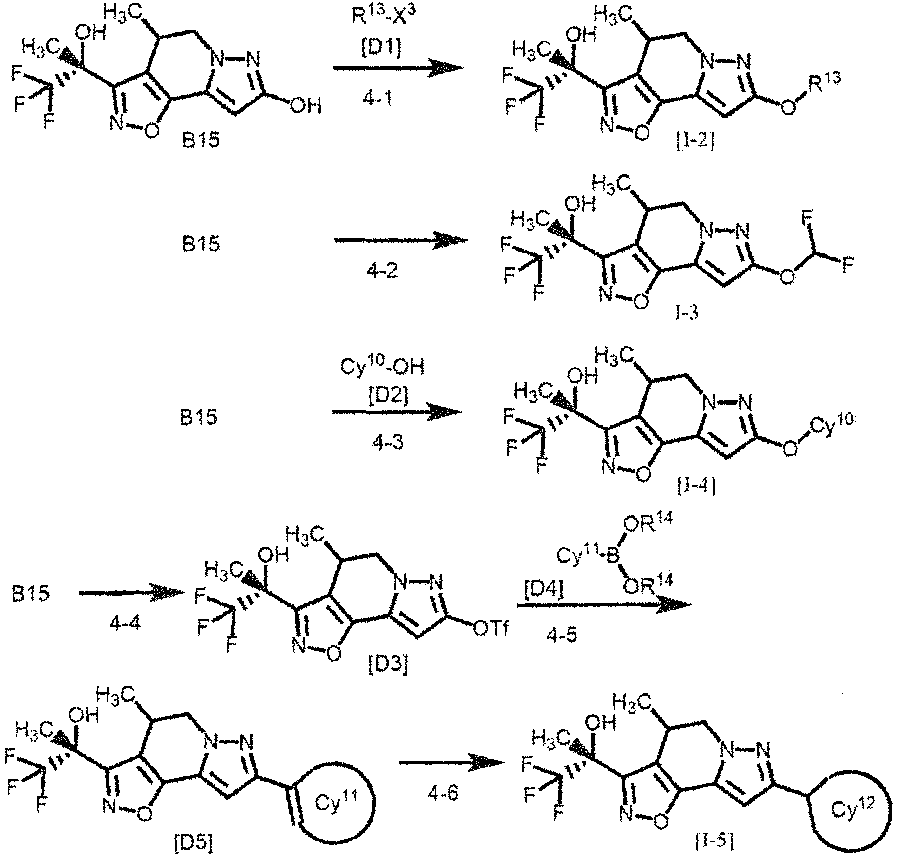
wherein X³ is a leaving group such as bromo, p-toluenesulfonyloxy, and the like;
R¹³ is
   (1) C₁₋₆ alkyl wherein the C₁₋₆ alkyl is optionally substituted by one substituent selected from the group consisting of
      (a) hydroxy,
      (b) cyano,
      (c) C₁₋₄ alkoxy,
      (d) C₃₋₆ cycloalkyl optionally substituted by one cyano,
      (e) phenyl, and
      (f) 4- to 6-membered saturated heterocyclyl having one oxygen atom, or
   (2) halo C₁₋₄ alkyl;
Cy¹⁰ is
   (1) 4- to 6-membered saturated heterocyclyl having one oxygen atom, or
   (2) C₃₋₆ cycloalkyl wherein the C₃₋₆ cycloalkyl is optionally substituted by 1 or 2 substituents independently selected from the group consisting of C₁₋₆ alkyl, hydroxy, and cyano;
Cy¹¹ is 4- to 6-membered unsaturated heterocyclyl having one oxygen atom;
Cy¹² is 4- to 6-membered saturated heterocyclyl having one oxygen atom; and
each R¹⁴ is independently hydrogen or C₁₋₄ alkyl, and one R¹⁴ may be bonded to the other R¹⁴ to form a ring.

### Step 4-1

Compound [1-2] can be obtained by reacting compound B15 with compound [D1]. For example, compound [1-2] can be obtained by reacting compound B15 with compound [D1] in a solvent in the presence of a base at room temperature to 90°C.

Examples of the base include potassium carbonate and cesium carbonate.

Examples of the solvent include N,N-dimethylformamide.

### Step 4-2

Compound 1-3 can be obtained by reacting compound B15 with sodium chlorodifluoroacetate in a solvent in the presence of a base at room temperature to 100°C.

Examples of the base include potassium carbonate.

Examples of the solvent include N,N-dimethylformamide.

### Step 4-3

Compound [1-4] can be obtained by a Mitsunobu reaction of compound B15 and compound [D2]. For example, compound [1-4] can be obtained by reacting compound B15 with phosphine and azodicarboxylic acid diester in a solvent at room temperature to 100°C.

Examples of the phosphine include trioctylphosphine, tributylphosphine and triphenylphosphine.

Examples of the azodicarboxylic acid diester include diisopropyl azodicarboxylate and di-tert-butyl azodicarboxylate.

Examples of the solvent include toluene, tetrahydrofuran, and 2-methyltetrahydrofuran.

### Step 4-4

Compound [D3] can be obtained by reacting compound B15 with trifluoromethanesulfonic anhydride in a solvent in the presence of a base at 0°C to room temperature.

Examples of the base include triethylamine.

Examples of the solvent include dichloromethane and chloroform.

### Step 4-5

Compound [D5] can be obtained by Suzuki coupling of compound [D3] and compound [D4]. For example, compound [D5] can be obtained by reacting compound [D3] with compound [D4] in a solvent in the presence of a base and a palladium catalyst at room temperature to 100°C.

Examples of the base include potassium carbonate and tripotassium phosphate.

Examples of the palladium catalyst include XPhos Pd G4.

Examples of the solvent include 1,4-dioxane, water, and toluene.

Compound [D4] may be a commercially available product, or may be obtained by appropriately converting a commercially available product by a method well known to those of ordinary skill in the art.

### Step 4-6

Compound [1-5] can be obtained by reducing the double bond of compound [D5]. For example, compound [1-5] can be obtained by catalytic reduction of compound [D5] in a solvent under a hydrogen atmosphere in the presence of a palladium catalyst at room temperature.

Examples of the palladium catalyst include palladium carbon.

Examples of the solvent include methanol, ethanol, tetrahydrofuran, and ethyl acetate.

### [Production Method 5]

Compounds of the formulas [1-6] and [1-7] can be obtained by Production Method 5 shown by the following scheme. 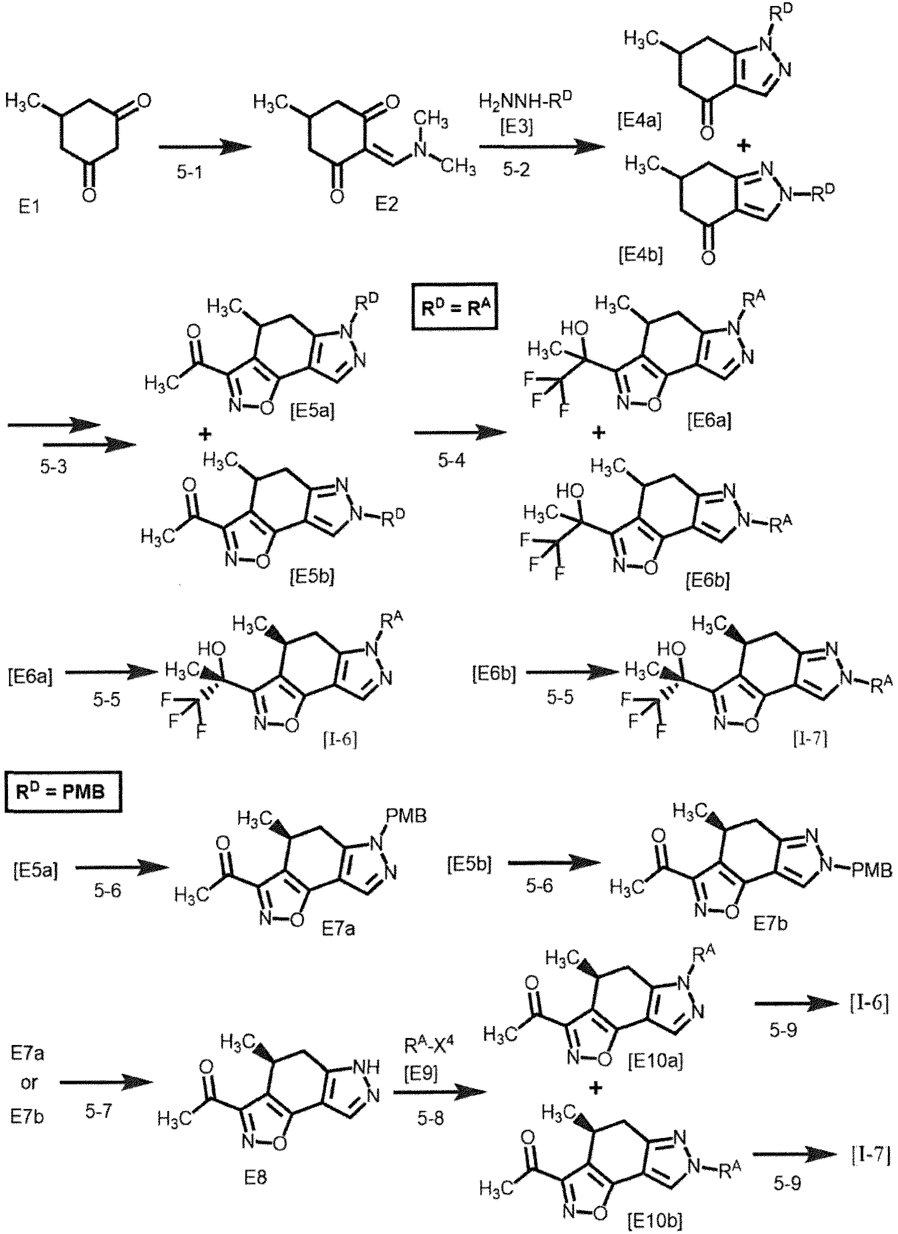
wherein R^{D} is R^{A} or p-methoxybenzyl (PMB);
X⁴ is a leaving group such as p-toluenesulfonyloxy, methanesulfonyloxy, and the like; and
other each symbol is as defined for the aforementioned formula [I] .

### Steps 5-1 and 5-2

Compounds [E4a] and [E4b] can be obtained by a pyrazole cyclization reaction using compound E1 and compound [E3]. For example, compounds [E4a] and [E4b] can be obtained by reacting compound E1 with N,N-dimethylformamide dimethyl acetal in a solvent at 100°C to 120°C, and reacting the obtained compound with compound [E3] in the presence of an acid at 100°C to 200°C. After isolating the intermediate compound E2, the intermediate may be reacted with compound [E3]. A microwave device may also be used as necessary. The reaction may be performed without using a solvent.

Examples of the acid include acetic acid.

Examples of the solvent include ethanol and water.

Compounds E1 and [E3] may be commercially available products, or may be obtained by appropriately converting a commercially available product by a method well known to those of ordinary skill in the art.

### Step 5-3

Compounds [E5a] and [E5b] can be obtained using compounds [E4a] and [E4b] by reactions similar to those in Steps 2-7 to 2-9.

### Step 5-4

When R^{D} is R^{A}, compounds [E6a] and [E6b] can be obtained by reacting compounds [E5a] and [E5b] with (trifluoromethyl)trimethylsilane, and purifying the obtained compounds by column chromatography. For example, the reaction with (trifluoromethyl)trimethylsilane can be performed by an operation similar to that in Step 2-10.

### Step 5-5

Compounds [1-6] and [1-7] can be obtained by purifying compounds [E6a] and [E6b] by chiral column chromatography. The steric configuration of compounds [1-6] and [1-7] can be determined, for example, by X-ray crystal structure analysis.

When R^{D} is p-methoxybenzyl (PMB), the compounds [1-6] and [1-7] can also be obtained by Steps 5-6 to 5-9.

### Step 5-6

Compounds E7a and E7b can be obtained by respectively purifying compounds [E5a] and [E5b] by chiral column chromatography. The steric configuration of compounds E7a and E7b can be determined, for example, by X-ray crystal structure analysis.

### Step 5-7

Compound E8 can be obtained by deprotection of compound E7a or E7b. For example, compound E8 can be obtained by treating compounds E7a and E7b with an acid at room temperature to 100°C. Where necessary, a microwave device may also be used. Where necessary, a cation scavenger may also be used.

Examples of the acid include trifluoroacetic acid.

Examples of the cation scavenger include anisole.

### Step 5-8

Compounds [E10a] and [E10b] can be obtained by reacting compound E8 with compound [E9]. For example, compounds [E10a] and [E10b] can be obtained by reacting compound E8 with compound [E9] in a solvent in the presence of a base at 80°C to 100°C. Where necessary, an additive may be added.

Examples of the base include potassium carbonate and cesium carbonate.

Examples of the solvent include N,N-dimethylformamide.

Examples of the additive include sodium iodide.

Compound [E9] may be a commercially available product, or may be obtained by appropriately converting a commercially available product by a method well known to those of ordinary skill in the art.

### Step 5-9

Compounds [1-6] and [1-7] can be obtained using compounds [E10a] or [E10b] and by the reaction of Step 5-4.

### [Production Method 6]

Compounds of the formulas [II-1] and [11-2] can be obtained by Production Method 6 shown by the following scheme. 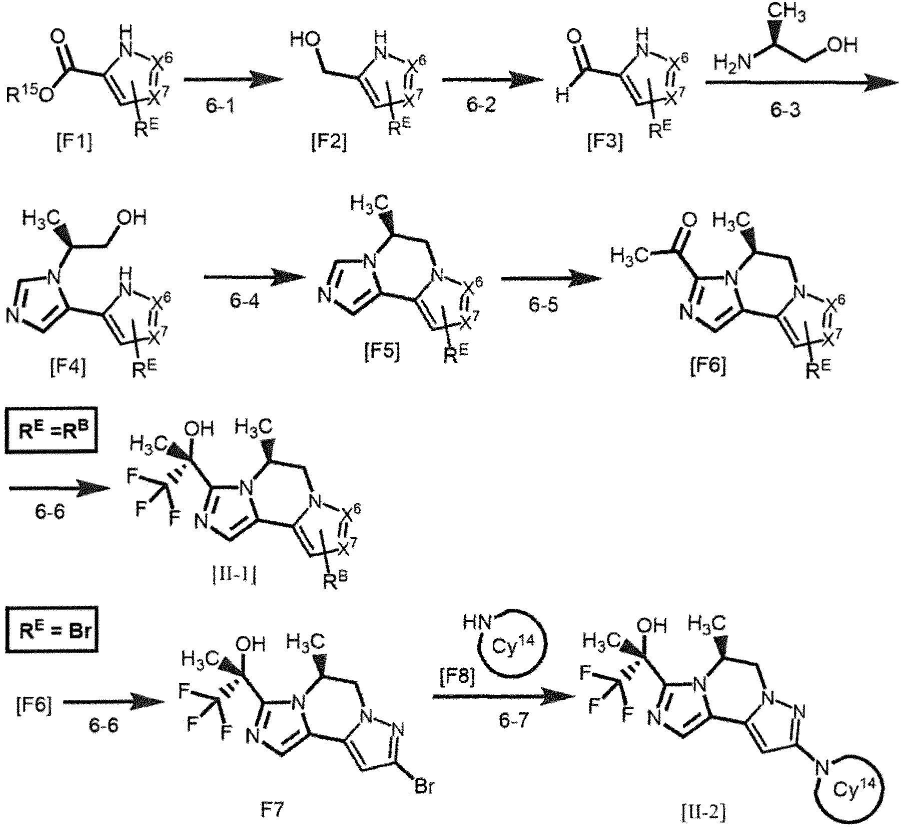
wherein R^{E} is R^{B} or bromo;
R¹⁵ is C₁₋₄ alkyl; and
Cy¹⁴ is
   (1) 4- to 6-membered saturated heterocyclyl having 1 or 2 nitrogen atoms or having one nitrogen atom and one oxygen atom,
   (2) 6- to 10-membered saturated fused heterocyclyl having 1 or 2 nitrogen atoms or having one nitrogen atom and one oxygen atom wherein the saturated fused heterocyclyl is optionally substituted by 1 or 2 halogens,
   (3) 6- to 10-membered spiro heterocyclyl having 1 or 2 nitrogen atoms or having one nitrogen atom and one oxygen atom wherein the spiro heterocyclyl is optionally substituted by 1 or 2 halogens, or
   (4) 5- to 10-membered bridged heterocyclyl having 1 or 2 nitrogen atoms or having one nitrogen atom and one oxygen atom wherein the bridged heterocyclyl is optionally substituted by 1 or 2 substituents independently selected from the group consisting of halogen, cyano, and hydroxy C₁₋₄ alkyl.

### Step 6-1

Compound [F2] can be obtained by reducing the ester group of compound [F1]. For example, compound [F2] can be obtained by reacting compound [F1] with a reducing agent in a solvent at -40°C to room temperature.

Examples of the reducing agent include lithium aluminum hydride, diisobutylaluminum hydride, and lithium borohydride.

Examples of the solvent include tetrahydrofuran, diethyl ether, and cyclopentyl methyl ether.

Compound [F1] may be a commercially available product, or may be obtained by appropriately converting a commercially available product by a method well known to those of ordinary skill in the art.

### Step 6-2

Compound [F3] can be obtained by oxidation of the hydroxy group of compound [F2]. For example, compound [F3] can be obtained by reacting compound [F2] with an oxidizing agent in a solvent under ice-cooling to room temperature.

Examples of the oxidizing agent include manganese dioxide, Dess-Martin periodinane, and sulfur trioxide-pyridine complex.

Examples of the solvent include tetrahydrofuran, 1,2-dimethoxyethane, toluene, dimethyl sulfoxide, chloroform, and dichloromethane.

### Step 6-3

Compound [F4] can be obtained by an imination reaction of compound [F3] and (S)-2-aminopropan-1-ol, and a cyclization reaction using p-toluenesulfonylmethyl isocyanide. For example, an imination reaction is performed by reacting compound [F3] and (S)-2-aminopropan-1-ol in a solvent at room temperature to 60°C. Then, the resultant product is reacted with p-toluenesulfonylmethyl isocyanide in a solvent in the presence of a base under ice-cooling to room temperature, whereby compound [F4] can be obtained.

Examples of the solvent of the imination reaction include methanol, 1,2-dimethoxyethane, and N,N-dimethylformamide.

Examples of the base include potassium carbonate.

Examples of the solvent of the cyclization reaction include methanol, 1,2-dimethoxyethane, and N,N-dimethylformamide.

### Step 6-4

Compound [F5] can be obtained by an intramolecular Mitsunobu reaction of compound [F4]. For example, compound [F5] can be obtained by reacting compound [F4] with phosphine and azodicarboxylic acid diester in a solvent at room temperature to 100°C.

Examples of the phosphine include trioctylphosphine, tributylphosphine, and triphenylphosphine.

Examples of the azodicarboxylic acid diester include diisopropyl azodicarboxylate and di-tert-butyl azodicarboxylate.

Examples of the solvent include toluene, tetrahydrofuran, and 2-methyltetrahydrofuran.

### Step 6-5

Compound [F6] can be obtained by reacting compound [F5] with N-methoxy-N-methylacetamide. For example, compound [F6] can be obtained by reacting compound [F5] with N-methoxy-N-methylacetamide in a solvent at -78°C to room temperature in the presence of a base.

Examples of the base include n-butyllithium and lithium diisopropylamide.

Examples of the solvent include cyclopentyl methyl ether, tetrahydrofuran, and toluene.

### Step 6-6

When R^{E} is R^{B}, compound [II-1] can be obtained by reacting compound [F6] with (trifluoromethyl)trimethylsilane. For example, compound [II-1] can be obtained by an operation similar to that in Step 2-10.

The methyl group of compound [F6] becomes a steric hindrance and the reaction proceeds diastereoselectively, whereby compound [11-1] is obtained as an optically active form.

### Step 6-7

When X⁶ is nitrogen, X⁷ is carbon, and R^{E} is bromo bonded to X⁷, compound F7 is obtained by Step 6-6. Compound [11-2] can be obtained by reacting compound F7 with compound [F8]. For example, Compound [11-2] can be obtained by reacting compound F7 with compound [F8] in a solvent in the presence of a base and a palladium catalyst at room temperature to 120°C.

Examples of the base include sodium tert-butoxide.

Examples of the palladium catalyst include XPhos Pd G4 and RuPhos Pd G4.

Examples of the solvent include 1,4-dioxane and toluene.

Compound [F8] may be a commercially available product, or may be obtained by appropriately converting a commercially available product by a method well known to those of ordinary skill in the art. Compound [F8] may also be a salt with an acid.

### [Production Method 7]

Compounds of the formulas [11-3] to [11-5] can be obtained by Production Method 7 shown by the following scheme. 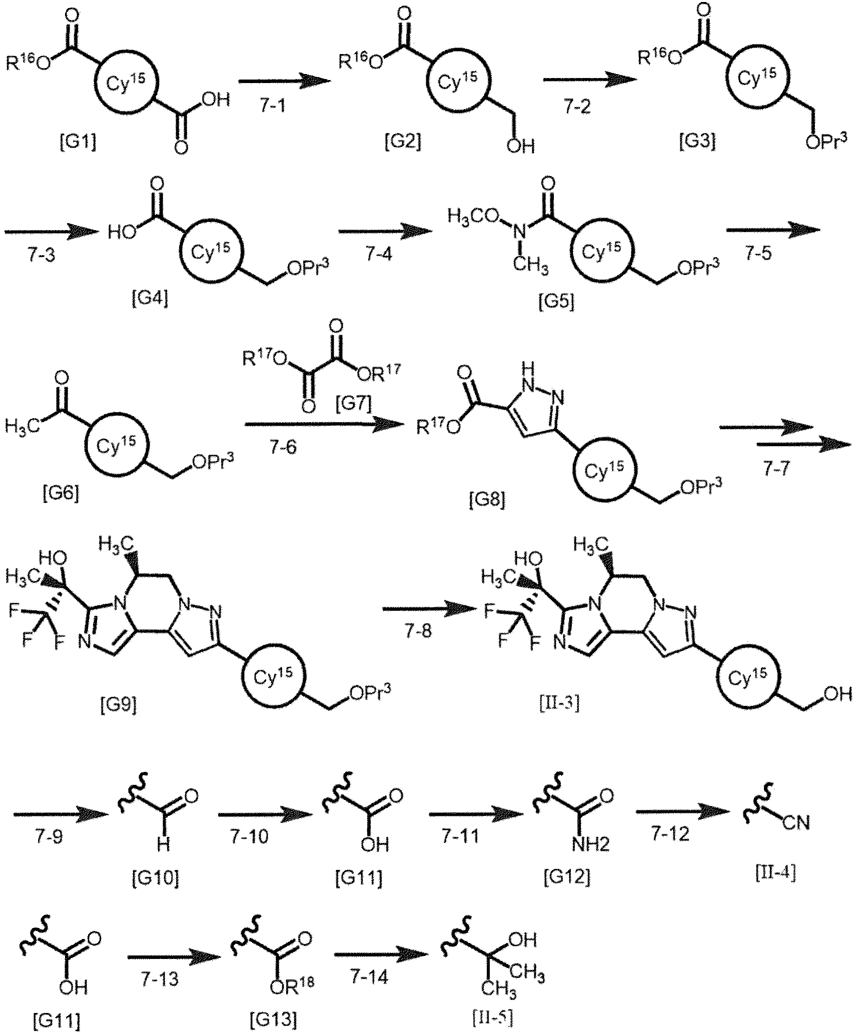
wherein R¹⁶, R¹⁷ and R¹⁸ are each independently C₁₋₉ alkyl;
Cy¹⁵ is bridged C₅₋₁₀ cycloalkylene or 5- to 10-membered bridged heterocyclylene having 1 or 2 hetero atoms independently selected from a nitrogen atom and an oxygen atom; and
Pr³ is a hydroxy-protecting group such as 2-tetrahydropyranyl and the like.

### Step 7-1

Compound [G2] can be obtained by reducing the carboxy group of compound [G1]. For example, compound [G2] can be obtained by an operation similar to that in Step 2-2.

Compound [G1] may be a commercially available product, or may be obtained by appropriately converting a commercially available product by a method well known to those of ordinary skill in the art.

### Step 7-2

Compound [G3] can be obtained by protecting the hydroxy group of compound [G2]. For example, when Pr³ is 2-tetrahydropyranyl, compound [G3] can be obtained by reacting compound [G2] with 3,4-dihydro-2H-pyran in a solvent in the presence of an acid under ice-cooling to room temperature.

Examples of the acid include pyridinium p-toluenesulfonate.

Examples of the solvent include acetonitrile.

### Step 7-3

Compound [G4] can be obtained by hydrolysis of the ester of compound [G3]. For example, compound [G4] can be obtained by treating compound [G3] with an alkali in a solvent under ice-cooling to 80°C.

Examples of the alkali include lithium hydroxide and sodium hydroxide.

Examples of the solvent include methanol, ethanol, and water.

### Step 7-4

Compound [G5] can be obtained by an amidation reaction of compound [G4] and N,O-dimethylhydroxylamine. For example, compound [G5] can be obtained by reacting compound [G4] with N,O-dimethylhydroxylamine in a solvent in the presence of a base and a condensing agent under ice-cooling to room temperature.

Examples of the base include diisopropylethylamine and triethylamine.

Examples of the condensing agent include 1-[bis(dimethylamino)methylene]-1H-1,2,3-triazolo[4,5-b]pyridinium 3-oxide hexafluorophosphate (HATU) and 1-ethyl-3-(3-dimethylaminopropyl)carbodiimide hydrochloride (WSC·HCl).

Examples of the solvent include pyridine and N,N-dimethylformamide.

### Step 7-5

Compound [G6] can be obtained by reacting compound [G5] with methylmagnesium halide. For example, compound [G5] can be obtained by the same operation as in Step 2-9.

### Step 7-6

Compound [G8] can be obtained by a pyrazole cyclization reaction using compound [G6] and compound [G7]. For example, compound [G8] can be obtained by reacting compound [G6] with compound [G7] in a solvent in the presence of a base under ice-cooling to room temperature, and then reacting the obtained compound with hydrazine under ice-cooling to room temperature. The intermediate resulting from the reaction with compound [G7] may be isolated and the intermediate may be reacted with hydrazine.

Examples of the base include potassium tert-butoxide.

Examples of the solvent include tetrahydrofuran.

Compound [G7] may be a commercially available product, or may be obtained by appropriately converting a commercially available product by a method well known to those of ordinary skill in the art.

### Step 7-7

Compound [G9] can be obtained using compound [G8] by reactions similar to those in Steps 6-1 to 6-6.

### Step 7-8

Compound [11-3] can be obtained by deprotection of compound [G9]. For example, when Pr³ is 2-tetrahydropyranyl, compound [11-3] can be obtained by treating compound [G9] with an acid in a solvent at room temperature to 70°C.

Examples of the acid include 10-camphorsulfonic acid.

Examples of the solvent include methanol.

### Step 7-9

Compound [G10] can be obtained by oxidation of a hydroxy group of compound [11-3]. For example, compound [G10] can be obtained by an operation similar to that in Step 6-2.

### Step 7-10

Compound [G11] can be obtained by reacting compound [G10] with an oxidizing agent in a solvent in the presence of an additive and a scavenger under ice-cooling to room temperature.

Examples of the oxidizing agent include sodium chlorite.

Examples of the additive include sodium dihydrogen phosphate dihydrate and disodium hydrogen phosphate.

Examples of the scavenger include 2-methyl-2-butene.

Examples of the solvent include tert-butanol and water.

### Step 7-11

Compound [G12] can be obtained by an amidation reaction of compound [G11] and ammonia. For example, compound [G12] can be obtained by an operation similar to that in Step 7-4.

### Step 7-12

Compound [11-4] can be obtained by a cyanation reaction of compound [G12]. For example, compound [11-4] can be obtained by reacting compound [G12] with an acid anhydride in a solvent in the presence of a base under ice-cooling to room temperature.

Examples of the base include triethylamine.

Examples of the acid anhydride include trifluoroacetic anhydride.

Examples of the solvent include 1,4-dioxane and tetrahydrofuran.

### Step 7-13

Compound [G13] can be obtained by an esterification reaction of compound [G11]. For example, compound [G13] can be obtained by reacting compound [G11] with trimethylsilyldiazomethane in a solvent under ice-cooling to room temperature.

Examples of the solvent include toluene and methanol.

### Step 7-14

Compound [11-5] can be obtained by reacting compound [G13] with two equivalents of methylmagnesium halide. For example, compound [11-5] can be obtained by an operation similar to that in Step 2-9.

### [Production Method 8]

The compounds of the formulas [11-6] and [11-7] can be obtained by Production Method 8 shown by the following scheme. 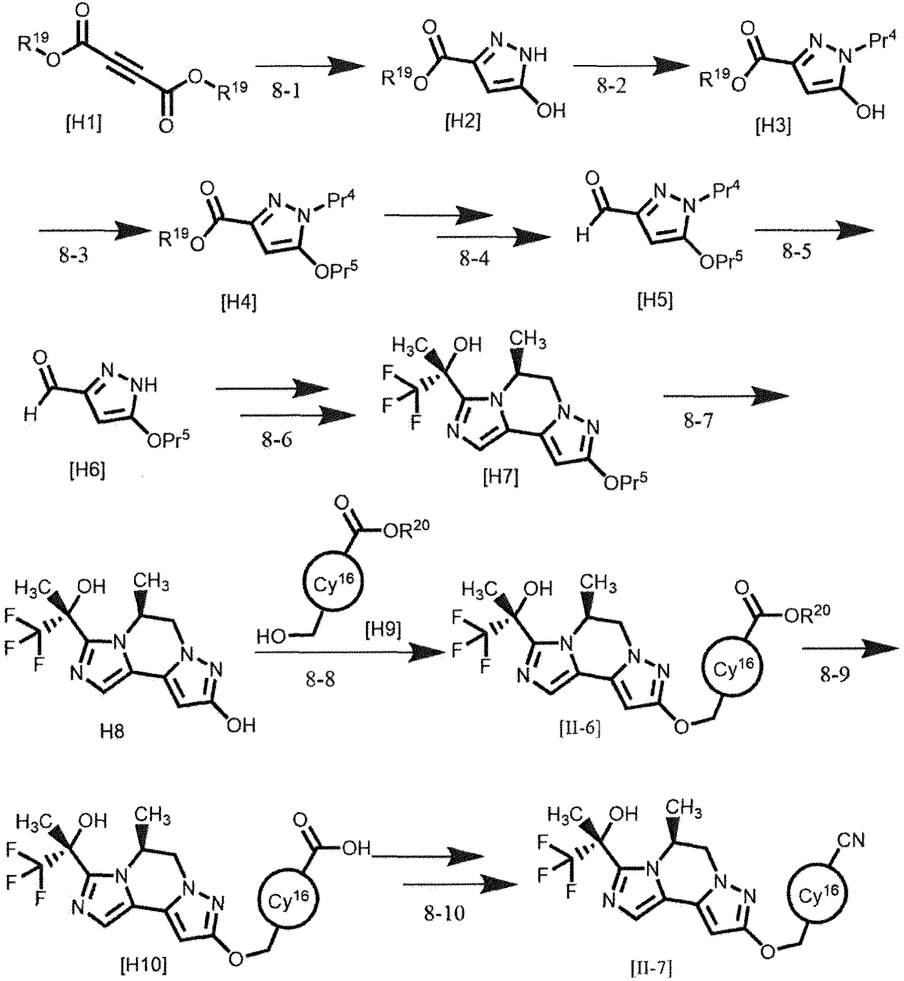
wherein R¹⁹ and R²⁰ are each independently C₁₋₄ alkyl;
Pr⁴ is a pyrazole-protecting group such as 2-tetrahydropyranyl and the like;
Pr⁵ is a hydroxy-protecting group such as benzyl and the like; and
Cy¹⁶ is bridged C₅₋₁₀ cycloalkylene.

### Step 8-1

Compound [H2] can be obtained by reacting compound [H1] with hydrazine. For example, compound [H2] can be obtained by reacting compound [H1] with hydrazine in a solvent at room temperature. Where necessary, the reaction may be performed in the presence of an acid.

Examples of the solvent include acetonitrile, toluene and ethanol.

Examples of the acid include acetic acid.

Compound [H1] may be a commercially available product, or may be obtained by appropriately converting a commercially available product by a method well known to those of ordinary skill in the art.

### Step 8-2

Compound [H3] can be obtained by introduction of a protecting group into pyrazole of compound [H2]. For example, when Pr⁴ is 2-tetrahydropyranyl, compound [H3] can be obtained by reacting compound [H2] with 3,4-dihydro-2H-pyran in a solvent in the presence of an acid at room temperature.

Examples of the solvent include acetonitrile and N,N-dimethylformamide.

Examples of the acid include pyridinium p-toluenesulfonate and p-toluenesulfonic acid.

### Step 8-3

Compound [H4] can be obtained by protecting the hydroxy group of compound [H3]. For example, when Pr⁵ is benzyl, compound [H4] can be obtained by reacting compound [H3] with benzyl halide in a solvent in the presence of a base at room temperature.

Examples of the benzyl halide include benzyl chloride and benzyl bromide.

Examples of the solvent include N-methylpyrrolidone, N,N-dimethylformamide, acetonitrile, toluene, isopropyl acetate, tetrahydrofuran, and dimethyl sulfoxide.

Examples of the base include potassium carbonate, sodium carbonate, cesium carbonate, lithium carbonate, potassium tert-butoxide, potassium acetate, potassium phosphate, 1,8-diazabicyclo[5.4.0]undec-7-ene (DBU), and N,N-diisopropylethylamine.

### Step 8-4

Compound [H5] can be obtained using compound [H4] by reactions similar to those in Steps 6-1 and 6-2.

### Step 8-5

Compound [H6] can be obtained by deprotection of the protecting group of pyrazole of compound [H5]. For example, when Pr⁴ is 2-tetrahydropyranyl, compound [H6] can be obtained by treating compound [H5] with an acid in a solvent at room temperature.

Examples of the acid include hydrochloric acid, methanesulfonic acid, sulfuric acid, and phosphoric acid.

Examples of the solvent include 1,2-dimethoxyethane.

### Step 8-6

Compound [H7] can be obtained using compound [H6] by reactions similar to those in Step s 6-3 to 6-6.

### Step 8-7

Compound H8 can be obtained by deprotection of compound [H7]. For example, compound H8 can be obtained by treating compound [H7] with an acid at room temperature to 50°C. Compound H8 may be obtained as a salt with an acid used.

Examples of the acid include concentrated hydrochloric acid.

### Step 8-8

Compound [11-6] can be obtained by a Mitsunobu reaction of compound H8 and compound [H9]. For example, compound [11-6] can be obtained by an operation similar to that in Step 4-3.

Compound [H9] may be a commercially available product, or may be obtained by appropriately converting a commercially available product by a method well known to those of ordinary skill in the art.

### Step 8-9

Compound [H10] can be obtained by hydrolysis of the ester of compound [11-6]. For example, compound [H10] can be obtained by an operation similar to that in Step 7-3.

### Step 8-10

Compound [11-7] can be obtained using compound [H10] by reactions similar to those in Steps 7-11 and 7-12.

### [Production Method 9]

The compound of the formula [11-8] can be obtained by Production Method 9 shown by the following scheme. 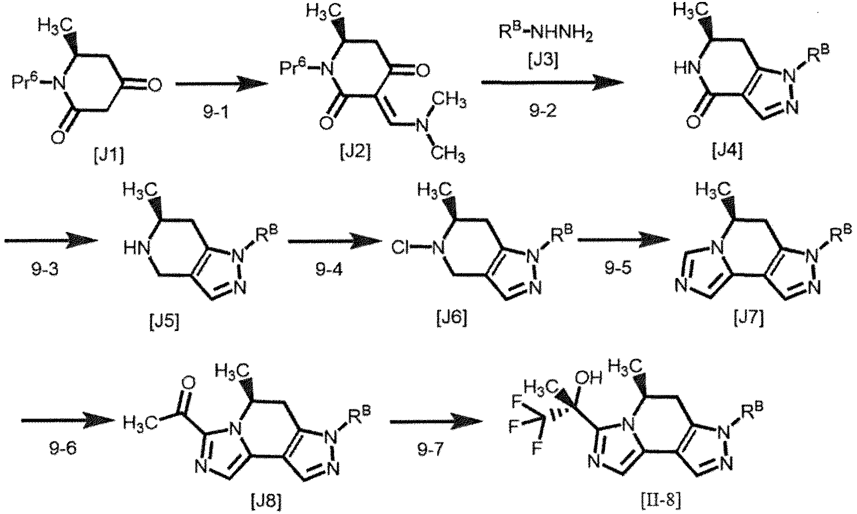
wherein Pr⁶ is an amino-protecting group such as tert-butoxycarbonyl and the like; and
other each symbol is as defined for the formula [II].

### Steps 9-1 and 9-2

Compound [J4] can be obtained by a pyrazole cyclization reaction using compound [J1] and compound [J3]. For example, compound [J2] can be obtained by reacting compound [J1] with N,N-dimethylformamide dimethyl acetal in a solvent at room temperature. Thereafter, compound [J4] can be obtained by reacting compound [J2] with compound [J3] in a solvent in the presence of an acid at 100°C to 200°C. When Pr⁶ is tert-butoxycarbonyl, deprotection also proceeds.

Examples of the solvent of the reaction with N,N-dimethylformamide dimethyl acetal include toluene.

Examples of the solvent of the cyclization reaction include ethanol, isopropanol, and water.

Examples of the acid include acetic acid.

Compound [J1] and compound [J3] may be commercially available products, or may be obtained by appropriately converting a commercially available product by a method well known to those of ordinary skill in the art.

### Step 9-3

Compound [J5] can be obtained by reducing compound [J4]. For example, compound [J5] can be obtained by reacting compound [J4] with a reducing agent in a solvent at room temperature to 90°C.

Examples of the reducing agent include borane and lithium aluminum hydride.

Examples of the solvent include tetrahydrofuran.

### Step 9-4

Compound [J6] can be obtained by chlorination of compound [J5]. For example, compound [J6] can be obtained by reacting compound [J5] with a chlorinating agent in a solvent in the presence of a base under ice-cooling to room temperature.

Examples of the base include 1,8-diazabicyclo[5.4.0]undec-7-ene (DBU).

Examples of the chlorinating agent include N-chlorosuccinimide.

Examples of the solvent include dichloromethane.

### Step 9-5

Compound [J7] can be obtained by an imination reaction of compound [J6] and a cyclization reaction using p-toluenesulfonylmethyl isocyanide. For example, the imination reaction is performed by reacting compound [J6] with a base in a solvent at room temperature. Then the resultant product is reacted with p-toluenesulfonylmethyl isocyanide in a solvent in the presence of a base under ice-cooling to room temperature, whereby compound [J7] can be obtained.

Examples of the solvent of the imination reaction include methanol and 1,2-dimethoxyethane.

Examples of the base of the imination reaction include sodium hydroxide.

Examples of the base of the cyclization reaction include potassium carbonate.

Examples of the solvent of the cyclization reaction include tetrahydrofuran and 1,2-dimethoxyethane.

### Step 9-6

Compound [J8] can be obtained by an acylation reaction of compound [J7]. For example, compound [J8] can be obtained by reacting compound [J7] with N-methoxy-N-methylacetamide in a solvent in the presence of a base.

Examples of the base include lithium diisopropylamide.

Examples of the solvent include tetrahydrofuran.

### Step 9-7

Compound [11-8] can be obtained by reacting compound [J8] with (trifluoromethyl)trimethylsilane. For example, compound [II-8] can be obtained by an operation similar to that in Step 2-10.

The methyl group of compound [J8] becomes a steric hindrance and the reaction proceeds diastereoselectively, whereby compound [11-8] is obtained as an optically active form.

### [Production Method 10]

The compound of the formulas [III] can be obtained by Production Method 10 shown by the following scheme. 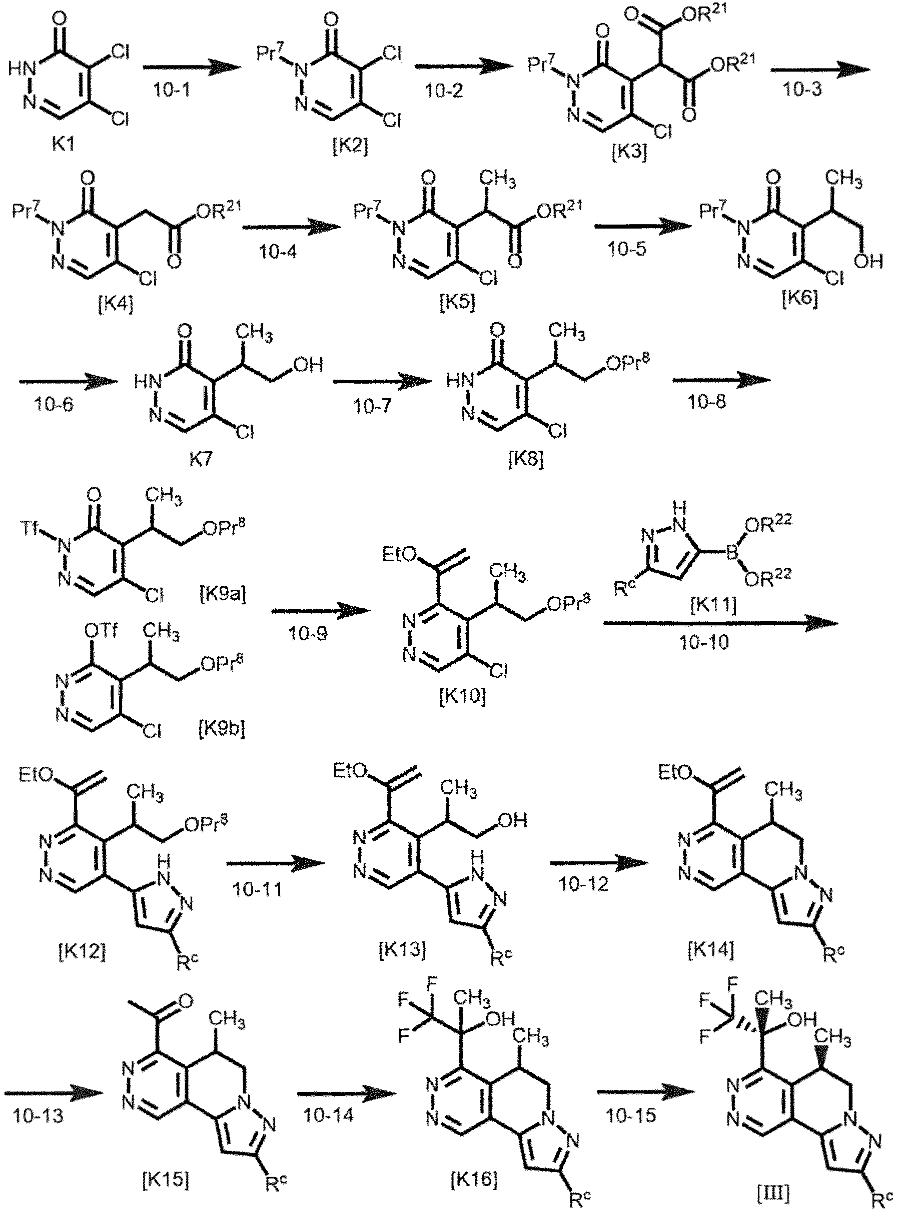
wherein Pr⁷ is a protecting group such as p-methoxybenzyl and the like;
Pr⁸ is a hydroxy-protecting group such as tert-butyldimethylsilyl and the like;
R²¹ is C₁₋₄ alkyl;
each R²² is independently hydrogen or C₁₋₄ alkyl, and one R²² may be bonded to the other R²² to form a ring; and
other each symbol is as defined for the aforementioned formula [III].

### Step 10-1

Compound [K2] can be obtained by protection of compound K1. For example, when Pr⁷ is p-methoxybenzyl, compound [K2] can be obtained by reacting compound K1 with p-methoxybenzyl halide in a solvent in the presence of a base. Where necessary, an additive may also be used.

Examples of the p-methoxybenzyl halide include p-methoxybenzyl chloride.

Examples of the base include potassium hydroxide and cesium carbonate.

Examples of the additive include tetrabutylammonium bromide.

Examples of the solvent include toluene and N,N-dimethylformamide.

Compound [K1] may be a commercially available product, or may be obtained by appropriately converting a commercially available product by a method well known to those of ordinary skill in the art.

### Step 10-2

Compound [K3] can be obtained by reacting compound [K2] with malonic acid diester. For example, compound [K3] can be obtained by reacting compound [K2] with malonic acid diester in a solvent in the presence of a base.

Examples of the malonic acid diester include diethyl malonate.

Examples of the base include sodium tert-pentoxide.

Examples of the solvent include tetrahydrofuran.

### Step 10-3

Compound [K4] can be obtained by heating compound [K3] in a solvent at 100°C to 140°C.

Examples of the solvent include water and dimethyl sulfoxide.

### Step 10-4

Compound [K5] can be obtained by methylation of compound [K4]. For example, compound [K5] can be obtained by reacting compound [K4] with a methylating agent in a solvent in the presence of a base at -78°C to room temperature.

Examples of the base include lithium bis(trimethylsilyl)amide.

Examples of the methylating agent include methyl iodide.

Examples of the solvent include tetrahydrofuran.

### Step 10-5

Compound [K6] can be obtained by reduction of the ester group of compound [K5]. For example, compound [K6] can be obtained by reacting compound [K5] with a reducing agent in a solvent under ice-cooling.

Examples of the reducing agent include lithium aluminum hydride.

Examples of the solvent include tetrahydrofuran, diethyl ether, and cyclopentyl methyl ether.

### Step 10-6

Compound K7 can be obtained by deprotection of compound [K6]. For example, when Pr⁷ is p-methoxybenzyl, compound K7 can be obtained by reacting compound [K6] with cerium(IV) ammonium nitrate in a solvent under ice-cooling to room temperature.

Examples of the solvent include acetonitrile and water.

### Step 10-7

Compound [K8] can be obtained by protection of the hydroxy group of compound K7. For example, when Pr⁸ is tert-butyldimethylsilyl, compound [K8] can be obtained by reacting compound K7 with tert-butyldimethylsilyl chloride in a solvent in the presence of a base under ice-cooling to room temperature.

Examples of the base include imidazole and 4-dimethylaminopyridine.

Examples of the solvent include N,N-dimethylformamide.

### Step 10-8

Compounds [K9a] and [K9b] can be obtained by reacting compound [K8] with trifluoromethanesulfonic anhydride in a solvent in the presence of a base under ice-cooling.

Examples of the base include pyridine.

Examples of the solvent include dichloromethane.

### Step 10-9

compound [K10] can be obtained by a coupling reaction using compounds [K9a] and [K9b] and an organotin reagent. For example, compound [K10] can be obtained by reacting compounds [K9a] and [K9b] with an organotin reagent in a solvent in the presence of a palladium catalyst and an additive at 100°C to 130°C.

Examples of the palladium catalyst include tetrakis(triphenylphosphine)palladium.

Examples of the additive include lithium chloride and copper iodide.

Examples of the organotin reagent include tributyl(1-ethoxyvinyl)stannane.

Examples of the solvent include 1,4-dioxane.

### Step 10-10

Compound [K12] can be obtained by Suzuki coupling of compound [K10] and compound [K11]. For example, compound [K12] can be obtained by an operation similar to that in Step 4-5.

Compound [K11] may be a commercially available product, or may be obtained by appropriately converting a commercially available product by a method well known to those of ordinary skill in the art.

### Step 10-11

Compound [K13] can be obtained by deprotection of compound [K12]. For example, when Pr⁸ is tert-butyldimethylsilyl, compound [K13] can be obtained by reacting compound [K12] with a fluorine reagent in a solvent under ice-cooling to room temperature.

Examples of the fluorine reagent include tetrabutylammonium fluoride.

Examples of the solvent include tetrahydrofuran.

### Step 10-12

Compound [K14] can be obtained by an intramolecular Mitsunobu reaction of compound [K13]. For example, compound [K14] can be obtained by an operation similar to that in Step 6-4.

### Step 10-13

Compound [K15] can be obtained by treating compound [K14] with an acid in a solvent at room temperature to 60°C.

Examples of the acid include hydrochloric acid.

Examples of the solvent include methanol and tetrahydrofuran.

### Step 10-14

Compound [K16] can be obtained by reacting compound [K15] with (trifluoromethyl)trimethylsilane. For example, compound [K16] can be obtained by an operation similar to that in Step 2-10.

### Step 10-15

Compound [III] can be obtained by purifying compound [K16] by chiral column chromatography. The steric configuration of compound [III] can be determined, for example, by X-ray crystal structure analysis.

### Estimation of steric configuration

WO 2019/151274 discloses PDHK inhibitors of Example 3 and the like, in which the absolute configuration of the ring carbon substituted by the methyl group is S and the absolute configuration of the carbon substituted by the hydroxy group is R. Example 20 (racemate) of the present invention showed an hPDHK2 inhibitory activity of IC₅₀ = 22.6 nM. Example 28, which is an optically active form of Example 20, showed an activity of IC₅₀ = 8.5 nM, whereas the inhibitory activity of its enantiomer was low. From these results, the steric configuration of Example 28 could be estimated. In this way, the steric configuration can be estimated from the inhibitory activity of each enantiomer.

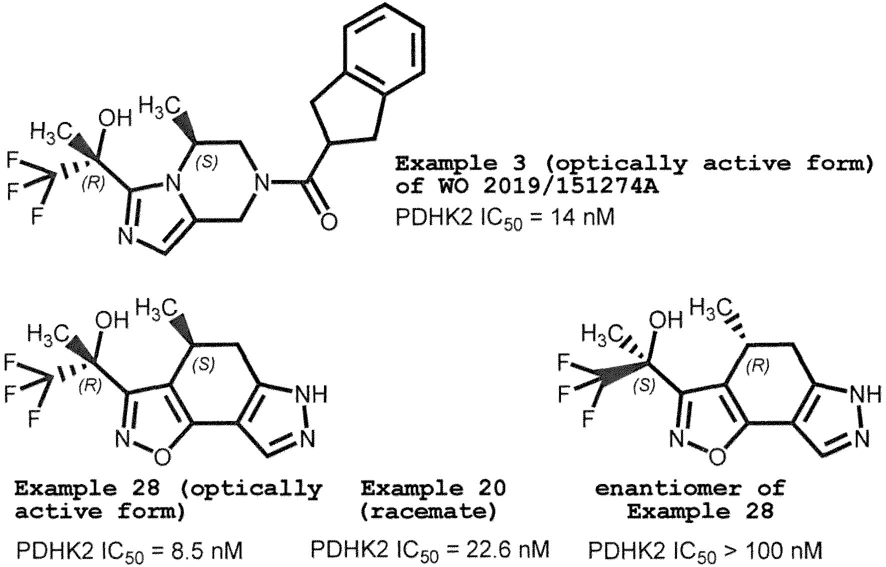

### [Example]

The production method of the compound of the formula [I], the formula [II] or the formula [III] or a pharmaceutically acceptable salt thereof of the present invention is specifically explained by way of the following Production Examples. However, the production method of the compound or a pharmaceutically acceptable salt thereof is not limited by the Production Examples.

Unless otherwise specified, % shows wt%. Unless otherwise specified, the ratio of a mixed solvent is a volume mixing ratio.

In the Examples, abbreviations mean the following.
DMSO: dimethyl sulfoxide
DMF: N,N-dimethylformamide
THF: tetrahydrofuran
M: mol/L
N: normality
HATU: 1-[bis(dimethylamino)methylene]-1H-1,2,3-triazolo[4,5-b]pyridinium 3-oxide hexafluorophosphate
WSC-HCl: 1-ethyl-3-(3-dimethylaminopropyl)carbodiimide hydrochloride
HOBt-H₂O: 1-hydroxybenzotriazole monohydrate

The measurement results of ¹H-NMR are indicated using the following abbreviations.
s: singlet, d: doublet, dd: double doublet, dt: double triplet, t: triplet, q: quartet, dq: double quartet, m: multiplet, brs: broad singlet, brm: broad multiplet, J: coupling constant, Hz: Hertz

¹H-NMR spectrum was measured in CDCl₃ or DMSO-D₆ using tetramethylsilane as an internal standard, and all δ values are shown in ppm.

### Production Example 1

### Synthesis of (R)-1,1,1-trifluoro-2-((R)-6-methyl-3-(trifluoromethyl)-5,6-dihydroisoxazolo[5,4-c][1,2,4]triazolo[4,3-a]pyridin-7-yl)propan-2-ol (Example 1)

### Step 1

### (R)-4-methyl-3-((R)-1,1,1-trifluoro-2-hydroxypropan-2-yl)-5,6-dihydroisoxazolo[5,4-c]pyridin-7(4H)-one

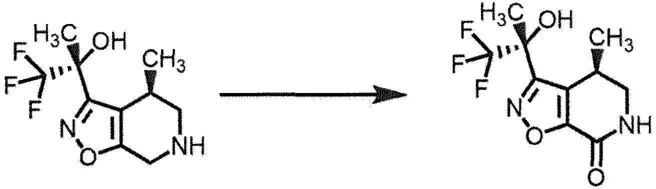

(R)-1,1,1-Trifluoro-2-((R)-4-methyl-4,5,6,7-tetrahydroisoxazolo[5,4-c]pyridin-3-yl)propan-2-ol (1.5 g) was mixed with acetonitrile (27 ml) and water (3 ml). Under ice-cooling, tetrabutylammonium iodide (0.221 g) and iodosobenzene (1.978 g) were added, and the mixture was stirred at room temperature for 1 hr. Under ice-cooling, tetrabutylammonium iodide (0.221 g) and iodosobenzene (1.978 g) were added, and the mixture was stirred at room temperature for 100 min. To the reaction mixture was added saturated sodium thiosulfate aqueous solution, and the mixture was stirred for 1 hr. The mixture was extracted with ethyl acetate. The organic layer was washed twice with saturated aqueous sodium chloride solution, and dried over sodium sulfate. Sodium sulfate was filtered off, and the filtrate was concentrated under reduced pressure. The obtained residue was purified by silica gel column chromatography (hexane:ethyl acetate=100:0 to 0:100) to give the title compound (1.19 g).
¹H-NMR (400 MHz, DMSO-D₆) 1.21 (d, J=6.95Hz, 3H), 1.76 (s, 3H), 3.03 - 3.22 (m, 2H), 3.60 - 3.68 (m, 1H), 7.28 (s, 1H), 8.20 - 8.13 (m, 1H)

### Step 2

### (R)-4-methyl-3-((R)-1,1,1-trifluoro-2-hydroxypropan-2-yl)-5,6-dihydroisoxazolo[5,4-c]pyridin-7(4H)-thione

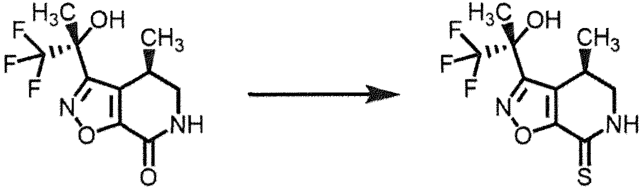

(R)-4-Methyl-3-((R)-1,1,1-trifluoro-2-hydroxypropan-2-yl)-5,6-dihydroisoxazolo[5,4-c]pyridin-7(4H)-one (1.76 g) was mixed with toluene (35.2 ml). At room temperature, pyridine (2.68 ml) and 2,4-bis(4-methoxyphenyl)-1,3-dithia-2,4-diphosphetane-2,4-disulfide (1.886 g), and the mixture was stirred at 120°C for 3 hr. The reaction mixture was concentrated under reduced pressure, and the obtained residue was purified by silica gel column chromatography (hexane:ethyl acetate=100:0 to 0:100) to give the title compound (0.648 g). ¹H-NMR (400 MHz, DMSO-D₆) 1.16 (d, J=6.94Hz, 3H), 1.76 (s, 3H), 3.13 - 3.21 (m, 1H), 3.27 - 3.35 (m, 1H), 3.56 - 3.66 (m, 1H), 7.30 (s, 1H), 10.49 (brs, 1H)

### Step 3

### (R)-1,1,1-trifluoro-2-((R)-7-hydrazinylidene-4-methyl-4,5,6,7-tetrahydroisoxazolo[5,4-c]pyridin-3-yl)propan-2-ol

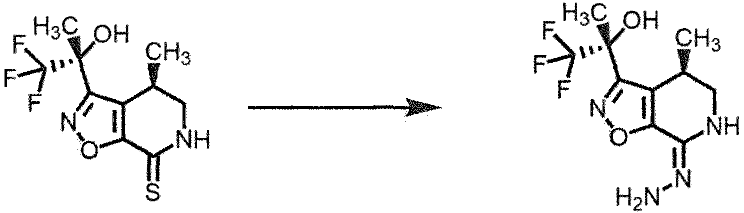

(R)-4-Methyl-3-((R)-1,1,1-trifluoro-2-hydroxypropan-2-yl)-5,6-dihydroisoxazolo[5,4-c]pyridin-7(4H)-thione (0.075 g) obtained in the previous step was mixed with 2-propanol (0.75 ml). At room temperature, hydrazine monohydrate (0.067 g) was added, and the mixture was stirred at 120°C for 40 min. The reaction mixture was concentrated under reduced pressure to give a crude product (0.075 g) of the title compound.

### Step 4

### (R)-1,1,1-trifluoro-2-((R)-6-methyl-3-(trifluoromethyl)-5,6-dihydroisoxazolo[5,4-c][1,2,4]triazolo[4,3-a]pyridin-7-yl)propan-2-ol

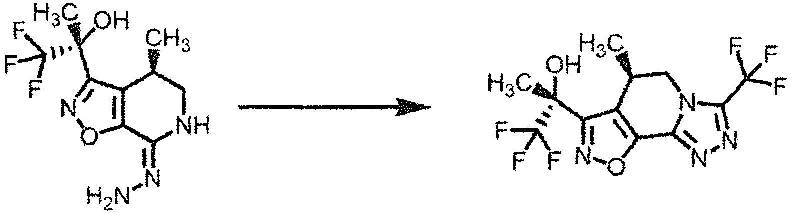

(R)-1,1,1-Trifluoro-2-((R)-7-hydrazinylidene-4-methyl-4,5,6,7-tetrahydroisoxazolo[5,4-c]pyridin-3-yl)propan-2-ol (0.075 g) obtained in the previous step was mixed with chloroform (0.15 ml). At room temperature, trifluoroacetic acid (0.45 ml) and trifluoroacetic anhydride (0.113 ml) were added, and the mixture was stirred overnight. The mixture was stirred at 70°C for 2 hr. At room temperature, saturated aqueous sodium hydrogen carbonate solution was added, and the mixture was extracted with ethyl acetate and the organic layer was washed with water and saturated aqueous sodium chloride solution. After filtration through a phase separator, the residue was concentrated under reduced pressure. The obtained residue was purified by silica gel column chromatography (hexane:ethyl acetate=80:20 to 50:50) to give the title compound (0.066 g).
¹H-NMR (400 MHz, DMSO-D₆) 1.21 (d, J=6.94Hz, 3H), 1.82 (s, 3H), 3.56 - 3.60 (m, 1H), 4.40 (d, J=3.24Hz, 2H), 7.49 (s, 1H)

### Production Example 2-1

### Synthesis of (R)-1,1,1-trifluoro-2-((R)-8-(2-hydroxy-2-methylpropoxy)-4-methyl-4,5-dihydroisoxazolo[5,4-c]pyrazolo[1,5-a]pyridin-3-yl)propan-2-ol (Example 31)

### Step 1

### 4-ethoxy-2-methyl-4-oxobutyric acid

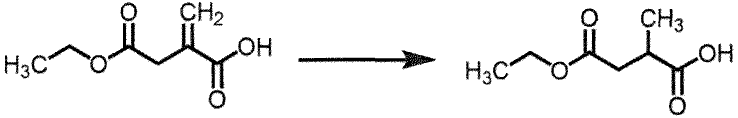

4-Ethoxy-2-methylene-4-oxobutyric acid (25.0 g) and 10 w/w% palladium carbon (2.5 g) were mixed with methanol (200 ml). Under hydrogen atmosphere, the mixture was stirred at room temperature overnight. The reaction mixture was filtered through celite, and the filtrate was concentrated under reduced pressure. The obtained residue was azeotroped with toluene. The obtained residue was filtered through celite again, and the filtrate was concentrated under reduced pressure to give a crude product (25.0 g) of the title compound.

### Step 2

### ethyl 4-hydroxy-3-methylbutyrate

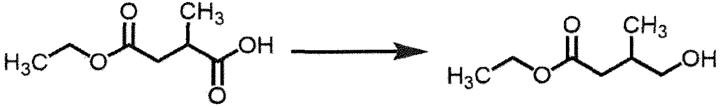

The crude product (21.7 g) of 4-ethoxy-2-methyl-4-oxobutyric acid obtained in the previous step was dissolved in tetrahydrofuran (217 ml). Under ice-cooling, to this solution was added dropwise 0.89 M borane/tetrahydrofuran solution (168 ml). The reaction mixture was stirred at room temperature for 3 hr. Under ice-cooling, methanol (43.5 ml) was added dropwise to the reaction mixture, and the mixture was stirred at room temperature for 30 min. The reaction mixture was concentrated under reduced pressure, water was added to the residue, and the mixture was extracted with ethyl acetate. The organic layer was washed with saturated aqueous sodium chloride solution and dried over magnesium sulfate. Magnesium sulfate was filtered off, and the filtrate was concentrated under reduced pressure to give a crude product (18.4 g) of the title compound.

### Step 3

### ethyl 3-methyl-4-((methylsulfonyl)oxy)butanoate

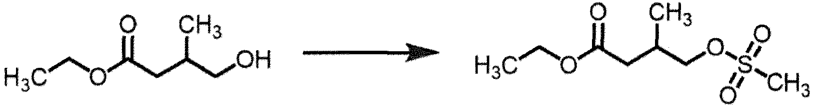

The crude product (18.4 g) of ethyl 4-hydroxy-3-methylbutyrate obtained in the previous step was mixed with dichloromethane (147 ml). Under ice-cooling, triethylamine (26.3 ml) was added to the reaction mixture, and then methanesulfonic anhydride (24.1 g) was added in 3 portions. The reaction mixture was stirred at 0°C for 1 hr. To the reaction mixture was added saturated aqueous sodium hydrogen carbonate solution, and the mixture was extracted with ethyl acetate. The organic layer was washed successively with 1N hydrochloric acid and saturated aqueous sodium chloride solution, and dried over sodium sulfate. Sodium sulfate was filtered off, and the filtrate was concentrated under reduced pressure to give a crude product (27.4 g) of the title compound.

### Step 4

### ethyl 3-(benzyloxy)-1-(4-ethoxy-2-methyl-4-oxobutyl)-1H-pyrazole-5-carboxylate

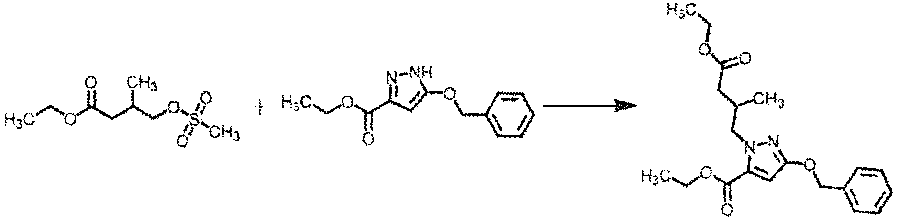

The crude product (27.4 g) of ethyl 3-methyl-4-((methylsulfonyl)oxy)butanoate obtained in the previous step was mixed with dimethylformamide (219 ml). At room temperature, to the reaction mixture were added a crude product (27.4 g) of ethyl 3-(benzyloxy)-1H-pyrazole-5-carboxylate obtained in Auxiliary Step 1 and cesium carbonate (54.3 g). After heating at 80°C for 2 hr, the mixture was stirred at room temperature overnight. Water was added to the reaction mixture, and the mixture was extracted with toluene. The organic layer was washed successively with saturated ammonium chloride aqueous solution and saturated aqueous sodium chloride solution, and dried over sodium sulfate. Sodium sulfate was filtered off, and the filtrate was concentrated under reduced pressure to give a crude product (45 g) of the title compound.

### Step 5

### ethyl 2-(benzyloxy)-6-methyl-4-oxo-4,5,6,7-tetrahydropyrazolo[1,5-a]pyridine-5-carboxylate

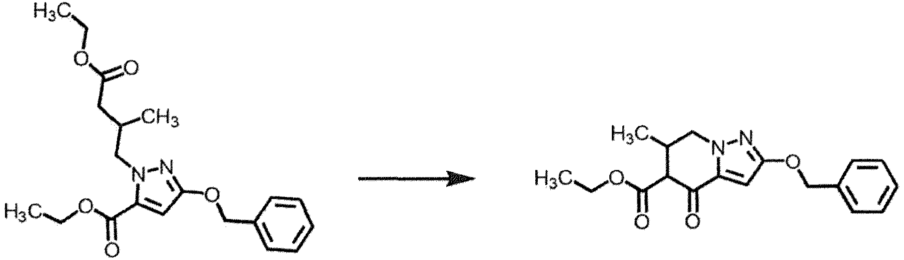

The crude product (45.0 g) of ethyl 3-(benzyloxy)-1-(4-ethoxy-2-methyl-4-oxobutyl)-1H-pyrazole-5-carboxylate obtained in the previous step was mixed with toluene (319 ml). Under water cooling, to the reaction mixture was added potassium tert-butoxide (13.1 g), and the mixture was heated under reflux at 120°C for 2 hr. Saturated ammonium chloride aqueous solution was added to the reaction mixture at room temperature, and the mixture was extracted with ethyl acetate. The organic layer was washed successively with water and saturated aqueous sodium chloride solution, and dried over sodium sulfate. Sodium sulfate was filtered off, and the filtrate was concentrated under reduced pressure to give a crude product (37.2 g) of the title compound.

### Step 6

### 2-(benzyloxy)-6-methyl-6,7-dihydropyrazolo[1,5-a]pyridin-4(5H)-one

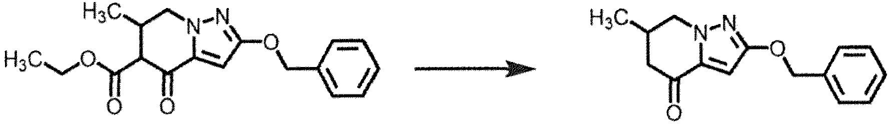

The crude product (37.2 g) of ethyl 2-(benzyloxy)-6-methyl-4-oxo-4,5,6,7-tetrahydropyrazolo[1,5-a]pyridine-5-carboxylate obtained in the previous step was mixed with dimethyl sulfoxide (372 ml) and water (74 ml). At room temperature, to the reaction mixture was added lithium chloride (11.1 g), and the mixture was heated under reflux at 150°C for 8 hr. Water was added to the reaction mixture at room temperature, and the mixture was extracted with toluene. The organic layer was washed successively with water and saturated aqueous sodium chloride solution, and dried over sodium sulfate. Sodium sulfate was filtered off, and the filtrate was concentrated under reduced pressure. The obtained residue was purified by silica gel column chromatography (hexane:ethyl acetate=95:5 to 50:50) to give the title compound (21.0 g).
¹H-NMR (400 MHz, DMSO-D₆) 1.07 (d, J=6.47Hz, 3H), 2.41 - 2.63 (m, 3H), 3.86 (dd, J=12.72, 9.25Hz, 1H), 4.22 - 4.27 (m, 1H), 5.17 (s, 2H), 6.25 (s, 1H), 7.31 - 7.45 (m, 5H)

### Step 7

### ethyl 8-(benzyloxy)-9b-hydroxy-4-methyl-3a,4,5,9b-tetrahydroisoxazolo[5,4-c]pyrazolo[1,5-a]pyridine-3-carboxylate

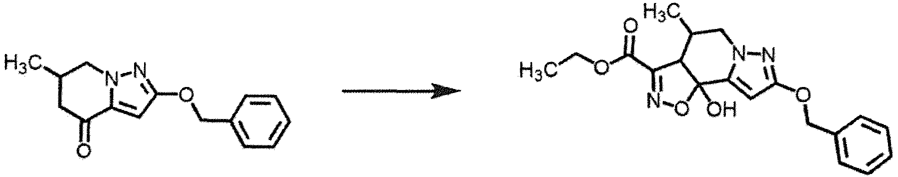

Ethyl 2-chloro-2-(hydroxyimino)acetate (8.73 g) was mixed with tetrahydrofuran (123 ml). To this solution was added dropwise 1.3 M lithium bis(trimethylsilyl)amide/tetrahydrofuran solution (44.3 ml) at -78°C to prepare nitrile oxide solution. In a separate reaction vessel, 2-(benzyloxy)-6-methyl-6,7-dihydropyrazolo[1,5-a]pyridin-4(5H)-one (12.3 g) obtained in the previous step was mixed with tetrahydrofuran (123 ml). To this solution was added dropwise 1.3 M lithium bis(trimethylsilyl)amide/tetrahydrofuran solution (40.6 ml) at -78°C. This reaction mixture was added dropwise through a cannula to the aforementioned nitrile oxide solution cooled to -78°C. The reaction mixture was stirred at -78°C for 30 min, and allowed to warm to room temperature over 1 hr. To the reaction mixture was added dropwise 2N hydrochloric acid (120 ml) at room temperature, and the mixture was extracted with ethyl acetate. The organic layer was washed with saturated aqueous sodium chloride solution, and filtered through a phase separator. The above-mentioned operation was also performed on 2-(benzyloxy)-6-methyl-6,7-dihydropyrazolo[1,5-a]pyridin-4(5H)-one (10.0 g). The filtrates were combined and concentrated under reduced pressure. The obtained residue was purified by silica gel column chromatography (ethyl acetate:hexane=10:90 to 43:57) to give a crude product (12.5 g) of the title compound.

### Step 8

### ethyl 8-(benzyloxy)-4-methyl-4,5-dihydroisoxazolo[5,4-c]pyrazolo[1,5-a]pyridine-3-carboxylate

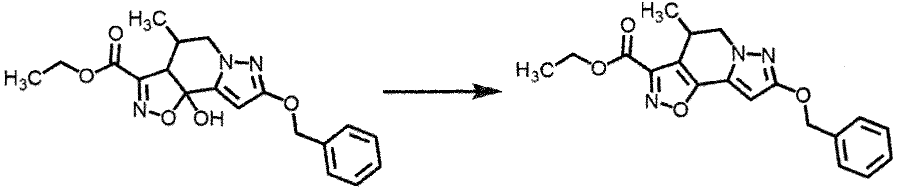

The crude product (12.5 g) of ethyl 8-(benzyloxy)-9b-hydroxy-4-methyl-3a,4,5,9b-tetrahydroisoxazolo[5,4-c]pyrazolo[1,5-a]pyridine-3-carboxylate obtained in the previous step was dissolved in tetrahydrofuran (125 ml). Under ice-cooling, to the reaction mixture were successively added methanesulfonic anhydride (10.2 g) and triethylamine (14 ml). The mixture was stirred at 0°C for 30 min, after which stirred at 45°C for 30 min. The reaction mixture was concentrated under reduced pressure. The obtained residue was purified by silica gel column chromatography (ethyl acetate:hexane=5:95 to ethyl acetate:hexane=50:50) to give the title compound (9.7 g). ¹H-NMR (400 MHz, DMSO-D₆) 1.21 (d, J=7.48Hz, 3H), 1.35 (t, J=7.11Hz, 3H), 3.51 - 3.55 (m, 1H), 4.13 - 4.23 (m, 2H), 4.38 - 4.44 (m, 2H), 5.21 (s, 2H), 6.39 (s, 1H), 7.35 - 7.50 (m, 5H)

### Step 9

### ethyl (R)-8-(benzyloxy)-4-methyl-4,5-dihydroisoxazolo[5,4-c]pyrazolo[1,5-a]pyridine-3-carboxylate

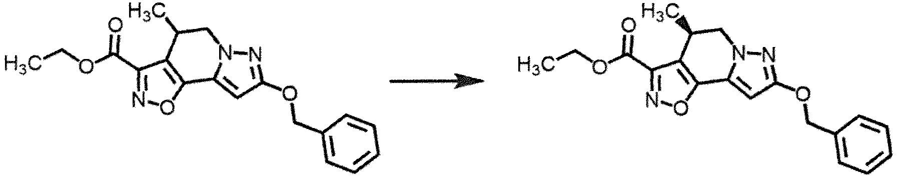

Ethyl 8-(benzyloxy)-4-methyl-4,5-dihydroisoxazolo[5,4-c]pyrazolo[1,5-a]pyridine-3-carboxylate (2.0 g) obtained in the previous step was optically resolved using Japan Analytical Industry Co., Ltd. automatic recycling preparative HPLC (apparatus name: LaboACE LC-7080, column: Daicel CHIRALPAK IA, 20 mm(I.D.) x 250 mm(L), 5 µm, mobile phase flow rate: 20 ml/min, mobile phase mixing ratio: isocratic, hexane:ethanol=85:15) to give the title compound (0.93 g) as the second peak fraction.
¹H-NMR (400 MHz, DMSO-D₆) 1.21 (d, J=7.17Hz, 3H), 1.35 (t, J=7.05Hz, 3H), 3.52 - 3.54 (m, 1H), 4.15 - 4.21 (m, 2H), 4.37 - 4.45 (m, 2H), 5.21 (s, 2H), 6.39 (s, 1H), 7.36 - 7.48 (m, 5H)

### Step 10

### (R)-1-(8-(benzyloxy)-4-methyl-4,5-dihydroisoxazolo[5,4-c]pyrazolo[1,5-a]pyridin-3-yl)ethan-1-one

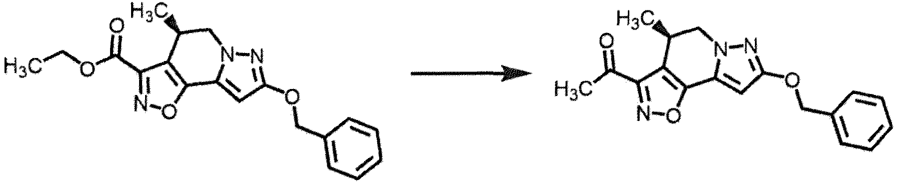

Ethyl (R)-8-(benzyloxy)-4-methyl-4,5-dihydroisoxazolo[5,4-c]pyrazolo[1,5-a]pyridine-3-carboxylate (0.93 g) obtained in the previous step was dissolved in 2-methyltetrahydrofuran (18.6 ml). Under ice-cooling, a mixture of triethylamine (2.2 ml) and 1.08 M methylmagnesium bromide/tetrahydrofuran solution (5.3 ml) was added dropwise, and the mixture was stirred at 0°C for 1 hr. To the reaction mixture was added saturated ammonium chloride aqueous solution, and the mixture was extracted with ethyl acetate. The organic layer was washed successively with water and saturated aqueous sodium chloride solution, and filtered through a phase separator, the filtrate was concentrated under reduced pressure. To the obtained residue were added ethyl acetate (8 ml) and hexane (42 ml), and the mixture was stirred at room temperature. The precipitated solid was collected by filtration to give the title compound (0.64 g).
¹H-NMR (400 MHz, DMSO-D₆) 1.18 (d, J=6.73Hz, 3H), 2.63 (s, 3H), 3.53 - 3.55 (m, 1H), 4.12 - 4.18 (m, 2H), 5.21 (s, 2H), 6.39 (s, 1H), 7.36 - 7.44 (m, 5H)

### Step 11

### (R)-2-((R)-8-(benzyloxy)-4-methyl-4,5-dihydroisoxazolo[5,4-c]pyrazolo[1,5-a]pyridin-3-yl)-1,1,1-trifluoropropan-2-ol

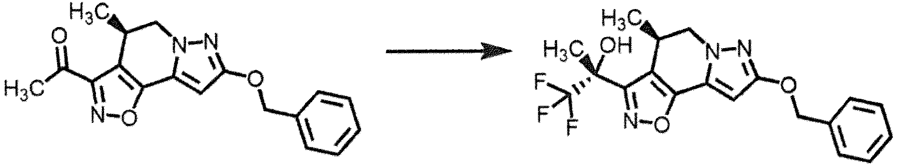

(R)-1-(8-(Benzyloxy)-4-methyl-4,5-dihydroisoxazolo[5,4-c]pyrazolo[1,5-a]pyridin-3-yl)ethan-1-one (0.63 g) obtained in the previous step was mixed with tetrahydrofuran (12.6 ml). Under ice-cooling, to the mixture was added cesium fluoride (0.059 g), and then (trifluoromethyl)trimethylsilane (0.42 ml) was added dropwise. The reaction mixture was stirred at room temperature for 3 hr, methanol (12.6 ml) and potassium carbonate (0.54 g) were added at room temperature, and the mixture was stirred at room temperature for 2 hr. To the reaction mixture was added saturated ammonium chloride aqueous solution, and the mixture was extracted with ethyl acetate. The organic layer was washed successively with water and saturated aqueous sodium chloride solution, filtered through a phase separator, and the filtrate was concentrated under reduced pressure. The obtained residue was purified by silica gel column chromatography (ethyl acetate:hexane=2:98 to 35:65) to give the title compound (0.4 g).
¹H-NMR (400 MHz, DMSO-D₆) 1.20 (d, J=6.94Hz, 3H), 1.78 (s, 3H), 3.41 - 3.42 (m, 1H), 4.12 (d, J=3.24Hz, 2H), 5.20 (s, 2H), 6.32 (s, 1H), 7.30 - 7.48 (m, 6H)

### Step 12

### (R)-4-methyl-3-((R)-1,1,1-trifluoro-2-hydroxypropan-2-yl)-4,5-dihydroisoxazolo[5,4-c]pyrazolo[1,5-a]pyridin-8-ol

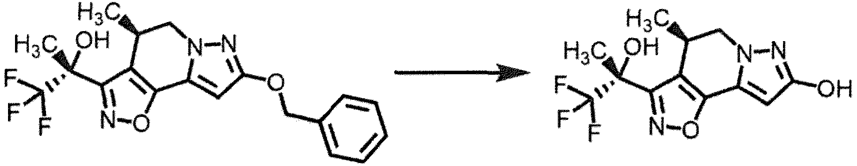

(R)-2-((R)-8-(Benzyloxy)-4-methyl-4,5-dihydroisoxazolo[5,4-c]pyrazolo[1,5-a]pyridin-3-yl)-1,1,1-trifluoropropan-2-ol (1.35 g) obtained in the previous step and 10 w/w% palladium carbon (0.27 g) were mixed with methanol (13.5 ml) and tetrahydrofuran (27 ml). Under hydrogen atmosphere, the mixture was stirred overnight at room temperature. The reaction mixture was filtered through celite, and the filtrate was concentrated under reduced pressure to give the title compound (1.1 g).
¹H-NMR (400 MHz, DMSO-D₆) 1.19 (d, J=6.94Hz, 3H), 1.77 (s, 3H), 3.36 - 3.39 (m, 1H), 4.01 - 4.02 (m, 2H), 5.96 (s, 1H), 7.26 (s, 1H), 10.17 (s, 1H)

### Step 13

### isopropyl 2-(((R)-4-methyl-3-((R)-1,1,1-trifluoro-2-hydroxypropan-2-yl)-4,5-dihydroisoxazolo[5,4-c]pyrazolo[1,5-a]pyridin-8-yl)oxy)acetate

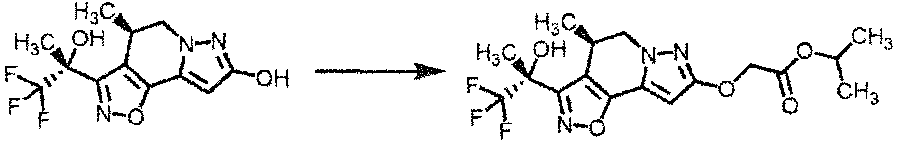

(R)-4-Methyl-3-((R)-1,1,1-trifluoro-2-hydroxypropan-2-yl)-4,5-dihydroisoxazolo[5,4-c]pyrazolo[1,5-a]pyridin-8-ol (0.07 g) obtained in the previous step was mixed with dimethylformamide (1.4 ml). At room temperature, potassium carbonate (0.064 g) and isopropyl bromoacetate (0.063 ml) were added, and the mixture was stirred at 80°C for 1 hr. The reaction mixture was purified by reversed-phase silica gel chromatography (acetonitrile:water=5:95 to 100:0) to give the title compound (0.072 g).
¹H-NMR (400 MHz, DMSO-D₆) 1.17 - 1.19 (m, 9H), 1.78 (s, 3H), 3.38 - 3.42 (m, 1H), 4.03 - 4.09 (m, 2H), 4.77 (s, 2H), 4.96 - 5.02 (m, 1H), 6.30 (s, 1H), 7.29 (s, 1H)

### Step 14

### (R)-1,1,1-trifluoro-2-((R)-8-(2-hydroxy-2-methylpropoxy)-4-methyl-4,5-dihydroisoxazolo[5,4-c]pyrazolo[1,5-a]pyridin-3-yl)propan-2-ol

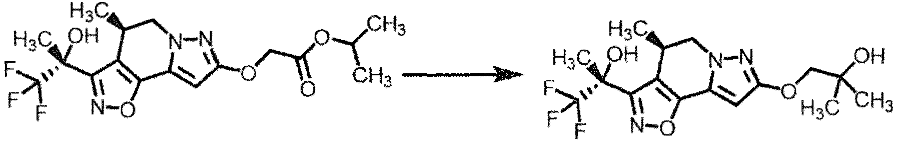

Isopropyl 2-(((R)-4-methyl-3-((R)-1,1,1-trifluoro-2-hydroxypropan-2-yl)-4,5-dihydroisoxazolo[5,4-c]pyrazolo[1,5-a]pyridin-8-yl)oxy)acetate (0.063 g) obtained in the previous step was mixed with toluene (1.3 ml). Under ice-cooling, 1.08 M methylmagnesium bromide/tetrahydrofuran solution (0.6 ml) was added, and the mixture was stirred at room temperature for 1 hr. To the reaction mixture was added saturated ammonium chloride aqueous solution, and the mixture was extracted with ethyl acetate. The organic layer was washed successively with water and saturated aqueous sodium chloride solution, and filtered through a phase separator. The filtrate was concentrated under reduced pressure. The obtained residue was purified by silica gel chromatography (hexane:ethyl acetate=67:33 to ethyl acetate) to give the title compound (0.056 g).
¹H-NMR (400 MHz, DMSO-D₆) 1.18 - 1.21 (m, 9H), 1.78 (s, 3H), 3.39 - 3.41 (m, 1H), 3.87 (s, 2H), 4.08 - 4.10 (m, 2H), 4.62 (s, 1H), 6.25 (s, 1H), 7.29 (s, 1H)

### Auxiliary Step 1 ethyl 3-(benzyloxy)-1H-pyrazole-5-carboxylate

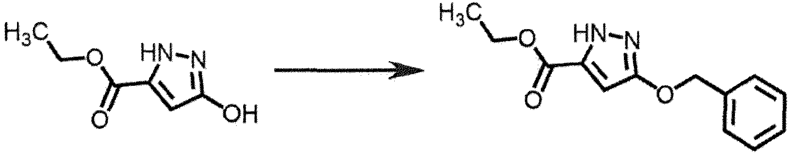

Ethyl 3-hydroxy-1H-pyrazole-5-carboxylate (10 g) was mixed with tetrahydrofuran (100 ml). To the mixture were added benzyl alcohol (8.0 ml) and triphenylphosphine (18.5 g). Under ice-cooling, diisopropyl azodicarboxylate (13.7 ml) was added dropwise, and the mixture was stirred at room temperature overnight. The reaction mixture was concentrated under reduced pressure, ethyl acetate (70 ml) and hexane (140 ml) were added, and the mixture was stirred at room temperature. The precipitated solid was filtered off, and the filtrate was concentrated under reduced pressure. The obtained residue was purified by silica gel column chromatography (hexane:ethyl acetate=92:8 to hexane:ethyl acetate=47:53) to give a crude product (20 g) of the title compound.

### Production Example 2-2

### Synthesis of (R)-1,1,1-trifluoro-2-((R)-4-methyl-4,5-dihydroisoxazolo[5,4-c]pyrazolo[1,5-a]pyridin-3-yl)propan-2-ol (Example 60)

### Step 1

### (R)-4-methyl-3-((R)-1,1,1-trifluoro-2-hydroxypropan-2-yl)-4,5-dihydroisoxazolo[5,4-c]pyrazolo[1,5-a]pyridin-8-yl trifluoromethanesulfonate

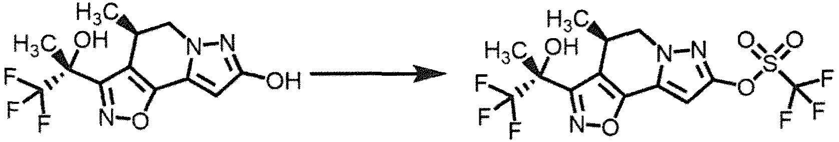

(R)-4-Methyl-3-((R)-1,1,1-trifluoro-2-hydroxypropan-2-yl)-4,5-dihydroisoxazolo[5,4-c]pyrazolo[1,5-a]pyridin-8-ol (0.15 g) was mixed with chloroform (1.5 ml). Under ice-cooling, triethylamine (0.138 ml) and trifluoromethanesulfonic anhydride (0.10 ml) were added, and the mixture was stirred for 1 hr. The reaction mixture was purified by silica gel column chromatography (ethyl acetate:hexane=8:92 to 66:34) to give the title compound (0.2006 g).
¹H-NMR (400 MHz, DMSO-D₆) 1.17 (d, J=7.40Hz, 3H), 1.77 (s, 3H), 3.46 - 3.47 (m, 1H), 4.29 - 4.31 (m, 2H), 7.07 (s, 1H), 7.35 (s, 1H)

### Step 2

### (R)-1,1,1-trifluoro-2-((R)-4-methyl-4,5-dihydroisoxazolo[5,4-c]pyrazolo[1,5-a]pyridin-3-yl)propan-2-ol

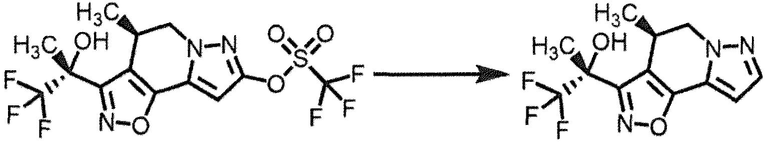

(R)-4-Methyl-3-((R)-1,1,1-trifluoro-2-hydroxypropan-2-yl)-4,5-dihydroisoxazolo[5,4-c]pyrazolo[1,5-a]pyridin-8-yl trifluoromethanesulfonate (0.10 g) obtained in the previous step, 10 w/w% palladium carbon (0.05 g), and sodium acetate (0.094 g) were mixed with methanol (1.0 ml). Under hydrogen atmosphere, the mixture was stirred at room temperature for 4 hr. The reaction mixture was filtered through celite, and the filtrate was concentrated under reduced pressure. The obtained residue was purified by reversed-phase silica gel column chromatography (acetonitrile:water=5:95 to acetonitrile) to give the title compound (0.0594 g).
¹H-NMR (400 MHz, DMSO-D₆) 1.16 (d, J=6.94Hz, 3H), 1.78 (s, 3H), 3.44 - 3.46 (m, 1H), 4.25 (dd, J=13.00, 5.20Hz, 1H), 4.34 (d, J=13.00Hz, 1H), 6.79 (d, J=1.85Hz, 1H), 7.29 (s, 1H), 7.64 (d, J=2.08Hz, 1H)

### Production Example 2-3

### Synthesis of 2-methyl-4-(((R)-4-methyl-3-((R)-1,1,1-trifluoro-2-hydroxypropan-2-yl)-4,5-dihydroisoxazolo[5,4-c]pyrazolo[1,5-a]pyridin-8-yl)oxy)butan-2-ol (Example 62)

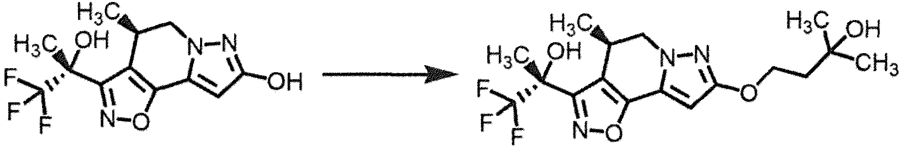

(R)-4-Methyl-3-((R)-1,1,1-trifluoro-2-hydroxypropan-2-yl)-4,5-dihydroisoxazolo[5,4-c]pyrazolo[1,5-a]pyridin-8-ol (0.06 g) was mixed with dimethylformamide (1 ml). Potassium carbonate (0.041 g) and 4-bromo-2-methylbutan-2-ol (0.036 g) were added at room temperature, and the mixture was stirred at 80°C for 3 hr. Water was added to the reaction mixture at room temperature, and the mixture was extracted with ethyl acetate. The organic layer was washed with water and saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=12:88 to ethyl acetate) and reversed-phase silica gel chromatography (acetonitrile:water=5:95 to 100:0) to give the title compound (0.052 g).
¹H-NMR (400 MHz, DMSO-D₆) 1.15 (s, 6H), 1.19 (d, J=7.17Hz, 3H), 1.77 (s, 3H), 1.83 (t, J=7.23Hz, 2H), 3.38 - 3.40 (m, 1H), 4.09 (d, J=3.01Hz, 2H), 4.21 (t, J=7.23Hz, 2H), 4.35 (s, 1H), 6.23 (s, 1H), 7.29 (s, 1H)

### Production Example 2-4

### Synthesis of (R)-1,1,1-trifluoro-2-((R)-4-methyl-8-((tetrahydro-2H-pyran-4-yl)oxy)-4,5-dihydroisoxazolo[5,4-c]pyrazolo[1,5-a]pyridin-3-yl)propan-2-ol (Example 59)

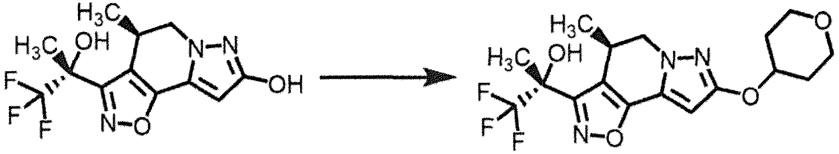

(R)-4-Methyl-3-((R)-1,1,1-trifluoro-2-hydroxypropan-2-yl)-4,5-dihydroisoxazolo[5,4-c]pyrazolo[1,5-a]pyridin-8-ol (0.05 g) was mixed with tetrahydrofuran (0.5 ml). At room temperature, to the reaction mixture were added triphenylphosphine (0.065 g), di-tert-butyl azodicarboxylate (0.057 g) and tetrahydro-2H-pyran-4-ol (0.016 g), and the mixture was stirred at room temperature for 1 hr. The reaction mixture was purified by reversed-phase silica gel chromatography (acetonitrile:water=5:95 to 100:0), and then purified by silica gel column chromatography (ethyl acetate:hexane=20:80 to 55:45) to give the title compound (0.047 g).
¹H-NMR (400 MHz, DMSO-D₆) 1.19 (d, J=6.94Hz, 3H), 1.60 - 1.63 (m, 2H), 1.77 (s, 3H), 2.00 - 2.02 (m, 2H), 3.41 - 3.47 (m, 3H), 3.84 - 3.86 (m, 2H), 4.09 (d, J=3.24Hz, 2H), 4.66 - 4.67 (m, 1H), 6.28 (s, 1H), 7.27 (s, 1H)

### Production Example 2-5

### Synthesis of (1R,4r)-1-methyl-4-(((R)-4-methyl-3-((R)-1,1,1-trifluoro-2-hydroxypropan-2-yl)-4,5-dihydroisoxazolo[5,4-c]pyrazolo[1,5-a]pyridin-8-yl)oxy)cyclohexan-1-ol (Example 65)

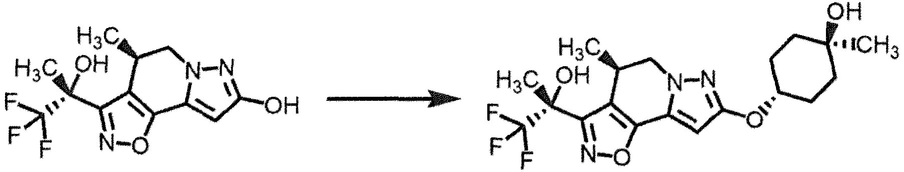

(R)-4-Methyl-3-((R)-1,1,1-trifluoro-2-hydroxypropan-2-yl)-4,5-dihydroisoxazolo[5,4-c]pyrazolo[1,5-a]pyridin-8-ol (0.07 g) was mixed with tetrahydrofuran (0.7 ml). At room temperature, to the reaction mixture were added triphenylphosphine (0.091 g), di-tert-butyl azodicarboxylate (0.036 g) and cis-1-methylcyclohexane-1,4-diol (0.036 g), and the mixture was stirred at room temperature for 1 hr. The reaction mixture was purified by reversed-phase silica gel chromatography (acetonitrile:water=5:95 to 100:0), and then purified by silica gel column chromatography (ethyl acetate:hexane=20:80 to 65:35) to give the title compound (0.045 g). The steric configuration of the title compound was determined by X-ray crystal structure analysis.
¹H-NMR (400 MHz, DMSO-D₆) 1.16 (d, J=17.11Hz, 3H), 1.19 (q, J=6.94Hz, 3H), 1.38 - 1.41 (m, 2H), 1.57 - 1.66 (m, 4H), 1.77 (s, 3H), 1.89 - 1.93 (m, 2H), 3.37 - 3.41 (m, 1H), 4.08 (d, J=3.24Hz, 2H), 4.16 (s, 1H), 4.56 - 4.61 (m, 1H), 6.24 (s, 1H), 7.27 (s, 1H)

### Production Example 2-6

### Synthesis of (R)-1,1,1-trifluoro-2-((R)-8-(fluoromethoxy)-4-methyl-4,5-dihydroisoxazolo[5,4-c]pyrazolo[1,5-a]pyridin-3-yl)propan-2-ol (Example 64)

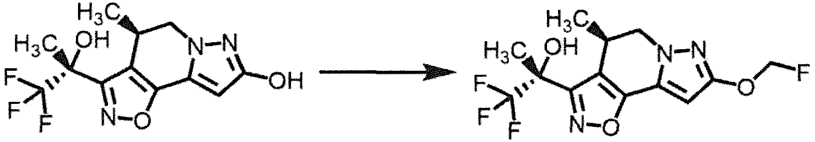

(R)-4-Methyl-3-((R)-1,1,1-trifluoro-2-hydroxypropan-2-yl)-4,5-dihydroisoxazolo[5,4-c]pyrazolo[1,5-a]pyridin-8-ol (0.2 g) was mixed with dimethylformamide (2 ml). At room temperature, potassium carbonate (0.182 g) and fluoromethyl 4-methoxybenzenesulfonate (0.162 g) were added, and the mixture was stirred at 80°C for 3 hr. Water was added to the reaction mixture at room temperature, and the mixture was extracted with ethyl acetate. The organic layer was washed with water and saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=12:88 to ethyl acetate), reversed-phase silica gel chromatography (acetonitrile:water=5:95 to 100:0), and amino silica gel column chromatography (ethyl acetate:hexane=8:92 to 88:12) to give the title compound (0.099 g).
¹H-NMR (400 MHz, DMSO-D₆) 1.20 (d, J=6.94Hz, 3H), 1.78 (s, 3H), 3.42 - 3.44 (m, 1H), 4.16 (s, 2H), 5.81 (q, J=2.39Hz, 1H), 5.95 (q, J=2.39Hz, 1H), 6.47 (s, 1H), 7.30 (s, 1H)

### Production Example 2-7

### Synthesis of (1R,3r)-1-methyl-3-(((R)-4-methyl-3-((R)-1,1,1-trifluoro-2-hydroxypropan-2-yl)-4,5-dihydroisoxazolo[5,4-c]pyrazolo[1,5-a]pyridin-8-yl)oxy)cyclobutane-1-carbonitrile (Example 63)

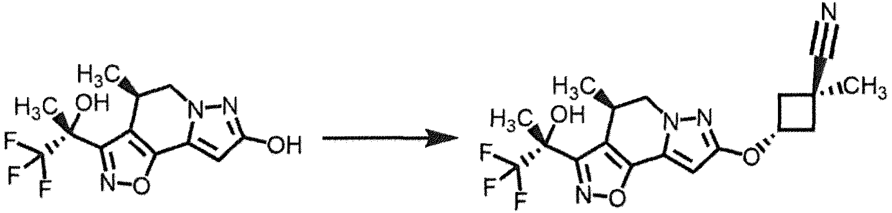

(R)-4-Methyl-3-((R)-1,1,1-trifluoro-2-hydroxypropan-2-yl)-4,5-dihydroisoxazolo[5,4-c]pyrazolo[1,5-a]pyridin-8-ol (0.1 g) was mixed with tetrahydrofuran (2 ml). At room temperature, to the reaction mixture were added triphenylphosphine (0.13 g) and 3-hydroxy-1-methylcyclobutane-1-carbonitrile (0.044 g). Under heating at 70°C, di-tert-butyl azodicarboxylate (0.114 g) was added, and the mixture was stirred for 45 min. To the reaction mixture were added triphenylphosphine (0.065 g), 3-hydroxy-1-methylcyclobutane-1-carbonitrile (0.022 g), and di-tert-butyl azodicarboxylate (0.057 g), and the mixture was stirred for 75 min. The reaction mixture was purified by silica gel column chromatography (ethyl acetate:hexane=6:94 to 50:50), and then purified by reversed-phase silica gel chromatography (acetonitrile:water=5:95 to 100:0) to give the title compound (0.073 g).
¹H-NMR (400 MHz, DMSO-D₆) 1.19 (d, J=7.17Hz, 3H), 1.54 (s, 3H), 1.77 (s, 3H), 2.26 - 2.27 (m, 2H), 2.95 - 3.01 (m, 2H), 3.38 - 3.41 (m, 1H), 4.10 - 4.10 (m, 2H), 4.97 (t, J=7.28Hz, 1H), 6.28 (s, 1H), 7.28 (s, 1H)

### Production Example 2-8

### Synthesis of (R)-1,1,1-trifluoro-2-((R)-4-methyl-8-(tetrahydro-2H-pyran-4-yl)-4,5-dihydroisoxazolo[5,4-c]pyrazolo[1,5-a]pyridin-3-yl)propan-2-ol (Example 61)

### Step 1

### (R)-2-((R)-8-(3,6-dihydro-2H-pyran-4-yl)-4-methyl-4,5-dihydroisoxazolo[5,4-c]pyrazolo[1,5-a]pyridin-3-yl)-1,1,1-trifluoropropan-2-ol

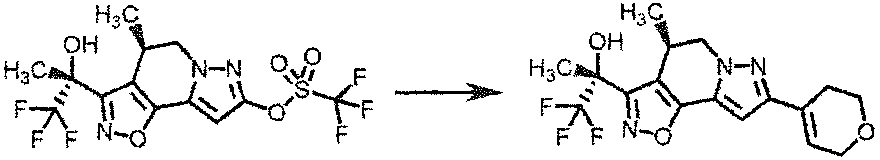

(R)-4-Methyl-3-((R)-1,1,1-trifluoro-2-hydroxypropan-2-yl)-4,5-dihydroisoxazolo[5,4-c]pyrazolo[1,5-a]pyridin-8-yl trifluoromethanesulfonate (100 mg) obtained in the previous step, methanesulfonato(2-dicyclohexylphosphino-2',4',6'-triisopropyl-1,1'-biphenyl)(2'-methylamino-1,1'-biphenyl-2-yl)palladium(II) (59.4 mg), 3,6-dihydro-2H-pyran-4-boronic acid pinacol ester (72.4 mg), and tripotassium phosphate (146 mg) were mixed with 1,4-dioxane (1.2 ml)-water (0.6 ml). Under an argon atmosphere, the reaction mixture was stirred at 70°C for 1 hr. At room temperature, water was added to the reaction mixture, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=12:88 to ethyl acetate) to give the title compound (54.5 mg).
¹H-NMR (400 MHz, CDCl₃) 1.21 - 1.28 (m, 3H), 1.91 (s, 3H), 2.55 - 2.62 (m, 2H), 2.91 (s, 1H), 3.41 - 3.51 (m, 1H), 3.91 (t, J=5.55Hz, 2H), 4.19 - 4.35 (m, 4H), 6.28 - 6.32 (m, 1H), 6.66 (s, 1H)

### Step 2

### (R)-1,1,1-trifluoro-2-((R)-4-methyl-8-(tetrahydro-2H-pyran-4-yl)-4,5-dihydroisoxazolo[5,4-c]pyrazolo[1,5-a]pyridin-3-yl)propan-2-ol

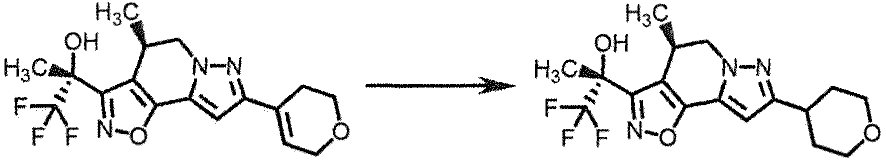

(R)-2-((R)-8-(3,6-Dihydro-2H-pyran-4-yl)-4-methyl-4,5-dihydroisoxazolo[5,4-c]pyrazolo[1,5-a]pyridin-3-yl)-1,1,1-trifluoropropan-2-ol (54.5 mg) obtained in the previous step and palladium carbon (27.3 mg) were mixed with ethanol (1 ml). Under hydrogen atmosphere, the mixture was stirred at room temperature for 4 hr. Palladium carbon was filtered off from the reaction mixture by using celite as an aid, and the filtrate was concentrated under reduced pressure. The obtained residue was purified by silica gel column chromatography (ethyl acetate:hexane=12:88 to ethyl acetate) to give the title compound (36.2 mg).
¹H-NMR (400 MHz, DMSO-D₆) 1.15 (d, J=6.94Hz, 3H), 1.58 - 1.71 (m, 2H), 1.76 (s, 3H), 1.79 - 1.88 (m, 2H), 2.80 - 2.93 (m, 1H), 3.36 - 3.49 (m, 3H), 3.83 - 3.93 (m, 2H), 4.17 (dd, J=13.41, 5.09Hz, 1H), 4.24 (d, J=12.02Hz, 1H), 6.65 (s, 1H), 7.25 (s, 1H)

### Production Example 3-1

### Synthesis of (1S,4r)-1-methyl-4-((S)-4-methyl-3-((R)-1,1,1-trifluoro-2-hydroxypropan-2-yl)-4,5-dihydro-7H-isoxazolo[5,4-e]indazol-7-yl)cyclohexan-1-ol and enantiomer thereof (Example 55) and (1S,4r)-1-methyl-4-((S)-4-methyl-3-((R)-1,1,1-trifluoro-2-hydroxypropan-2-yl)-4,5-dihydro-6H-isoxazolo[5,4-e]indazol-6-yl)cyclohexan-1-ol and enantiomer thereof (Example 56)

### Step 1

### 2-((dimethylamino)methylene)-5-methylcyclohexane-1,3-dione

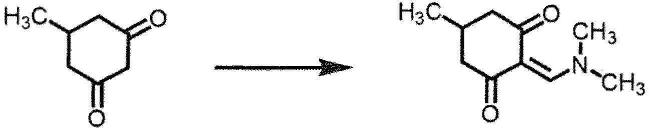

5-Methylcyclohexane-1,3-dione (5.4 g) was mixed with N,N-dimethylformamide dimethyl acetal (10.2 g). The reaction mixture was heated under reflux at 110°C for 1 hr. The reaction mixture was concentrated under reduced pressure, and azeotroped with toluene. To the obtained residue was added a mixed solution of hexane:ethyl acetate=90:10, and the mixture was stirred at room temperature. The precipitated solid was collected by filtration to give the title compound (7.5 g). ¹H-NMR (400 MHz, DMSO-D₆) 0.96 (d, J=5.78Hz, 3H), 2.05 - 2.08 (m, 3H), 2.30 - 2.38 (m, 2H), 3.04 (s, 3H), 3.38 (s, 3H), 7.96 (s, 1H)

### Step 2

### mixture of 1-(4-methoxybenzyl)-6-methyl-1,5,6,7-tetrahydro-4H-indazol-4-one and 2-(4-methoxybenzyl)-6-methyl-2,5,6,7-tetrahydro-4H-indazol-4-one

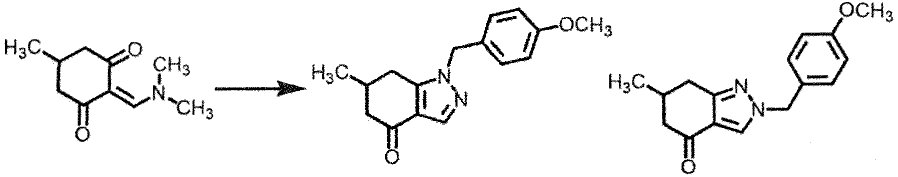

2-((Dimethylamino)methylene)-5-methylcyclohexane-1,3-dione (5.0 g) obtained in the previous step was dissolved in ethanol (50 ml) and acetic acid (25 ml). At room temperature, to the reaction mixture was added (4-methoxybenzyl)hydrazine hydrochloride (5.5 g), and the mixture was heated under reflux at 125°C for 2 hr. The reaction mixture was concentrated under reduced pressure, 1 M sodium hydroxide aqueous solution was added to the residue, and the mixture was extracted with ethyl acetate. The organic layer was washed successively with sodium hydrogen carbonate aqueous solution and saturated aqueous sodium chloride solution, and dried over sodium sulfate. Sodium sulfate was filtered off, and the filtrate was concentrated under reduced pressure. The obtained residue was purified by silica gel column chromatography (hexane:ethyl acetate=80:20 to 20:80), and azeotroped with toluene to give a crude product (7.4 g) of the title compound.

### Step 3

### mixture of ethyl 8b-hydroxy-6-(4-methoxybenzyl)-4-methyl-3a,5,6,8b-tetrahydro-4H-isoxazolo[5,4-e]indazole-3-carboxylate and ethyl 8b-hydroxy-7-(4-methoxybenzyl)-4-methyl-3a,5,7,8b-tetrahydro-4H-isoxazolo[5,4-e]indazole-3-carboxylate

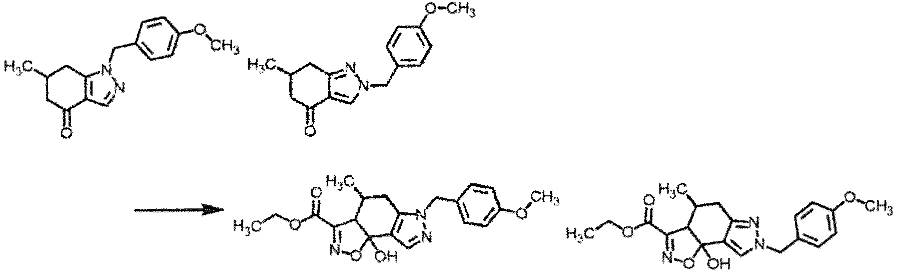

Ethyl 2-chloro-2-(hydroxyimino)acetate (4.7 g) was mixed with tetrahydrofuran (70 ml). To this solution was added dropwise 1.3 M lithium bis(trimethylsilyl)amide/tetrahydrofuran solution (25.9 ml) at -78°C to prepare a nitrile oxide solution. In a separate reaction vessel, the mixture (7.0 g) of 1-(4-methoxybenzyl)-6-methyl-1,5,6,7-tetrahydro-4H-indazol-4-one and 2-(4-methoxybenzyl)-6-methyl-2,5,6,7-tetrahydro-4H-indazol-4-one obtained in the previous step was mixed with tetrahydrofuran (70 ml). To this solution was added dropwise 1.3 M lithium bis(trimethylsilyl)amide/tetrahydrofuran solution (29.9 ml) at -78°C. The reaction mixture was added dropwise through a cannula to the aforementioned nitrile oxide solution cooled to -78°C. The reaction mixture was allowed to warm to 0°C over 1 hr. To the reaction mixture was added dropwise 2N hydrochloric acid (51.8 ml) at room temperature, then saturated aqueous sodium hydrogen carbonate solution was added, and the mixture was extracted with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=40:60 to 90:10) to give a crude product (12.5 g) of the title compound.

### Step 4

### mixture of ethyl 6-(4-methoxybenzyl)-4-methyl-5,6-dihydro-4H-isoxazolo[5,4-e]indazole-3-carboxylate and ethyl 7-(4-methoxybenzyl)-4-methyl-5,7-dihydro-4H-isoxazolo[5,4-e]indazole-3-carboxylate

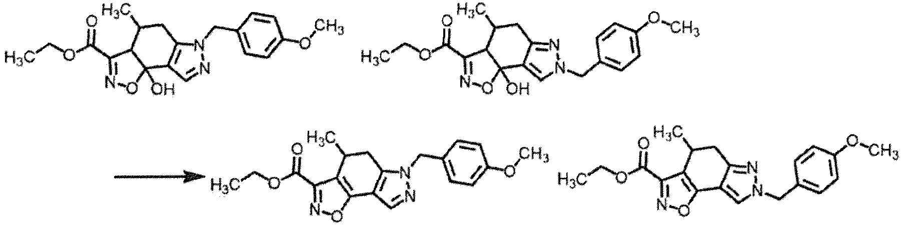

The mixture (9.65 g) of ethyl 8b-hydroxy-6-(4-methoxybenzyl)-4-methyl-3a,5,6,8b-tetrahydro-4H-isoxazolo[5,4-e]indazole-3-carboxylate and ethyl 8b-hydroxy-7-(4-methoxybenzyl)-4-methyl-3a,5,7,8b-tetrahydro-4H-isoxazolo[5,4-e]indazole-3-carboxylate obtained in the previous step was dissolved in tetrahydrofuran (77 ml). Under ice-cooling, to the reaction mixture were successively added methanesulfonic anhydride (5.68 g) and triethylamine (10.5 ml). The reaction mixture was stirred at 0°C for 30 min. At room temperature, to the reaction mixture was added triethylamine (10.5 ml), and the mixture was heated under reflux at 70°C overnight. To the reaction mixture was added water at room temperature, and the mixture was extracted with ethyl acetate. The organic layer was washed successively with saturated ammonium chloride aqueous solution and saturated aqueous sodium chloride solution, and dried over magnesium sulfate. Magnesium sulfate was filtered off, and the filtrate was concentrated under reduced pressure. The obtained residue was purified by silica gel column chromatography (ethyl acetate:hexane=20:80 to 60:40) to give a crude product (6.7 g) of the title compound.

### Step 5

### mixture of 1-(6-(4-methoxybenzyl)-4-methyl-5,6-dihydro-4H-isoxazolo[5,4-e]indazol-3-yl)ethan-1-one and 1-(7-(4-methoxybenzyl)-4-methyl-5,7-dihydro-4H-isoxazolo[5,4-e]indazol-3-yl)ethan-1-one

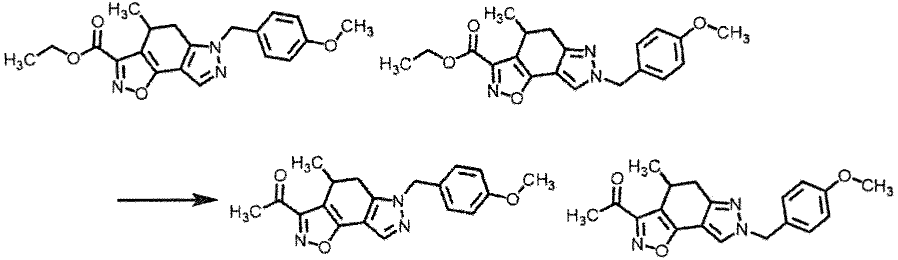

The mixture (6.7 g) of ethyl 6-(4-methoxybenzyl)-4-methyl-5,6-dihydro-4H-isoxazolo[5,4-e]indazole-3-carboxylate and ethyl 7-(4-methoxybenzyl)-4-methyl-5,7-dihydro-4H-isoxazolo[5,4-e]indazole-3-carboxylate obtained in the previous step was dissolved in toluene (66.8 ml). Under ice-cooling, a mixture of triethylamine (12.7 ml) and 1.08 M methylmagnesium bromide/tetrahydrofuran solution (23.6 ml) was added dropwise, and the mixture was stirred at 0°C for 1 hr. Under ice-cooling, a mixture of triethylamine (1.27 ml) and 1.08 M methylmagnesium bromide/tetrahydrofuran solution (2.4 ml) was further added dropwise, and the mixture was stirred at 0°C for 1 hr. To the reaction mixture was added 2N hydrochloric acid, and the mixture was extracted with ethyl acetate. The organic layer was washed successively with saturated aqueous sodium hydrogen carbonate solution and saturated aqueous sodium chloride solution, and dried over magnesium sulfate. Magnesium sulfate was filtered off, and the filtrate was concentrated under reduced pressure. The obtained residue was purified by silica gel column chromatography (ethyl acetate:hexane=30:70 to 60:40) to give a crude product (5.0 g) of the title compound.

### Step 6 1-(4-methyl-5,6-dihydro-4H-isoxazolo[5,4-e]indazol-3-yl)ethan-1-one

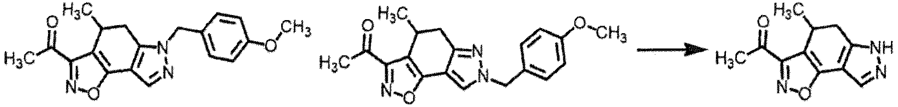

The mixture (1.2 g) of 1-(6-(4-methoxybenzyl)-4-methyl-5,6-dihydro-4H-isoxazolo[5,4-e]indazol-3-yl)ethan-1-one and 1-(7-(4-methoxybenzyl)-4-methyl-5,7-dihydro-4H-isoxazolo[5,4-e]indazol-3-yl)ethan-1-one obtained in the previous step was mixed with trifluoroacetic acid (11.6 ml). To the reaction mixture was added anisole (0.75 ml), and the mixture was stirred at 130°C for 1 hr under microwave (Biotage (registered trademark) Initiator+) radiation. To the reaction mixture was added toluene, and the mixture was concentrated under reduced pressure. The obtained residue was purified by silica gel column chromatography (ethyl acetate:hexane=20:80 to 85:15) to give the title compound (0.71 g).
¹H-NMR (400 MHz, DMSO-D₆) 1.00 (d, J=6.73Hz, 3H), 2.59 (s, 3H), 2.75 - 2.79 (m, 1H), 2.94 - 3.00 (m, 1H), 3.48 - 3.50 (m, 1H), 8.22 (s, 1H), 13.09 (s, 1H)

### Step 7

### mixture of 1-(7-((1r,4r)-4-(methoxymethoxy)-4-methylcyclohexyl)-4-methyl-5,7-dihydro-4H-isoxazolo[5,4-e]indazol-3-yl)ethan-1-one and 1-(6-((1r,4r)-4-(methoxymethoxy)-4-methylcyclohexyl)-4-methyl-5,6-dihydro-4H-isoxazolo[5,4-e]indazol-3-yl)ethan-1-one

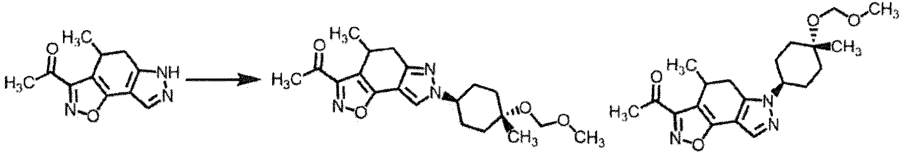

1-(4-Methyl-5,6-dihydro-4H-isoxazolo[5,4-e]indazol-3-yl)ethan-1-one (0.050 g) obtained in the previous step was mixed with dimethylformamide (0.75 ml). To the reaction mixture were added cesium carbonate (0.23 g) and cis-4-(methoxymethoxy)-4-methylcyclohexyl methanesulfonate (0.15 g) obtained in Auxiliary Step 3, and the mixture was stirred at 80°C for 4 hr. The reaction mixture was purified by reversed-phase silica gel chromatography (acetonitrile:water=0:100 to 100:0), and the crude product was purified by silica gel column chromatography (ethyl acetate:hexane=2:98 to 30:70) to give a crude product (0.043 g) of the title compound.

### Step 8

### mixture of (R)-1,1,1-trifluoro-2-((S)-7-((1r,4S)-4-(methoxymethoxy)-4-methylcyclohexyl)-4-methyl-5,7-dihydro-4H-isoxazolo[5,4-e]indazol-3-yl)propan-2-ol and (S)-1,1,1-trifluoro-2-((R)-7-((1r,4R)-4-(methoxymethoxy)-4-methylcyclohexyl)-4-methyl-5,7-dihydro-4H-isoxazolo[5,4-e]indazol-3-yl)propan-2-ol, and mixture of (R)-1,1,1-trifluoro-2-((S)-6-((1r,4S)-4-(methoxymethoxy)-4-methylcyclohexyl)-4-methyl-5,6-dihydro-4H-isoxazolo[5,4-e]indazol-3-yl)propan-2-ol and (S)-1,1,1-trifluoro-2-((R)-6-((1r,4R)-4-(methoxymethoxy)-4-methylcyclohexyl)-4-methyl-5,6-dihydro-4H-isoxazolo[5,4-e]indazol-3-yl)propan-2-ol

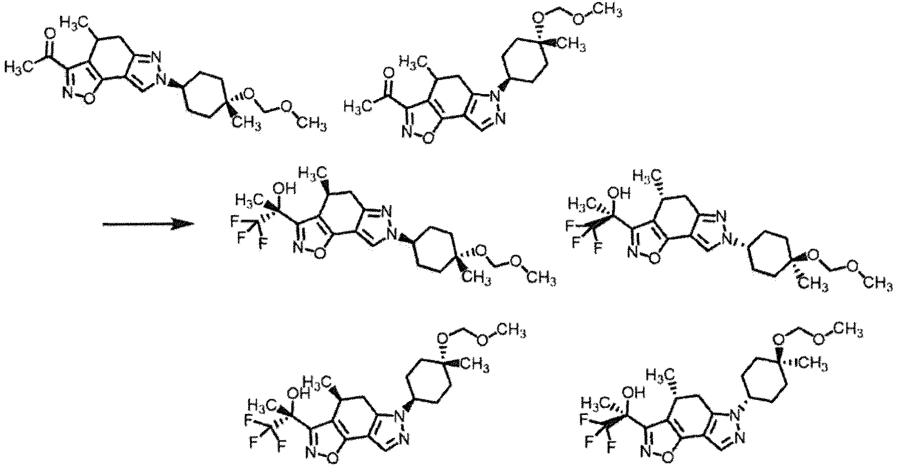

The mixture (0.043 g) of 1-(7-((1r,4r)-4-(methoxymethoxy)-4-methylcyclohexyl)-4-methyl-5,7-dihydro-4H-isoxazolo[5,4-e]indazol-3-yl)ethan-1-one and 1-(6-((1r,4r)-4-(methoxymethoxy)-4-methylcyclohexyl)-4-methyl-5,6-dihydro-4H-isoxazolo[5,4-e]indazol-3-yl)ethan-1-one obtained in the previous step was mixed with tetrahydrofuran (0.86 ml). Under ice-cooling, to the mixture was added cesium fluoride (0.004 g), and then (trifluoromethyl)trimethylsilane (0.033 ml) was added dropwise. The reaction mixture was stirred for 1 hr while raising the temperature to room temperature. Methanol (0.86 ml) and potassium carbonate (0.095 g) were added at room temperature, and the mixture was stirred at room temperature for 30 min. To the reaction mixture was added saturated ammonium chloride aqueous solution, and the mixture was extracted with ethyl acetate. The organic layer was washed with water and saturated aqueous sodium chloride solution, filtered through a phase separator, and the filtrate was concentrated under reduced pressure. The obtained residue was purified by preparative thin layer silica gel chromatography (dichloromethane:ethyl acetate=6:1, developed 3 times) to give the title compound (0.009 g) and a diastereomer (0.004 g) thereof each as a racemate.
¹H-NMR (400 MHz, DMSO-D₆) 1.03 (d, J=6.94Hz, 3H), 1.28 (s, 3H), 1.62 - 1.75 (m, 7H), 1.90 - 2.01 (m, 4H), 2.71 (d, J=16.35Hz, 1H), 2.85 (dd, J=16.30, 6.82Hz, 1H), 3.27 (s, 3H), 3.36 - 3.37 (m, 1H), 4.23 - 4.24 (m, 1H), 4.70 (s, 2H), 7.03 (s, 1H), 8.23 (s, 1H)
¹H-NMR (400 MHz, DMSO-D₆) 1.05 (d, J=6.73Hz, 3H), 1.29 (s, 3H), 1.67 - 2.03 (m, 11H), 2.89 - 3.05 (m, 2H), 3.27 (s, 3H), 3.38 - 3.44 (m, 1H), 4.32 - 4.35 (m, 1H), 4.71 (s, 2H), 7.04 (s, 1H), 7.78 (s, 1H)

### Step 9

### mixture of (1S,4r)-1-methyl-4-((S)-4-methyl-3-((R)-1,1,1-trifluoro-2-hydroxypropan-2-yl)-4,5-dihydro-7H-isoxazolo[5,4-e]indazol-7-yl)cyclohexan-1-ol and (1R,4r)-1-methyl-4-((R)-4-methyl-3-((S)-1,1,1-trifluoro-2-hydroxypropan-2-yl)-4,5-dihydro-7H-isoxazolo[5,4-e]indazol-7-yl)cyclohexan-1-ol

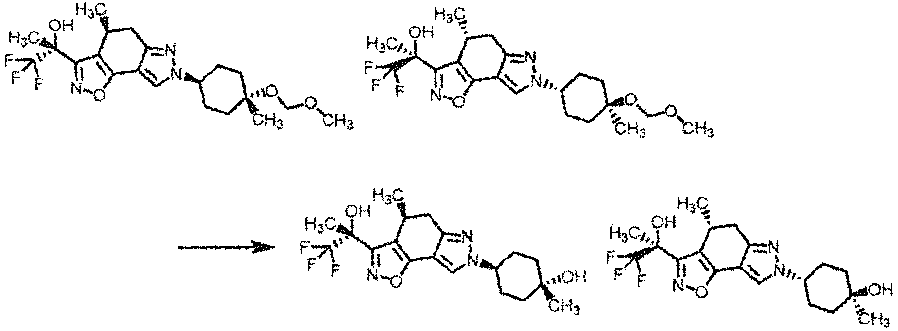

The mixture (0.009 g) of (R)-1,1,1-trifluoro-2-((S)-7-((1r,4S)-4-(methoxymethoxy)-4-methylcyclohexyl)-4-methyl-5,7-dihydro-4H-isoxazolo[5,4-e]indazol-3-yl)propan-2-ol and (S)-1,1,1-trifluoro-2-((R)-7-((1r,4R)-4-(methoxymethoxy)-4-methylcyclohexyl)-4-methyl-5,7-dihydro-4H-isoxazolo[5,4-e]indazol-3-yl)propan-2-ol obtained in the previous step was mixed with tetrahydrofuran (0.09 ml). At room temperature, to the reaction mixture was added 6N hydrochloric acid (0.033 ml), and the mixture was stirred for 1 hr. The reaction mixture was further stirred at 45°C for 30 min. Under ice-cooling, to the reaction mixture was added saturated aqueous sodium hydrogen carbonate solution, and the mixture was extracted with ethyl acetate. The organic layer was washed with saturated aqueous sodium chloride solution, and dried over sodium sulfate. Sodium sulfate was filtered off, and the filtrate was concentrated under reduced pressure to give the title compound (0.008 g) as a racemate.
¹H-NMR (400 MHz, DMSO-D₆) 1.05 (d, J=6.73Hz, 3H), 1.20 (s, 3H), 1.56 - 1.64 (m, 4H), 1.74 (s, 3H), 1.83 - 1.96 (m, 4H), 2.94 - 3.01 (m, 2H), 3.39 - 3.40 (m, 1H), 4.25 - 4.26 (m, 1H), 4.43 (s, 1H), 7.03 (s, 1H), 7.77 (s, 1H)

### Step 10

### mixture of (1S,4r)-1-methyl-4-((S)-4-methyl-3-((R)-1,1,1-trifluoro-2-hydroxypropan-2-yl)-4,5-dihydro-6H-isoxazolo[5,4-e]indazol-6-yl)cyclohexan-1-ol and (1R,4r)-1-methyl-4-((R)-4-methyl-3-((S)-1,1,1-trifluoro-2-hydroxypropan-2-yl)-4,5-dihydro-6H-isoxazolo[5,4-e]indazol-6-yl)cyclohexan-1-ol

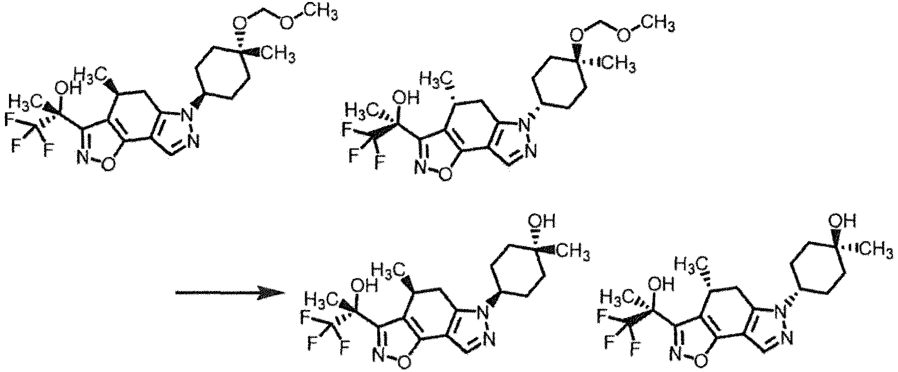

The mixture (0.004 g) of (R)-1,1,1-trifluoro-2-((S)-6-((1r,4S)-4-(methoxymethoxy)-4-methylcyclohexyl)-4-methyl-5,6-dihydro-4H-isoxazolo[5,4-e]indazol-3-yl)propan-2-ol and (S)-1,1,1-trifluoro-2-((R)-6-((1r,4R)-4-(methoxymethoxy)-4-methylcyclohexyl)-4-methyl-5,6-dihydro-4H-isoxazolo[5,4-e]indazol-3-yl)propan-2-ol obtained in the previous step was mixed with tetrahydrofuran (0.04 ml). At room temperature, to the reaction mixture was added 6N hydrochloric acid (0.015 ml), and the mixture was stirred for 1 hr. The reaction mixture was further stirred at 45°C for 30 min. Under ice-cooling, to the reaction mixture was added saturated aqueous sodium hydrogen carbonate solution, and the mixture was extracted with ethyl acetate. The organic layer was washed with saturated aqueous sodium chloride solution, and dried over sodium sulfate.
Sodium sulfate was filtered off, and the filtrate was concentrated under reduced pressure to give the title compound (0.036 g) as a racemate.
¹H-NMR (400 MHz, DMSO-D₆) 1.05 (d, J=6.73Hz, 3H), 1.20 (s, 3H), 1.56 - 1.64 (m, 4H), 1.74 (s, 3H), 1.83 - 1.96 (m, 4H), 2.94 - 3.01 (m, 2H), 3.39 - 3.40 (m, 1H), 4.25 - 4.26 (m, 1H), 4.43 (s, 1H), 7.03 (s, 1H), 7.77 (s, 1H)

### Auxiliary Step 2

### cis-4-hydroxy-4-methylcyclohexyl methanesulfonate

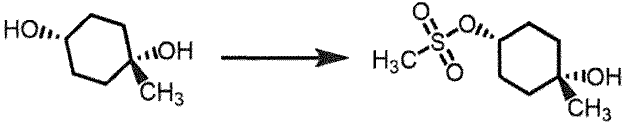

cis-1-Methylcyclohexane-1,4-diol (1.0 g) was mixed with dichloromethane (10 ml). Under ice-cooling, triethylamine (2.1 ml), methanesulfonic anhydride (2.0 g), and 4-dimethylaminopyridine (0.094 g) were added, and the mixture was stirred at room temperature for 2 hr. The reaction mixture was concentrated under reduced pressure. The obtained residue was purified by silica gel column chromatography (ethyl acetate:hexane=50:50 to ethyl acetate) to give the title compound (0.37 g).
¹H-NMR (400 MHz, DMSO-D₆) 1.09 (s, 3H), 1.34 - 1.44 (m, 2H), 1.56 - 1.59 (m, 2H), 1.70 - 1.84 (m, 4H), 3.14 (s, 3H), 4.21 (brs, 1H), 4.51 - 4.58 (m, 1H)

### Auxiliary Step 3

### cis-4-(methoxymethoxy)-4-methylcyclohexyl methanesulfonate

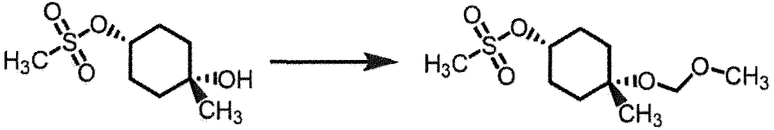

cis-4-Hydroxy-4-methylcyclohexyl methanesulfonate (1.1 g) obtained in the previous step was mixed with dichloromethane (20 ml). Under ice-cooling, chloromethyl methyl ether (0.74 ml) and N,N-diisopropylethylamine (2.1 ml) were added, and the mixture was stirred at room temperature overnight. To the reaction mixture was added water, and the mixture was extracted with ethyl acetate. The organic layer was washed successively with water and saturated aqueous sodium chloride solution, and filtered through a phase separator. The filtrate was concentrated under reduced pressure. The obtained residue was purified by silica gel column chromatography (ethyl acetate:hexane=10:90 to 55:45) to give the title compound (1.0 g).
¹H-NMR (400 MHz, DMSO-D₆) 1.13 (s, 3H), 1.38 - 1.45 (m, 2H), 1.71 - 1.85 (m, 6H), 3.14 (s, 3H), 3.27 (s, 3H), 4.55 - 4.60 (m, 1H), 4.64 (s, 2H)

Using (R)-1,1,1-trifluoro-2-((S)-4-methyl-5,6-dihydro-4H-isoxazolo[5,4-e]indazol-3-yl)propan-2-ol obtained in the below-mentioned Production Example 3-2, Step 3, the reactions of Production Example 3-1, Step 7 to Step 10, are performed, whereby (1S,4r)-1-methyl-4-((S)-4-methyl-3-((R)-1,1,1-trifluoro-2-hydroxypropan-2-yl)-4,5-dihydro-7H-isoxazolo[5,4-e]indazol-7-yl)cyclohexan-1-ol (Example 105 which is an optically active form of Example 55) and (1S,4r)-1-methyl-4-((S)-4-methyl-3-((R)-1,1,1-trifluoro-2-hydroxypropan-2-yl)-4,5-dihydro-6H-isoxazolo[5,4-e]indazol-6-yl)cyclohexan-1-ol (optically active form of Example 56) can be obtained.

### Production Example 3-2

### Synthesis of (R)-1,1,1-trifluoro-2-((S)-4-methyl-5,6-dihydro-4H-isoxazolo[5,4-e]indazol-3-yl)propan-2-ol (Example 28)

### Step 1

### (S)-1-(6-(4-methoxybenzyl)-4-methyl-5,6-dihydro-4H-isoxazolo[5,4-e]indazol-3-yl)ethan-1-one

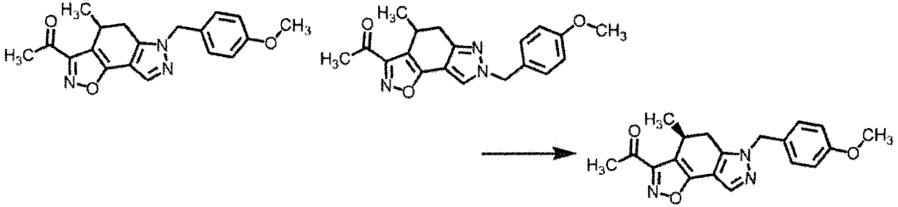

The mixture (0.85 g) of 1-(6-(4-methoxybenzyl)-4-methyl-5,6-dihydro-4H-isoxazolo[5,4-e]indazol-3-yl)ethan-1-one and 1-(7-(4-methoxybenzyl)-4-methyl-5,7-dihydro-4H-isoxazolo[5,4-e]indazol-3-yl)ethan-1-one was subjected to optical resolution using recycling preparative HPLC (apparatus name: Japan Analytical Industry Co., Ltd. LaboACE LC-7080, column: Daicel CHIRALPAK IG, 20 mm(I.D.) x 250 mm(L), 5 µm, mobile phase flow rate: 20 ml/min, mobile phase mixing ratio: isocratic, hexane:ethanol=40:60). The third peak fraction and the fourth peak fraction were recycled once and then fractionated to give the title compound (0.35 g) as the third peak fraction. ¹H-NMR (400 MHz, DMSO-D₆) 0.97 (d, J=7.48Hz, 3H), 2.58 (s, 3H), 2.96 - 3.02 (m, 2H), 3.47 - 3.53 (m, 1H), 3.72 (s, 3H), 5.33 (d, J=2.99Hz, 2H), 6.90 (d, J=8.23Hz, 2H), 7.15 (d, J=8.23Hz, 2H), 7.87 (s, 1H)

### Step 2

### (R)-1,1,1-trifluoro-2-((S)-6-(4-methoxybenzyl)-4-methyl-5,6-dihydro-4H-isoxazolo[5,4-e]indazol-3-yl)propan-2-ol

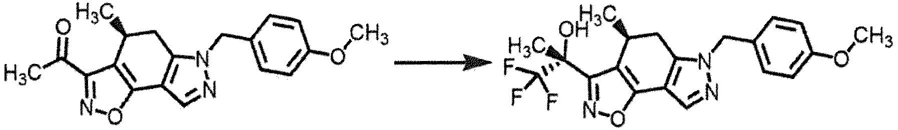

(S)-1-(6-(4-Methoxybenzyl)-4-methyl-5,6-dihydro-4H-isoxazolo[5,4-e]indazol-3-yl)ethan-1-one (0.025 g) obtained in the previous step was mixed with tetrahydrofuran (0.5 ml). Under ice-cooling, to the mixture was added cesium fluoride (0.002 g), and then (trifluoromethyl)trimethylsilane (0.016 ml) was added dropwise. The reaction mixture was stirred for 1 hr while raising the temperature to room temperature. At room temperature, methanol (0.5 ml) and potassium carbonate (0.02 g) were added, and the mixture was stirred at room temperature for 30 min. To the reaction mixture was added saturated ammonium chloride aqueous solution, and the mixture was extracted with ethyl acetate. The organic layer was washed with water and saturated aqueous sodium chloride solution, filtered through a phase separator, and the filtrate was concentrated under reduced pressure. The obtained residue was purified by preparative thin layer silica gel chromatography (hexane:ethyl acetate=3:2) to give a crude product (0.015 g) of the title compound.
¹H-NMR (400 MHz, DMSO-D₆) 1.00 (d, J=6.73Hz, 3H), 1.74 (s, 3H), 2.84 (dd, J=16.83, 7.48Hz, 1H), 3.02 (d, J=16.83Hz, 1H), 3.37 - 3.38 (m, 1H), 3.72 (s, 3H), 5.32 (d, J=2.99Hz, 2H), 6.89 (d, J=8.23Hz, 2H), 7.03 (s, 1H), 7.14 (d, J=8.23Hz, 2H), 7.81 (s, 1H)

### Step 3

### (R)-1,1,1-trifluoro-2-((S)-4-methyl-5,6-dihydro-4H-isoxazolo[5,4-e]indazol-3-yl)propan-2-ol

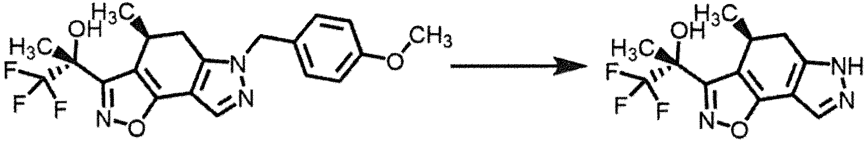

(R)-1,1,1-Trifluoro-2-((S)-6-(4-methoxybenzyl)-4-methyl-5,6-dihydro-4H-isoxazolo[5,4-e]indazol-3-yl)propan-2-ol (0.015 g) obtained in the previous step and anisole (0.008 ml) were mixed with trifluoroacetic acid (0.75 ml). The reaction mixture was stirred at 130°C for 30 min under microwave (Biotage (registered trademark) Initiator+) radiation. To the reaction mixture was added toluene, and the mixture concentrated under reduced pressure. The obtained residue was purified by silica gel column chromatography (ethyl acetate:hexane=30:70 to ethyl acetate) to give the title compound (0.009 g).
¹H-NMR (400 MHz, DMSO-D₆) 1.01 (d, J=6.94Hz, 3H), 1.73 (s, 3H), 2.62 - 2.92 (m, 2H), 3.32 - 3.41 (m, 1H), 7.02 (s, 1H), 8.13 (s, 1H), 12.98 (s, 1H)

### Production Example 3-3

### Synthesis of (1S,3r)-1-methyl-3-((S)-4-methyl-3-((R)-1,1,1-trifluoro-2-hydroxypropan-2-yl)-4,5-dihydro-6H-isoxazolo[5,4-e]indazol-6-yl)cyclobutane-1-carbonitrile and enantiomer thereof (Example 53), (1S,3r)-1-methyl-3-((S)-4-methyl-3-((R)-1,1,1-trifluoro-2-hydroxypropan-2-yl)-4,5-dihydro-7H-isoxazolo[5,4-e]indazol-7-yl)cyclobutane-1-carbonitrile and enantiomer thereof (Example 54), (1R,3s)-1-methyl-3-((S)-4-methyl-3-((R)-1,1,1-trifluoro-2-hydroxypropan-2-yl)-4,5-dihydro-6H-isoxazolo[5,4-e]indazol-6-yl)cyclobutane-1-carbonitrile and enantiomer thereof (Example 57), and (1R,3s)-1-methyl-3-((S)-4-methyl-3-((R)-1,1,1-trifluoro-2-hydroxypropan-2-yl)-4,5-dihydro-7H-isoxazolo[5,4-e]indazol-7-yl)cyclobutane-1-carbonitrile and enantiomer thereof (Example 58)

### Step 1

### mixture of 3-(3-acetyl-4-methyl-4,5-dihydro-7H-isoxazolo[5,4-e]indazol-7-yl)-1-methylcyclobutane-1-carbonitrile and 3-(3-acetyl-4-methyl-4,5-dihydro-6H-isoxazolo[5,4-e]indazol-6-yl)-1-methylcyclobutane-1-carbonitrile

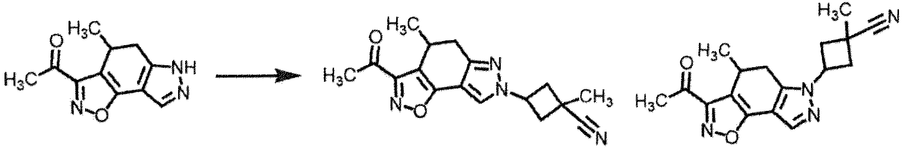

(1-(4-Methyl-5,6-dihydro-4H-isoxazolo[5,4-e]indazol-3-yl)ethan-1-one (0.1 g) obtained in the previous step was mixed with dimethylformamide (1.5 ml). To the reaction mixture were added cesium carbonate (0.45 g), sodium iodide (0.14 g), and 3-cyano-3-methylcyclobutyl 4-methylbenzenesulfonate (0.24 g) obtained in Auxiliary Step 5, and the mixture was stirred at 80°C for 2 hr. The reaction mixture was purified by reversed-phase silica gel chromatography (acetonitrile:water=0:100 to 100:0) to give a crude product (0.16 g) of the title compound.

### Step 2

### mixture of (1S,3r)-1-methyl-3-((S)-4-methyl-3-((R)-1,1,1-trifluoro-2-hydroxypropan-2-yl)-4,5-dihydro-6H-isoxazolo[5,4-e]indazol-6-yl)cyclobutane-1-carbonitrile and (1R,3r)-1-methyl-3-((R)-4-methyl-3-((S)-1,1,1-trifluoro-2-hydroxypropan-2-yl)-4,5-dihydro-6H-isoxazolo[5,4-e]indazol-6-yl)cyclobutane-1-carbonitrile, mixture of (1S,3r)-1-methyl-3-((S)-4-methyl-3-((R)-1,1,1-trifluoro-2-hydroxypropan-2-yl)-4,5-dihydro-7H-isoxazolo[5,4-e]indazol-7-yl)cyclobutane-1-carbonitrile and (1R,3r)-1-methyl-3-((R)-4-methyl-3-((S)-1,1,1-trifluoro-2-hydroxypropan-2-yl)-4,5-dihydro-7H-isoxazolo[5,4-e]indazol-7-yl)cyclobutane-1-carbonitrile, mixture of (1R,3s)-1-methyl-3-((S)-4-methyl-3-((R)-1,1,1-trifluoro-2-hydroxypropan-2-yl)-4,5-dihydro-6H-isoxazolo[5,4-e]indazol-6-yl)cyclobutane-1-carbonitrile and (1S,3s)-1-methyl-3-((R)-4-methyl-3-((S)-1,1,1-trifluoro-2-hydroxypropan-2-yl)-4,5-dihydro-6H-isoxazolo[5,4-e]indazol-6-yl)cyclobutane-1-carbonitrile, and mixture of (1R,3s)-1-methyl-3-((S)-4-methyl-3-((R)-1,1,1-trifluoro-2-hydroxypropan-2-yl)-4,5-dihydro-7H-isoxazolo[5,4-e]indazol-7-yl)cyclobutane-1-carbonitrile and (1S,3s)-1-methyl-3-((R)-4-methyl-3-((S)-1,1,1-trifluoro-2-hydroxypropan-2-yl)-4,5-dihydro-7H-isoxazolo[5,4-e]indazol-7-yl)cyclobutane-1-carbonitrile

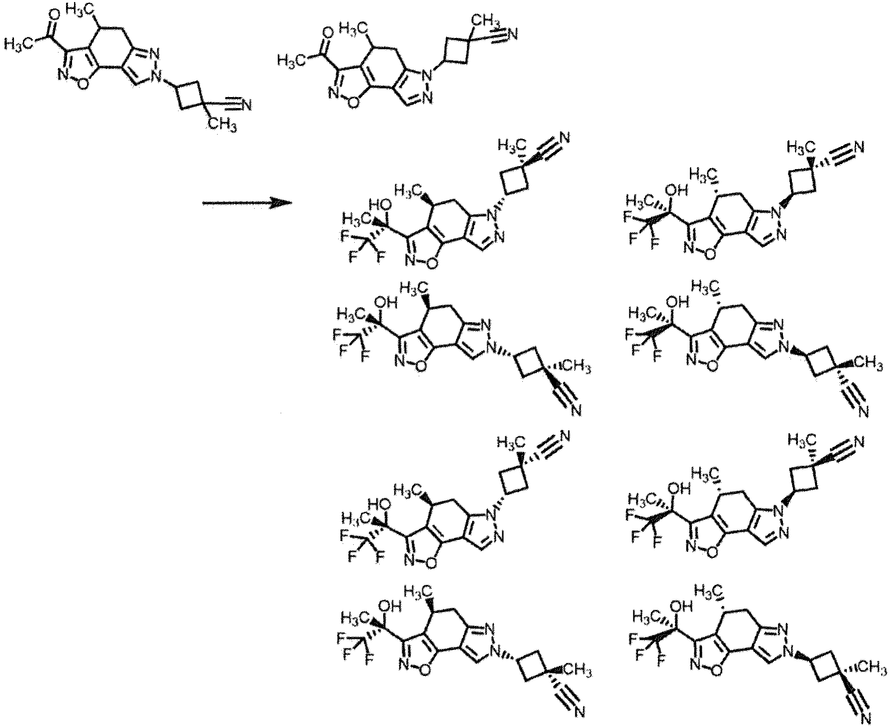

The mixture (0.16 g) of 3-(3-acetyl-4-methyl-4,5-dihydro-7H-isoxazolo[5,4-e]indazol-7-yl)-1-methylcyclobutane-1-carbonitrile and 3-(3-acetyl-4-methyl-4,5-dihydro-6H-isoxazolo[5,4-e]indazol-6-yl)-1-methylcyclobutane-1-carbonitrile obtained in the previous step was mixed with tetrahydrofuran (3.3 ml). Under ice-cooling, to the mixture was added cesium fluoride (0.016 g), and then (trifluoromethyl)trimethylsilane (0.15 ml) was added dropwise. The reaction mixture was stirred for 1 hr while raising the temperature to room temperature. At room temperature, methanol (3.3 ml) and potassium carbonate (0.44 g) were added, and the mixture was stirred at room temperature for 30 min. To the reaction mixture was added saturated ammonium chloride aqueous solution, and the mixture was extracted with ethyl acetate. The organic layer was washed with water and saturated aqueous sodium chloride solution, filtered through a phase separator, and the filtrate was concentrated under reduced pressure. The obtained residue was purified by preparative thin layer silica gel chromatography (hexane:ethyl acetate=2:1). The crude product was purified by preparative thin layer silica gel chromatography (dichloromethane:ethyl acetate=10:1, developed three times) to give the title compounds (0.007 g), (0.003 g), (0.003 g), and (0.009 g) in the order from lowest polarity, each as a racemate.
¹H-NMR (400 MHz, DMSO-D₆) 1.03 (d, J=6.94Hz, 3H), 1.56 (s, 3H), 1.73 (s, 3H), 2.59 (dd, J=11.97, 9.02Hz, 1H), 2.73 (dd, J=11.97, 8.67Hz, 1H), 2.91 - 3.03 (m, 4H), 3.37 - 3.39 (m, 1H), 5.13 - 5.15 (m, 1H), 7.03 (s, 1H), 7.88 (s, 1H)
¹H-NMR (400 MHz, DMSO-D₆) 1.03 (d, J=6.94Hz, 3H), 1.57 (s, 3H), 1.74 (s, 3H), 2.64 - 2.67 (m, 2H), 2.75 (d, J=15.95Hz, 1H), 2.87 (dd, J=15.95, 6.47Hz, 1H), 2.96 - 2.98 (m, 2H), 3.37 - 3.38 (m, 1H), 5.06 - 5.11 (m, 1H), 7.06 (s, 1H), 8.27 (s, 1H)
¹H-NMR (400 MHz, DMSO-D₆) 1.04 (d, J=6.73Hz, 3H), 1.62 (s, 3H), 1.74 (s, 3H), 2.58 - 2.64 (m, 2H), 2.88 - 3.14 (m, 4H), 3.39 - 3.41 (m, 1H), 5.20 - 5.22 (m, 1H), 7.05 (s, 1H), 7.92 (s, 1H)
¹H-NMR (400 MHz, DMSO-D₆) 1.04 (d, J=6.73Hz, 3H), 1.59 (s, 3H), 1.74 (s, 3H), 2.61 - 2.63 (m, 2H), 2.77 (d, J=15.71Hz, 1H), 2.88 (dd, J=15.71, 6.73Hz, 1H), 2.96 - 3.02 (m, 2H), 3.36 - 3.39 (m, 1H), 5.11 - 5.13 (m, 1H), 7.05 (d, J=3.74Hz, 1H), 8.24 (s, 1H)

### Auxiliary Step 4

### 3-hydroxy-1-methylcyclobutane-1-carbonitrile

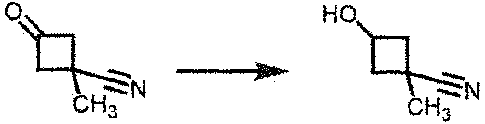

1-Methyl-3-oxocyclobutane-1-carbonitrile (0.76 g) was mixed with methanol (7.6 ml). To the reaction mixture was added sodium borohydride (0.079 g), and the mixture was stirred at room temperature for 2 hr. To the reaction mixture was added water, and the mixture was extracted with ethyl acetate. The organic layer was washed with water and saturated aqueous sodium chloride solution, filtered through a phase separator, and the filtrate was concentrated under reduced pressure to give a crude product (0.75 g) of the title compound.

### Auxiliary Step 5

### 3-cyano-3-methylcyclobutyl 4-methylbenzenesulfonate

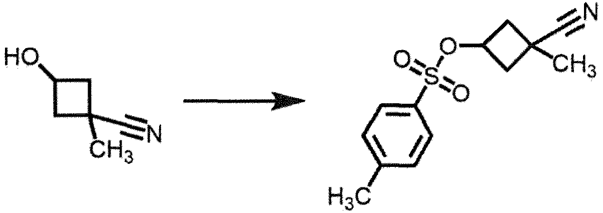

The crude product (0.34 g) of 3-hydroxy-1-methylcyclobutane-1-carbonitrile obtained in the previous step was mixed with dichloromethane (3.4 ml). To the reaction mixture were added p-toluenesulfonyl chloride (0.7 g), 4-dimethylaminopyridine (0.14 g), and triethylamine (0.46 g), and the mixture was stirred at room temperature overnight. To the reaction mixture was added saturated ammonium chloride aqueous solution, and the mixture was extracted with ethyl acetate. The organic layer was washed with water and saturated aqueous sodium chloride solution, and filtered through a phase separator. The filtrate was concentrated under reduced pressure. The obtained residue was purified by silica gel column chromatography (ethyl acetate:hexane=20:80 to 60:40) to give a crude product (0.47 g) of the title compound.

Using (R)-1,1,1-trifluoro-2-((S)-4-methyl-5,6-dihydro-4H-isoxazolo[5,4-e]indazol-3-yl)propan-2-ol obtained in Production Example 3-2, Step 3, the reactions of Production Example 3-3, Step 1 and Step 2, are performed, whereby (1S,3r)-1-methyl-3-((S)-4-methyl-3-((R)-1,1,1-trifluoro-2-hydroxypropan-2-yl)-4,5-dihydro-6H-isoxazolo[5,4-e]indazol-6-yl)cyclobutane-1-carbonitrile (optically active form of Example 53), (1S,3r)-1-methyl-3-((S)-4-methyl-3-((R)-1,1,1-trifluoro-2-hydroxypropan-2-yl)-4,5-dihydro-7H-isoxazolo[5,4-e]indazol-7-yl)cyclobutane-1-carbonitrile (optically active form of Example 54), (1R,3s)-1-methyl-3-((S)-4-methyl-3-((R)-1,1,1-trifluoro-2-hydroxypropan-2-yl)-4,5-dihydro-6H-isoxazolo[5,4-e]indazol-6-yl)cyclobutane-1-carbonitrile (optically active form of Example 57), and (1R,3s)-1-methyl-3-((S)-4-methyl-3-((R)-1,1,1-trifluoro-2-hydroxypropan-2-yl)-4,5-dihydro-7H-isoxazolo[5,4-e]indazol-7-yl)cyclobutane-1-carbonitrile (optically active form of Example 58) can be obtained.

### Production Example 4

### Synthesis of (S)-1,1,1-trifluoro-2-((S)-5-methyl-3-phenyl-4,5-dihydro-3H-imidazo[1,5-a]pyrazolo[4,3-c]pyridin-7-yl)propan-2-ol (Example 73)

### Step 1

### tert-butyl (S,E)-3-((dimethylamino)methylene)-6-methyl-2,4-dioxopiperidine-1-carboxylate

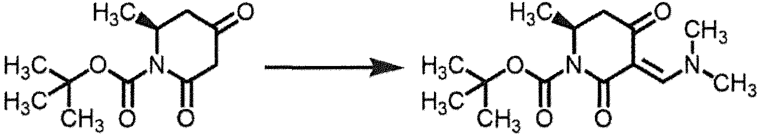

tert-Butyl (S)-2-methyl-4,6-dioxopiperidine-1-carboxylate (1.5 g) was mixed with toluene (7.5 ml). To the reaction mixture was added N,N-dimethylformamide dimethyl acetal (1.8 ml), and the mixture was stirred at room temperature for 1 hr. The reaction mixture was concentrated under reduced pressure to give the title compound (1.5 g).
¹H-NMR (400 MHz, DMSO-D₆) 1.20 (d, J=6.35Hz, 3H), 1.45 (s, 9H), 2.14 (dd, J=16.41, 2.08Hz, 1H), 2.78 (dd, J=16.41, 6.35Hz, 1H), 3.04 (s, 3H), 3.38 (s, 3H), 4.31 - 4.35 (m, 1H), 7.99 (s, 1H)

### Step 2

### (S)-6-methyl-1-phenyl-1,5,6,7-tetrahydro-4H-pyrazolo[4,3-c]pyridin-4-one

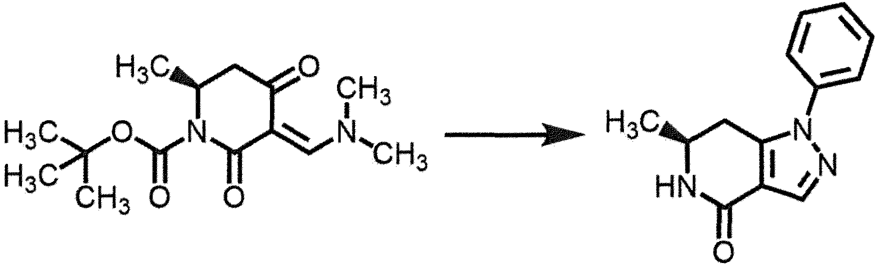

tert-Butyl (S,E)-3-((dimethylamino)methylene)-6-methyl-2,4-dioxopiperidine-1-carboxylate (1.0 g) obtained in the previous step was dissolved in isopropanol (5.0 ml), acetic acid (0.53 ml), and water (7.0 ml). To the reaction mixture was added phenylhydrazine (0.38 g), and the mixture was heated under reflux for 2 hr. To the reaction mixture was added toluene (5.0 ml), and the mixture was further heated under reflux for 2 hr. Water was added to the reaction mixture, and the mixture was extracted with ethyl acetate. The organic layer was washed with saturated aqueous sodium chloride solution, and filtered through a phase separator. The filtrate was concentrated under reduced pressure. The obtained residue was purified by silica gel column chromatography (ethyl acetate:hexane=40:60 to ethyl acetate, thereafter ethyl acetate:methanol=85:15) to give the title compound (0.54 g). ¹H-NMR (400 MHz, DMSO-D₆) 1.22 (d, J=5.78Hz, 3H), 2.88 (dd, J=16.24, 10.52Hz, 1H), 3.06 (dd, J=16.24, 5.78Hz, 1H), 3.74 - 3.77 (m, 1H), 7.39 (s, 1H), 7.45 (tt, J=7.05, 1.73Hz, 1H), 7.54 - 7.61 (m, 4H), 7.94 (s, 1H)

### Step 3

### (S)-6-methyl-1-phenyl-4,5,6,7-tetrahydro-1H-pyrazolo[4,3-c]pyridine

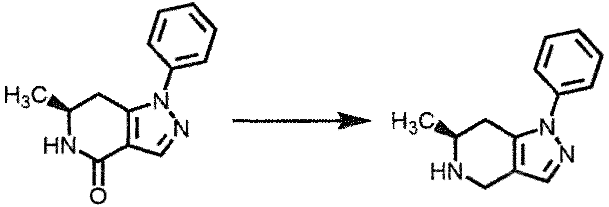

Lithium aluminum hydride (0.27 g) was dissolved in tetrahydrofuran (10 ml). To the reaction mixture was added (S)-6-methyl-1-phenyl-1,5,6,7-tetrahydro-4H-pyrazolo[4,3-c]pyridin-4-one (0.53 g) obtained in the previous step, and the mixture was heated under reflux at 90°C for 1 hr. To the reaction mixture was added lithium aluminum hydride (0.27 g), and the mixture was further heated under reflux at 90°C for 1 hr. At room temperature, to the reaction mixture were successively added water (0.53 ml), 4 M sodium hydroxide aqueous solution (0.53 ml), and water (1.5 ml). Insoluble material was filtered off through celite, and the filtrate was concentrated under reduced pressure. The obtained residue was purified by SCX column chromatography (methanol to 1N ammonia /methanol solution) to give the title compound (0.47 g). ¹H-NMR (400 MHz, CDCl₃) 1.29 (d, J=6.24Hz, 3H), 2.52 (dd, J=16.07, 9.83Hz, 1H), 2.74 - 2.78 (m, 1H), 2.94 - 2.99 (m, 1H), 3.96 - 4.01 (m, 2H), 7.31 - 7.34 (m, 1H), 7.43 - 7.51 (m, 5H)

### Step 4

### (S)-5-chloro-6-methyl-1-phenyl-4,5,6,7-tetrahydro-1H-pyrazolo[4,3-c]pyridine

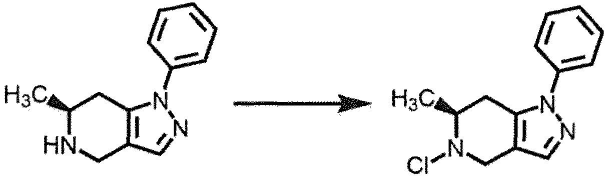

(S)-6-Methyl-1-phenyl-4,5,6,7-tetrahydro-1H-pyrazolo[4,3-c]pyridine (0.28 g) obtained in the previous step was dissolved in dichloromethane (2.7 ml). At room temperature, to the reaction mixture was added N-chlorosuccinimide (5.68 g), and the mixture was stirred for 30 min. At room temperature, to the reaction mixture was added 1,8-diazabicyclo[5.4.0]-7-undecene (0.21 ml), and the mixture was further stirred for 30 min. To the reaction mixture was added water at room temperature, and the mixture was filtered through silica gel (10 g). The filtrate was concentrated under reduced pressure to give a crude product (0.12 g) of the title compound.

### Step 5

### (S)-5-methyl-3-phenyl-4,5-dihydro-3H-imidazo[1,5-a]pyrazolo[4,3-c]pyridine

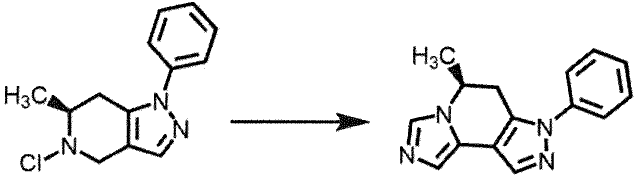

The crude product (0.12 g) of (S)-5-chloro-6-methyl-1-phenyl-4,5,6,7-tetrahydro-1H-pyrazolo[4,3-c]pyridine obtained in the previous step was dissolved in 1,2-dimethoxyethane (3.6 ml) and methanol (0.9 ml). At room temperature, potassium hydroxide (0.03 g) was added, and the mixture was stirred for 30 min. At room temperature, 2 M sodium hydroxide aqueous solution (0.097 ml) was added and the mixture was further stirred for 30 min. After confirming the disappearance of the starting material, to the mixture were added potassium carbonate (0.4 g) and p-toluenesulfonylmethyl isocyanide (0.42 g), and the mixture was stirred at room temperature for 2 days. The reaction mixture was purified by reversed-phase silica gel chromatography (acetonitrile:water=0:100 to 100:0) to give the title compound (0.036 g).
¹H-NMR (400 MHz, CDCl₃) 1.59 (d, J=5.90Hz, 3H), 2.98 (dd, J=15.95, 8.55Hz, 1H), 3.28 (dd, J=15.95, 5.90Hz, 1H), 4.37 - 4.40 (m, 1H), 7.17 (s, 1H), 7.38 - 7.43 (m, 1H), 7.48 - 7.54 (m, 4H), 7.59 (s, 1H), 7.85 (s, 1H)

### Step 6

### (S)-1-(5-methyl-3-phenyl-4,5-dihydro-3H-imidazo[1,5-a]pyrazolo[4,3-c]pyridin-7-yl)ethan-1-one

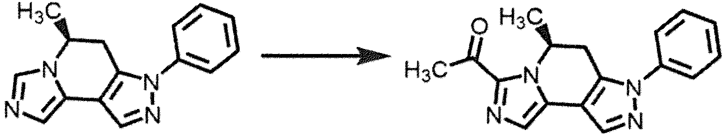

(S)-5-Methyl-3-phenyl-4,5-dihydro-3H-imidazo[1,5-a]pyrazolo[4,3-c]pyridine (0.034 g) obtained in the previous step was dissolved in tetrahydrofuran (1.0 ml). 2 M Lithium diisopropylamide/tetrahydrofuran-heptane-ethylbenzene solution (0.065 ml) was added dropwise to the reaction mixture at -78°C, and the mixture was stirred for 30 min. To the mixture was added N-methoxy-N-methylacetamide (0.033 ml), and the mixture was stirred at -78°C for 1 hr. To the mixture was added dropwise 1 M lithium diisopropylamide/tetrahydrofuran-heptane-ethylbenzene solution (0.068 ml), and the mixture was stirred at -78°C for 1 hr. At room temperature, to the reaction mixture was added saturated ammonium chloride aqueous solution, and the mixture was extracted with ethyl acetate. The organic layer was washed successively with water and saturated aqueous sodium chloride solution, and filtered through a phase separator. The filtrate was concentrated under reduced pressure. The obtained residue was purified by silica gel column chromatography (hexane to ethyl acetate:hexane=33:67) to give a crude product (0.018 g) of the title compound.

### Step 7

### (S)-1,1,1-trifluoro-2-((S)-5-methyl-3-phenyl-4,5-dihydro-3H-imidazo[1,5-a]pyrazolo[4,3-c]pyridin-7-yl)propan-2-ol

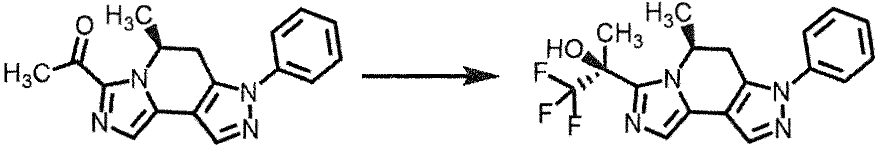

The crude product (0.018 g) of (S)-1-(5-methyl-3-phenyl-4,5-dihydro-3H-imidazo[1,5-a]pyrazolo[4,3-c]pyridin-7-yl)ethan-1-one obtained in the previous step was mixed with tetrahydrofuran (1 ml). Under ice-cooling, to the mixture was added cesium fluoride (0.005 g), and then (trifluoromethyl)trimethylsilane (0.018 ml) was added dropwise. The reaction mixture was stirred for 1 hr while raising the temperature to room temperature. At room temperature, methanol (0.5 ml) and potassium carbonate (0.026 g) were added, and the mixture was stirred at room temperature for 1 hr. To the reaction mixture was added saturated ammonium chloride aqueous solution, and the mixture was extracted with ethyl acetate. The organic layer was washed with water and saturated aqueous sodium chloride solution, filtered through a phase separator, and the filtrate was concentrated under reduced pressure. The obtained residue was purified by reversed-phase silica gel chromatography (acetonitrile:water=5:95 to 100:0) to give the title compound (0.017 g).
¹H-NMR (400 MHz, DMSO-D₆) 1.09 (d, J=6.24Hz, 3H), 1.81 (s, 3H), 2.99 (d, J=16.93Hz, 1H), 3.46 (dd, J=16.93, 7.28Hz, 1H), 5.33 - 5.35 (m, 1H), 7.05 (s, 1H), 7.08 (s, 1H), 7.42 - 7.43 (m, 1H), 7.53 - 7.59 (m, 4H), 7.97 (s, 1H)

### Production Example 5

### Synthesis of (R)-2-((S)-9-(6,6-difluoro-2-azaspiro[3.3]heptan-2-yl)-5-methyl-5,6-dihydroimidazo[1,5-a]pyrazolo[5,1-c]pyrazin-3-yl)-1,1,1-trifluoropropan-2-ol (Example 84)

### Step 1

### (S)-2-(5-(3-bromo-1H-pyrazol-5-yl)-1H-imidazol-1-yl)propan-1-ol

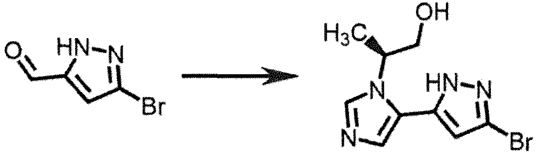

3-Bromo-1H-pyrazole-5-carbaldehyde (2.41 g) and (S)-2-aminopropan-1-ol (1.034 g) were mixed with methanol (24.1 ml), and the mixture was stirred at room temperature overnight. To the mixture was added 1,2-dimethoxyethane (48.2 ml). Under ice-cooling, p-toluenesulfonylmethyl isocyanide (4.03 g) and potassium carbonate (5.71 g) were added, and the mixture was stirred at room temperature overnight. Water was added to the reaction mixture, and the mixture was extracted twice with ethyl acetate. The organic layer was washed twice with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=25:75 to ethyl acetate, thereafter ethyl acetate:methanol=50:50), and azeotroped twice with THF to give a crude product (3.12 g) of the title compound.

### Step 2

### (S)-9-bromo-5-methyl-5,6-dihydroimidazo[1,5-a]pyrazolo[5,1-c]pyrazine

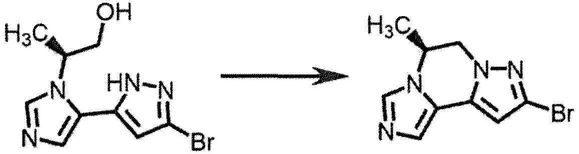

The crude product (2.91 g) of (S)-2-(5-(3-bromo-1H-pyrazol-5-yl)-1H-imidazol-1-yl)propan-1-ol obtained in the previous step was mixed with tetrahydrofuran (58.2 ml). Under ice-cooling, triphenylphosphine (3.38 g) and diisopropyl azodicarboxylate (2.60 g) were added, and the mixture was stirred at room temperature for 1 hr. The reaction mixture was concentrated under reduced pressure, and purified by silica gel column chromatography (ethyl acetate:hexane=25:75 to ethyl acetate, thereafter ethyl acetate:methanol=50:50) to give the title compound (2.23 g).
¹H-NMR (400 MHz, DMSO-D₆) 1.48 (d, J=6.47Hz, 3H), 4.14 (dd, J=13.29, 7.74Hz, 1H), 4.48 (dd, J=13.29, 4.51Hz, 1H), 4.67 - 4.78 (m, 1H), 6.67 (s, 1H), 7.31 (s, 1H), 7.93 (s, 1H)

### Step 3

### (S)-1- (9-bromo-5-methyl-5,6-dihydroimidazo[1,5-a]pyrazolo[5,1-c]pyrazin-3-yl)ethan-1-one

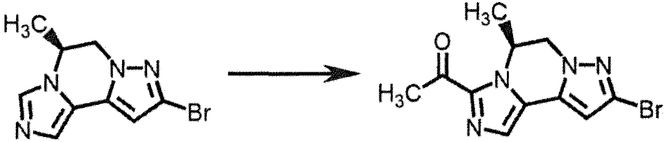

(S)-9-Bromo-5-methyl-5,6-dihydroimidazo[1,5-a]pyrazolo[5,1-c]pyrazine (1.5 g) obtained in the previous step was mixed with tetrahydrofuran (15 ml). The mixture was cooled to -78°C, and 2 M lithium diisopropylamide/tetrahydrofuran-heptane-ethylbenzene solution (2.96 ml) was added dropwise. The mixture was stirred at -78°C for 1 hr. To the mixture was added N-methoxy-N-methylacetamide (1.452 ml), and the mixture was stirred at -78°C for 1 hr. To the mixture was added dropwise 2 M lithium diisopropylamide/tetrahydrofuran-heptane-ethylbenzene solution (2.96 ml), and the mixture was stirred at -78°C for 1 hr. To the reaction mixture was added saturated ammonium chloride aqueous solution, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. To the obtained residue was added ethanol/water=1/2 (4.5 ml), and the mixture was stirred at room temperature. The precipitated solid was collected by filtration to give a crude product (1.373 g) of the title compound.

### Step 4

### (R)-2-((S)-9-bromo-5-methyl-5,6-dihydroimidazo[1,5-a]pyrazolo[5,1-c]pyrazin-3-yl)-1,1,1-trifluoropropan-2-ol

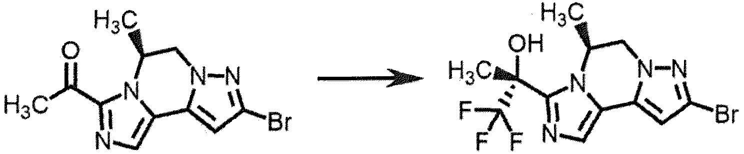

(S)-1-(9-Bromo-5-methyl-5,6-dihydroimidazo[1,5-a]pyrazolo[5,1-c]pyrazin-3-yl)ethan-1-one crude product (1.373 g) obtained in the previous step was mixed with N,N-dimethylacetamide (6.42 ml). Under ice-cooling, to the mixture was added lithium acetate (0.086 g), and then trifluoromethyltrimethylsilane (0.966 ml) was added dropwise. The mixture was stirred under ice-cooling for 25 min. Under ice-cooling, methanol (0.642 ml) and potassium carbonate (0.721 g) were added, and the mixture was stirred at room temperature for 1 hr. To the reaction mixture was added water, and the mixture was extracted twice with ethyl acetate. The organic layer was washed successively with 1N aqueous hydrochloric acid solution, water, and saturated aqueous sodium chloride solution. The aqueous layers were combined and extracted again with ethyl acetate, and the organic layer was washed with 1N potassium hydrogen sulfate aqueous solution. The 1N potassium hydrogen sulfate aqueous solution layer was extracted again with ethyl acetate. All organic layers were combined, washed with saturated aqueous sodium hydrogen carbonate solution and saturated aqueous sodium chloride solution, and dried over sodium sulfate. Sodium sulfate was filtered off, and the filtrate was concentrated under reduced pressure to give a crude product (1.669 g) containing the title compound. The crude product (1.421 g) was mixed with acetonitrile (11.37 ml). To the mixture was added phosphoric acid (0.673 g), and the mixture was stirred at room temperature for 1 hr. Under ice-cooling, the mixture was stirred for 1 hr, and the precipitated solid was collected by filtration to give a phosphate (1.533 g) of the title compound. Using the crude product (0.1 g) containing the title compound and by a similar method, a phosphate (0.1057 g) of the title compound was obtained. The phosphate (1.6387 g) of the title compound was mixed with ethyl acetate (16.22 ml). To the mixture was added saturated aqueous sodium hydrogen carbonate solution (16.22 ml), and the mixture was stirred at room temperature for 1 hr. After layer separation, the aqueous layer was extracted with ethyl acetate. The organic layers were combined and washed successively with water and saturated aqueous sodium chloride solution, and dried over sodium sulfate. Sodium sulfate was filtered off, and the filtrate was concentrated under reduced pressure to give the title compound (1.264 g).
¹H-NMR (400 MHz, DMSO-D₆) 1.24 (d, J=6.47Hz, 3H), 1.81 (s, 3H), 4.30 (dd, J=13.52, 4.05Hz, 1H), 4.39 (d, J=13.52Hz, 1H), 5.28 - 5.41 (m, 1H), 6.71 (s, 1H), 7.31 (s, 2H)

### Step 5

### (R)-2-((S)-9-(6,6-difluoro-2-azaspiro[3.3]heptan-2-yl)-5-methyl-5,6-dihydroimidazo[1,5-a]pyrazolo[5,1-c]pyrazin-3-yl)-1,1,1-trifluoropropan-2-ol

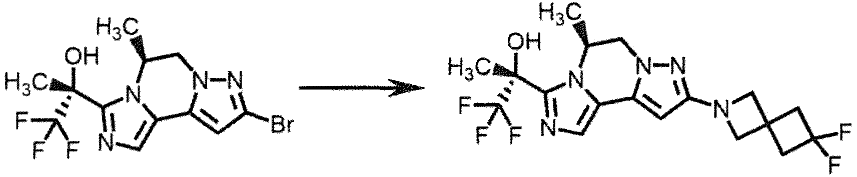

(R)-2-((S)-9-Bromo-5-methyl-5,6-dihydroimidazo[1,5-a]pyrazolo[5,1-c]pyrazin-3-yl)-1,1,1-trifluoropropan-2-ol (200 mg) obtained in the previous step, 6,6-difluoro-2-azaspiro[3.3]heptane 2,2,2-trifluoroacetate (382 mg), sodium tert-butoxide (346 mg), and methanesulfonato(2-dicyclohexylphosphino-2',4',6'-triisopropyl-1,1'-biphenyl)(2'-methylamino-1,1'-biphenyl-2-yl)palladium(II) (89 mg) were mixed with toluene (1.88 ml). Under an argon atmosphere, the reaction mixture was stirred at 120°C for 1.5 hr. At room temperature, water was added to the reaction mixture, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. The obtained residue was successively purified by silica gel column chromatography (ethyl acetate:hexane=12:88 to ethyl acetate) and reversed-phase silica gel chromatography (acetonitrile:water=5:95 to acetonitrile) to give the title compound (154.8 mg).
¹H-NMR (400 MHz, DMSO-D₆) 1.26 (d, J=6.47Hz, 3H), 1.81 (s, 3H), 2.83 (t, J=12.60Hz, 4H), 3.87 (s, 4H), 4.10 - 4.16 (m, 2H), 5.28 - 5.30 (m, 1H), 5.80 (s, 1H), 7.20 (s, 1H), 7.26 (s, 1H)

### Production Example 6

### Synthesis of 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)bicyclo[2.2.1]heptane-1-carbonitrile (Example 86)

### Step 1

### methyl 4-(hydroxymethyl)bicyclo[2.2.1]heptane-1-carboxylate

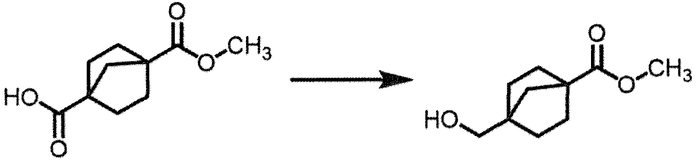

4-(Methoxycarbonyl)bicyclo[2.2.1]heptane-1-carboxylic acid (5 g) was mixed with THF (50 ml). Under ice-cooling, to the mixture was added dropwise 0.89 M borane-THF complex/THF solution (31.2 ml), and the mixture was stirred for 10 min. The reaction mixture was warmed to room temperature and stirred for 2 hr. Under ice-cooling, to the reaction mixture was added dropwise saturated aqueous sodium hydrogen carbonate solution, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=12:88 to ethyl acetate) to give the title compound (4.57 g).
¹H-NMR (400 MHz, CDCl₃) 1.29 - 1.43 (m, 3H), 1.55 (s, 2H), 1.60 - 1.74 (m, 4H), 1.93 - 2.06 (m, 2H), 3.65 - 3.72 (m, 2H), 3.66 (s, 3H)

### Step 2

### methyl 4-(((tetrahydro-2H-pyran-2-yl)oxy)methyl)bicyclo[2.2.1]heptane-1-carboxylate

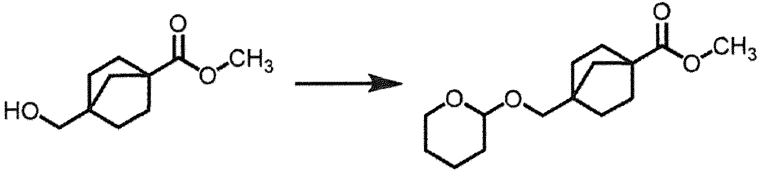

Methyl 4-(hydroxymethyl)bicyclo[2.2.1]heptane-1-carboxylate (4.57 g) obtained in the previous step was mixed with acetonitrile (42.3 ml). At room temperature, pyridinium p-toluenesulfonate (1.153 g) and 3,4-dihydro-2H-pyran (2.316 g) were added, and the mixture was stirred at room temperature overnight. The reaction mixture was concentrated under reduced pressure. The obtained residue was purified by silica gel column chromatography (ethyl acetate:hexane=2:98 to 18:82) to give the title compound (6.254 g).
¹H-NMR (400 MHz, CDCl₃) 1.31 - 1.43 (m, 2H), 1.43 - 1.89 (m, 12H), 1.91 - 2.05 (m, 2H), 3.36 (d, J=9.94Hz, 1H), 3.44 - 3.53 (m, 1H), 3.66 (s, 3H), 3.75 - 3.87 (m, 2H), 4.53 - 4.60 (m, 1H)

### Step 3

### 4-(((tetrahydro-2H-pyran-2-yl)oxy)methyl)bicyclo[2.2.1]heptane-1-carboxylic acid

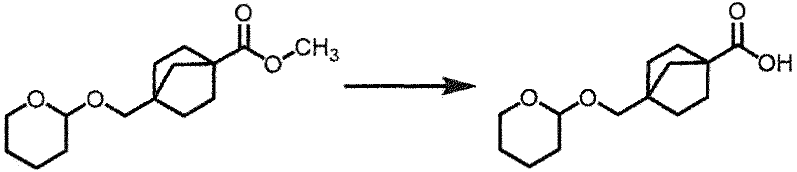

Methyl 4-(((tetrahydro-2H-pyran-2-yl)oxy)methyl)bicyclo[2.2.1]heptane-1-carboxylate (6.254 g) obtained in the previous step was mixed with ethanol (30 ml). To the reaction mixture was added 4N sodium hydroxide aqueous solution (13.98 ml), and the mixture was stirred at 80°C for 2 hr and allowed to stand at room temperature for 3 days. The reaction mixture was stirred at 80°C for 6 hr. After cooling to room temperature, 2N aqueous hydrochloric acid solution (28 ml) was added, and the mixture was concentrated under reduced pressure. The obtained residue was extracted twice with ethyl acetate, washed with saturated aqueous sodium chloride solution, and dried over sodium sulfate. Sodium sulfate was filtered off, and the filtrate was concentrated under reduced pressure to give a crude product (5.69 g) of the title compound.

### Step 4

### N-methoxy-N-methyl-4-(((tetrahydro-2H-pyran-2-yl)oxy)methyl)bicyclo[2.2.1]heptane-1-carboxamide

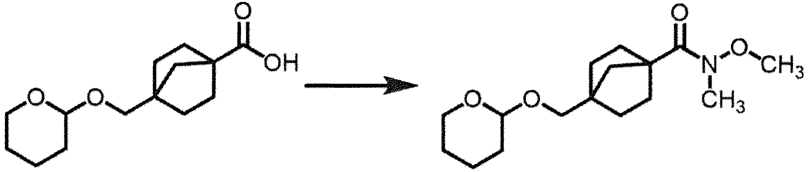

The crude product (5.69 g) of 4-(((tetrahydro-2H-pyran-2-yl)oxy)methyl)bicyclo[2.2.1]heptane-1-carboxylic acid obtained in the previous step and N,O-dimethylhydroxylamine hydrochloride (2.62 g) were mixed with pyridine (28.4 ml). To the mixture was added 1-ethyl-3-(3-dimethylaminopropyl)carbodiimide hydrochloride (5.15 g), and the mixture was stirred at room temperature overnight. To the reaction mixture were added N,O-dimethylhydroxylamine hydrochloride (2.62 g) and 1-ethyl-3-(3-dimethylaminopropyl)carbodiimide hydrochloride (5.15 g), and the mixture was stirred at room temperature for 2 hr. To the reaction mixture was added 1N aqueous hydrochloric acid solution, and the mixture was extracted with ethyl acetate. The organic layer was washed successively with 1N aqueous hydrochloric acid solution, water, and saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=5:95 to 50:50) to give the title compound (6.18 g).
¹H-NMR (400 MHz, CDCl₃) 1.34 - 1.62 (m, 6H), 1.62 - 1.74 (m, 5H), 1.75 - 1.92 (m, 5H), 3.17 (s, 3H), 3.37 (d, J=9.94Hz, 1H), 3.43 - 3.52 (m, 1H), 3.65 (s, 3H), 3.79 (d, J=9.94Hz, 1H), 3.79 - 3.88 (m, 1H), 4.55 - 4.59 (m, 1H)

### Step 5

### 1-(4-(((tetrahydro-2H-pyran-2-yl)oxy)methyl)bicyclo[2.2.1]heptan-1-yl)ethan-1-one

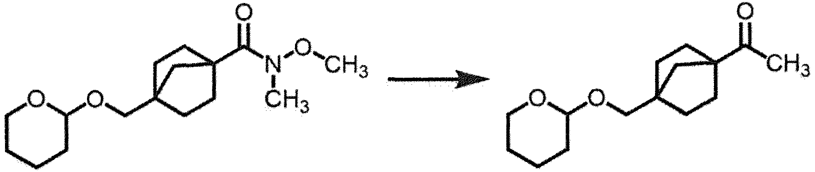

Under an argon atmosphere, N-methoxy-N-methyl-4-(((tetrahydro-2H-pyran-2-yl)oxy)methyl)bicyclo[2.2.1]heptane-1-carboxamide (6.18 g) obtained in the previous step was mixed with toluene (57.8 ml). Under ice-cooling, 1.04 M methylmagnesium bromide/tetrahydrofuran solution (22.42 ml) was added dropwise, and the mixture was stirred for 30 min. At room temperature, saturated ammonium chloride aqueous solution and water were added, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=8:92 to 66:34) to give the title compound (4.59 g).
¹H-NMR (400 MHz, CDCl₃) 1.33 - 1.97 (m, 16H), 2.15 (s, 3H), 3.37 (d, J=9.71Hz, 1H), 3.43 - 3.55 (m, 1H), 3.76 - 3.88 (m, 2H), 4.52 - 4.60 (m, 1H)

### Step 6

### ethyl 2,4-dioxo-4-(4-(((tetrahydro-2H-pyran-2-yl)oxy)methyl)bicyclo[2.2.1]heptan-1-yl)butanoate

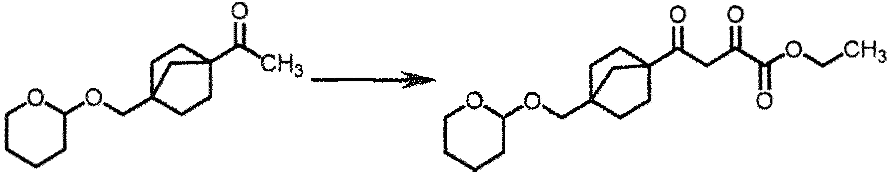

1-(4-(((Tetrahydro-2H-pyran-2-yl)oxy)methyl)bicyclo[2.2.1]heptan-1-yl)ethan-1-one (4.59 g) obtained in the previous step was mixed with tetrahydrofuran (45.9 ml). Under ice-cooling, to the mixture was added diethyl oxalate (489 mg), 1 M potassium tert-butoxide/tetrahydrofuran solution (21.83 ml) was added dropwise, and the mixture was stirred for 30 min. To the reaction mixture were added 1 M aqueous hydrochloric acid solution (21.3 ml), and the mixture was extracted with ethyl acetate. The organic layer was washed with saturated aqueous sodium chloride solution, and dried over sodium sulfate. Sodium sulfate was filtered off, and the filtrate was concentrated under reduced pressure, and azeotroped with ethanol to give a crude product (6.41 g).

### Step 7

### ethyl 3-(4-(((tetrahydro-2H-pyran-2-yl)oxy)methyl)bicyclo[2.2.1]heptan-1-yl)-1H-pyrazole-5-carboxylate

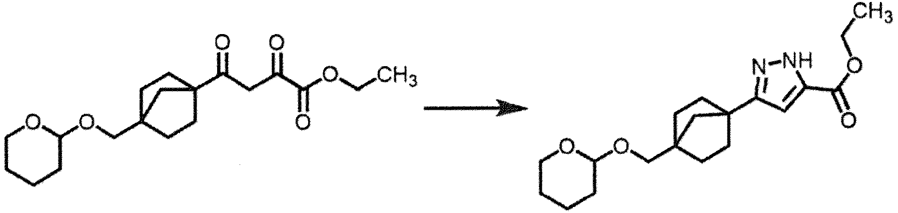

The crude product (6.41 g) of ethyl 2,4-dioxo-4-(4-(((tetrahydro-2H-pyran-2-yl)oxy)methyl)bicyclo[2.2.1]heptan-1-yl)butanoate obtained in the previous step was mixed with ethanol (64.1 ml). Under ice-cooling, hydrazine monohydrate (0.926 ml) was added dropwise, and the mixture was stirred at room temperature overnight. The reaction mixture was concentrated under reduced pressure. The obtained residue was purified by silica gel column chromatography (ethyl acetate:hexane=8:92 to 63:37) to give the title compound (7.148 g).
¹H-NMR (400 MHz, CDCl₃) 1.37 (t, J=7.17Hz, 3H), 1.41 - 1.99 (m, 16H), 3.41 (d, J=9.71Hz, 1H), 3.44 - 3.55 (m, 1H), 3.79 - 3.88 (m, 2H), 4.37 (q, J=7.17Hz, 2H), 4.56 - 4.61 (m, 1H), 6.64 (s, 1H), 10.26 (brs, 1H)

### Step 8

### (3-(4-(((tetrahydro-2H-pyran-2-yl)oxy)methyl)bicyclo[2.2.1]heptan-1-yl)-1H-pyrazol-5-yl)methanol

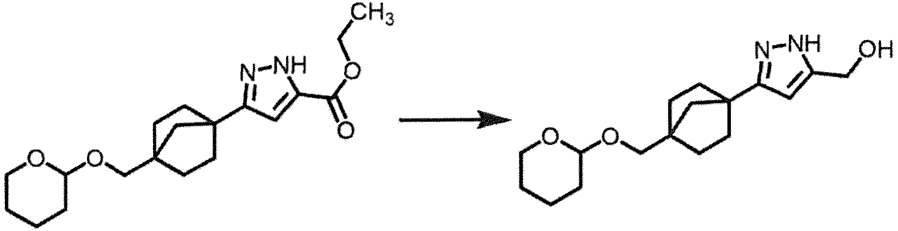

Ethyl 3-(4-(((tetrahydro-2H-pyran-2-yl)oxy)methyl)bicyclo[2.2.1]heptan-1-yl)-1H-pyrazole-5-carboxylate (7.148 g) obtained in the previous step was mixed with tetrahydrofuran (100 ml). Under ice-cooling, to the mixture was added dropwise 2 M lithium aluminum hydride/tetrahydrofuran solution (16.94 ml), and the mixture was stirred for 50 min. Under ice-cooling, to the reaction mixture was added saturated Rochelle salt aqueous solution, and the mixture was stirred at room temperature for 1 hr and extracted with ethyl acetate. The organic layer was washed with saturated aqueous sodium chloride solution, and dried over sodium sulfate. Sodium sulfate was filtered off, and the filtrate was concentrated under reduced pressure. To the obtained residue was added ethyl acetate/hexane=1/2, and the mixture was stirred at room temperature. The precipitated solid was collected by filtration to give the title compound (4.94 g).
¹H-NMR (400 MHz, DMSO-D₆) 1.30 - 1.91 (m, 16H), 3.33 - 3.45 (m, 2H), 3.65 - 3.78 (m, 2H), 4.28 - 4.41 (m, 2H), 4.52 - 4.57 (m, 1H), 4.88 (s, 1H), 5.94 (brs, 1H), 12.17 (brs, 1H)

### Step 9

### 3-(4-(((tetrahydro-2H-pyran-2-yl)oxy)methyl)bicyclo[2.2.1]heptan-1-yl)-1H-pyrazole-5-carbaldehyde

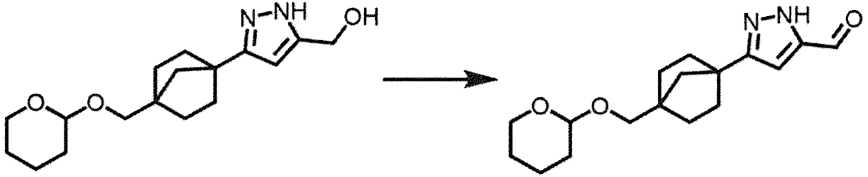

(3-(4-(((Tetrahydro-2H-pyran-2-yl)oxy)methyl)bicyclo[2.2.1]heptan-1-yl)-1H-pyrazol-5-yl)methanol (2.5 g) obtained in the previous step was mixed with 1,2-dimethoxyethane (100 ml). Manganese dioxide (7.5 g) was added, and the mixture was stirred at room temperature overnight. The reaction mixture was filtered through celite, and the filtrate was concentrated under reduced pressure to give a crude product (2.4 g) of the title compound.

### Step 10

### (2S)-2-(5-(5-(4-(((tetrahydro-2H-pyran-2-yl)oxy)methyl)bicyclo[2.2.1]heptan-1-yl)-1H-pyrazol-3-yl)-1H-imidazol-1-yl)propan-1-ol

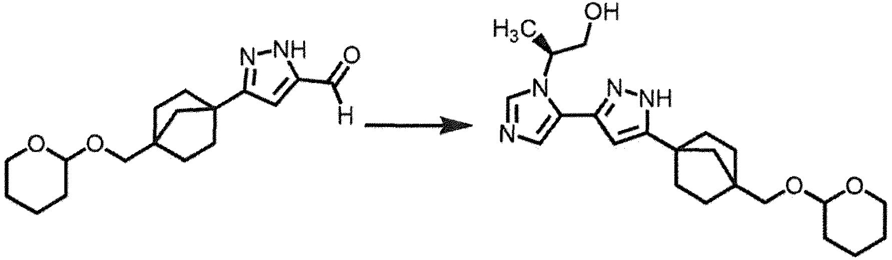

The crude product (2.4 g) of 3-(4-(((tetrahydro-2H-pyran-2-yl)oxy)methyl)bicyclo[2.2.1]heptan-1-yl)-1H-pyrazole-5-carbaldehyde obtained in the previous step and (S)-2-aminopropan-1-ol (0.574 g) were mixed with 1,2-dimethoxyethane (32.2 ml) and methanol (11.08 ml), and the mixture was stirred at room temperature for 2 hr. The reaction mixture was concentrated under reduced pressure. To the obtained residue were successively added DMF (22.15 ml), potassium carbonate (2.012 g), and p-toluenesulfonylmethyl isocyanide (1.705 g), and the mixture was stirred at room temperature for 3 days. At room temperature, water was added to the reaction mixture, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:methanol=2:98 to 50:50) to give the title compound (2.11 g).
¹H-NMR (400 MHz, CDCl₃) 1.39 - 2.12 (m, 20H), 3.42 (d, J=9.71Hz, 1H), 3.47 - 3.54 (m, 1H), 3.68 - 3.77 (m, 1H), 3.80 - 3.89 (m, 2H), 3.95 (dd, J=11.44, 3.58Hz, 1H), 4.56 - 4.62 (m, 1H), 4.87 - 4.99 (m, 1H), 6.22 (s, 1H), 7.15 (d, J=0.92Hz, 1H), 7.66 (d, J=0.92Hz, 1H)

### Step 11

### (5S)-5-methyl-9-(4-(((tetrahydro-2H-pyran-2-yl)oxy)methyl)bicyclo[2.2.1]heptan-1-yl)-5,6-dihydroimidazo[1,5-a]pyrazolo[5,1-c]pyrazine

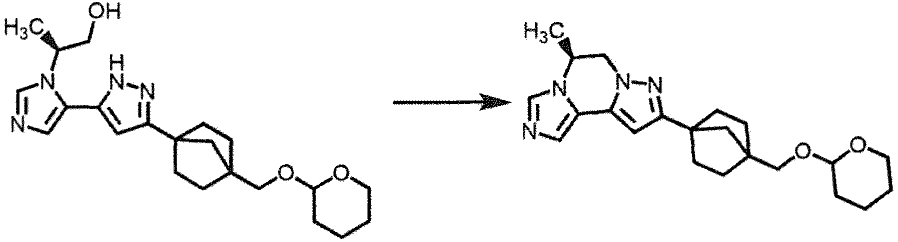

(2S)-2-(5-(5-(4-(((Tetrahydro-2H-pyran-2-yl)oxy)methyl)bicyclo[2.2.1]heptan-1-yl)-1H-pyrazol-3-yl)-1H-imidazol-1-yl)propan-1-ol (2.11 g) obtained in the previous step and triphenylphosphine (1.492 g) were mixed with tetrahydrofuran (38 ml). Under ice-cooling, diisopropyl azodicarboxylate (1.224 g) was added dropwise, and the mixture was stirred at room temperature for 1 hr. The reaction mixture was purified by silica gel column chromatography (ethyl acetate:methanol=2:98 to 60:40) to give the title compound (1.74 g).
¹H-NMR (400 MHz, CDCl₃) 1.39 - 2.06 (m, 19H), 3.42 (d, J=9.94Hz, 1H), 3.45 - 3.53 (m, 1H), 3.80 - 3.91 (m, 2H), 4.08 (dd, J=12.95, 8.21Hz, 1H), 4.44 (dd, J=12.95, 4.39Hz, 1H), 4.53 - 4.64 (m, 2H), 6.27 (s, 1H), 7.25 (s, 1H), 7.59 (s, 1H)

### Step 12

### 1-((5S)-5-methyl-9-(4-(((tetrahydro-2H-pyran-2-yl)oxy)methyl)bicyclo[2.2.1]heptan-1-yl)-5,6-dihydroimidazo[1,5-a]pyrazolo[5,1-c]pyrazin-3-yl)ethan-1-one

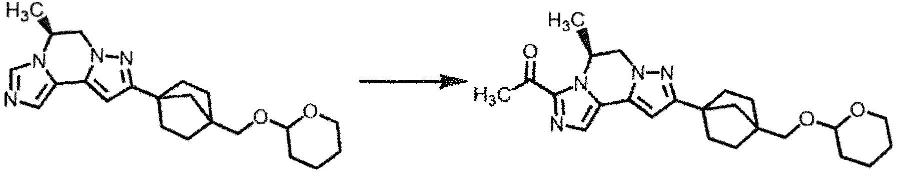

(5S)-5-Methyl-9-(4-(((tetrahydro-2H-pyran-2-yl)oxy)methyl)bicyclo[2.2.1]heptan-1-yl)-5,6-dihydroimidazo[1,5-a]pyrazolo[5,1-c]pyrazine (1.74 g) obtained in the previous step was mixed with tetrahydrofuran (17.4 ml). The mixture was cooled to -78°C, and 2 M lithium diisopropylamide/tetrahydrofuran-heptane-ethylbenzene solution (2.274 ml) was added dropwise. The mixture was stirred at - 78°C for 30 min. To the mixture was added N-methoxy-N-methylacetamide (1.115 ml), and the mixture was stirred at - 78°C for 30 min. To the mixture was added dropwise 2 M lithium diisopropylamide/tetrahydrofuran-heptane-ethylbenzene solution (2.274 ml), and the mixture was stirred at -78°C for 1 hr. To the reaction mixture was added saturated ammonium chloride aqueous solution, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=12:88 to ethyl acetate, thereafter ethyl acetate:methanol=60:40) to give the title compound (1.4 g). ¹H-NMR (400 MHz, CDCl₃) 1.38 (d, J=6.70Hz, 3H), 1.41 - 1.92 (m, 14H), 1.92 - 2.05 (m, 2H), 2.67 (s, 3H), 3.42 (d, J=9.94Hz, 1H), 3.46 - 3.54 (m, 1H), 3.80 - 3.91 (m, 2H), 4.34 (d, J=3.01Hz, 2H), 4.57 - 4.64 (m, 1H), 5.74 - 5.86 (m, 1H), 6.39 (s, 1H), 7.33 (s, 1H)

### Step 13

### (2R)-1,1,1-trifluoro-2-((5S)-5-methyl-9-(4-(((tetrahydro-2H-pyran-2-yl)oxy)methyl)bicyclo[2.2.1]heptan-1-yl)-5,6-dihydroimidazo[1,5-a]pyrazolo[5,1-c]pyrazin-3-yl)propan-2-ol

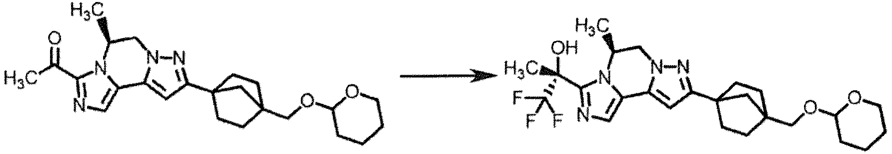

1-((5S)-5-Methyl-9-(4-(((tetrahydro-2H-pyran-2-yl)oxy)methyl)bicyclo[2.2.1]heptan-1-yl)-5,6-dihydroimidazo[1,5-a]pyrazolo[5,1-c]pyrazin-3-yl)ethan-1-one (1.4 g) obtained in the previous step was azeotroped with toluene, and mixed with N,N-dimethylacetamide (6.55 ml). Under ice-cooling, to the mixture were added lithium acetate (0.061 g) and trifluoromethyltrimethylsilane (0.686 ml), and the mixture was stirred for 30 min. Under ice-cooling, methanol (0.655 ml) and potassium carbonate (0.512 g) were added, and the mixture was stirred at room temperature for 1 hr. To the reaction mixture was added water, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=12:88 to ethyl acetate) to give the title compound (1.32 g).
¹H-NMR (400 MHz, CDCl₃) 1.36 (d, J=6.70Hz, 3H), 1.41 - 2.04 (m, 19H), 3.15 (s, 1H), 3.42 (d, J=9.94Hz, 1H), 3.45 - 3.53 (m, 1H), 3.80 - 3.90 (m, 2H), 4.25 - 4.36 (m, 2H), 4.57 - 4.64 (m, 1H), 5.26 - 5.37 (m, 1H), 6.28 (s, 1H), 7.20 (s, 1H)

### Step 14

### (R)-1,1,1-trifluoro-2-((S)-9-(4-(hydroxymethyl)bicyclo[2.2.1]heptan-1-yl)-5-methyl-5,6-dihydroimidazo[1,5-a]pyrazolo[5,1-c]pyrazin-3-yl)propan-2-ol

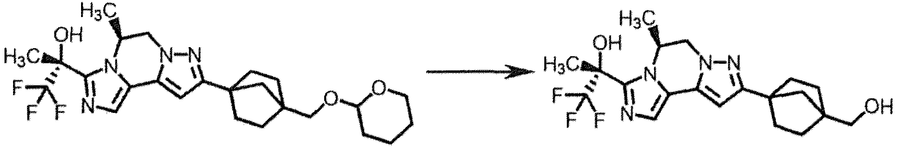

(2R)-1,1,1-Trifluoro-2-((5S)-5-methyl-9-(4-(((tetrahydro-2H-pyran-2-yl)oxy)methyl)bicyclo[2.2.1]heptan-1-yl)-5,6-dihydroimidazo[1,5-a]pyrazolo[5,1-c]pyrazin-3-yl)propan-2-ol (650 mg) obtained in the previous step was mixed with methanol (6.5 ml). To the mixture was added 10-camphorsulfonic acid (153 mg), and the mixture was stirred at room temperature for 3 hr. To the reaction mixture was added saturated aqueous sodium hydrogen carbonate solution, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=24:76 to ethyl acetate, thereafter ethyl acetate:methanol=90:10) to give the title compound (520 mg).
¹H-NMR (400 MHz, DMSO-D₆) 1.23 (d, J=6.47Hz, 3H), 1.25 - 1.36 (m, 2H), 1.46 (s, 2H), 1.57 - 1.72 (m, 4H), 1.80 (s, 3H), 1.83 - 1.95 (m, 2H), 3.47 (d, J=5.32Hz, 2H), 4.21 (dd, J=13.52, 4.05Hz, 1H), 4.33 (d, J=13.52Hz, 1H), 4.42 (t, J=5.32Hz, 1H), 5.24 - 5.38 (m, 1H), 6.37 (s, 1H), 7.21 (s, 1H), 7.23 (s, 1H)

### Step 15

### 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)bicyclo[2.2.1]heptane-1-carbaldehyde

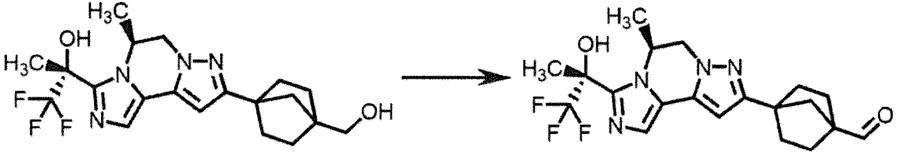

(R)-1,1,1-Trifluoro-2-((S)-9-(4-(hydroxymethyl)bicyclo[2.2.1]heptan-1-yl)-5-methyl-5,6-dihydroimidazo[1,5-a]pyrazolo[5,1-c]pyrazin-3-yl)propan-2-ol (250 mg) obtained in the previous step was mixed with chloroform (2.363 ml)-acetonitrile (2.363 ml). Under ice-cooling, Dess-Martin periodinane (293 mg) was added, and the mixture was stirred at room temperature overnight. To the reaction mixture were added 5% sodium thiosulfate aqueous solution and saturated aqueous sodium hydrogen carbonate solution, and the mixture was stirred at room temperature for 1 hr, and extracted twice with ethyl acetate. The organic layer was washed successively with 10% potassium carbonate aqueous solution, saturated aqueous sodium hydrogen carbonate solution, and saturated aqueous sodium chloride solution, and dried over sodium sulfate. Sodium sulfate was filtered off, and the filtrate was concentrated under reduced pressure to give a crude product (239 mg) of the title compound.

### Step 16

### 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)bicyclo[2.2.1]heptane-1-carboxylic acid

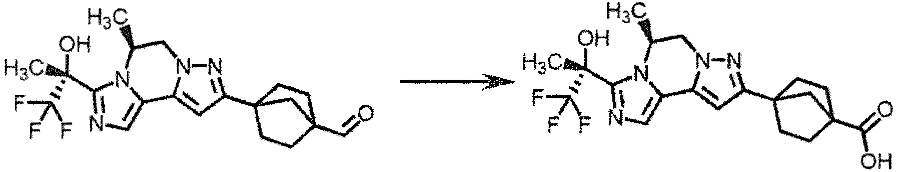

The crude product (239 mg) of 4-((S)-5-methyl-3-((R)-1,1, 1-trifluoro-2-hydroxypropan-2-yl)-5, 6-dihydroimida zo[1,5-a]pyrazolo[5,1-c]pyrazin-9-yl)bicyclo[2.2.1]heptane-1-carbaldehyde obtained in the previous step was mixed with tert-butanol (1.882 ml) and water (0.941 ml). To the mixture were added 2-methyl-2-butene (0.61 ml), sodium dihydrogen phosphate (449 mg), and sodium chlorite (195 mg), and the mixture was stirred at room temperature for 2 hr. To the reaction mixture was added 5% sodium thiosulfate aqueous solution, and the mixture was stirred at room temperature for 30 min. The mixture was purified by reversed-phase silica gel chromatography (acetonitrile:water=5:95 to acetonitrile). To the obtained crude product was added water, the pH was adjusted to 6 to 7 with 1N aqueous hydrochloric acid solution, and the mixture was extracted three times with ethyl acetate. The organic layer was washed with saturated aqueous sodium chloride solution, and dried over sodium sulfate. Sodium sulfate was filtered off, and the filtrate was concentrated under reduced pressure to give a crude product (295 mg) of the title compound.

### Step 17

### 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)bicyclo[2.2.1]heptane-1-carboxamide

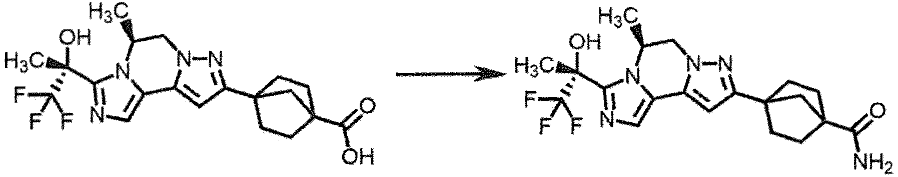

The crude product (295 mg) of 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)bicyclo[2.2.1]heptane-1-carboxylic acid obtained in the previous step was mixed with DMF (2.445 ml). Under ice-cooling, 7 M ammonia/methanol solution (0.247 ml) and 1-[bis(dimethylamino)methylene]-1H-1,2,3-triazolo[4,5-b]pyridinium 3-oxide hexafluorophosphate (329 mg) were added, and the mixture was stirred at room temperature for 3 hr. To the reaction mixture was added water, and the mixture was extracted with ethyl acetate. Sodium chloride was added to the aqueous layer, and the mixture was extracted three times with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:methanol=99:1 to 60:40) to give the title compound (302.1 mg).
¹H-NMR (400 MHz, CDCl₃) 1.36 (d, J=6.70Hz, 3H), 1.76 - 1.89 (m, 4H), 1.95 - 2.00 (m, 5H), 2.04 - 2.10 (m, 4H), 3.71 - 3.82 (m, 1H), 4.26 - 4.37 (m, 2H), 5.29 - 5.43 (m, 2H), 5.59 (brs, 1H), 6.27 (s, 1H), 7.22 (s, 1H)

### Step 18

### 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)bicyclo[2.2.1]heptane-1-carbonitrile

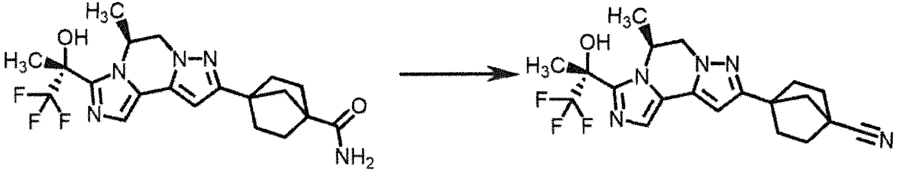

4-((S)-5-Methyl-3-((R)-1,1,1-trifluoro-2-hydroxypropan-2-yl)-5,6-dihydroimidazo[1,5-a]pyrazolo[5,1-c]pyrazin-9-yl)bicyclo[2.2.1]heptane-1-carboxamide (100 mg) obtained in the previous step was mixed with tetrahydrofuran (0.75 ml). Under ice-cooling, triethylamine (0.123 ml) and trifluoroacetic anhydride (0.0375 ml) were added, and the mixture was stirred for 1 hr. Under ice-cooling, to the reaction mixture was added trifluoroacetic anhydride (0.025 ml), and the mixture was stirred for 30 min. At room temperature, to the reaction mixture was added saturated aqueous sodium hydrogen carbonate solution, and the mixture was extracted twice with ethyl acetate. The organic layer was washed successively with water and saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=12:88 to ethyl acetate) to give the title compound (61.6 mg). ¹H-NMR (400 MHz, DMSO-D₆) 1.22 (d, J=6.47Hz, 3H), 1.64 - 1.74 (m, 2H), 1.80 (s, 3H), 1.83 - 1.92 (m, 2H), 1.92 - 2.10 (m, 6H), 4.23 (dd, J=13.41, 3.93Hz, 1H), 4.35 (d, J=13.41Hz, 1H), 5.27 - 5.39 (m, 1H), 6.45 (s, 1H), 7.23 (s, 1H), 7.25 (s, 1H)

### Production Example 7

### Synthesis of 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)bicyclo[2.2.2]octane-1-carbonitrile (Example 71)

### Step 1

### methyl 4-(hydroxymethyl)bicyclo[2.2.2]octane-1-carboxylate

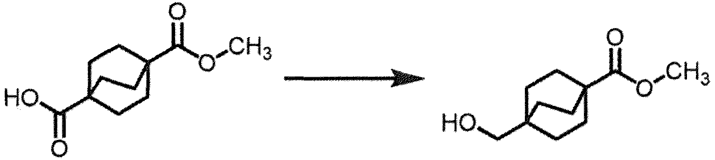

4-(Methoxycarbonyl)bicyclo[2.2.2]octane-1-carboxylic acid (3 g) was mixed with THF (45 ml). The reaction solution was cooled at 0°C, and 0.89 M borane-THF complex THF solution (15.88 ml) was added dropwise. The reaction mixture was stirred at room temperature for 2 hr. The reaction solution was cooled to 0°C, and saturated aqueous sodium hydrogen carbonate solution was added dropwise. The reaction mixture was extracted with ethyl acetate. The organic layer was washed with saturated aqueous sodium chloride solution, and sodium sulfate was added. Sodium sulfate was filtered off, and the filtrate was concentrated under reduced pressure. The obtained residue was purified by silica gel column chromatography (hexane:ethyl acetate=100:0 to 0:100) to give the title compound (2.8 g).
¹H-NMR (400 MHz, CDCl₃) 1.23 (t, J=5.60Hz, 1H), 1.41 - 1.48 (m, 6H), 1.76 - 1.83 (m, 6H), 3.29 (d, J=5.60Hz, 2H), 3.64 (s, 3H)

### Step 2

### methyl 4-(((tetrahydro-2H-pyran-2-yl)oxy)methyl)bicyclo[2.2.2]octane-1-carboxylate

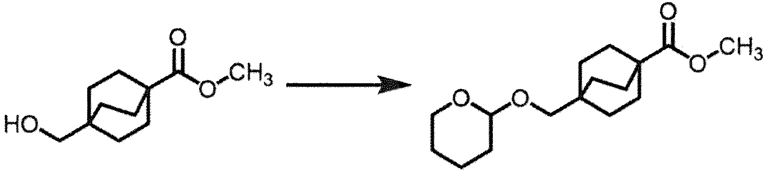

Methyl 4-(hydroxymethyl)bicyclo[2.2.2]octane-1-carboxylate (2.12 g) obtained in the previous step was mixed with acetonitrile (30 ml). At room temperature, 3,4-dihydro-2H-pyran (1.075 ml) and pyridinium p-toluenesulfonate (0.537 g) were added, and the mixture was stirred at room temperature for 18 hr. The reaction mixture was added to silica gel (10 g) and eluted with ethyl acetate. The obtained residue was purified by silica gel column chromatography (hexane:ethyl acetate=100:0 to 0:100) to give the title compound (2.86 g). ¹H-NMR (400 MHz, DMSO-D₆) 1.38 - 1.45 (m, 10H), 1.54 - 1.61 (m, 1H), 1.64 - 1.71 (m, 7H), 2.89 - 2.93 (m, 1H), 3.26 - 3.29 (m, 1H), 3.36 - 3.41 (m, 1H), 3.55 (s, 3H), 3.64 - 3.70 (m, 1H), 4.48 - 4.45 (m, 1H)

### Step 3

### 4-(((tetrahydro-2H-pyran-2-yl)oxy)methyl)bicyclo[2.2.2]octane-1-carboxylic acid

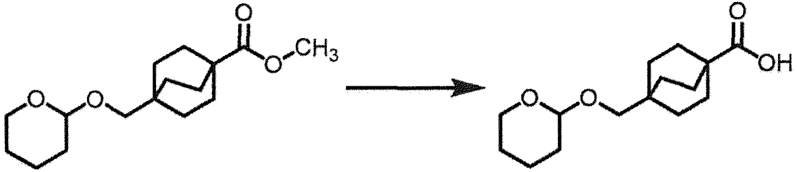

Methyl 4-(((tetrahydro-2H-pyran-2-yl)oxy)methyl)bicyclo[2.2.2]octane-1-carboxylate (3.28 g) was mixed with ethanol (32.8 ml). 2N Sodium hydroxide aqueous solution (6.97 ml) was added, and the mixture was stirred at 60°C for 18 hr. The reaction mixture was stirred at 70°C for 2 hr. To the reaction mixture was added 2N sodium hydroxide aqueous solution (3 ml), and the mixture was stirred at 70°C for 3 hr. The reaction mixture was stirred at 80°C for 5 hr. At room temperature, 2N hydrogen chloride solution (9.97 ml) was added, and the mixture was concentrated under reduced pressure. The obtained residue was extracted with ethyl acetate, and the organic layer was washed with saturated aqueous sodium chloride solution, and sodium sulfate was added. Sodium sulfate was filtered off, and the filtrate was concentrated under reduced pressure to give a crude product (3.12 g) of the title compound.

### Step 4

### N-methoxy-N-methyl-4-(((tetrahydro-2H-pyran-2-yl)oxy)methyl)bicyclo[2.2.2]octane-1-carboxamide

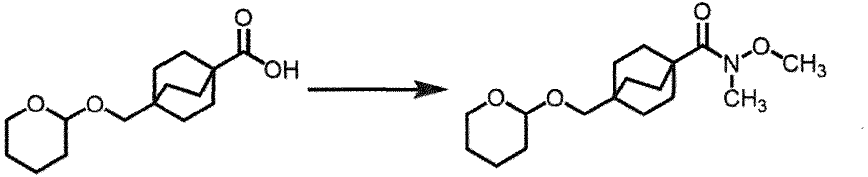

4-(((Tetrahydro-2H-pyran-2-yl)oxy)methyl)bicyclo[2.2.2]octane-1-carboxylic acid (3.02 g) obtained in the previous step was mixed with pyridine (30.2 ml). To the mixture were added 1-ethyl-3-(3-dimethylaminopropyl)carbodiimide hydrochloride (2.59 g) and N,O-dimethylhydroxylamine hydrochloride (1.317 g). The reaction mixture was stirred at room temperature for 18 hr. To the mixture were added 1-ethyl-3-(3-dimethylaminopropyl)carbodiimide hydrochloride (2.59 g) and N,O-dimethylhydroxylamine hydrochloride (1.317 g), and the mixture was stirred at 60°C for 2 hr. At room temperature, silica gel (50 g) and ethyl acetate were added to the reaction mixture, and silica gel was filtered off. The filtrate was concentrated under reduced pressure, and the obtained residue was purified by silica gel column chromatography (hexane:ethyl acetate=100:0 to 0:100) to give the title compound (3.2 g). ¹H-NMR (400 MHz, CDCl₃) 1.42 - 1.57 (m, 10H), 1.62 - 1.73 (m, 1H), 1.78 - 1.92 (m, 7H), 2.92 - 2.97 (m, 1H), 3.14 (s, 3H), 3.39 - 3.43 (m, 1H), 3.44 - 3.50 (m, 1H), 3.64 (s, 3H), 3.77 - 3.86 (m, 1H), 4.53 - 4.49 (m, 1H)

### Step 5

### 1-(4-(((tetrahydro-2H-pyran-2-yl)oxy)methyl)bicyclo[2.2.2]octan-1-yl)ethan-1-one

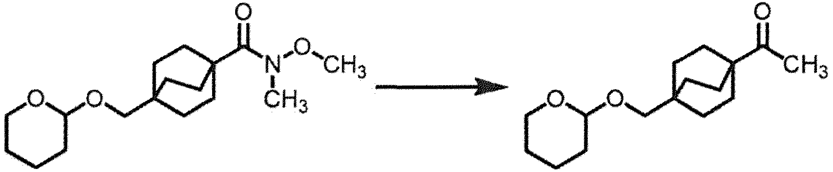

Under an argon atmosphere, N-methoxy-N-methyl-4-(((tetrahydro-2H-pyran-2-yl)oxy)methyl)bicyclo[2.2.2]octane-1-carboxamide (3.2 g) obtained in the previous step was mixed with tetrahydrofuran (32 ml). The mixture was ice-cooled, 1.04 M methylmagnesium bromide/tetrahydrofuran solution (11.86 ml) was added dropwise, and the mixture was stirred for 1 hr. To the reaction mixture was added saturated ammonium chloride aqueous solution. The reaction mixture was extracted with ethyl acetate. The organic layer was washed with saturated aqueous sodium chloride solution, and sodium sulfate was added. Sodium sulfate was filtered off, and the filtrate was concentrated under reduced pressure. The obtained residue was purified by silica gel column chromatography (hexane:ethyl acetate=100:0 to 0:100) to give the title compound (1.26 g). ¹H-NMR (400 MHz, CDCl₃) 1.47 - 1.56 (m, 10H), 1.64 - 1.74 (m, 7H), 1.77 - 1.84 (m, 1H), 2.07 (s, 3H), 2.92 - 2.98 (m, 1H), 3.40 - 3.44 (m, 1H), 3.44 - 3.51 (m, 1H), 3.78 - 3.83 (m, 1H), 4.52 - 4.49 (m, 1H)

### Step 6

### ethyl 2,4-dioxo-4-(4-(((tetrahydro-2H-pyran-2-yl)oxy)methyl)bicyclo[2.2.2]octan-1-yl)butanoate

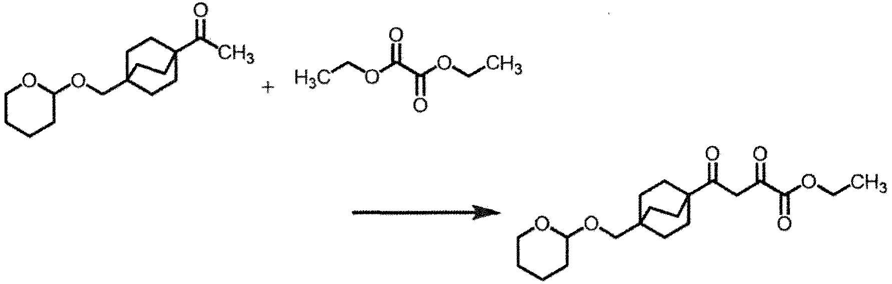

1-(4-(((Tetrahydro-2H-pyran-2-yl)oxy)methyl)bicyclo[2.2.2]octan-1-yl)ethan-1-one (1.26 g) obtained in the previous step was mixed with tetrahydrofuran (12.6 ml). To the mixture was added diethyl oxalate (0.768 ml). The mixture was ice-cooled, 1 M potassium-tert-butoxide/tetrahydrofuran solution (5.68 ml) was added dropwise, and the mixture was stirred for 2 hr. To the reaction mixture was added 1N hydrogen chloride solution (5.68 ml), and the mixture was extracted with ethyl acetate. The organic layer was washed with saturated aqueous sodium chloride solution, and sodium sulfate was added. Sodium sulfate was filtered off. The filtrate was concentrated under reduced pressure to give a crude product (1.733 g) of the title compound.

### Step 7

### ethyl 5-(4-(((tetrahydro-2H-pyran-2-yl)oxy)methyl)bicyclo[2.2.2]octan-1-yl)-1H-pyrazole-3-carboxylate

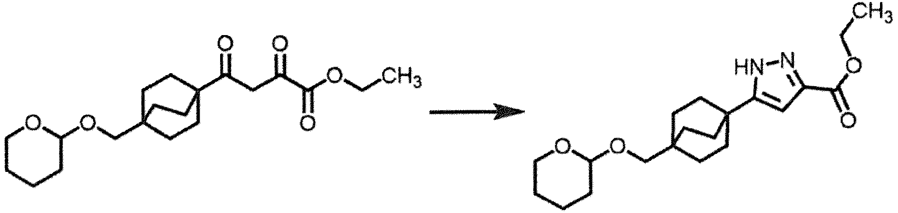

Ethyl 2,4-dioxo-4-(4-(((tetrahydro-2H-pyran-2-yl)oxy)methyl)bicyclo[2.2.2]octan-1-yl)butanoate (1.73 g) obtained in the previous step was mixed with ethanol (17.3 ml). Under ice-cooling, hydrazine monohydrate (0.241 ml) was added. The mixture was stirred at room temperature for 15 hr. The reaction mixture was concentrated under reduced pressure. The obtained residue was purified by silica gel column chromatography (hexane:ethyl acetate=100:0 to 0:100) to give the title compound (1.53 g).
¹H-NMR (400 MHz, DMSO-D₆) 1.25 (t, J=7.05Hz, 3H), 1.41 - 1.79 (m, 18H), 2.92 - 2.97 (m, 1H), 3.31 - 3.34 (m, 1H), 3.37 - 3.43 (m, 1H), 3.65 - 3.73 (m, 1H), 4.22 (q, J=7.05Hz, 2H), 4.49 (s, 1H), 6.39 (s, 1H), 13.12 (brs, 1H)

### Step 8

### (5-(4-(((tetrahydro-2H-pyran-2-yl)oxy)methyl)bicyclo[2.2.2]octan-1-yl)-1H-pyrazol-3-yl)methanol

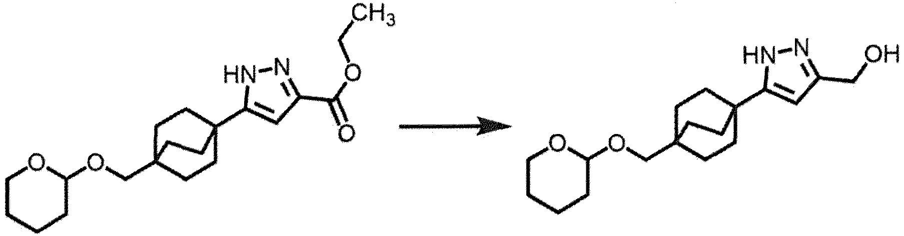

Under an argon atmosphere, lithium aluminum hydride (0.129 g) was mixed with tetrahydrofuran (12.45 ml). The mixture was ice-cooled, and a solution of ethyl 5-(4-(((tetrahydro-2H-pyran-2-yl)oxy)methyl)bicyclo[2.2.2]octan-1-yl)-1H-pyrazole-3-carboxylate (0.615 g) obtained in the previous step in tetrahydrofuran (6 ml) was added dropwise. The mixture was stirred for 150 min, and saturated potassium sodium tartrate aqueous solution was added dropwise to the reaction mixture. After stirring at room temperature for 1 hr, the reaction mixture was extracted with ethyl acetate. The organic layer was washed with saturated aqueous sodium chloride solution, and sodium sulfate was added. Sodium sulfate was filtered off, and the filtrate was concentrated under reduced pressure to give a crude product (0.544 g) of the title compound.

### Step 9

### 5-(4-(((tetrahydro-2H-pyran-2-yl)oxy)methyl)bicyclo[2.2.2]octan-1-yl)-1H-pyrazole-3-carbaldehyde

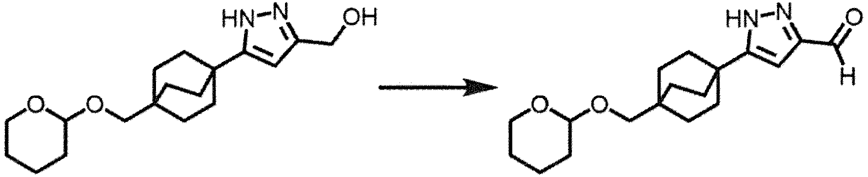

(5-(4-(((Tetrahydro-2H-pyran-2-yl)oxy)methyl)bicyclo[2.2.2]octan-1-yl)-1H-pyrazol-3-yl)methanol (0.632 g) was mixed with 1,2-dimethoxyethane (20 ml). Manganese dioxide (1.58 g) was added, and the mixture was stirred at room temperature for 16 hr. The reaction mixture was filtered through celite, and the filtrate was concentrated under reduced pressure to give a crude product (0.582 g) of the title compound.

### Step 10

### (2S)-2-(5-(3-(4-(((tetrahydro-2H-pyran-2-yl)oxy)methyl)bicyclo[2.2.2]octan-1-yl)-1H-pyrazol-5-yl)-1H-imidazol-1-yl)propan-1-ol

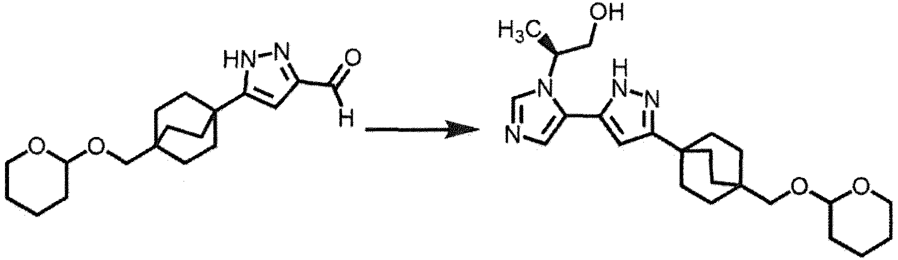

5-(4-(((Tetrahydro-2H-pyran-2-yl)oxy)methyl)bicyclo[2.2.2]octan-1-yl)-1H-pyrazole-3-carbaldehyde (0.582 g) obtained in the previous step was mixed with DMF (5 ml). At room temperature, (S)-2-aminopropan-1-ol (0.144 g) was added, and the mixture was stirred at room temperature for 20 hr. p-Toluenesulfonylmethyl isocyanide (0.430 g) and potassium carbonate (0.505 g) were added, and the mixture was stirred at room temperature for 43 hr. The reaction mixture was filtered through celite, and the filtrate was concentrated under reduced pressure. To the obtained residue was added water, and the mixture was extracted with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=25:75 to ethyl acetate, thereafter ethyl acetate:methanol=60:40) to give a crude product (0.458 g) of the title compound.

### Step 11

### (5S)-5-methyl-9-(4-(((tetrahydro-2H-pyran-2-yl)oxy)methyl)bicyclo[2.2.2]octan-1-yl)-5,6-dihydroimidazo[1,5-a]pyrazolo[5,1-c]pyrazine

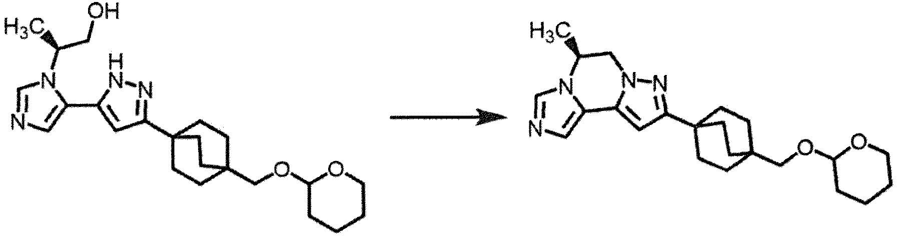

(2S)-2-(5-(3-(4-(((Tetrahydro-2H-pyran-2-yl)oxy)methyl)bicyclo[2.2.2]octan-1-yl)-1H-pyrazol-5-yl)-1H-imidazol-1-yl)propan-1-ol (0.458 g) obtained in the previous step was mixed with tetrahydrofuran (9.16 ml). At room temperature, triphenylphosphine (0.348 g) and diisopropyl azodicarboxylate (0.285 ml) were added, and the mixture was stirred at room temperature for 1 hr. The reaction mixture was concentrated under reduced pressure. The obtained residue was purified by silica gel column chromatography (ethyl acetate:hexane=25:75 to ethyl acetate, thereafter ethyl acetate:methanol=60:40) to give the title compound (0.43 g). ¹H-NMR (400 MHz, DMSO-D₆) 1.39 - 1.53 (m, 13H), 1.56 - 1.66 (m, 1H), 1.69 - 1.81 (m, 7H), 2.92 - 2.97 (m, 1H), 3.30 - 3.34 (m, 1H), 3.37 - 3.46 (m, 1H), 3.65 - 3.75 (m, 1H), 4.00 - 4.07 (m, 1H), 4.37 - 4.44 (m, 1H), 4.47 - 4.50 (m, 1H), 4.64 (s, 1H), 6.29 (s, 1H), 7.21 (s, 1H), 7.85 (s, 1H)

### Step 12

### 1-((5S)-5-methyl-9-(4-(((tetrahydro-2H-pyran-2-yl)oxy)methyl)bicyclo[2.2.2]octan-1-yl)-5,6-dihydroimidazo[1,5-a]pyrazolo[5,1-c]pyrazin-3-yl)ethan-1-one

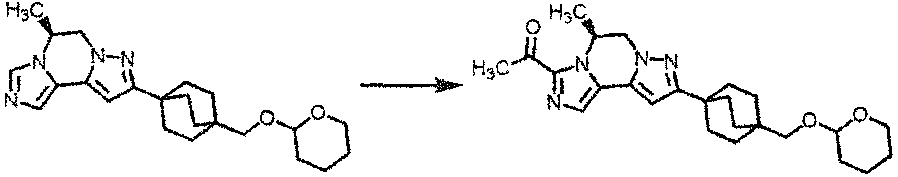

(5S)-5-Methyl-9-(4-(((tetrahydro-2H-pyran-2-yl)oxy)methyl)bicyclo[2.2.2]octan-1-yl)-5,6-dihydroimidazo[1,5-a]pyrazolo[5,1-c]pyrazine (0.43 g) obtained in the previous step was mixed with tetrahydrofuran (8.66 ml). Under cooling in an acetone-dry ice bath, 2 M lithium diisopropylamide/tetrahydrofuran solution (0.515 ml) was added dropwise, and the mixture was stirred for 1 hr. To the mixture was added N-methoxy-N-methylacetamide (0.286 ml), and the mixture was stirred for 1 hr. To the mixture was added dropwise 2 M lithium diisopropylamide/tetrahydrofuran solution (0.596 ml), and the mixture was stirred for 1 hr. Water was added to the reaction mixture, and the mixture was extracted with ethyl acetate. The organic layer was washed with saturated aqueous sodium chloride solution, and sodium sulfate was added. Sodium sulfate was filtered off, and the filtrate was concentrated under reduced pressure. The obtained residue was purified by silica gel column chromatography (hexane:ethyl acetate=100:0 to 0:100) to give the title compound (0.338 g). ¹H-NMR (400 MHz, DMSO-D₆) 1.22 (d, J=6.70Hz, 3H), 1.41 - 1.53 (m, 10H), 1.56 - 1.62 (m, 1H), 1.68 - 1.83 (m, 7H), 2.56 (s, 3H), 2.92 - 2.98 (m, 1H), 3.30 - 3.35 (m, 1H), 3.38 - 3.44 (m, 1H), 3.66 - 3.73 (m, 1H), 4.34 - 4.37 (m, 2H), 4.52 - 4.48 (m, 1H), 5.59 - 5.62 (m, 1H), 6.52 (s, 1H), 7.47 (s, 1H)

### Step 13

### (2R)-1,1,1-trifluoro-2-((5S)-5-methyl-9-(4-(((tetrahydro-2H-pyran-2-yl)oxy)methyl)bicyclo[2.2.2]octan-1-yl)-5,6-dihydroimidazo[1,5-a]pyrazolo[5,1-c]pyrazin-3-yl)propan-2-ol

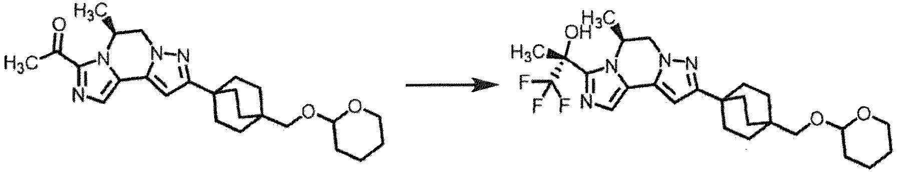

1-((5S)-5-Methyl-9-(4-(((tetrahydro-2H-pyran-2-yl)oxy)methyl)bicyclo[2.2.2]octan-1-yl)-5,6-dihydroimidazo[1,5-a]pyrazolo[5,1-c]pyrazin-3-yl)ethan-1-one (0.43 g) was mixed with tetrahydrofuran (4 ml). Under ice-cooling, to the mixture was added cesium fluoride (0.023 g), and (trifluoromethyl)trimethylsilane (0.228 ml) was added dropwise. The reaction mixture was stirred at room temperature for 1 hr. Under ice-cooling, methanol (2 ml) and potassium carbonate (0.138 g) were added, and the mixture was stirred at room temperature for 16 hr. Water was added, and the mixture was extracted with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (hexane:ethyl acetate=100:0 to 0:100) to give a crude product (0.287 g) of the title compound.

### Step 14

### (R)-1,1,1-trifluoro-2-((S)-9-(4-(hydroxymethyl)bicyclo[2.2.2]octan-1-yl)-5-methyl-5,6-dihydroimidazo[1,5-a]pyrazolo[5,1-c]pyrazin-3-yl)propan-2-ol

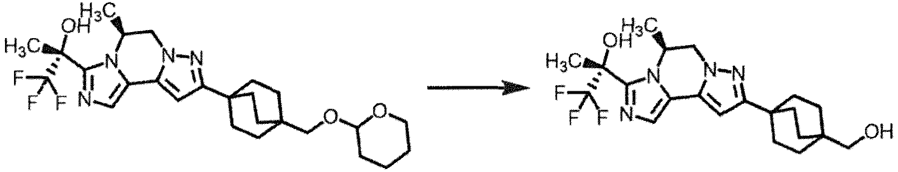

(2R)-1,1,1-Trifluoro-2-((5S)-5-methyl-9-(4-(((tetrahydro-2H-pyran-2-yl)oxy)methyl)bicyclo[2.2.2]octan-1-yl)-5,6-dihydroimidazo[1,5-a]pyrazolo[5,1-c]pyrazin-3-yl)propan-2-ol (0.287 g) obtained in the previous step was mixed with methanol (5 ml). 10-Camphorsulfonic acid (0.065 g) was added, and the mixture was stirred at 70°C for 1 hr. The reaction mixture was added to silica gel (10 g), and eluted with ethyl acetate. The obtained solution was concentrated under reduced pressure, and the residue was purified by silica gel column chromatography (hexane:ethyl acetate=100:0 to 0:100) to give the title compound (0.23 g).
¹H-NMR (400 MHz, DMSO-D₆) 1.21 (d, J=6.47Hz, 3H), 1.37 - 1.44 (m, 6H), 1.72 - 1.78 (m, 7H), 1.80 (s, 3H), 3.05 (d, J=5.55Hz, 2H), 4.16 - 4.23 (m, 1H), 4.29 - 4.34 (m, 2H), 5.34 - 5.27 (m, 1H), 6.33 (s, 1H), 7.20 (s, 1H), 7.24 (s, 1H)

### Step 15

### 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)bicyclo[2.2.2]octane-1-carbaldehyde

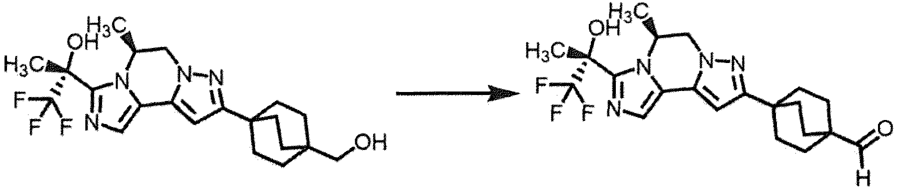

(R)-1,1,1-Trifluoro-2-((S)-9-(4-(hydroxymethyl)bicyclo[2.2.2]octan-1-yl)-5-methyl-5,6-dihydroimidazo[1,5-a]pyrazolo[5,1-c]pyrazin-3-yl)propan-2-ol (0.21 g) obtained in the previous step was mixed with chloroform (6 ml). Under ice-cooling, Dess-Martin periodinane (0.252 g) were added, and the mixture was stirred at room temperature for 16 hr. At room temperature, Dess-Martin periodinane (0.150 g) was added, and the mixture was stirred for 2 hr. Saturated sodium thiosulfate aqueous solution and saturated aqueous sodium hydrogen carbonate solution were added, and the mixture was extracted with ethyl acetate. The organic layer was washed with saturated aqueous sodium chloride solution, and dried over sodium sulfate. Sodium sulfate was filtered off, and the filtrate was concentrated under reduced pressure to give a crude product (0.20 g) of the title compound.

### Step 16

### 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)bicyclo[2.2.2]octane-1-carboxylic acid

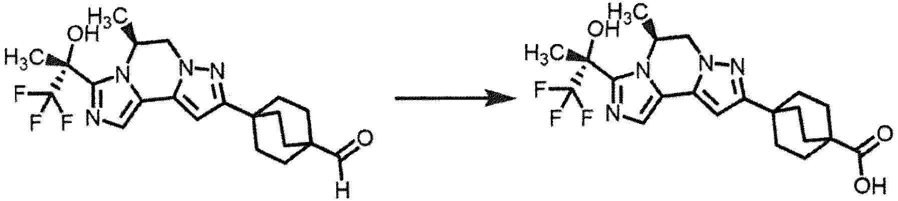

4-((S)-5-Methyl-3-((R)-1,1,1-trifluoro-2-hydroxypropan-2-yl)-5,6-dihydroimidazo[1,5-a]pyrazolo[5,1-c]pyrazin-9-yl)bicyclo[2.2.2]octane-1-carbaldehyde (0.2 g) obtained in the previous step was mixed with tert-butanol (6 ml) and water (3 ml). At room temperature, 2-methyl-2-butene (0.502 ml), disodium hydrogen phosphate (0.336 g), and sodium chlorite (0.128 g) were added, and the mixture was stirred for 17 hr. The reaction mixture was extracted with ethyl acetate. The aqueous layer was extracted twice with ethyl acetate. The organic layers were combined and dried over sodium sulfate. Sodium sulfate was filtered off, and the filtrate was concentrated under reduced pressure to give a crude product (0.118 g) of the title compound.

### Step 17

### 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)bicyclo[2.2.2]octane-1-carboxamide

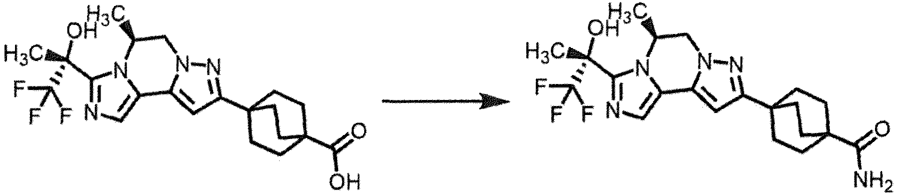

4-((S)-5-Methyl-3-((R)-1,1,1-trifluoro-2-hydroxypropan-2-yl)-5,6-dihydroimidazo[1,5-a]pyrazolo[5,1-c]pyrazin-9-yl)bicyclo[2.2.2]octane-1-carboxylic acid (0.03 g) obtained in the previous step was mixed with DMF (2 ml). At room temperature, 1-[bis(dimethylamino)methylene]-1H-1,2,3-triazolo[4,5-b]pyridinium 3-oxide hexafluorophosphate (0.039 g) and 7 M ammonia/methanol solution (0.029 ml) were added, and the mixture was stirred for 3 hr. To the reaction mixture was added water, and the mixture was extracted with ethyl acetate. The organic layer was washed twice with saturated aqueous sodium chloride solution, and dried over sodium sulfate. Sodium sulfate was filtered off, and the filtrate was concentrated under reduced pressure. The obtained residue was purified by silica gel column chromatography (hexane:ethyl acetate=100:0 to 0:100) to give a crude product (0.026 g).

### Step 18

### 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)bicyclo[2.2.2]octane-1-carbonitrile

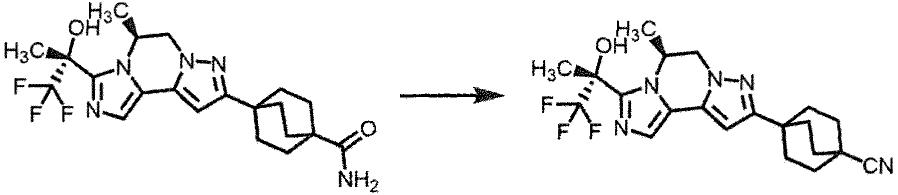

4-((S)-5-Methyl-3-((R)-1,1,1-trifluoro-2-hydroxypropan-2-yl)-5,6-dihydroimidazo[1,5-a]pyrazolo[5,1-c]pyrazin-9-yl)bicyclo[2.2.2]octane-1-carboxamide (0.026 g) obtained in the previous step was mixed with tetrahydrofuran (2 ml). Under ice-cooling, triethylamine (0.041 ml) and trifluoroacetic anhydride (0.013 ml) were added, and the mixture was stirred for 1 hr. The reaction mixture was added to silica gel (10 g), and eluted with ethyl acetate. The obtained solution was concentrated under reduced pressure, and the residue was purified by silica gel column chromatography (hexane:ethyl acetate=100:0 to 0:100) to give the title compound (0.018 g).
¹H-NMR (400 MHz, DMSO-D₆) 1.22 (d, J=6.73Hz, 3H), 1.80 - 1.86 (m, 9H), 1.94 - 1.99 (m, 6H), 4.19 - 4.25 (m, 1H), 4.30 - 4.36 (m, 1H), 5.36 - 5.28 (m, 1H), 6.37 (s, 1H), 7.23 (s, 1H), 7.27 (s, 1H)

### Production Example 8

Synthesis of 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)bicyclo[2.1.1]hexane-1-carbonitrile (Example 91)

### Step 1

### methyl 4-(hydroxymethyl)bicyclo[2.1.1]hexane-1-carboxylate

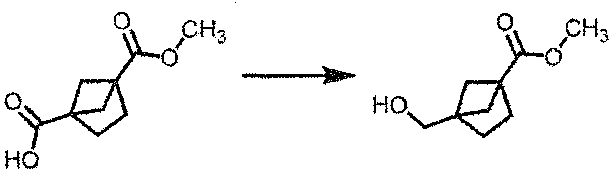

4-(Methoxycarbonyl)bicyclo[2.1.1]hexane-1-carboxylic acid (1.1 g) was mixed with THF (11 ml). Under ice-cooling, to the mixture was added dropwise 0.89 M borane-THF complex/THF solution (7.38 ml), and the mixture was stirred for 10 min. The reaction mixture was warmed to room temperature and stirred for 2 hr. Under ice-cooling, to the reaction mixture was added dropwise saturated aqueous sodium hydrogen carbonate solution, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=24:76 to ethyl acetate) to give the title compound (835.8 mg).
¹H-NMR (400 MHz, CDCl₃) 1.29 (t, J=5.09Hz, 1H), 1.37 - 1.45 (m, 2H), 1.62 - 1.69 (m, 2H), 1.75 - 1.84 (m, 2H), 1.93 - 2.00 (m, 2H), 3.68 (s, 3H), 3.71 (d, J=5.09Hz, 2H)

### Step 2

### methyl 4-(((tetrahydro-2H-pyran-2-yl)oxy)methyl)bicyclo[2.1.1]hexane-1-carboxylate

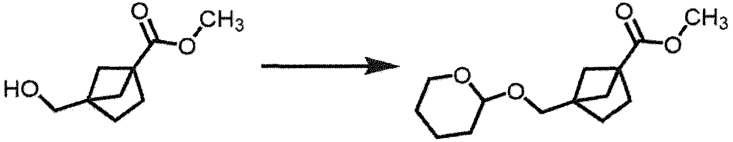

Methyl 4-(hydroxymethyl)bicyclo[2.1.1]hexane-1-carboxylate (835.8 mg) obtained in the previous step was mixed with acetonitrile (7.857 ml). At room temperature, pyridinium p-toluenesulfonate (232 mg) and 3,4-dihydro-2H-pyran (466 mg) were added, and the mixture was stirred at room temperature overnight. The reaction mixture was concentrated. The obtained residue was purified by silica gel column chromatography (ethyl acetate:hexane=5:95 to 36:64) to give a mixture (1.2375 g) containing the title compound.

### Step 3

### 4-(((tetrahydro-2H-pyran-2-yl)oxy)methyl)bicyclo[2.1.1]hexane-1-carboxylic acid

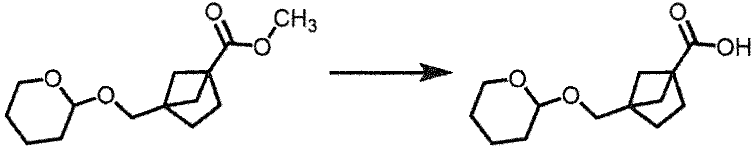

The mixture (1.2375 g) containing methyl 4-(((tetrahydro-2H-pyran-2-yl)oxy)methyl)bicyclo[2.1.1]hexane-1-carboxylate obtained in the previous step was mixed with methanol (5.87 ml)-tetrahydrofuran (5.87 ml). 2N Sodium hydroxide aqueous solution (2.77 ml) was added, and the mixture was stirred at room temperature for 1.5 hr. To the reaction mixture was added 2N sodium hydroxide aqueous solution (2.77 ml), and the mixture was stirred at 80°C for 3 hr. After cooling to room temperature, 2N aqueous hydrochloric acid solution (5.54 ml) was added, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure to give a crude product (1.25 g) of the title compound.

### Step 4

### N-methoxy-N-methyl-4-(((tetrahydro-2H-pyran-2-yl)oxy)methyl)bicyclo[2.1.1]hexane-1-carboxamide

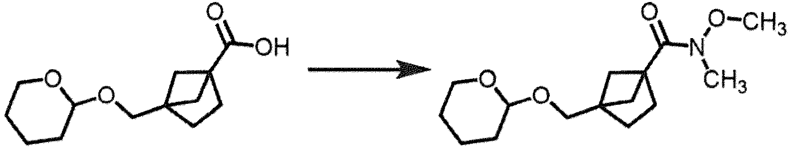

The crude product (1.25 g) of 4-(((tetrahydro-2H-pyran-2-yl)oxy)methyl)bicyclo[2.1.1]hexane-1-carboxylic acid obtained in the previous step and N,O-dimethylhydroxylamine hydrochloride (0.676 g) were mixed with pyridine (11.1 ml). To the mixture was added 1-ethyl-3-(3-dimethylaminopropyl)carbodiimide hydrochloride (1.329 g), and the mixture was stirred at room temperature overnight. To the reaction mixture were added N,O-dimethylhydroxylamine hydrochloride (0.676 g) and 1-ethyl-3-(3-dimethylaminopropyl)carbodiimide hydrochloride (1.329 g), and the mixture was stirred at room temperature for 3 hr. Water was added to the reaction mixture, and the mixture was extracted with ethyl acetate. The organic layer was washed successively with 1N aqueous hydrochloric acid solution, saturated aqueous sodium hydrogen carbonate solution, and saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=12:88 to ethyl acetate) to give the title compound (1.17 g).
¹H-NMR (400 MHz, CDCl₃) 1.45 - 1.63 (m, 6H), 1.63 - 1.88 (m, 6H), 1.93 - 2.00 (m, 2H), 3.17 (s, 3H), 3.42 - 3.52 (m, 2H), 3.63 (s, 3H), 3.79 - 3.87 (m, 2H), 4.54 - 4.59 (m, 1H)

### Step 5

### 1-(4-(((tetrahydro-2H-pyran-2-yl)oxy)methyl)bicyclo[2.1.1]hexan-1-yl)ethan-1-one

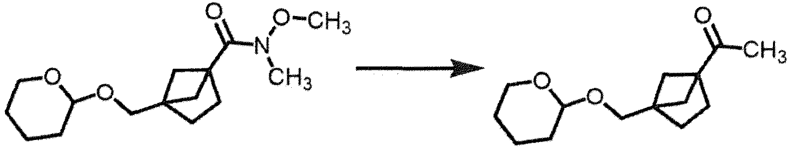

N-Methoxy-N-methyl-4-(((tetrahydro-2H-pyran-2-yl)oxy)methyl)bicyclo[2.1.1]hexane-1-carboxamide (1.17 g) obtained in the previous step was mixed with toluene (57.8 ml). Under ice-cooling, 1.04 M methylmagnesium bromide/tetrahydrofuran solution (4.57 ml) was added dropwise, and the mixture was stirred for 30 min. Under ice-cooling, saturated ammonium chloride aqueous solution and water were added, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=12:88 to ethyl acetate) to give the title compound (0.741 g).
¹H-NMR (400 MHz, CDCl₃) 1.36 - 1.44 (m, 2H), 1.45 - 1.63 (m, 4H), 1.63 - 1.87 (m, 6H), 1.87 - 1.94 (m, 2H), 2.13 (s, 3H), 3.39 - 3.52 (m, 2H), 3.77 - 3.88 (m, 2H), 4.53 - 4.58 (m, 1H)

### Step 6

### ethyl 2,4-dioxo-4-(4-(((tetrahydro-2H-pyran-2-yl)oxy)methyl)bicyclo[2.1.1]hexan-1-yl)butanoate

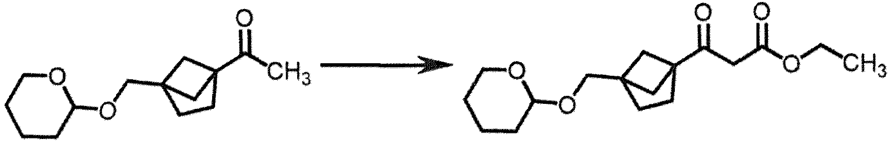

1-(4-(((Tetrahydro-2H-pyran-2-yl)oxy)methyl)bicyclo[2.1.1]hexan-1-yl)ethan-1-one (741 mg) obtained in the previous step was mixed with tetrahydrofuran (7.41 ml). Under ice-cooling, to the mixture was added diethyl oxalate (500 mg), 1 M potassium tert-butoxide/tetrahydrofuran solution (3.731 ml) was added dropwise, and the mixture was stirred for 1 hr. To the reaction mixture were added 1 M aqueous hydrochloric acid solution (3.731 ml), and the mixture was extracted with ethyl acetate. The organic layer was washed with saturated aqueous sodium chloride solution, and dried over sodium sulfate. Sodium sulfate was filtered off, and the filtrate was concentrated under reduced pressure, and azeotroped with ethanol to give a crude product (1.069 g) of the title compound.

### Step 7

### ethyl 3-(4-(((tetrahydro-2H-pyran-2-yl)oxy)methyl)bicyclo[2.1.1]hexan-1-yl)-1H-pyrazole-5-carboxylate

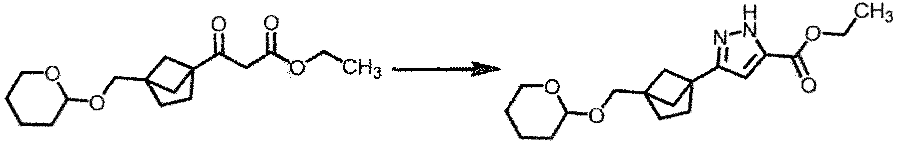

The crude product (1.069 g) of ethyl 2,4-dioxo-4-(4-(((tetrahydro-2H-pyran-2-yl)oxy)methyl)bicyclo[2.1.1]hexan-1-yl)butanoate obtained in the previous step and ethanol (10.52 ml) were mixed. Under ice-cooling, hydrazine monohydrate (0.158 ml) was added dropwise, and the mixture was stirred at room temperature overnight. The reaction mixture was concentrated under reduced pressure. The obtained residue was purified by silica gel column chromatography (ethyl acetate:hexane=12:88 to ethyl acetate) to give the title compound (1.03 g).
¹H-NMR (400 MHz, CDCl₃) 1.37 (t, J=7.05Hz, 3H), 1.43 - 1.91 (m, 12H), 1.95 - 2.05 (m, 2H), 3.44 - 3.53 (m, 2H), 3.79 - 3.92 (m, 2H), 4.37 (q, J=7.05Hz, 2H), 4.56 - 4.61 (m, 1H), 6.65 (s, 1H), 10.23 (brs, 1H)

### Step 8

### (3-(4-(((tetrahydro-2H-pyran-2-yl)oxy)methyl)bicyclo[2.1.1]hexan-1-yl)-1H-pyrazol-5-yl)methanol

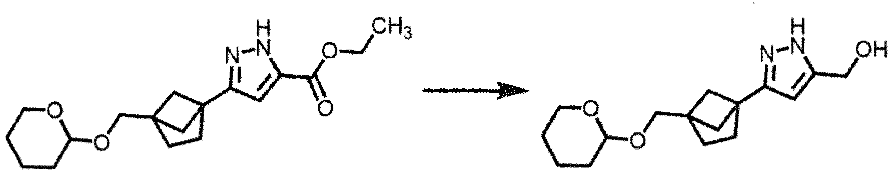

Ethyl 3-(4-(((tetrahydro-2H-pyran-2-yl)oxy)methyl)bicyclo[2.1.1]hexan-1-yl)-1H-pyrazole-5-carboxylate (1.03 g) obtained in the previous step was mixed with tetrahydrofuran (15.93 ml). Under ice-cooling, to the mixture was added dropwise 2 M lithium aluminum hydride/tetrahydrofuran solution (2.8 ml), and the mixture was stirred for 50 min. Under ice-cooling, to the reaction mixture was added saturated Rochelle salt aqueous solution, and the mixture was stirred at room temperature for 1 hr and extracted twice with ethyl acetate. The organic layer was washed with saturated aqueous sodium chloride solution, and dried over sodium sulfate. Sodium sulfate was filtered off, and the filtrate was concentrated under reduced pressure to give a crude product (0.819 g) of the title compound.

### Step 9

### 3-(4-(((tetrahydro-2H-pyran-2-yl)oxy)methyl)bicyclo[2.1.1]hexan-1-yl)-1H-pyrazole-5-carbaldehyde

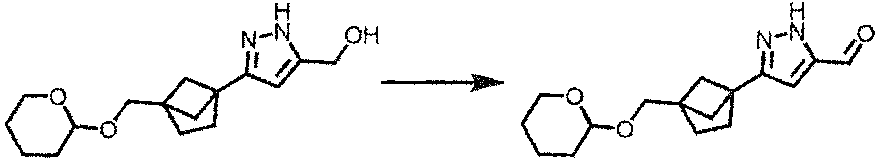

The crude product (0.819 g) of (3-(4-(((tetrahydro-2H-pyran-2-yl)oxy)methyl)bicyclo[2.1.1]hexan-1-yl)-1H-pyrazol-5-yl)methanol obtained in the previous step was mixed with 1,2-dimethoxyethane (24.56 ml). Manganese dioxide (2.456 g) was added, and the mixture was stirred at room temperature overnight. The reaction mixture was filtered through celite, and the filtrate was concentrated under reduced pressure to give a crude product (0.7343 g) of the title compound.

### Step 10

### (2S)-2-(5-(5-(4-(((tetrahydro-2H-pyran-2-yl)oxy)methyl)bicyclo[2.1.1]hexan-1-yl)-1H-pyrazol-3-yl)-1H-imidazol-1-yl)propan-1-ol

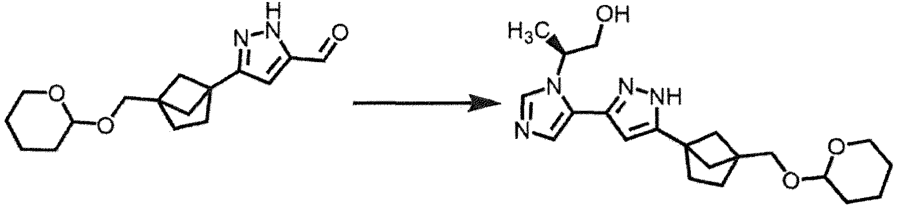

The crude product (734.3 mg) of 3-(4-(((tetrahydro-2H-pyran-2-yl)oxy)methyl)bicyclo[2.1.1]hexan-1-yl)-1H-pyrazole-5-carbaldehyde obtained in the previous step and (S)-2-aminopropan-1-ol (199 mg) were mixed with 1,2-dimethoxyethane (14.7 ml), and the mixture was stirred at room temperature for 1.5 hr. The reaction mixture was concentrated under reduced pressure. To the obtained residue were successively added DMF (7.343 ml), potassium carbonate (1.049 g), and p-toluenesulfonylmethyl isocyanide (0.741 g), and the mixture was stirred at room temperature for 4 days. At room temperature, water was added to the reaction mixture, and the mixture was extracted three times with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:methanol=2:98 to 50:50) to give a crude product (767.6 mg) of the title compound.

### Step 11

### (5S)-5-methyl-9-(4-(((tetrahydro-2H-pyran-2-yl)oxy)methyl)bicyclo[2.1.1]hexan-1-yl)-5,6-dihydroimidazo[1,5-a]pyrazolo[5,1-c]pyrazine

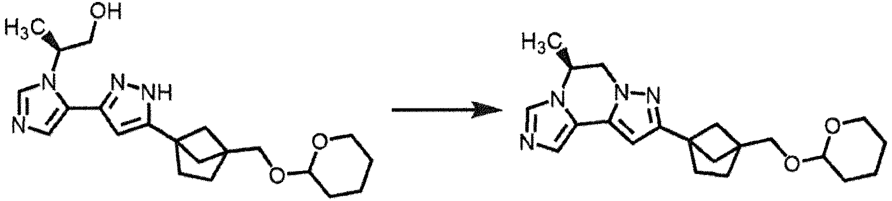

The crude product (767.6 mg) of (2S)-2-(5-(5-(4-(((tetrahydro-2H-pyran-2-yl)oxy)methyl)bicyclo[2.1.1]hexan-1-yl)-1H-pyrazol-3-yl)-1H-imidazol-1-yl)propan-1-ol obtained in the previous step and triphenylphosphine (569 mg) were mixed with tetrahydrofuran (15.352 ml). Under ice-cooling, diisopropyl azodicarboxylate (439 mg) was added dropwise, and the mixture was stirred at room temperature for 1 hr. Under ice-cooling, to the reaction mixture were added triphenylphosphine (142 mg) and diisopropyl azodicarboxylate (110 mg), and the mixture was stirred at room temperature for 1 hr. The reaction mixture was purified by silica gel column chromatography (ethyl acetate:methanol=2:98 to 60:40) to give the title compound (620.1 mg).
¹H-NMR (400 MHz, CDCl₃) 1.43 - 1.78 (m, 12H), 1.78 - 1.91 (m, 3H), 1.97 - 2.06 (m, 2H), 3.44 - 3.55 (m, 2H), 3.80 - 3.91 (m, 2H), 4.09 (dd, J=13.06, 7.98Hz, 1H), 4.45 (dd, J=13.06, 4.51Hz, 1H), 4.52 - 4.64 (m, 2H), 6.28 (s, 1H), 7.26 (s, 1H), 7.59 (s, 1H)

### Step 12

### 1-((5S)-5-methyl-9-(4-(((tetrahydro-2H-pyran-2-yl)oxy)methyl)bicyclo[2.1.1]hexan-1-yl)-5,6-dihydroimidazo[1,5-a]pyrazolo[5,1-c]pyrazin-3-yl)ethan-1-one

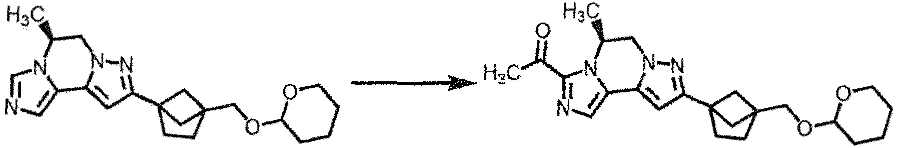

(5S)-5-Methyl-9-(4-(((tetrahydro-2H-pyran-2-yl)oxy)methyl)bicyclo[2.1.1]hexan-1-yl)-5,6-dihydroimidazo[1,5-a]pyrazolo[5,1-c]pyrazine (620.1 mg) obtained in the previous step was mixed with tetrahydrofuran (12.402 ml). The mixture was cooled to -78°C, and 2 M lithium diisopropylamide/tetrahydrofuran-heptane-ethylbenzene solution (0.841 ml) was added dropwise. The reaction mixture was stirred at -78°C for 30 min. To the mixture was added N-methoxy-N-methylacetamide (0.412 ml), and the mixture was stirred at -78°C for 30 min. To the mixture was added dropwise 2 M lithium diisopropylamide/tetrahydrofuran-heptane-ethylbenzene solution (0.841 ml), and the mixture was stirred at -78°C for 1 hr. To the reaction mixture was added saturated ammonium chloride aqueous solution, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=16:84 to ethyl acetate, thereafter ethyl acetate:methanol=50:50) to give the title compound (443 mg). ¹H-NMR (400 MHz, CDCl₃) 1.39 (d, J=6.94Hz, 3H), 1.46 - 1.92 (m, 12H), 1.99 - 2.07 (m, 2H), 2.68 (s, 3H), 3.46 - 3.54 (m, 2H), 3.82 - 3.91 (m, 2H), 4.31 - 4.40 (m, 2H), 4.58 - 4.63 (m, 1H), 5.75 - 5.86 (m, 1H), 6.41 (s, 1H), 7.34 (s, 1H)

### Step 13

### (2R)-1,1,1-trifluoro-2-((5S)-5-methyl-9-(4-(((tetrahydro-2H-pyran-2-yl)oxy)methyl)bicyclo[2.1.1]hexan-1-yl)-5,6-dihydroimidazo[1,5-a]pyrazolo[5,1-c]pyrazin-3-yl)propan-2-ol

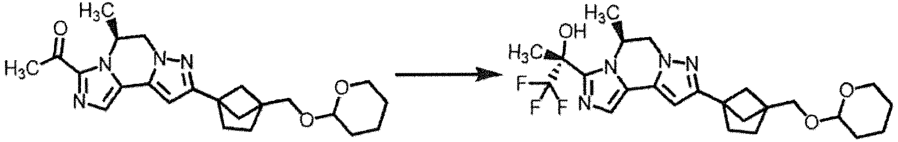

1-((5S)-5-Methyl-9-(4-(((tetrahydro-2H-pyran-2-yl)oxy)methyl)bicyclo[2.1.1]hexan-1-yl)-5,6-dihydroimidazo[1,5-a]pyrazolo[5,1-c]pyrazin-3-yl)ethan-1-one (443 mg) obtained in the previous step and N,N-dimethylacetamide (1.994 ml) were mixed. Under ice-cooling, to the mixture were added lithium acetate (19.23 mg) and trifluoromethyltrimethylsilane (0.216 ml), and the mixture was stirred for 1 hr. Under ice-cooling, methanol (0.199 ml) and potassium carbonate (0.161 g) were added, and the mixture was stirred at room temperature for 1 hr. To the reaction mixture was added water, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=12:88 to ethyl acetate) to give the title compound (409.4 mg).
¹H-NMR (400 MHz, CDCl₃) 1.37 (d, J=5.98Hz, 3H), 1.45 - 1.94 (m, 12H), 1.96 - 2.08 (m, 5H), 3.11 (s, 1H), 3.45 - 3.57 (m, 2H), 3.80 - 3.95 (m, 2H), 4.26 - 4.40 (m, 2H), 4.59 - 4.66 (m, 1H), 5.28 - 5.39 (m, 1H), 6.31 (s, 1H), 7.23 (s, 1H)

### Step 14

### (R)-1,1,1-trifluoro-2-((S)-9-(4-(hydroxymethyl)bicyclo[2.1.1]hexan-1-yl)-5-methyl-5,6-dihydroimidazo[1,5-a]pyrazolo[5,1-c]pyrazin-3-yl)propan-2-ol

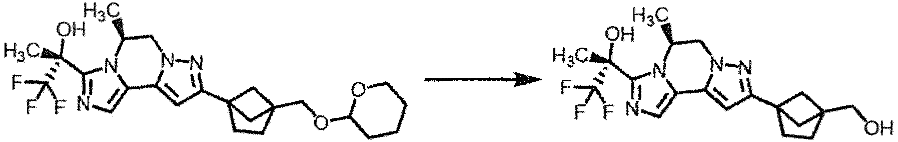

(2R)-1,1,1-Trifluoro-2-((5S)-5-methyl-9-(4-(((tetrahydro-2H-pyran-2-yl)oxy)methyl)bicyclo[2.1.1]hexan-1-yl)-5,6-dihydroimidazo[1,5-a]pyrazolo[5,1-c]pyrazin-3-yl)propan-2-ol (409.4 mg) obtained in the previous step was mixed with methanol (4.094 ml). To the mixture was added 10-camphorsulfonic acid (39.6 mg), and the mixture was stirred at room temperature overnight. The reaction solution was stirred at 70°C for 3 hr. To the reaction mixture was added saturated aqueous sodium hydrogen carbonate solution, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:methanol=99:1 to 80:20) to give the title compound (349.3 mg).
¹H-NMR (400 MHz, DMSO-D₆) 1.23 (d, J=6.47Hz, 3H), 1.28 - 1.33 (m, 2H), 1.55 - 1.63 (m, 2H), 1.65 - 1.70 (m, 2H), 1.80 (s, 3H), 1.87 - 1.94 (m, 2H), 3.51 (d, J=5.32Hz, 2H), 4.22 (dd, J=13.52, 4.28Hz, 1H), 4.34 (d, J=13.52Hz, 1H), 4.39 - 4.46 (m, 1H), 5.26 - 5.37 (m, 1H), 6.38 (s, 1H), 7.22 (s, 1H), 7.24 (s, 1H)

### Step 15

### 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)bicyclo[2.1.1]hexane-1-carbaldehyde

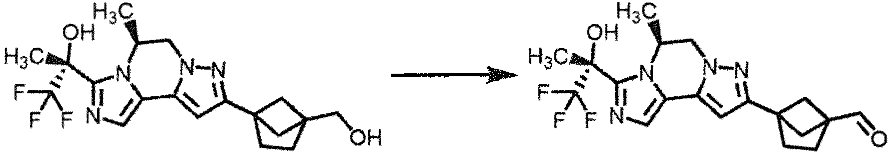

(R)-1,1,1-Trifluoro-2-((S)-9-(4-(hydroxymethyl)bicyclo[2.1.1]hexan-1-yl)-5-methyl-5,6-dihydroimidazo[1,5-a]pyrazolo[5,1-c]pyrazin-3-yl)propan-2-ol (175 mg) obtained in the previous step was mixed with chloroform (4.946 ml)-acetonitrile (2.473 ml). Under ice-cooling, Dess-Martin periodinane (212 mg) was added, and the mixture was stirred at room temperature overnight. 5% Sodium thiosulfate aqueous solution and saturated aqueous sodium hydrogen carbonate solution were added, and the mixture was stirred at room temperature for 1 hr and extracted twice with ethyl acetate. The organic layer was washed successively with 10% potassium carbonate aqueous solution, saturated aqueous sodium hydrogen carbonate solution, and saturated aqueous sodium chloride solution, and dried over sodium sulfate. Sodium sulfate was filtered off, and the filtrate was concentrated under reduced pressure to give a crude product (161 mg) of the title compound.

### Step 16

### 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)bicyclo[2.1.1]hexane-1-carboxylic acid

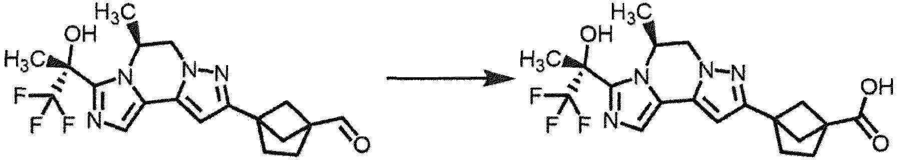

The crude product (161 mg) of 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)bicyclo[2.1.1]hexane-1-carbaldehyde obtained in the previous step was mixed with tert-butanol (1.255 ml) and water (0.627 ml). 2-Methyl-2-butene (0.421 ml), sodium dihydrogen phosphate (239 mg), and sodium chlorite (135 mg) were added, and the mixture was stirred at room temperature for 2 hr. To the reaction mixture was added 5% sodium thiosulfate aqueous solution, and the mixture was stirred at room temperature for 30 min and extracted three times with ethyl acetate. The mixture was purified by reversed-phase silica gel chromatography (acetonitrile:water=5:95 to acetonitrile). To the obtained crude product was added water, and the mixture was adjusted to pH 6 to 7 with 1N aqueous hydrochloric acid solution, and extracted three times with ethyl acetate. The organic layer was washed with saturated aqueous sodium chloride solution, and dried over sodium sulfate. Sodium sulfate was filtered off, and the filtrate was concentrated under reduced pressure to give a crude product (163 mg) of the title compound.

### Step 17

### 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)bicyclo[2.1.1]hexane-1-carboxamide

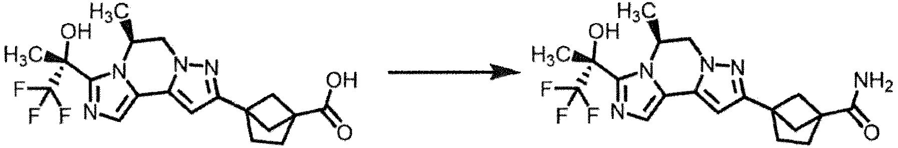

The crude product (161 mg) of 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)bicyclo[2.1.1]hexane-1-carboxylic acid obtained in the previous step was mixed with DMF (1.633 ml). Under ice-cooling, 7 M ammonia/methanol solution (0.171 ml) and 1-[bis(dimethylamino)methylene]-1H-1,2,3-triazolo[4,5-b]pyridinium 3-oxide hexafluorophosphate (227 mg) were added, and the mixture was stirred at room temperature overnight. Under ice-cooling, 7 M ammonia/methanol solution (0.085 ml) and 1-[bis(dimethylamino)methylene]-1H-1,2,3-triazolo[4,5-b]pyridinium 3-oxide hexafluorophosphate (75.7 mg) were added, and the mixture was stirred at room temperature for 3 hr. The reaction mixture was purified by reversed-phase silica gel chromatography (acetonitrile:water=5:95 to acetonitrile) to give the title compound (95 mg).
¹H-NMR (400 MHz, DMSO-D₆) 1.23 (d, J=6.47Hz, 3H), 1.57 - 1.61 (m, 2H), 1.80 (s, 3H), 1.84 - 1.90 (m, 2H), 1.90 - 1.97 (m, 4H), 4.23 (dd, J=13.64, 3.93Hz, 1H), 4.35 (d, J=13.64Hz, 1H), 5.27 - 5.37 (m, 1H), 6.42 (s, 1H), 6.86 (brs, 1H), 7.14 (brs, 1H), 7.23 (s, 1H), 7.25 (s, 1H)

### Step 18

### 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)bicyclo[2.1.1]hexane-1-carbonitrile

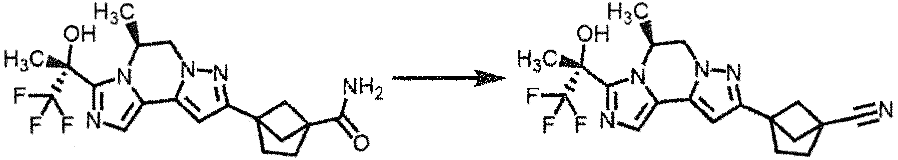

4-((S)-5-Methyl-3-((R)-1,1,1-trifluoro-2-hydroxypropan-2-yl)-5,6-dihydroimidazo[1,5-a]pyrazolo[5,1-c]pyrazin-9-yl)bicyclo[2.1.1]hexane-1-carboxamide (95 mg) obtained in the previous step was mixed with tetrahydrofuran (0.886 ml). Under ice-cooling, triethylamine (0.151 ml) and trifluoroacetic anhydride (0.0459 ml) were added, and the mixture was stirred for 30 min. Under ice-cooling, to the reaction mixture was added trifluoroacetic anhydride (0.015 ml), and the mixture was stirred for 30 min. At room temperature, to the reaction mixture was added saturated aqueous sodium hydrogen carbonate solution, and the mixture was extracted twice with ethyl acetate. The organic layer was washed successively with water and saturated aqueous sodium chloride solution, and dried over sodium sulfate. Sodium sulfate was filtered off, and the filtrate was concentrated under reduced pressure. To the obtained residue was added ethyl acetate/hexane=1/1, and the mixture was stirred at room temperature. The precipitated solid was collected by filtration to give the title compound (79.5 mg).
¹H-NMR (400 MHz, DMSO-D₆) 1.22 (d, J=6.47Hz, 3H), 1.76 - 1.84 (m, 5H), 1.95 - 2.02 (m, 2H), 2.07 - 2.13 (m, 2H), 2.20 - 2.27 (m, 2H), 4.24 (dd, J=13.64, 3.93Hz, 1H), 4.36 (d, J=12.72Hz, 1H), 5.27 - 5.38 (m, 1H), 6.49 (s, 1H), 7.24 (s, 1H), 7.26 (s, 1H)

### Production Example 9-1

### Synthesis of 1-((S)-5-methyl-3-((R)-1,1,1-trifluoro-2-hydroxypropan-2-yl)-5,6-dihydroimidazo[1,5-a]pyrazolo[5,1-c]pyrazin-9-yl)-2-oxabicyclo[2.2.2]octane-4-carbonitrile (Example 93)

### Step 1

### ethyl 1-(hydroxymethyl)-4-methylenecyclohexane-1-carboxylate

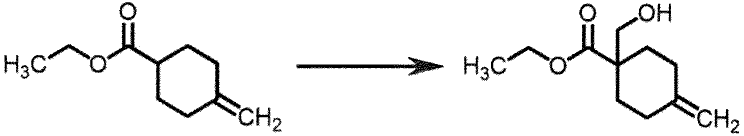

Ethyl 4-methylenecyclohexane-1-carboxylate (23.98 g) was mixed with tetrahydrofuran (480 ml). The mixture was cooled to -78°C, and 2 M lithium diisopropylamide/tetrahydrofuran-heptane-ethylbenzene solution (82 ml) was added dropwise. The mixture was stirred at -78°C for 30 min. To the mixture was added formaldehyde (6.42 g), and the mixture was stirred at - 78°C for 30 min, and then stirred at room temperature for 4 hr. To the reaction mixture was added saturated ammonium chloride aqueous solution, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=4:96 to 70:30) to give the title compound (21.1 g).
¹H-NMR (400 MHz, DMSO-D₆) 1.17 (t, J=7.09Hz, 3H), 1.21 - 1.33 (m, 2H), 1.94 - 2.06 (m, 4H), 2.10 - 2.20 (m, 1H), 3.39 (d, J=5.55Hz, 2H), 4.07 (q, J=7.09Hz, 2H), 4.59 (s, 2H), 4.81 (t, J=5.55Hz, 1H)

### Step 2

### ethyl 1-(iodomethyl)-2-oxabicyclo[2.2.2]octane-4-carboxylate

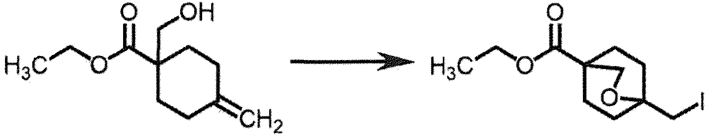

Ethyl 1-(hydroxymethyl)-4-methylenecyclohexane-1-carboxylate (13.65 g) obtained in the previous step was mixed with dichloromethane (341 ml). Under ice-cooling, to the mixture was added dropwise bis(2,4,6-trimethylpyridine)iodonium hexafluorophosphate (37.2 g)/dichloromethane (137 ml) solution, and the mixture was stirred for 1 hr. The reaction mixture was concentrated under reduced pressure, 5% sodium thiosulfate aqueous solution was added, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=5:95 to 31:69) to give the title compound (10.176 g).
¹H-NMR (400 MHz, CDCl₃) 1.24 (t, J=7.11Hz, 3H), 1.68 - 1.83 (m, 2H), 1.90 - 2.06 (m, 6H), 3.16 (s, 2H), 4.00 (s, 2H), 4.12 (q, J=7.11Hz, 2H)

### Step 3

### ethyl 1-(acetoxymethyl)-2-oxabicyclo[2.2.2]octane-4-carboxylate

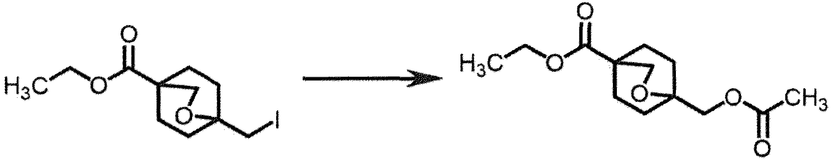

Ethyl 1-(iodomethyl)-2-oxabicyclo[2.2.2]octane-4-carboxylate (10.176 g) obtained in the previous step, potassium acetate (9.24 g), and 18-crown-6 (1.659 g) were mixed with dimethyl sulfoxide (50.9 ml), and the mixture was stirred at 110°C for 2 days and at 120°C for 1 day. At room temperature, to the reaction mixture was added saturated ammonium chloride aqueous solution, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=8:92 to 48:52) to give the title compound (7.3 g).
¹H-NMR (400 MHz, CDCl₃) 1.22 (t, J=7.09Hz, 3H), 1.53 - 1.64 (m, 2H), 1.86 - 2.03 (m, 6H), 2.08 (s, 3H), 3.93 (s, 2H), 3.98 (s, 2H), 4.10 (q, J=7.09Hz, 2H)

### Step 4

### ethyl 1-(hydroxymethyl)-2-oxabicyclo[2.2.2]octane-4-carboxylate

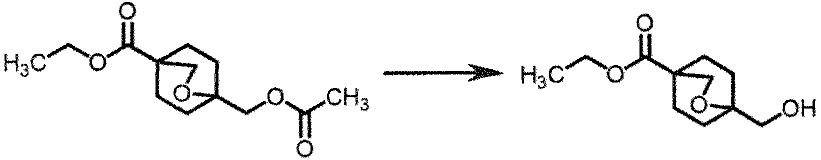

Ethyl 1-(acetoxymethyl)-2-oxabicyclo[2.2.2]octane-4-carboxylate (7.3 g) obtained in the previous step was mixed with ethanol (51.1 ml). Under ice-cooling, to the mixture was added dropwise 20% sodium ethoxide/ethanol solution (22.03 ml), and the mixture was stirred at room temperature for 5 hr. The reaction mixture was concentrated under reduced pressure, saturated ammonium chloride aqueous solution was added, and the mixture was extracted with ethyl acetate. The organic layer was washed with saturated aqueous sodium chloride solution, and dried over sodium sulfate. Sodium sulfate was filtered off, and the filtrate was concentrated under reduced pressure to give the title compound (6.0 g).
¹H-NMR (400 MHz, CDCl₃) 1.24 (t, J=7.11Hz, 3H), 1.46 - 1.63 (m, 2H), 1.84 (t, J=6.73Hz, 1H), 1.88 - 2.04 (m, 6H), 3.40 (d, J=6.73Hz, 2H), 3.98 (s, 2H), 4.12 (q, J=7.11Hz, 2H)

### Step 5

### ethyl 1-((benzyloxy)methyl)-2-oxabicyclo[2.2.2]octane-4-carboxylate

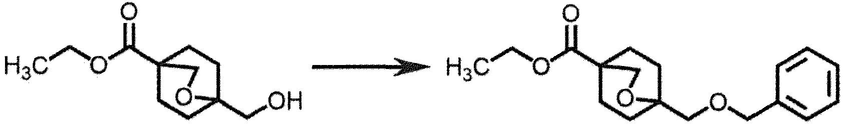

Ethyl 1-(hydroxymethyl)-2-oxabicyclo[2.2.2]octane-4-carboxylate (6.0 g) obtained in the previous step was mixed with tetrahydrofuran (60 ml). Under ice-cooling, to the mixture were added benzyl bromide (6.71 g) and potassium tert-butoxide (4.09 g), and the mixture was stirred for 1 hr. To the reaction mixture was added saturated ammonium chloride aqueous solution, and the mixture was extracted with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=5:95 to 24:76) to give the title compound (7.21 g).
¹H-NMR (400 MHz, CDCl₃) 1.22 (t, J=7.17Hz, 3H), 1.55 - 1.68 (m, 2H), 1.84 - 2.02 (m, 6H), 3.27 (s, 2H), 3.98 (s, 2H), 4.09 (q, J=7.17Hz, 2H), 4.55 (s, 2H), 7.23 - 7.35 (m, 5H)

### Step 6

### (1-((benzyloxy)methyl)-2-oxabicyclo[2.2.2]octan-4-yl)methanol

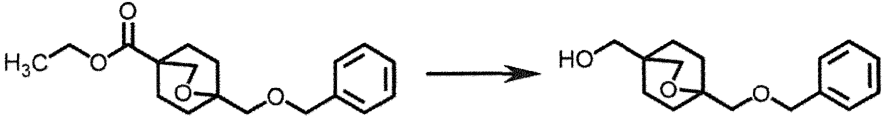

Ethyl 1-((benzyloxy)methyl)-2-oxabicyclo[2.2.2]octane-4-carboxylate (3.2 g) obtained in the previous step was mixed with tetrahydrofuran (32 ml). Under ice-cooling, to the mixture was added dropwise 2 M lithium aluminum hydride/tetrahydrofuran solution (6.31 ml), and the mixture was stirred for 30 min. Under ice-cooling, to the reaction mixture were successively added dropwise water (0.48 ml), 4N sodium hydroxide aqueous solution (0.48 ml), and water (1.44 ml), and the mixture was stirred at room temperature for 1 hr. Solid was filtered off using celite as an aid, and the filtrate was concentrated under reduced pressure to give a crude product (2.82 g) of the title compound.

### Step 7

### 1-((benzyloxy)methyl)-4-(((tetrahydro-2H-pyran-2-yl)oxy)methyl)-2-oxabicyclo[2.2.2]octane

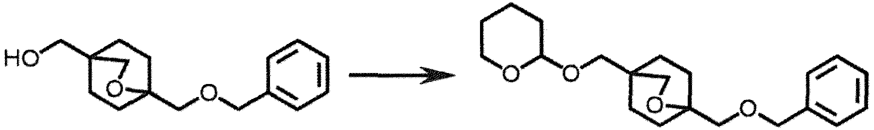

The crude product (2.76 g) of (1-((benzyloxy)methyl)-2-oxabicyclo[2.2.2]octan-4-yl)methanol obtained in the previous step was mixed with acetonitrile (27.6 ml). To the mixture were added pyridinium p-toluenesulfonate (0.528 g) and 3,4-dihydro-2H-pyran (1.061 g), and the mixture was stirred at room temperature overnight. The reaction mixture was concentrated under reduced pressure, and the obtained residue was purified by silica gel column chromatography (ethyl acetate:hexane=2:98 to 20:80) to give the title compound (3.575 g).
¹H-NMR (400 MHz, CDCl₃) 1.40 - 1.97 (m, 14H), 2.98 (d, J=9.71Hz, 1H), 3.26 (s, 2H), 3.40 - 3.52 (m, 2H), 3.72 - 3.83 (m, 3H), 4.49 (t, J=3.24Hz, 1H), 4.55 (s, 2H), 7.22 - 7.33 (m, 5H)

### Step 8

### (4-(((tetrahydro-2H-pyran-2-yl)oxy)methyl)-2-oxabicyclo[2.2.2]octan-1-yl)methanol

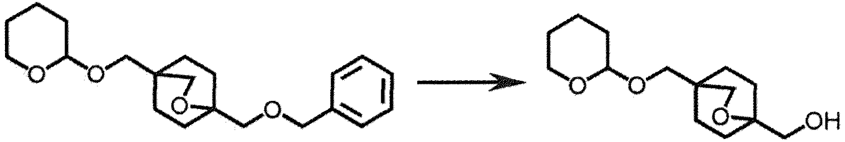

1-((Benzyloxy)methyl)-4-(((tetrahydro-2H-pyran-2-yl)oxy)methyl)-2-oxabicyclo[2.2.2]octane (3.275 g) obtained in the previous step was mixed with tetrahydrofuran (16.38 ml) and ethanol (16.38 ml). To the mixture was added palladium carbon (0.983 g) and the mixture was stirred overnight under a hydrogen atmosphere at room temperature. Palladium carbon was filtered off from the reaction mixture by using celite as an aid, and the filtrate was concentrated under reduced pressure to give a crude product (2.1 g) of the title compound.

### Step 9

### 4-(((tetrahydro-2H-pyran-2-yl)oxy)methyl)-2-oxabicyclo[2.2.2]octane-1-carbaldehyde

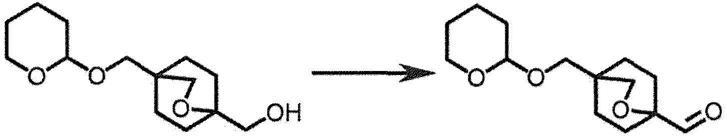

The crude product (2.1 g) containing (4-(((tetrahydro-2H-pyran-2-yl)oxy)methyl)-2-oxabicyclo[2.2.2]octan-1-yl)methanol obtained in the previous step was mixed with chloroform (24.22 ml). Under ice-cooling, to the mixture was added Dess-Martin periodinane (4.81 g), and the mixture was stirred at room temperature overnight. Under ice-cooling, to the reaction mixture was added Dess-Martin periodinane (2.405 g), and the mixture was stirred at room temperature for 4 hr. To the reaction mixture were added 5% sodium thiosulfate aqueous solution and saturated aqueous sodium hydrogen carbonate solution, and the mixture was stirred at room temperature for 1 hr, and extracted twice with ethyl acetate. The organic layer was washed successively with 10% potassium carbonate aqueous solution, saturated aqueous sodium hydrogen carbonate solution, and saturated aqueous sodium chloride solution, and dried over magnesium sulfate. Magnesium sulfate was filtered off, and the filtrate was concentrated under reduced pressure to give the title compound (1.277 g).
¹H-NMR (400 MHz, CDCl₃) 1.42 - 2.00 (m, 14H), 3.01 (d, J=9.71Hz, 1H), 3.43 - 3.52 (m, 2H), 3.72 - 3.82 (m, 1H), 3.86 (s, 2H), 4.46 - 4.53 (m, 1H), 9.57 (s, 1H)

### Step 10

### 1-(4-(((tetrahydro-2H-pyran-2-yl)oxy)methyl)-2-oxabicyclo[2.2.2]octan-1-yl)ethan-1-ol

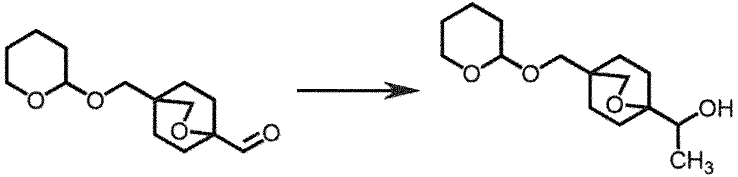

4-(((Tetrahydro-2H-pyran-2-yl)oxy)methyl)-2-oxabicyclo[2.2.2]octane-1-carbaldehyde (1.277 g) obtained in the previous step was mixed with tetrahydrofuran (12.39 ml). Under ice-cooling, to the mixture was added dropwise 1 M methylmagnesium bromide/tetrahydrofuran solution (7.02 ml), and the mixture was stirred for 30 min. Under ice-cooling, to the reaction mixture was added saturated ammonium chloride aqueous solution, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=12:88 to ethyl acetate) to give the title compound (863.9 mg).
¹H-NMR (400 MHz, CDCl₃) 1.04 (d, J=6.70Hz, 3H), 1.42 - 2.00 (m, 14H), 2.40 (d, J=2.77Hz, 1H), 2.96 - 3.01 (m, 1H), 3.41 - 3.52 (m, 2H), 3.53 - 3.62 (m, 1H), 3.72 - 3.82 (m, 3H), 4.47 - 4.51 (m, 1H)

### Step 11

### 1-(4-(((tetrahydro-2H-pyran-2-yl)oxy)methyl)-2-oxabicyclo[2.2.2]octan-1-yl)ethan-1-one

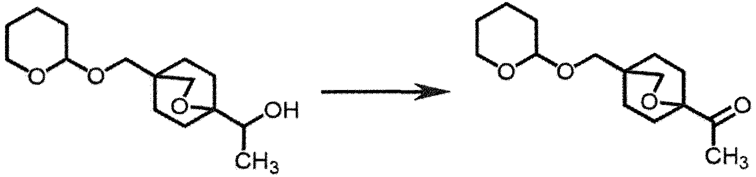

1-(4-(((Tetrahydro-2H-pyran-2-yl)oxy)methyl)-2-oxabicyclo[2.2.2]octan-1-yl)ethan-1-ol (863.9 mg) obtained in the previous step was mixed with chloroform (8.207 ml). Under ice-cooling, to the mixture was added Dess-Martin periodinane (1.931 g), and the mixture was stirred at room temperature overnight. Under ice-cooling, to the reaction mixture were added sodium thiosulfate aqueous solution and saturated aqueous sodium hydrogen carbonate solution added, and the mixture was stirred at room temperature for 30 min, and extracted twice with ethyl acetate. The organic layer was washed successively with 10% potassium carbonate aqueous solution, saturated aqueous sodium hydrogen carbonate solution, and saturated aqueous sodium chloride solution, and dried over sodium sulfate. Sodium sulfate was filtered off, and the filtrate was concentrated under reduced pressure and azeotroped with toluene to give the title compound (851 mg).
¹H-NMR (400 MHz, CDCl₃) 1.42 - 1.93 (m, 14H), 2.18 (s, 3H), 3.00 (d, J=9.71Hz, 1H), 3.42 - 3.53 (m, 2H), 3.71 - 3.88 (m, 3H), 4.47 - 4.52 (m, 1H)

### Step 12

### ethyl 2,4-dioxo-4-(4-(((tetrahydro-2H-pyran-2-yl)oxy)methyl)-2-oxabicyclo[2.2.2]octan-1-yl)butanoate

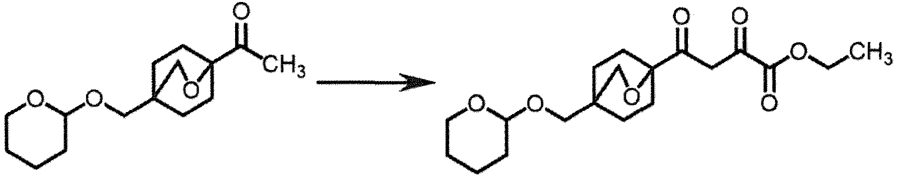

1-(4-(((Tetrahydro-2H-pyran-2-yl)oxy)methyl)-2-oxabicyclo[2.2.2]octan-1-yl)ethan-1-one (851 mg) obtained in the previous step was mixed with tetrahydrofuran (8.17 ml). Under ice-cooling, to the mixture was added diethyl oxalate (489 mg), 1 M potassium tert-butoxide/tetrahydrofuran solution (3.653 ml) was added dropwise, and the mixture was stirred for 30 min. Under ice-cooling, to the reaction mixture was added 1 M aqueous hydrochloric acid solution (21.3 ml), and the mixture was extracted with ethyl acetate. The organic layer was washed with saturated aqueous sodium chloride solution, and dried over sodium sulfate. Sodium sulfate was filtered off, and the filtrate was concentrated under reduced pressure and azeotroped with ethanol to give a crude product (1.168 g) of the title compound.

### Step 13

### ethyl 3-(4-(((tetrahydro-2H-pyran-2-yl)oxy)methyl)-2-oxabicyclo[2.2.2]octan-1-yl)-1H-pyrazole-5-carboxylate

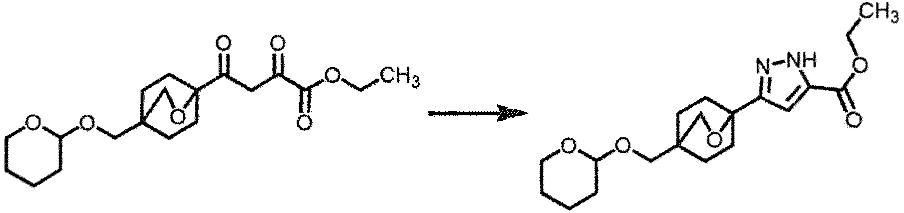

Ethyl 2,4-dioxo-4-(4-(((tetrahydro-2H-pyran-2-yl)oxy)methyl)-2-oxabicyclo[2.2.2]octan-1-yl)butanoate (1.168 g) obtained in the previous step was mixed with ethanol (11.2 ml). Under ice-cooling, hydrazine monohydrate (0.155 ml) was added dropwise, and the mixture was stirred at room temperature overnight. The reaction mixture was concentrated under reduced pressure. The obtained residue was purified by silica gel column chromatography (ethyl acetate:hexane=12:88 to ethyl acetate) to give the title compound (978.4 mg). ¹H-NMR (400 MHz, CDCl₃) 1.37 (t, J=7.09Hz, 3H), 1.44 - 1.90 (m, 10H), 1.93 - 2.04 (m, 2H), 2.04 - 2.17 (m, 2H), 3.04 (d, J=9.71Hz, 1H), 3.44 - 3.54 (m, 2H), 3.73 - 3.83 (m, 1H), 3.92 (s, 2H), 4.36 (q, J=7.09Hz, 2H), 4.48 - 4.55 (m, 1H), 6.60 (s, 1H)

### Step 14

### (3-(4-(((tetrahydro-2H-pyran-2-yl)oxy)methyl)-2-oxabicyclo[2.2.2]octan-1-yl)-1H-pyrazol-5-yl)methanol

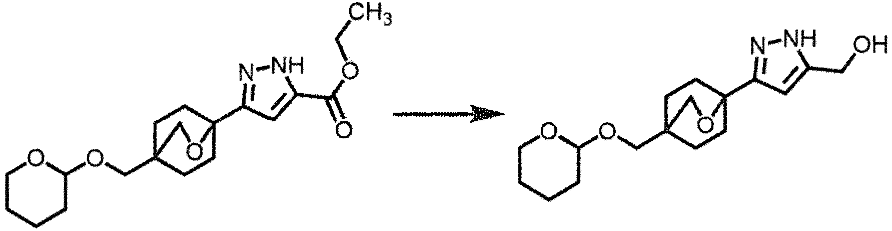

Ethyl 3-(4-(((tetrahydro-2H-pyran-2-yl)oxy)methyl)-2-oxabicyclo[2.2.2]octan-1-yl)-1H-pyrazole-5-carboxylate (978.4 mg) obtained in the previous step was mixed with tetrahydrofuran (14 ml). Under ice-cooling, to the mixture was added dropwise 2 M lithium aluminum hydride/tetrahydrofuran solution (2.255 ml), and the mixture was stirred for 50 min. Under ice-cooling, to the reaction mixture was added saturated Rochelle salt aqueous solution, and the mixture was stirred at room temperature for 1 hr, and extracted with ethyl acetate. The organic layer was washed with saturated aqueous sodium chloride solution, and dried over sodium sulfate. Sodium sulfate was filtered off, and the filtrate was concentrated under reduced pressure to give a crude product (769.6 mg) of the title compound.

### Step 15

### 3-(4-(((tetrahydro-2H-pyran-2-yl)oxy)methyl)-2-oxabicyclo[2.2.2]octan-1-yl)-1H-pyrazole-5-carbaldehyde

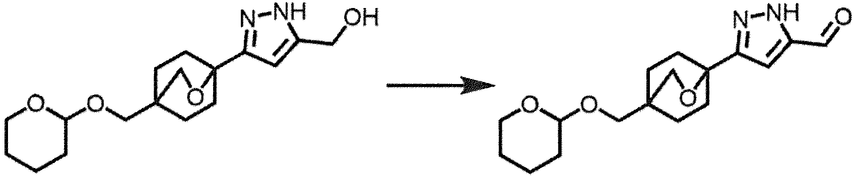

(3-(4-(((Tetrahydro-2H-pyran-2-yl)oxy)methyl)-2-oxabicyclo[2.2.2]octan-1-yl)-1H-pyrazol-5-yl)methanol (769.6 mg) obtained in the previous step was mixed with 1,2-dimethoxyethane (23.088 ml). Manganese dioxide (2.309 g) was added, and the mixture was stirred at room temperature overnight. The reaction mixture was filtered through celite, and the filtrate was concentrated under reduced pressure to give a crude product (617.8 mg) of the title compound.

### Step 16

### (2S)-2-(5-(3-(4-(((tetrahydro-2H-pyran-2-yl)oxy)methyl)-2-oxabicyclo[2.2.2]octan-1-yl)-1H-pyrazol-5-yl)-1H-imidazol-1-yl)propan-1-ol

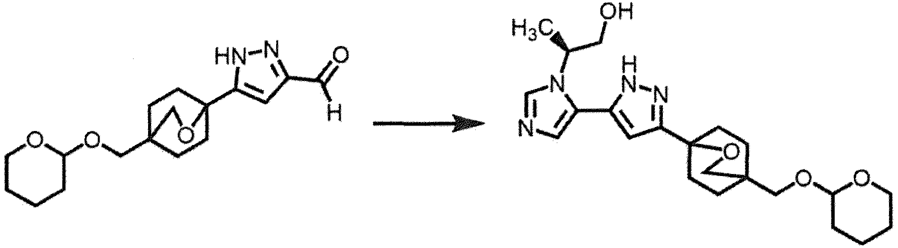

The crude product (617.8 mg) of 3-(4-(((tetrahydro-2H-pyran-2-yl)oxy)methyl)-2-oxabicyclo[2.2.2]octan-1-yl)-1H-pyrazole-5-carbaldehyde obtained in the previous step and (S)-2-aminopropan-1-ol (152 mg) were mixed with 1,2-dimethoxyethane (24.712 ml) and methanol (12.36 ml). The mixture was stirred at 60°C for 2.5 hr. The reaction mixture was concentrated under reduced pressure. To the obtained residue were successively added DMF (6.18 ml), potassium carbonate (799 mg), and p-toluenesulfonylmethyl isocyanide (565 mg), and the mixture was stirred at room temperature for 3 days. At room temperature, water was added to the reaction mixture, and the mixture was extracted three times with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:methanol=2:98 to 50:50) to give a crude product (550 mg) of the title compound.

### Step 17

### (5S)-5-methyl-9-(4-(((tetrahydro-2H-pyran-2-yl)oxy)methyl)-2-oxabicyclo[2.2.2]octan-1-yl)-5,6-dihydroimidazo[1,5-a]pyrazolo[5,1-c]pyrazine

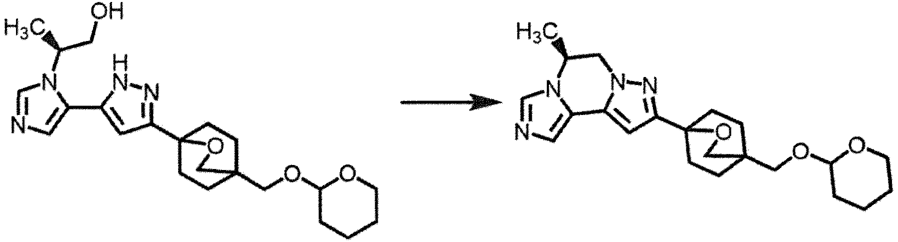

(2S)-2-(5-(3-(4-(((Tetrahydro-2H-pyran-2-yl)oxy)methyl)-2-oxabicyclo[2.2.2]octan-1-yl)-1H-pyrazol-5-yl)-1H-imidazol-1-yl)propan-1-ol (0.55 g) obtained in the previous step and triphenylphosphine (382 mg) were mixed with tetrahydrofuran (15.352 ml). Under ice-cooling, diisopropyl azodicarboxylate (295 mg) was added dropwise, and the mixture was stirred at room temperature for 1 hr. Under ice-cooling, triphenylphosphine (95.6 mg) and diisopropyl azodicarboxylate (73.7 mg) were added, and the mixture was stirred at room temperature for 1 hr. The reaction mixture was purified by silica gel column chromatography (ethyl acetate:methanol=2:98 to 60:40) to give the title compound (520 mg).
¹H-NMR (400 MHz, CDCl₃) 1.45 - 1.73 (m, 10H), 1.75 - 1.91 (m, 3H), 1.97 - 2.08 (m, 2H), 2.20 - 2.32 (m, 2H), 3.04 (d, J=9.71Hz, 1H), 3.45 - 3.53 (m, 2H), 3.75 - 3.84 (m, 1H), 3.94 (s, 2H), 4.09 (dd, J=13.18, 7.86Hz, 1H), 4.44 (dd, J=13.18, 4.39Hz, 1H), 4.49 - 4.62 (m, 2H), 6.35 (s, 1H), 7.25 (s, 1H), 7.58 (s, 1H)

### Step 18

### 1-((5S)-5-methyl-9-(4-(((tetrahydro-2H-pyran-2-yl)oxy)methyl)-2-oxabicyclo[2.2.2]octan-1-yl)-5,6-dihydroimidazo[1,5-a]pyrazolo[5,1-c]pyrazin-3-yl)ethan-1-one

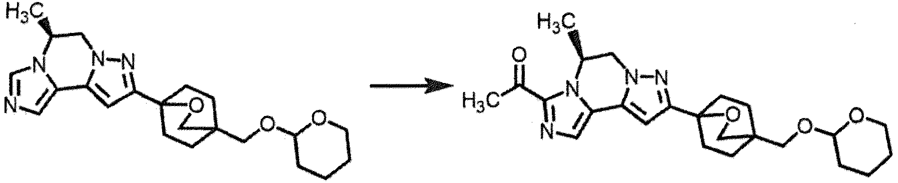

(5S)-5-Methyl-9-(4-(((tetrahydro-2H-pyran-2-yl)oxy)methyl)-2-oxabicyclo[2.2.2]octan-1-yl)-5,6-dihydroimidazo[1,5-a]pyrazolo[5,1-c]pyrazine (520 mg) obtained in the previous step was mixed with tetrahydrofuran (9.36 ml). The mixture was cooled to -78°C, and 2 M lithium diisopropylamide/tetrahydrofuran-heptane-ethylbenzene solution (0.587 ml) was added dropwise. The mixture was stirred at - 78°C for 30 min. To the mixture was added N-methoxy-N-methylacetamide (1.452 ml), and the mixture was stirred at - 78°C for 30 min. To the mixture was added dropwise 2 M lithium diisopropylamide/tetrahydrofuran-heptane-ethylbenzene solution (0.587 ml), and the mixture was stirred at -78°C for 1 hr. To the reaction mixture was added saturated ammonium chloride aqueous solution, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=16:84 to ethyl acetate, thereafter ethyl acetate:methanol=50:50) to give the title compound (356 mg). ¹H-NMR (400 MHz, CDCl₃) 1.36 (d, J=6.70Hz, 3H), 1.44 - 1.94 (m, 10H), 1.98 - 2.10 (m, 2H), 2.20 - 2.32 (m, 2H), 2.67 (s, 3H), 3.05 (d, J=9.71Hz, 1H), 3.45 - 3.54 (m, 2H), 3.75 - 3.84 (m, 1H), 3.95 (s, 2H), 4.29 - 4.42 (m, 2H), 4.49 - 4.54 (m, 1H), 5.73 - 5.85 (m, 1H), 6.49 (s, 1H), 7.33 (s, 1H)

### Step 19

### (2R)-1,1,1-trifluoro-2-((5S)-5-methyl-9-(4-(((tetrahydro-2H-pyran-2-yl)oxy)methyl)-2-oxabicyclo[2.2.2]octan-1-yl)-5,6-dihydroimidazo[1,5-a]pyrazolo[5,1-c]pyrazin-3-yl)propan-2-ol

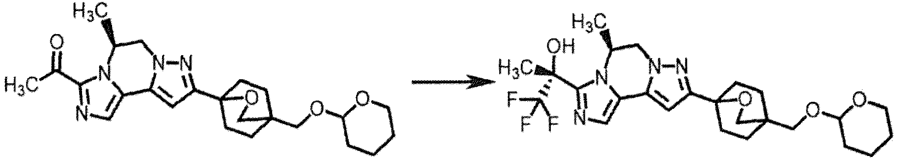

1-((5S)-5-Methyl-9-(4-(((tetrahydro-2H-pyran-2-yl)oxy)methyl)-2-oxabicyclo[2.2.2]octan-1-yl)-5,6-dihydroimidazo[1,5-a]pyrazolo[5,1-c]pyrazin-3-yl)ethan-1-one (356 mg) obtained in the previous step was azeotroped with toluene and mixed with N,N-dimethylacetamide (1.78 ml). Under ice-cooling, to the mixture were added lithium acetate (0.016 g) and trifluoromethyltrimethylsilane (0.966 ml), and the mixture was stirred for 1 hr. Under ice-cooling, methanol (0.642 ml) and potassium carbonate (0.721 g) were added, and the mixture was stirred at room temperature for 1 hr. To the reaction mixture was added water, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=16:84 to ethyl acetate) to give the title compound (317.5 mg).
¹H-NMR (400 MHz, CDCl₃) 1.33 (d, J=6.47Hz, 3H), 1.45 - 1.73 (m, 7H), 1.74 - 1.90 (m, 3H), 1.93 - 2.11 (m, 5H), 2.18 - 2.34 (m, 2H), 3.01 - 3.07 (m, 2H), 3.44 - 3.53 (m, 2H), 3.73 - 3.85 (m, 1H), 3.95 (s, 2H), 4.29 - 4.34 (m, 2H), 4.49 - 4.54 (m, 1H), 5.25 - 5.36 (m, 1H), 6.37 (s, 1H), 7.20 (s, 1H)

### Step 20

### (R)-1,1,1-trifluoro-2-((S)-9-(4-(hydroxymethyl)-2-oxabicyclo[2.2.2]octan-1-yl)-5-methyl-5,6-dihydroimidazo[1,5-a]pyrazolo[5,1-c]pyrazin-3-yl)propan-2-ol

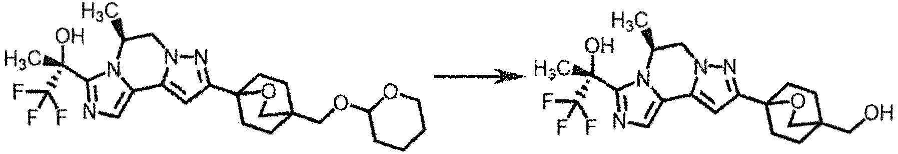

(2R)-1,1,1-Trifluoro-2-((5S)-5-methyl-9-(4-(((tetrahydro-2H-pyran-2-yl)oxy)methyl)-2-oxabicyclo[2.2.2]octan-1-yl)-5,6-dihydroimidazo[1,5-a]pyrazolo[5,1-c]pyrazin-3-yl)propan-2-ol (317.5 mg) obtained in the previous step was mixed with methanol (3.175 ml). To the mixture was added 10-camphorsulfonic acid (28.9 mg), and the mixture was stirred at room temperature overnight. To the reaction mixture was added 10-camphorsulfonic acid (144 mg), and the mixture was stirred at room temperature for 1 hr. To the reaction mixture was added saturated aqueous sodium hydrogen carbonate solution, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (methanol:ethyl acetate=1:99 to 20:80) to give the title compound (269.3 mg).
¹H-NMR (400 MHz, DMSO-D₆) 1.21 (d, J=6.47Hz, 3H), 1.43 - 1.55 (m, 2H), 1.58 - 1.69 (m, 2H), 1.80 (s, 3H), 1.84 - 1.99 (m, 2H), 2.00 - 2.14 (m, 2H), 3.12 (d, J=5.32Hz, 2H), 3.69 (s, 2H), 4.22 (dd, J=13.52, 4.05Hz, 1H), 4.33 (d, J=12.72Hz, 1H), 4.45 - 4.52 (m, 1H), 5.26 - 5.37 (m, 1H), 6.43 (s, 1H), 7.23 (s, 1H), 7.24 (s, 1H)

### Step 21

### 1-((S)-5-methyl-3-((R)-1,1,1-trifluoro-2-hydroxypropan-2-yl)-5,6-dihydroimidazo[1,5-a]pyrazolo[5,1-c]pyrazin-9-yl)-2-oxabicyclo[2.2.2]octane-4-carbaldehyde

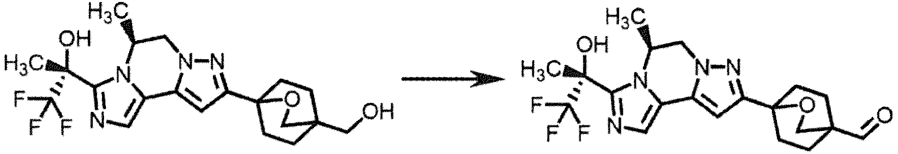

(R)-1,1,1-Trifluoro-2-((S)-9-(4-(hydroxymethyl)-2-oxabicyclo[2.2.2]octan-1-yl)-5-methyl-5,6-dihydroimidazo[1,5-a]pyrazolo[5,1-c]pyrazin-3-yl)propan-2-ol (135 mg) obtained in the previous step was mixed with chloroform (3.698 ml) and acetonitrile (1.849 ml). Under ice-cooling, Dess-Martin periodinane (147 mg) was added, and the mixture was stirred at room temperature overnight. 5% Sodium thiosulfate aqueous solution and saturated aqueous sodium hydrogen carbonate solution were added, and the mixture was stirred at room temperature for 1 hr, and extracted twice with ethyl acetate. The organic layer was washed successively with 10% potassium carbonate aqueous solution, saturated aqueous sodium hydrogen carbonate solution, and saturated aqueous sodium chloride solution, and dried over sodium sulfate. Sodium sulfate was filtered off, and the filtrate was concentrated under reduced pressure to give the title compound (124.6 mg).
¹H-NMR (400 MHz, CDCl₃) 1.33 (d, J=6.47Hz, 3H), 1.85 - 2.15 (m, 9H), 2.26 - 2.42 (m, 2H), 3.10 (s, 1H), 4.09 - 4.12 (m, 2H), 4.27 - 4.38 (m, 2H), 5.27 - 5.39 (m, 1H), 6.37 (s, 1H), 7.21 (s, 1H), 9.52 (s, 1H)

### Step 22

### 1-((S)-5-methyl-3-((R)-1,1,1-trifluoro-2-hydroxypropan-2-yl)-5,6-dihydroimidazo[1,5-a]pyrazolo[5,1-c]pyrazin-9-yl)-2-oxabicyclo[2.2.2]octane-4-carboxylic acid

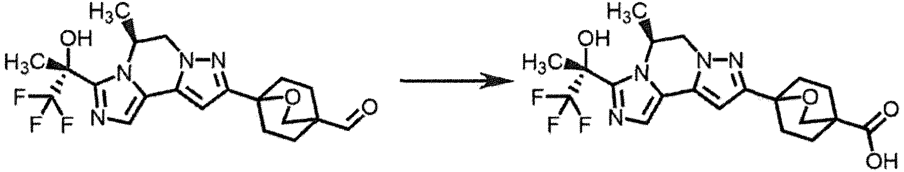

1-((S)-5-Methyl-3-((R)-1,1,1-trifluoro-2-hydroxypropan-2-yl)-5,6-dihydroimidazo[1,5-a]pyrazolo[5,1-c]pyrazin-9-yl)-2-oxabicyclo[2.2.2]octane-4-carbaldehyde (114.6 mg) obtained in the previous step was mixed with tert-butanol (0.871 ml) and water (0.435 ml). 2-Methyl-2-butene (0.272 ml), sodium dihydrogen phosphate (154 mg), and sodium chlorite (87 mg) were added, and the mixture was stirred at room temperature for 2 hr. Using the starting material (10 mg), a similar reaction was performed. The reaction mixtures were combined, 5% sodium thiosulfate aqueous solution was added, and the mixture was extracted three times with ethyl acetate. The organic layer was dried over sodium sulfate. Sodium sulfate was filtered off, and the filtrate was concentrated under reduced pressure to give a crude product (113 mg) of the title compound.

### Step 23

### 1-((S)-5-methyl-3-((R)-1,1,1-trifluoro-2-hydroxypropan-2-yl)-5,6-dihydroimidazo[1,5-a]pyrazolo[5,1-c]pyrazin-9-yl)-2-oxabicyclo[2.2.2]octane-4-carboxamide

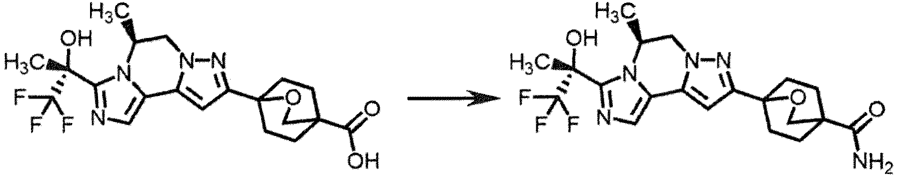

The crude product (113 mg) of 1-((S)-5-methyl-3-((R)-1,1,1-trifluoro-2-hydroxypropan-2-yl)-5,6-dihydroimidazo[1,5-a]pyrazolo[5,1-c]pyrazin-9-yl)-2-oxabicyclo[2.2.2]octane-4-carboxylic acid obtained in the previous step was mixed with DMF (1.273 ml). Under ice-cooling, 7 M ammonia/methanol solution (0.124 ml) and 1-[bis(dimethylamino)methylene]-1H-1,2,3-triazolo[4,5-b]pyridinium 3-oxide hexafluorophosphate (165 mg) were added, and the mixture was stirred at room temperature overnight. Under ice-cooling, to the reaction mixture were added 7 M ammonia/methanol solution (0.124 ml) and 1-[bis(dimethylamino)methylene]-1H-1,2,3-triazolo[4,5-b]pyridinium 3-oxide hexafluorophosphate (165 mg), and the mixture was stirred at room temperature for 3 hr. The reaction mixture was purified by reversed-phase chromatography (acetonitrile:water=5:95 to acetonitrile) to give the title compound (104.1 mg).
¹H-NMR (400 MHz, DMSO-D₆) 1.21 (d, J=6.47Hz, 3H), 1.80 (s, 3H), 1.85 - 1.99 (m, 6H), 2.03 - 2.20 (m, 2H), 3.88 (s, 2H), 4.23 (dd, J=13.18, 4.05Hz, 1H), 4.33 (d, J=13.18Hz, 1H), 5.26 - 5.39 (m, 1H), 6.45 (s, 1H), 6.92 (brs, 1H), 7.12 (brs, 1H), 7.23 (s, 1H), 7.25 (s, 1H)

### Step 24

### 1-((S)-5-methyl-3-((R)-1,1,1-trifluoro-2-hydroxypropan-2-yl)-5,6-dihydroimidazo[1,5-a]pyrazolo[5,1-c]pyrazin-9-yl)-2-oxabicyclo[2.2.2]octane-4-carbonitrile

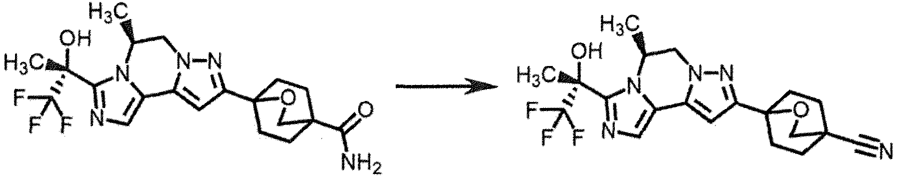

1-((S)-5-Methyl-3-((R)-1,1,1-trifluoro-2-hydroxypropan-2-yl)-5,6-dihydroimidazo[1,5-a]pyrazolo[5,1-c]pyrazin-9-yl)-2-oxabicyclo[2.2.2]octane-4-carboxamide (104.1 mg) obtained in the previous step was mixed with tetrahydrofuran (0.958 ml). Under ice-cooling, triethylamine (0.152 ml) and trifluoroacetic anhydride (0.0462 ml) were added, and the mixture was stirred for 30 min. Under ice-cooling, to the reaction mixture was added trifluoroacetic anhydride (0.0154 ml), and the mixture was stirred for 30 min. At room temperature, to the reaction mixture was added saturated aqueous sodium hydrogen carbonate solution, and the mixture was extracted twice with ethyl acetate. The organic layer was washed successively with water and saturated aqueous sodium chloride solution, and dried over sodium sulfate. Sodium sulfate was filtered off, and the filtrate was concentrated under reduced pressure. To the obtained residue was added ethyl acetate/hexane=1/1, and the mixture was stirred at room temperature. The precipitated solid was collected by filtration to give the title compound (68.4 mg).
¹H-NMR (400 MHz, DMSO-D₆) 1.20 (d, J=6.70Hz, 3H), 1.80 (s, 3H), 1.91 - 2.04 (m, 2H), 2.06 - 2.29 (m, 6H), 4.05 (s, 2H), 4.23 (dd, J=13.52, 4.05Hz, 1H), 4.34 (d, J=13.52Hz, 1H), 5.28 - 5.38 (m, 1H), 6.46 (s, 1H), 7.24 (s, 1H), 7.25 (s, 1H)

### Production Example 9-2

### Synthesis of (R)-1,1,1-trifluoro-2-((S)-9-(4-(2-hydroxypropan-2-yl)-2-oxabicyclo[2.2.2]octan-1-yl)-5-methyl-5,6-dihydroimidazo[1,5-a]pyrazolo[5,1-c]pyrazin-3-yl)propan-2-ol

### (Example 98)

### Step 1

### methyl 1-((S)-5-methyl-3-((R)-1,1,1-trifluoro-2-hydroxypropan-2-yl)-5,6-dihydroimidazo[1,5-a]pyrazolo[5,1-c]pyrazin-9-yl)-2-oxabicyclo[2.2.2]octane-4-carboxylate

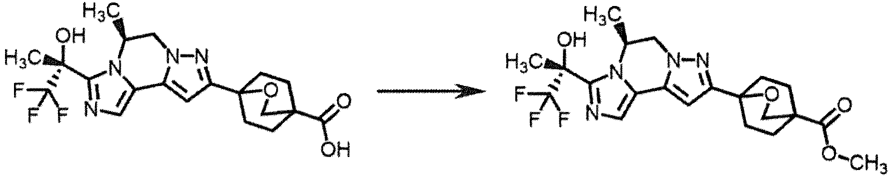

The crude product (133 mg) of 1-((S)-5-methyl-3-((R)-1,1,1-trifluoro-2-hydroxypropan-2-yl)-5,6-dihydroimidazo[1,5-a]pyrazolo[5,1-c]pyrazin-9-yl)-2-oxabicyclo[2.2.2]octane-4-carboxylic acid was mixed with methanol (3 ml). To the mixture was added dropwise 0.6 M trimethylsilyldiazomethane/hexane solution (0.675 ml) 3 times, and the mixture was stirred at room temperature for 30 min. The reaction mixture was concentrated under reduced pressure. The obtained residue was purified by silica gel column chromatography (ethyl acetate:hexane=24:76 to ethyl acetate) to give the title compound (114.2 mg).
¹H-NMR (400 MHz, DMSO-D₆) 1.21 (d, J=6.47Hz, 3H), 1.80 (s, 3H), 1.90 - 2.23 (m, 8H), 3.61 (s, 3H), 3.94 (s, 2H), 4.23 (dd, J=13.64, 4.16Hz, 1H), 4.34 (d, J=13.64Hz, 1H), 5.26 - 5.38 (m, 1H), 6.46 (s, 1H), 7.24 (s, 1H), 7.25 (s, 1H)

### Step 2

### (R)-1,1,1-trifluoro-2-((S)-9-(4-(2-hydroxypropan-2-yl)-2-oxabicyclo[2.2.2]octan-1-yl)-5-methyl-5,6-dihydroimidazo[1,5-a]pyrazolo[5,1-c]pyrazin-3-yl)propan-2-ol

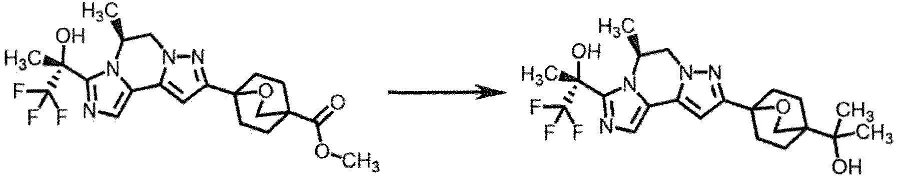

Methyl 1-((S)-5-methyl-3-((R)-1,1,1-trifluoro-2-hydroxypropan-2-yl)-5,6-dihydroimidazo[1,5-a]pyrazolo[5,1-c]pyrazin-9-yl)-2-oxabicyclo[2.2.2]octane-4-carboxylate (30 mg) was mixed with tetrahydrofuran (1.8 ml). Under ice-cooling, 1.04 M methylmagnesium bromide/tetrahydrofuran solution (0.317 ml) was added dropwise, and the mixture was stirred for 1 hr, and then stirred at room temperature for 3 hr. At room temperature, saturated ammonium chloride aqueous solution was added, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. The obtained residue was mixed with tetrahydrofuran (1.8 ml). At room temperature, 1.04 M methylmagnesium bromide/tetrahydrofuran solution (0.66 ml) was added dropwise, and the mixture was stirred for 2 hr. At room temperature, saturated ammonium chloride aqueous solution was added, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. The obtained residue was purified by preparative thin layer chromatography (ethyl acetate) to give the title compound (22.1 mg).
¹H-NMR (400 MHz, DMSO-D₆) 1.01 (s, 6H), 1.21 (d, J=6.47Hz, 3H), 1.53 - 1.65 (m, 2H), 1.67 - 1.94 (m, 7H), 1.98 - 2.12 (m, 2H), 3.80 (d, J=6.24Hz, 2H), 4.08 (s, 1H), 4.22 (dd, J=13.76, 4.05Hz, 1H), 4.33 (d, J=13.76Hz, 1H), 5.27 - 5.37 (m, 1H), 6.43 (s, 1H), 7.23 (s, 1H), 7.24 (s, 1H)

### Production Example 10-1

### Synthesis of 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)-2-oxabicyclo[2.2.2]octane-1-carbonitrile

### (Example 88)

### Step 1

### 1-((benzyloxy)methyl)-2-oxabicyclo[2.2.2]octane-4-carboxylic acid

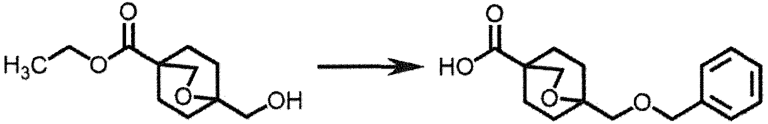

Ethyl 1-(hydroxymethyl)-2-oxabicyclo[2.2.2]octane-4-carboxylate (4.19 g) obtained in the previous step was mixed with tetrahydrofuran (30.8 ml). Under ice-cooling, to the mixture were added potassium tert-butoxide (2.624 g) and benzyl bromide (4.308 g), and the mixture was stirred for 1 hr. To the reaction mixture were added ethanol (30.8 ml) and 2N sodium hydroxide aqueous solution (27 ml), and the mixture was stirred at room temperature overnight. The reaction mixture was concentrated under reduced pressure, diisopropyl ether was added and the layers were separated. The organic layer was washed with 1N sodium hydroxide aqueous solution. The aqueous layers were combined, adjusted to pH 2 with 6N hydrochloric acid, and extracted three times with ethyl acetate. The organic layer was washed with saturated aqueous sodium chloride solution, and dried over sodium sulfate. Sodium sulfate was filtered off, and the filtrate was concentrated under reduced pressure to give the title compound (3.9 g). ¹H-NMR (400 MHz, DMSO-D₆) 1.53 - 1.93 (m, 8H), 3.23 (s, 2H), 3.79 (s, 2H), 4.47 (s, 2H), 7.21 - 7.41 (m, 5H), 12.37 (brs, 1H)

### Step 2

### 1-((benzyloxy)methyl)-N-methoxy-N-methyl-2-oxabicyclo[2.2.2]octane-4-carboxamide

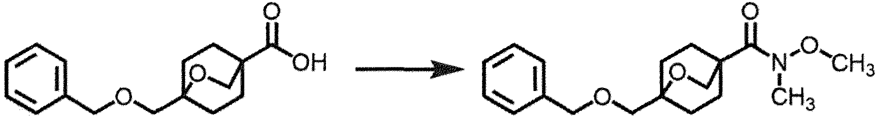

1-((Benzyloxy)methyl)-2-oxabicyclo[2.2.2]octane-4-carboxylic acid (2.5 g) was mixed with pyridine (12.5 ml). To the mixture were added 1-ethyl-3-(3-dimethylaminopropyl)carbodiimide hydrochloride (2.08 g) and N,O-dimethylhydroxylamine hydrochloride (1.059 g), and the mixture was stirred at room temperature for 16 hr. At room temperature, to the reaction mixture were added silica gel (30 g) and ethyl acetate. Silica gel was filtered off and eluted with ethyl acetate. The filtrate was concentrated under reduced pressure. The obtained residue was purified by silica gel column chromatography (hexane:ethyl acetate=100:0 to 0:100) to give the title compound (2.64 g).
¹H-NMR (400 MHz, CDCl₃) 1.51 - 2.14 (m, 8H), 3.13 (s, 3H), 3.27 (s, 2H), 3.65 (s, 3H), 4.10 (s, 2H), 4.55 (s, 2H), 7.25 - 7.34 (m, 5H)

### Step 3

### 1-(1-((benzyloxy)methyl)-2-oxabicyclo[2.2.2]octan-4-yl)ethan-1-one

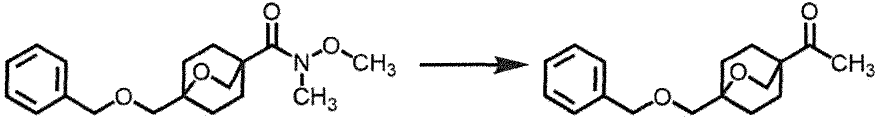

Under an argon atmosphere, 1-((benzyloxy)methyl)-N-methoxy-N-methyl-2-oxabicyclo[2.2.2]octane-4-carboxamide (2.64 g) obtained in the previous step was mixed with tetrahydrofuran (26.4 ml). The mixture was ice-cooled, 1.04 M methylmagnesium bromide/tetrahydrofuran solution (9.54 ml) was added dropwise, and the mixture was stirred for 80 min. Saturated ammonium chloride aqueous solution was added. The reaction mixture was extracted with ethyl acetate. The organic layer was washed with saturated aqueous sodium chloride solution, and sodium sulfate was added. Sodium sulfate was filtered off, and the filtrate was concentrated under reduced pressure. The obtained residue was purified by silica gel column chromatography (hexane:ethyl acetate=100:0 to 0:100) to give the title compound (1.62 g).
¹H-NMR (400 MHz, CDCl₃) 1.60 - 1.69 (m, 2H), 1.81 - 1.99 (m, 6H), 2.07 (s, 3H), 3.27 (s, 2H), 3.97 (s, 2H), 4.55 (s, 2H), 7.25 - 7.34 (m, 5H)

### Step 4

### ethyl 4-(1-((benzyloxy)methyl)-2-oxabicyclo[2.2.2]octan-4-yl)-2,4-dioxobutanoate

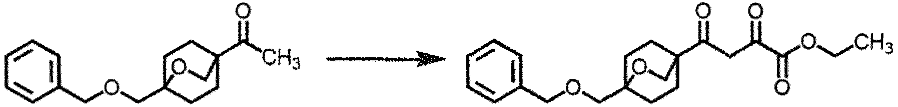

1-(1-((Benzyloxy)methyl)-2-oxabicyclo[2.2.2]octan-4-yl)ethan-1-one (2.13 g) was mixed with tetrahydrofuran (21.3 ml), and diethyl oxalate (1.156 ml) was added. The mixture was ice-cooled, 1 M potassium-tert-butoxide/tetrahydrofuran solution (9.32 ml) was added dropwise, and the mixture was stirred for 30 min. 1N Hydrogen chloride solution (9.32 ml) was added. The mixture was extracted with ethyl acetate and washed with saturated aqueous sodium chloride solution. To the organic layer was added sodium sulfate. Sodium sulfate was filtered off, and the filtrate was concentrated under reduced pressure to give a crude product (2.91 g) of the title compound.

### Step 5

### ethyl 5-(1-((benzyloxy)methyl)-2-oxabicyclo[2.2.2]octan-4-yl)-1H-pyrazole-3-carboxylate

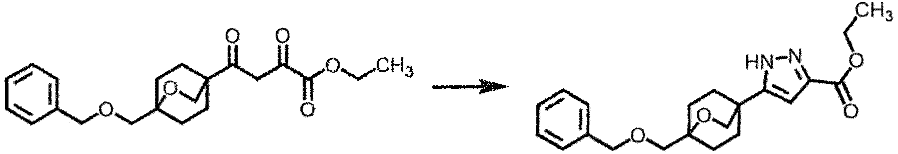

Ethyl 4-(1-((benzyloxy)methyl)-2-oxabicyclo[2.2.2]octan-4-yl)-2,4-dioxobutanoate (2.91 g) obtained in the previous step was mixed with ethanol (29.1 ml). Under ice-cooling, hydrazine monohydrate (0.397 ml) was added. The mixture was stirred at room temperature for 16 hr. The reaction mixture was concentrated under reduced pressure. The obtained residue was purified by silica gel column chromatography (hexane:ethyl acetate=100:0 to 0:100) to give the title compound (2.68 g). ¹H-NMR (400 MHz, CDCl₃) 1.37 (t, J=7.85Hz, 3H), 1.56 - 2.16 (m, 8H), 3.32 (s, 2H), 4.01 (s, 2H), 4.36 (q, J=7.85Hz, 2H), 4.57 (s, 2H), 6.63 (s, 1H), 7.25 - 7.35 (m, 5H), 10.65 (brs, 1H)

### Step 6

### (5-(1-((benzyloxy)methyl)-2-oxabicyclo[2.2.2]octan-4-yl)-1H-pyrazol-3-yl)methanol

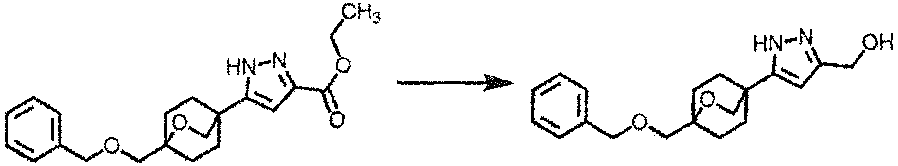

Under an argon atmosphere, lithium aluminum hydride (0.549 g) was mixed with tetrahydrofuran (70 ml). The mixture was ice-cooled. A solution of ethyl 5-(1-((benzyloxy)methyl)-2-oxabicyclo[2.2.2]octan-4-yl)-1H-pyrazole-3-carboxylate (2.68 g) obtained in the previous step in tetrahydrofuran (10 ml) was added dropwise. The reaction mixture was stirred at room temperature for 30 min. Saturated potassium sodium tartrate aqueous solution was added dropwise, and the mixture was stirred at room temperature for 1 hr. The reaction mixture was extracted with ethyl acetate. The organic layer was washed with saturated aqueous sodium chloride solution, and sodium sulfate was added. Sodium sulfate was filtered off. The filtrate was concentrated under reduced pressure to give a crude product (2.14 g) of the title compound.

### Step 7

### 5-(1-((benzyloxy)methyl)-2-oxabicyclo[2.2.2]octan-4-yl)-1H-pyrazole-3-carbaldehyde

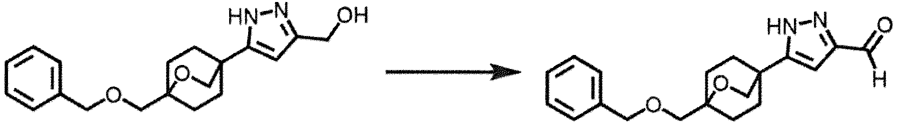

(5-(1-((Benzyloxy)methyl)-2-oxabicyclo[2.2.2]octan-4-yl)-1H-pyrazol-3-yl)methanol (1.14 g) obtained in the previous step was mixed with 1,2-dimethoxyethane (45.6 ml). Manganese dioxide (2.85 g) was added, and the mixture was stirred at room temperature for 22 hr. The reaction mixture was filtered through celite, and the filtrate was concentrated under reduced pressure to give a crude product (1.133 g) of the title compound.

### Step 8

### (S)-2-(5-(3-(1-((benzyloxy)methyl)-2-oxabicyclo[2.2.2]octan-4-yl)-1H-pyrazol-5-yl)-1H-imidazol-1-yl)propan-1-ol

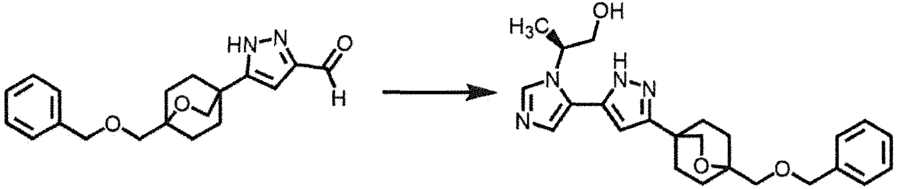

5-(1-((Benzyloxy)methyl)-2-oxabicyclo[2.2.2]octan-4-yl)-1H-pyrazole-3-carbaldehyde (1.133 g) obtained in the previous step was mixed with DMF (12 ml). At room temperature, (S)-2-aminopropan-1-ol (0.274 g) was added, and the mixture was stirred at room temperature for 16 hr. p-Toluenesulfonylmethyl isocyanide (0.813 g) and potassium carbonate (0.959 g) were added, and the mixture was stirred at room temperature for 71 hr. The reaction mixture was filtered through celite, and the filtrate was concentrated under reduced pressure. To the obtained residue was added water, and the mixture was extracted with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=25:75 to ethyl acetate, thereafter ethyl acetate:methanol=60:40) to give the title compound (1.2 g).

### Step 9

### (S)-9-(1-((benzyloxy)methyl)-2-oxabicyclo[2.2.2]octan-4-yl)-5-methyl-5,6-dihydroimidazo[1,5-a]pyrazolo[5,1-c]pyrazine

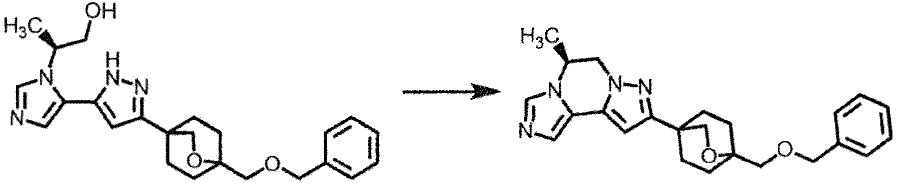

(S)-2-(5-(3-(1-((Benzyloxy)methyl)-2-oxabicyclo[2.2.2]octan-4-yl)-1H-pyrazol-5-yl)-1H-imidazol-1-yl)propan-1-ol (1.2 g) obtained in the previous step was mixed with tetrahydrofuran (24 ml). Under ice-cooling, triphenylphosphine (0.894 g) and diisopropyl azodicarboxylate (0.733 ml) were added, and the mixture was stirred at room temperature for 90 min. The reaction mixture was concentrated under reduced pressure. The obtained residue was purified by silica gel column chromatography (ethyl acetate:hexane=25:75 to ethyl acetate, thereafter ethyl acetate:methanol=60:40) to give the title compound (0.998 g).

### Step 10

### (S)-1-(9-(1-((benzyloxy)methyl)-2-oxabicyclo[2.2.2]octan-4-yl)-5-methyl-5,6-dihydroimidazo[1,5-a]pyrazolo[5,1-c]pyrazin-3-yl)ethan-1-one

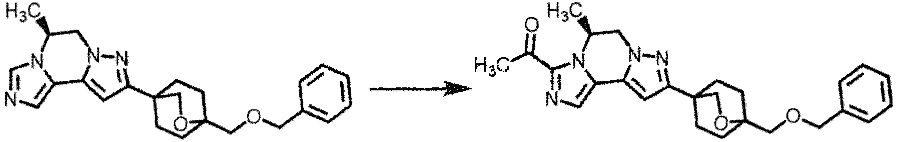

(S)-9-(1-((Benzyloxy)methyl)-2-oxabicyclo[2.2.2]octan-4-yl)-5-methyl-5,6-dihydroimidazo[1,5-a]pyrazolo[5,1-c]pyrazine (0.998 g) obtained in the previous step was mixed with tetrahydrofuran (19.96 ml). Under cooling in an acetone-dry ice bath, 2 M lithium diisopropylamide/tetrahydrofuran solution (1.172 ml) was added dropwise, and the mixture was stirred for 30 min. To the mixture was added N-methoxy-N-methylacetamide (0.605 ml), and the mixture was stirred for 30 min. To the mixture was added dropwise 2 M lithium diisopropylamide/tetrahydrofuran solution (1.357 ml), and the mixture was stirred for 90 min. Water was added, and the reaction mixture was extracted with ethyl acetate. The organic layer was washed with saturated aqueous sodium chloride solution, and sodium sulfate was added. Sodium sulfate was filtered off, and the filtrate was concentrated under reduced pressure. The obtained residue was purified by silica gel column chromatography (hexane:ethyl acetate=100:0 to 0:100) to give the title compound (0.899 g).

### Step 11

### (R)-2-((S)-9-(1-((benzyloxy)methyl)-2-oxabicyclo[2.2.2]octan-4-yl)-5-methyl-5,6-dihydroimidazo[1,5-a]pyrazolo[5,1-c]pyrazin-3-yl)-1,1,1-trifluoropropan-2-ol

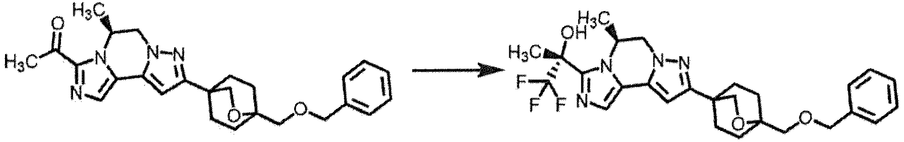

(S)-1-(9-(1-((Benzyloxy)methyl)-2-oxabicyclo[2.2.2]octan-4-yl)-5-methyl-5,6-dihydroimidazo[1,5-a]pyrazolo[5,1-c]pyrazin-3-yl)ethan-1-one (0.899 g) obtained in the previous step was mixed with tetrahydrofuran (10 ml). Under ice-cooling, to the mixture was added cesium fluoride (0.061 g), and (trifluoromethyl)trimethylsilane (0.596 ml) was added dropwise. The reaction mixture was stirred at room temperature for 1 hr. Under ice-cooling, methanol (5 ml) and potassium carbonate (0.362 g) were added, and the mixture was stirred at room temperature for 150 min. Water was added, and the mixture was extracted with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (hexane:ethyl acetate=100:0 to 0:100) to give the title compound (0.68 g).

### Step 12

### (S)-1,1,1-trifluoro-2-((S)-9-(1-(hydroxymethyl)-2-oxabicyclo[2.2.2]octan-4-yl)-5-methyl-5,6-dihydroimidazo[1,5-a]pyrazolo[5,1-c]pyrazin-3-yl)propan-2-ol

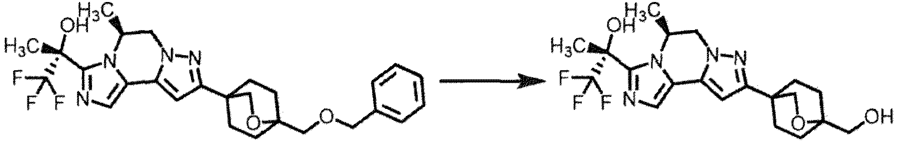

(R)-2-((S)-9-(1-((Benzyloxy)methyl)-2-oxabicyclo[2.2.2]octan-4-yl)-5-methyl-5,6-dihydroimidazo[1,5-a]pyrazolo[5,1-c]pyrazin-3-yl)-1,1,1-trifluoropropan-2-ol (0.68 g) obtained in the previous step was mixed with trifluoroacetic acid (8 ml), and the mixture was stirred at 90°C for 1 hr. The reaction mixture was concentrated under reduced pressure, and azeotroped with methanol. The residue was mixed with methanol (8 ml). Potassium carbonate (0.218 g) was added, and the mixture was stirred at room temperature for 1 hr. Water was added, and the mixture was extracted with ethyl acetate. The organic layer was washed with saturated aqueous sodium chloride solution, and dried over sodium sulfate. Sodium sulfate was filtered off, and the filtrate was concentrated under reduced pressure to give a crude product (0.55 g) of the title compound.

### Step 13

### 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)-2-oxabicyclo[2.2.2]octane-1-carbaldehyde

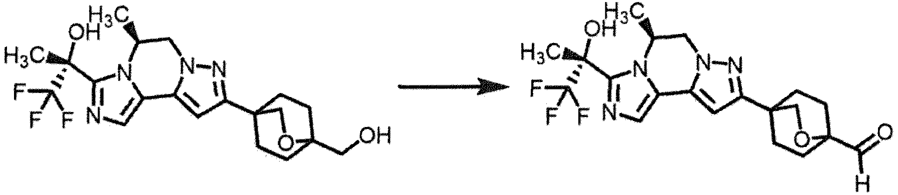

(S)-1,1,1-Trifluoro-2-((S)-9-(1-(hydroxymethyl)-2-oxabicyclo[2.2.2]octan-4-yl)-5-methyl-5,6-dihydroimidazo[1,5-a]pyrazolo[5,1-c]pyrazin-3-yl)propan-2-ol (0.28 g) obtained in the previous step was mixed with chloroform (6 ml). Under ice-cooling, Dess-Martin periodinane (0.252 g) was added, and the mixture was stirred at room temperature for 16 hr. Under ice-cooling, Dess-Martin periodinane (0.334 g) was added, and the mixture was stirred at room temperature for 17 hr. To the reaction mixture were added saturated sodium thiosulfate aqueous solution and saturated aqueous sodium hydrogen carbonate solution, and the mixture was extracted with ethyl acetate. The organic layer was washed with saturated aqueous sodium chloride solution, and dried over sodium sulfate. Sodium sulfate was filtered off, and the filtrate was concentrated under reduced pressure to give a crude product (0.294 g) of the title compound.

### Step 14

### 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)-2-oxabicyclo[2.2.2]octane-1-carboxylic acid

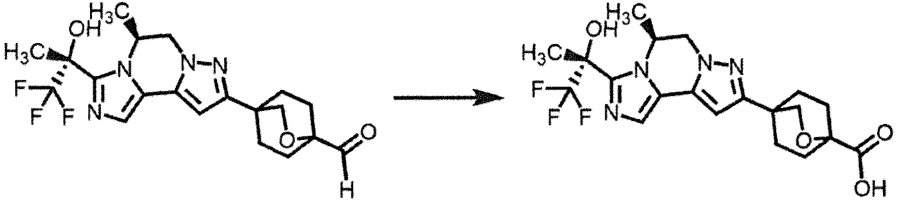

4-((S)-5-Methyl-3-((R)-1,1,1-trifluoro-2-hydroxypropan-2-yl)-5,6-dihydroimidazo[1,5-a]pyrazolo[5,1-c]pyrazin-9-yl)-2-oxabicyclo[2.2.2]octane-1-carbaldehyde (0.22 g) obtained in the previous step was mixed with acetone (6.6 ml). At room temperature, 2 M chromium trioxide sulfuric acid solution (0.259 ml) was added, and the mixture was stirred for 2 hr. The reaction mixture was purified by reversed-phase column chromatography (water:acetonitrile=90:10 to 0:100) to give a crude product (0.12 g).

### Step 15

### 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)-2-oxabicyclo[2.2.2]octane-1-carboxamide

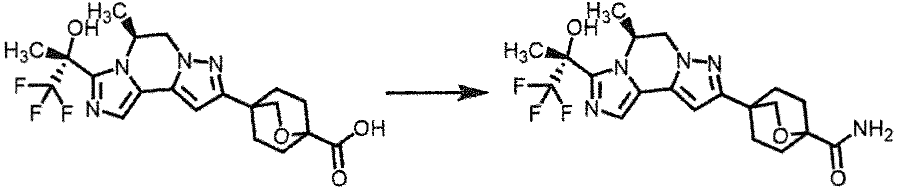

4-((S)-5-Methyl-3-((R)-1,1,1-trifluoro-2-hydroxypropan-2-yl)-5,6-dihydroimidazo[1,5-a]pyrazolo[5,1-c]pyrazin-9-yl)-2-oxabicyclo[2.2.2]octane-1-carboxylic acid (0.12 g) obtained in the previous step was mixed with DMF (2 ml). At room temperature, 1-[bis(dimethylamino)methylene]-1H-1,2,3-triazolo[4,5-b]pyridinium 3-oxide hexafluorophosphate (0.155 g) and 7 M ammonia/methanol solution (0.117 ml) were added, and the mixture was stirred for 16 hr. To the reaction mixture was added water, and the mixture was extracted with ethyl acetate. The organic layer was washed twice with saturated aqueous sodium chloride solution, and dried over sodium sulfate. Sodium sulfate was filtered off, and the filtrate was concentrated under reduced pressure. The obtained residue was purified by silica gel column chromatography (hexane:ethyl acetate=100:0 to 0:100) to give a crude product (0.065 g).

### Step 16

### 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)-2-oxabicyclo[2.2.2]octane-1-carbonitrile

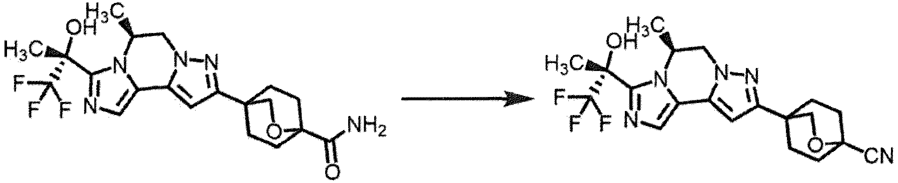

4-((S)-5-Methyl-3-((R)-1,1,1-trifluoro-2-hydroxypropan-2-yl)-5,6-dihydroimidazo[1,5-a]pyrazolo[5,1-c]pyrazin-9-yl)-2-oxabicyclo[2.2.2]octane-1-carboxamide (0.065 g) obtained in the previous step was mixed with tetrahydrofuran (2 ml). Under ice-cooling, triethylamine (0.103 ml) and trifluoroacetic anhydride (0.031 ml) were added, and the mixture was stirred for 1 hr. The reaction mixture was added to silica gel (10 g), and the mixture was eluted with ethyl acetate. The obtained solution was concentrated under reduced pressure, and the residue was purified by silica gel column chromatography (hexane:ethyl acetate=100:0 to 0:100) to give the title compound (0.044 g).
¹H-NMR (400 MHz, DMSO-D₆) 1.20 (d, J=6.47Hz, 3H), 1.80 (s, 3H), 1.92 - 2.18 (m, 6H), 2.25 - 2.35 (m, 2H), 3.94 (s, 2H), 4.20 - 4.25 (m, 1H), 4.32 - 4.37 (m, 1H), 5.36 - 5.28 (m, 1H), 6.44 (s, 1H), 7.23 (s, 1H), 7.26 (s, 1H)

### Production Example 10-2

### Synthesis of (R)-1,1,1-trifluoro-2-((S)-9-(1-(2-hydroxypropan-2-yl)-2-oxabicyclo[2.2.2]octan-4-yl)-5-methyl-5,6-dihydroimidazo[1,5-a]pyrazolo[5,1-c]pyrazin-3-yl)propan-2-ol

### (Example 96)

### Step 1

### methyl 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)-2-oxabicyclo[2.2.2]octane-1-carboxylate

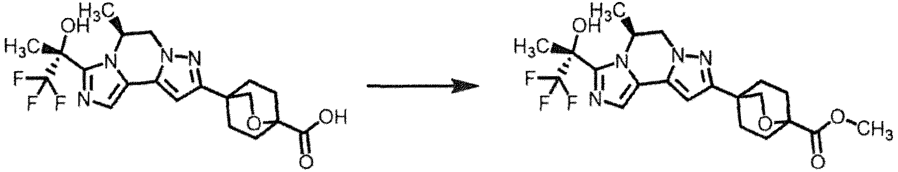

4-((S)-5-Methyl-3-((R)-1,1,1-trifluoro-2-hydroxypropan-2-yl)-5,6-dihydroimidazo[1,5-a]pyrazolo[5,1-c]pyrazin-9-yl)-2-oxabicyclo[2.2.2]octane-1-carboxylic acid (0.085 g) obtained in the previous step was mixed with methanol (3 ml). At room temperature, 0.6 M trimethylsilyldiazomethane/hexane solution (0.482 ml) was added, and the mixture was stirred for 15 min. The reaction mixture was concentrated under reduced pressure. The obtained residue was purified by silica gel column chromatography (hexane:ethyl acetate=100:0 to 0:100) to give a crude product (0.053 g) of the title compound.

### Step 2

### (R)-1,1,1-trifluoro-2-((S)-9-(1-(2-hydroxypropan-2-yl)-2-oxabicyclo[2.2.2]octan-4-yl)-5-methyl-5,6-dihydroimidazo[1,5-a]pyrazolo[5,1-c]pyrazin-3-yl)propan-2-ol

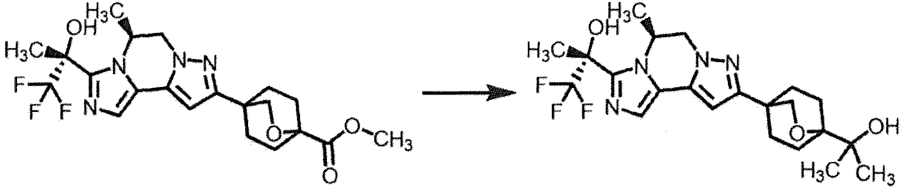

Under an argon atmosphere, methyl 4-((S)-5-methyl-3-((R)-1,1,1-trifluoro-2-hydroxypropan-2-yl)-5,6-dihydroimidazo[1,5-a]pyrazolo[5,1-c]pyrazin-9-yl)-2-oxabicyclo[2.2.2]octane-1-carboxylate (0.053 g) obtained in the previous step was mixed with tetrahydrofuran (2 ml). The mixture was ice-cooled, and 1.04 M methylmagnesium bromide/tetrahydrofuran solution (0.561 ml) was added. The mixture was stirred at room temperature for 17 hr. To the reaction mixture was added saturated ammonium chloride aqueous solution, and the mixture was extracted with ethyl acetate. The organic layer was washed with saturated aqueous sodium chloride solution, and sodium sulfate was added. Sodium sulfate was filtered off, and the filtrate was concentrated under reduced pressure. The obtained residue was purified by silica gel column chromatography (hexane:ethyl acetate=100:0 to 0:100) to give the title compound (0.037 g).
¹H-NMR (400 MHz, DMSO-D₆) 1.04 (s, 6H), 1.21 (d, J=6.47Hz, 3H), 1.64 - 1.72 (m, 2H), 1.78 - 1.96 (m, 9H), 3.84 (s, 2H), 3.93 (s, 1H), 4.19 - 4.25 (m, 1H), 4.32 - 4.37 (m, 1H), 5.36 - 5.27 (m, 1H), 6.40 (s, 1H), 7.22 (s, 1H), 7.26 (s, 1H)

### Production Example 11

### Synthesis of 3-((((S)-5-methyl-3-((R)-1,1,1-trifluoro-2-hydroxypropan-2-yl)-5,6-dihydroimidazo[1,5-a]pyrazolo[5,1-c]pyrazin-9-yl)oxy)methyl)bicyclo[1.1.1]pentane-1-carbonitrile

### (Example 97)

### Step 1

### (3-(benzyloxy)-1H-pyrazol-5-yl)methanol

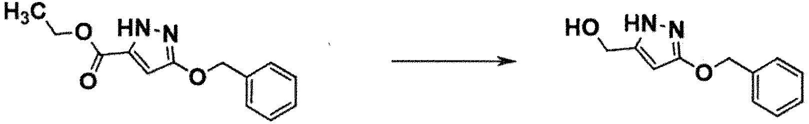

The crude product (19 g) of ethyl 3-(benzyloxy)-1H-pyrazole-5-carboxylate obtained in Auxiliary Step 1 was dissolved in cyclopentyl methyl ether (70 ml). Under ice-cooling, the solution was added dropwise to a solution of lithium borohydride (4.17 g) in cyclopentyl methyl ether (150 ml). The reaction mixture was stirred at 0°C for 5 min. Under ice-cooling, a solution of methanol (8 ml) in cyclopentyl methyl ether (50 ml), methanol (8 ml), and methanol (8 ml) were successively added dropwise. The reaction mixture was stirred at 0°C for 10 min. To the reaction mixture were added saturated ammonium chloride aqueous solution and 1N aqueous hydrochloric acid solution, and the mixture was extracted with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=16:84 to ethyl acetate, thereafter ethyl acetate:methanol=82:18) to give the title compound (8.2 g). ¹H-NMR (400 MHz, CDCl₃) 4.66 (s, 2H), 5.19 (s, 2H), 5.64 (s, 1H), 7.26 - 7.47 (m, 5H)

### Step 2

### 3-(benzyloxy)-1H-pyrazole-5-carbaldehyde

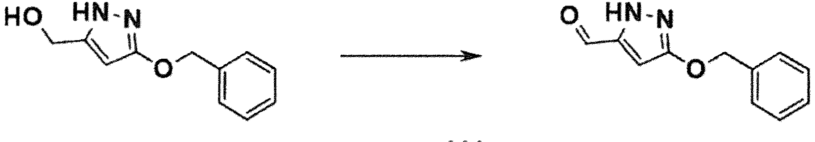

(3-(Benzyloxy)-1H-pyrazol-5-yl)methanol (8.2 g) obtained in the previous step was dissolved in 1,2-dimethoxyethane (164 ml). At room temperature, manganese dioxide (41 g) was added, and the mixture was stirred at 80°C for 1.5 hr. The mixture was allowed to cool to room temperature, and filtered through celite, and the filtrate was concentrated under reduced pressure to give a crude product (5.4 g) of the title compound.

### Step 3

### (S)-2-(5-(3-(benzyloxy)-1H-pyrazol-5-yl)-1H-imidazol-1-yl)propan-1-ol

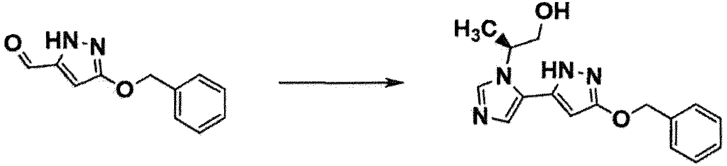

The crude product (5.4 g) of 3-(benzyloxy)-1H-pyrazole-5-carbaldehyde obtained in the previous step was mixed with methanol (54 ml). At room temperature, (S)-2-aminopropan-1-ol (2 g) was added, and the mixture was stirred at room temperature overnight. Under ice-cooling, 1,2-dimethoxyethane (164 ml), p-toluenesulfonylmethyl isocyanide (7.82 g), and potassium carbonate (11.07 g) were added, and the mixture was stirred at room temperature overnight. The reaction mixture was filtered through celite, and the filtrate was concentrated under reduced pressure. To the obtained residue was added water, and the mixture was extracted with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=25:75 to ethyl acetate, thereafter ethyl acetate:methanol=60:40) to give a crude product (4.1 g) of the title compound.

### Step 4

### (S)-9-(benzyloxy)-5-methyl-5,6-dihydroimidazo[1,5-a]pyrazolo[5,1-c]pyrazine

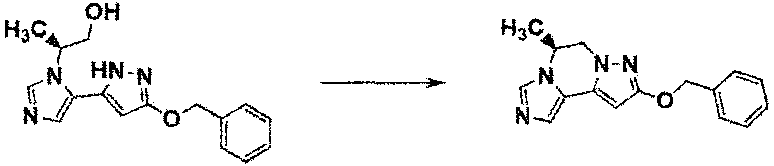

The crude product (4.1 g) of (S)-2-(5-(3-(benzyloxy)-1H-pyrazol-5-yl)-1H-imidazol-1-yl)propan-1-ol obtained in the previous step and triphenylphosphine (4.69 g) were mixed with tetrahydrofuran (123 ml). Under ice-cooling, di-tert-butyl azodicarboxylate (4.11 g) was added, and the mixture was stirred at room temperature overnight. The reaction mixture was concentrated under reduced pressure, and purified by silica gel column chromatography (ethyl acetate:hexane=25:75 to ethyl acetate, thereafter ethyl acetate:methanol=60:40) to give the title compound (2.98 g).
¹H-NMR (400 MHz, CDCl₃) 1.64 (d, J=6.70Hz, 3H), 3.97 (dd, J=12.95, 8.09Hz, 1H), 4.31 (dd, J=12.95, 4.39Hz, 1H), 4.54 - 4.65 (m, 1H), 5.21 (s, 2H), 5.86 (s, 1H), 7.26 (s, 1H), 7.29 - 7.49 (m, 5H), 7.59 (s, 1H)

### Step 5

### (S)-1-(9-(benzyloxy)-5-methyl-5,6-dihydroimidazo[1,5-a]pyrazolo[5,1-c]pyrazin-3-yl)ethan-1-one

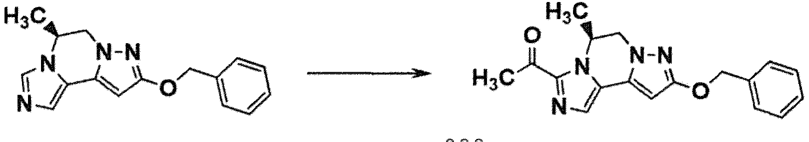

(S)-9-(Benzyloxy)-5-methyl-5,6-dihydroimidazo[1,5-a]pyrazolo[5,1-c]pyrazine (2.98 g) obtained in the previous step was mixed with tetrahydrofuran (29.8 ml). The mixture was cooled to -78°C, and 2 M lithium diisopropylamide/tetrahydrofuran-heptane-ethylbenzene solution (13.29 ml) was added dropwise. The mixture was stirred at - 78°C for 30 min. To the mixture was added N-methoxy-N-methylacetamide (5.25 ml), and the mixture was stirred at -78°C for 1 hr. To the reaction mixture were added saturated ammonium chloride aqueous solution and ethyl acetate, and the precipitated solid was collected by filtration. The filtrate was partitioned, and the organic layer was washed with saturated aqueous sodium chloride solution and dried over sodium sulfate. Sodium sulfate was filtered off, and the filtrate was concentrated under reduced pressure. To the obtained residue was added ethyl acetate/hexane=1/1 (10 ml), and the mixture was stirred at room temperature. The precipitated solid was collected by filtration. It was combined with the solid collected earlier by filtration and mixed with ethyl acetate. The mixture was concentrated under reduced pressure to give the title compound (2 g).
¹H-NMR (400 MHz, CDCl₃) 1.44 (d, J=6.58Hz, 3H), 2.69 (s, 3H), 4.19 (dd, J=13.45, 1.20Hz, 1H), 4.29 (dd, J=13.45, 4.63Hz, 1H), 5.24 (s, 2H), 5.75 - 5.84 (m, 1H), 5.98 (s, 1H), 7.32 - 7.50 (m, 6H)

### Step 6

### (R)-2-((S)-9-(benzyloxy)-5-methyl-5,6-dihydroimidazo[1,5-a]pyrazolo[5,1-c]pyrazin-3-yl)-1,1,1-trifluoropropan-2-ol

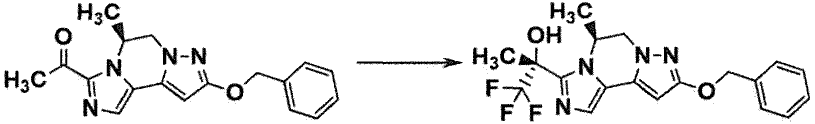

(S)-1-(9-(Benzyloxy)-5-methyl-5,6-dihydroimidazo[1,5-a]pyrazolo[5,1-c]pyrazin-3-yl)ethan-1-one (2 g) obtained in the previous step was mixed with tetrahydrofuran (29.8 ml). Under ice-cooling, to the mixture was added cesium fluoride (0.195 g), and then (trifluoromethyl)trimethylsilane (1.088 ml) was added dropwise. The reaction mixture was stirred at room temperature for 20 min. Under ice-cooling, methanol (24.84 ml) and potassium carbonate (1.065 g) were added, and the mixture was stirred at room temperature for 45 min. The reaction mixture was concentrated under reduced pressure, water was added, and the mixture was extracted with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=12:88 to 4:96) to give the title compound (2.2 g).
¹H-NMR (400 MHz, DMSO-D₆) 1.28 (d, J=6.70Hz, 3H), 1.80 (s, 3H), 4.12 - 4.24 (m, 2H), 5.15 (s, 2H), 5.27 - 5.36 (m, 1H), 6.04 (s, 1H), 7.23 (s, 1H), 7.28 (s, 1H), 7.29 - 7.46 (m, 5H)

### Step 7

### (S)-5-methyl-3-((R)-1,1,1-trifluoro-2-hydroxypropan-2-yl)-5,6-dihydroimidazo[1,5-a]pyrazolo[5,1-c]pyrazin-9-ol

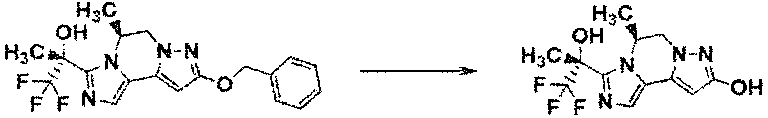

(R)-2-((S)-9-(Benzyloxy)-5-methyl-5,6-dihydroimidazo[1,5-a]pyrazolo[5,1-c]pyrazin-3-yl)-1,1,1-trifluoropropan-2-ol (2.2 g) obtained in the previous step and 10% palladium carbon (0.44 g) were mixed with ethanol (44 g). Under hydrogen atmosphere, the mixture was stirred at room temperature for 1.5 hr. The reaction mixture was filtered through celite, and the filtrate was concentrated under reduced pressure. The obtained residue was purified by silica gel column chromatography (ethyl acetate:hexane=16:84 to ethyl acetate, thereafter purified by ethyl acetate:methanol=80:20). To the obtained crude product were successively added ethyl acetate (6 ml) and hexane (6 ml), and the mixture was stirred at room temperature. The precipitated solid was collected by filtration to give the title compound (1.82 g). The steric configuration of the title compound was determined by X-ray crystal structure analysis.
¹H-NMR (400 MHz, DMSO-D₆) 1.28 (d, J=6.58Hz, 3H), 1.81 (s, 3H), 4.07 - 4.11 (m, 2H), 5.23 - 5.36 (m, 1H), 5.75 (s, 1H), 7.20 (s, 1H), 7.26 (brs, 1H), 9.89 (brs, 1H)

### Step 8

### methyl 3-((((S)-5-methyl-3-((R)-1,1,1-trifluoro-2-hydroxypropan-2-yl)-5,6-dihydroimidazo[1,5-a]pyrazolo[5,1-c]pyrazin-9-yl)oxy)methyl)bicyclo[1.1.1]pentane-1-carboxylate

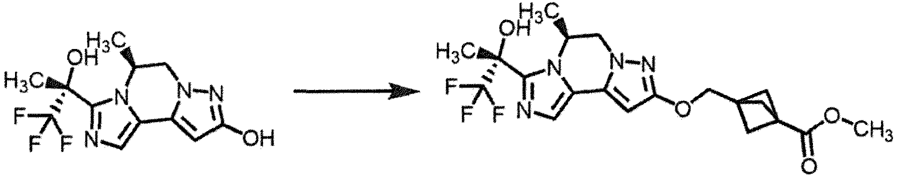

(S)-5-Methyl-3-((R)-1,1,1-trifluoro-2-hydroxypropan-2-yl)-5,6-dihydroimidazo[1,5-a]pyrazolo[5,1-c]pyrazin-9-ol (0.1 g) and methyl 3-(hydroxymethyl)bicyclo[1.1.1]pentane-1-carboxylate (0.078 g) were mixed with tetrahydrofuran (3 ml). Under heating at 70°C, triphenylphosphine (0.13 g) and diisopropyl azodicarboxylate (0.096 ml) were added, and the mixture was stirred for 15 min. The obtained residue was purified by silica gel column chromatography (hexane:ethyl acetate=100:0 to 0:100) to give a crude product (0.135 g).

### Step 9

### 3-((((S)-5-methyl-3-((R)-1,1,1-trifluoro-2-hydroxypropan-2-yl)-5,6-dihydroimidazo[1,5-a]pyrazolo[5,1-c]pyrazin-9-yl)oxy)methyl)bicyclo[1.1.1]pentane-1-carboxylic acid

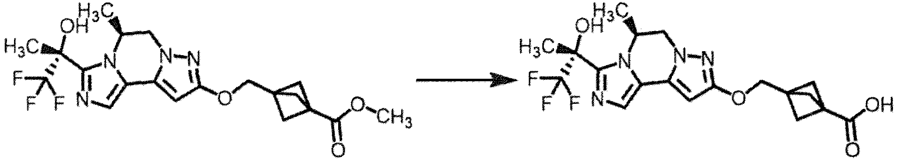

Methyl 3-((((S)-5-methyl-3-((R)-1,1,1-trifluoro-2-hydroxypropan-2-yl)-5,6-dihydroimidazo[1,5-a]pyrazolo[5,1-c]pyrazin-9-yl)oxy)methyl)bicyclo[1.1.1]pentane-1-carboxylate (0.135 g) obtained in the previous step was mixed with ethanol (2 ml). 2N Sodium hydroxide aqueous solution (1 ml) was added, and the mixture was stirred at 80°C for 8 hr. At room temperature, 2N hydrogen chloride solution (1 ml) was added, and the mixture was concentrated under reduced pressure. The obtained residue was extracted with ethyl acetate, and washed with saturated aqueous sodium chloride solution. Sodium sulfate was added. Sodium sulfate was filtered off. The filtrate was concentrated under reduced pressure to give a crude product (0.131 g) of the title compound.

### Step 10

### 3-((((S)-5-methyl-3-((R)-1,1,1-trifluoro-2-hydroxypropan-2-yl)-5,6-dihydroimidazo[1,5-a]pyrazolo[5,1-c]pyrazin-9-yl)oxy)methyl)bicyclo[1.1.1]pentane-1-carboxamide

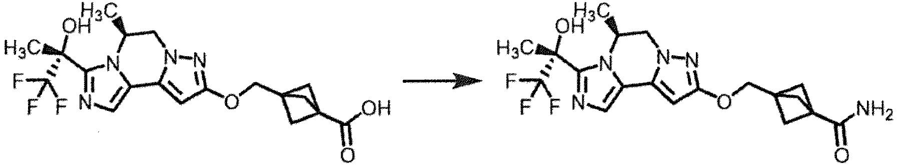

3-((((S)-5-Methyl-3-((R)-1,1,1-trifluoro-2-hydroxypropan-2-yl)-5,6-dihydroimidazo[1,5-a]pyrazolo[5,1-c]pyrazin-9-yl)oxy)methyl)bicyclo[1.1.1]pentane-1-carboxylic acid (0.131 g) obtained in the previous step was mixed with DMF (2 ml). At room temperature, 1-[bis(dimethylamino)methylene]-1H-1,2,3-triazolo[4,5-b]pyridinium 3-oxide hexafluorophosphate (0.175 g) and 7 M ammonia/methanol solution (0.132 ml) were added, and the mixture was stirred for 16 hr. Water was added, and the mixture was extracted with ethyl acetate. The organic layer was washed twice with saturated aqueous sodium chloride solution, and dried over sodium sulfate. Sodium sulfate was filtered off, and the filtrate was concentrated under reduced pressure. The obtained residue was purified by silica gel column chromatography (hexane:ethyl acetate=100:0 to 0:100) to give a crude product (0.095 g).

### Step 11

### 3-((((S)-5-methyl-3-((R)-1,1,1-trifluoro-2-hydroxypropan-2-yl)-5,6-dihydroimidazo[1,5-a]pyrazolo[5,1-c]pyrazin-9-yl)oxy)methyl)bicyclo[1.1.1]pentane-1-carbonitrile

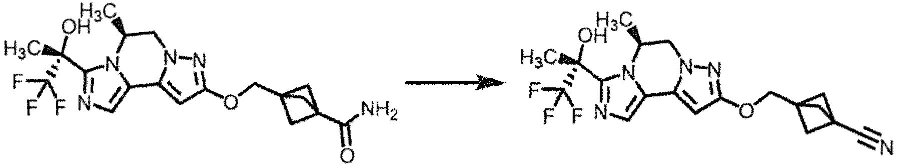

3-((((S)-5-Methyl-3-((R)-1,1,1-trifluoro-2-hydroxypropan-2-yl)-5,6-dihydroimidazo[1,5-a]pyrazolo[5,1-c]pyrazin-9-yl)oxy)methyl)bicyclo[1.1.1]pentane-1-carboxamide (0.095 g) obtained in the previous step was mixed with tetrahydrofuran (2 ml). Under ice-cooling, triethylamine (0.156 ml) and trifluoroacetic anhydride (0.047 ml) were added, and the mixture was stirred for 15 min. The reaction mixture was added to silica gel (10 g), and the mixture was eluted with ethyl acetate. The obtained filtrate was concentrated under reduced pressure, and the residue was purified by silica gel column chromatography (hexane:ethyl acetate=100:0 to 0:100) to give the title compound (0.077 g).
¹H-NMR (400 MHz, DMSO-D₆) 1.25 (d, J=6.47Hz, 3H), 1.80 (s, 3H), 2.23 (s, 6H), 4.09 (s, 2H), 4.12 - 4.15 (m, 2H), 5.34 - 5.27 (m, 1H), 5.97 (s, 1H), 7.21 (s, 1H), 7.28 (s, 1H)

### Production Example 12

### Synthesis of mixture of (R)-1,1,1-trifluoro-2-((R)-5-methyl-9-(trifluoromethyl)-5,6-dihydropyrazolo[1',5':1,2] pyrido[3, 4-d]pyridazin-4-yl)propan-2-ol and (S)-1,1,1-trifluoro-2-((S)-5-methyl-9-(trifluoromethyl)-5,6-dihydropyrazolo[1',5':1,2]pyrido[3,4-d]pyridazin-4-yl)propan-2-ol (Example 99) and (R)-1,1,1-trifluoro-2-((R)-5-methyl-9-(trifluoromethyl)-5,6-dihydropyrazolo[1',5':1,2]pyrido[3,4-d]pyridazin-4-yl)propan-2-ol (Example 100)

### Step 1

### 4,5-dichloro-2-(4-methoxybenzyl)pyridazin-3(2H)-one

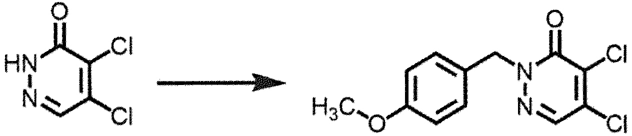

4,5-Dichloropyridazin-3(2H)-one (18.5 g) was dissolved in toluene (185 ml). At room temperature, to the reaction mixture were added 4-methoxybenzyl chloride (16.0 ml), tetrabutylammonium bromide (6.35 g), and 2N potassium hydroxide aqueous solution (59.1 ml), and the mixture was heated under reflux at 120°C for 7 hr. Water was added to the reaction mixture, and a partitioning operation was performed. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (hexane:ethyl acetate=88:12 to ethyl acetate). The obtained solid was washed with hexane to give the title compound (8.8 g).
¹H-NMR (400 MHz, DMSO-D₆) 3.73 (s, 3H), 5.21 (s, 2H), 6.88 - 6.91 (m, 2H), 7.26 - 7.28 (m, 2H), 8.22 (s, 1H)

### Step 2

### mixture of diethyl 2-(5-chloro-2-(4-methoxybenzyl)-3-oxo-2,3-dihydropyridazin-4-yl)malonate and diethyl 2-(5-chloro-1-(4-methoxybenzyl)-6-oxo-1,6-dihydropyridazin-4-yl)malonate

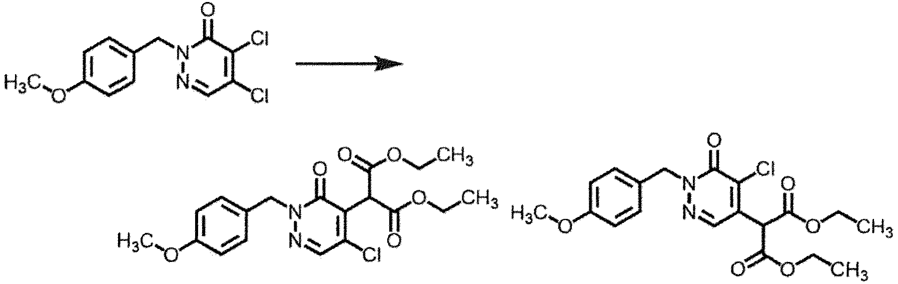

Diethyl malonate (21 ml) was dissolved in tetrahydrofuran (157 ml). At room temperature, to the reaction mixture was added sodium tert-pentoxide (15.2 g), and the mixture was stirred at room temperature for 15 min. To the reaction mixture was added 4,5-dichloro-2-(4-methoxybenzyl)pyridazin-3(2H)-one (15.7 g), and the mixture was stirred at 75°C for 3 hr. At room temperature, to the reaction mixture was added saturated ammonium chloride aqueous solution, and the mixture was extracted with ethyl acetate. The organic layer was washed successively with water and saturated aqueous sodium chloride solution, and filtered through a phase separator. The filtrate was concentrated under reduced pressure. The obtained residue was purified by silica gel column chromatography (hexane to ethyl acetate:hexane=50:50) to give a crude product (13.8 g) of the title compound.

### Step 3

### ethyl 2-(5-chloro-2-(4-methoxybenzyl)-3-oxo-2,3-dihydropyridazin-4-yl)acetate

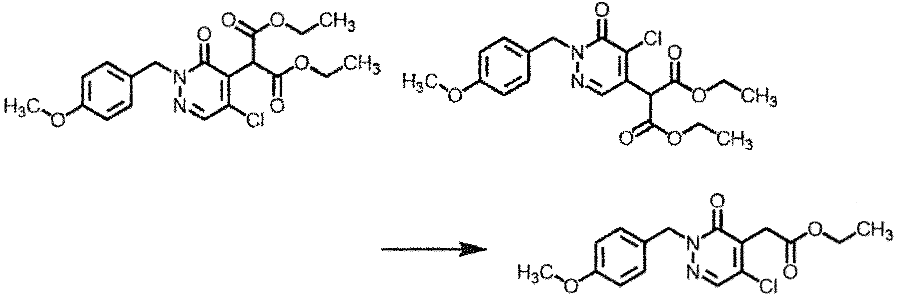

The mixture (13.75 g) of diethyl 2-(5-chloro-2-(4-methoxybenzyl)-3-oxo-2,3-dihydropyridazin-4-yl)malonate and diethyl 2-(5-chloro-1-(4-methoxybenzyl)-6-oxo-1,6-dihydropyridazin-4-yl)malonate obtained in the previous step was dissolved in dimethyl sulfoxide (53.6 ml). To the reaction mixture was added water (53.6 ml), and the mixture was stirred at 135°C overnight. At room temperature, water was added to the reaction mixture, and the mixture was extracted with ethyl acetate. The organic layer was washed successively with water and saturated aqueous sodium chloride solution, and filtered through a phase separator. The filtrate was concentrated under reduced pressure. The obtained residue was purified by silica gel column chromatography (hexane to ethyl acetate:hexane=50:50) to give the title compound (10.6 g). ¹H-NMR (400 MHz, DMSO-D₆) 1.15 (t, J=7.07Hz, 3H), 3.70 (s, 2H), 3.72 (s, 3H), 4.08 (q, J=7.07Hz, 2H), 5.18 (s, 2H), 6.88 - 6.90 (m, 2H), 7.23 - 7.26 (m, 2H), 8.13 (s, 1H)

### Step 4

### ethyl 2-(5-chloro-2-(4-methoxybenzyl)-3-oxo-2,3-dihydropyridazin-4-yl)propanoate

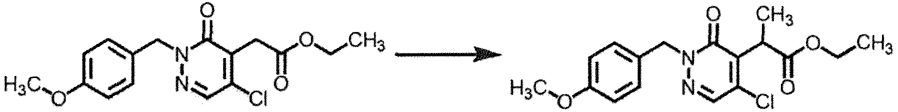

Ethyl 2-(5-chloro-2-(4-methoxybenzyl)-3-oxo-2,3-dihydropyridazin-4-yl)acetate (10.64 g) obtained in the previous step was dissolved in tetrahydrofuran (96 ml). At - 78°C, to the reaction mixture was added dropwise 1.08 M lithium bis(trimethylsilyl)amide/tetrahydrofuran solution (30.7 ml), and the mixture was stirred for 30 min. At -78°C, to the reaction mixture was added dropwise methyl iodide (2.1 ml), and the mixture was stirred for 3 hr while raising the temperature to room temperature. To the reaction mixture was added saturated ammonium chloride aqueous solution at room temperature, and the mixture was extracted with ethyl acetate. The organic layer was washed successively with water and saturated aqueous sodium chloride solution, and filtered through a phase separator. The filtrate was concentrated under reduced pressure. The obtained residue was purified by silica gel column chromatography (hexane to ethyl acetate:hexane=50:50) to give the title compound (9.3 g). ¹H-NMR (400 MHz, DMSO-D₆) 1.01 (t, J=7.06Hz, 3H), 1.30 (d, J=7.06Hz, 3H), 3.72 (s, 3H), 3.93 - 4.10 (m, 3H), 5.06 (d, J=13.99Hz, 1H), 5.26 (d, J=13.99Hz, 1H), 6.88 - 6.90 (m, 2H), 7.23 - 7.25 (m, 2H), 8.11 (s, 1H)

### Step 5

### 5-chloro-4-(1-hydroxypropan-2-yl)-2-(4-methoxybenzyl)pyridazin-3(2H)-one

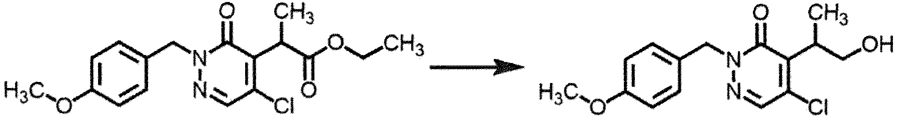

Ethyl 2-(5-chloro-2-(4-methoxybenzyl)-3-oxo-2,3-dihydropyridazin-4-yl)propanoate (9.3 g) obtained in the previous step was dissolved in diethyl ether (140 ml). Under ice-cooling, 2.0 M lithium borohydride/tetrahydrofuran solution (26.5 ml) was added dropwise, and the mixture was stirred at room temperature for 1 hr. Under ice-cooling, to the reaction mixture was added saturated ammonium chloride aqueous solution, and the mixture was extracted with ethyl acetate. The organic layer was washed successively with water and saturated aqueous sodium chloride solution, and filtered through a phase separator. The filtrate was concentrated under reduced pressure. The obtained residue was purified by silica gel column chromatography (ethyl acetate:hexane=25:75 to 67:33) to give the title compound (4.3 g).
¹H-NMR (400 MHz, DMSO-D₆) 1.16 (d, J=7.17Hz, 4H), 3.28 - 3.36 (m, 1H), 3.54 - 3.60 (m, 1H), 3.71 (s, 3H), 3.80 - 3.86 (m, 1H), 4.68 (t, J=5.66Hz, 1H), 5.12 (dd, J=18.50, 14.10Hz, 2H), 6.87 - 6.89 (m, 2H), 7.23 - 7.25 (m, 2H), 7.98 (s, 1H)

### Step 6

### 5-chloro-4-(1-hydroxypropan-2-yl)pyridazin-3(2H)-one

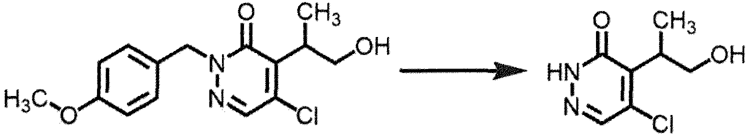

5-Chloro-4-(1-hydroxypropan-2-yl)-2-(4-methoxybenzyl)pyridazin-3(2H)-one (4.3 g) obtained in the previous step was mixed with acetonitrile (43 ml). Under ice-cooling, to the reaction mixture was added dropwise a mixed solution of cerium(IV) ammonium nitrate (18.3 g) and water (21.5 ml), and the mixture was stirred at room temperature for 2 hr. At room temperature, to the reaction mixture was added water, and the mixture was extracted with ethyl acetate. The organic layer was washed successively with water and saturated aqueous sodium chloride solution, and filtered through a phase separator. The filtrate was concentrated under reduced pressure. The obtained residue was purified by silica gel column chromatography (ethyl acetate:hexane=50:50 to ethyl acetate) to give the title compound (1.92 g).
¹H-NMR (400 MHz, DMSO-D₆) 1.18 (d, J=6.94Hz, 3H), 3.28 - 3.34 (m, 1H), 3.58 (dd, J=10.29, 6.47Hz, 1H), 3.85 (dd, J=10.29, 8.32Hz, 1H), 7.90 (s, 1H), 13.10 (s, 1H)

### Step 7

### 4-(1-((tert-butyldimethylsilyl)oxy)propan-2-yl)-5-chloropyridazin-3(2H)-one

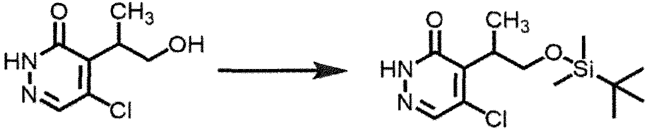

5-Chloro-4-(1-hydroxypropan-2-yl)pyridazin-3(2H)-one (1.92 g) obtained in the previous step was mixed with dimethylformamide (19.2 ml). At room temperature, imidazole (1.0 g), tert-butyldimethylchlorosilane (1.69 g), and 4-dimethylaminopyridine (0.12 g) were added, and the mixture was stirred for 30 min. The reaction mixture was purified by silica gel column chromatography (hexane to ethyl acetate:hexane=50:50) to give the title compound (2.74 g). ¹H-NMR (400 MHz, DMSO-D₆) 0.06 (s, 3H), 0.08 (s, 3H), 0.85 (s, 9H), 1.27 (d, J=7.17Hz, 3H), 3.40 - 3.49 (m, 1H), 3.82 (dd, J=9.42, 6.24Hz, 1H), 4.14 (t, J=9.42Hz, 1H), 8.00 (s, 1H), 13.22 (s, 1H)

### Step 8

### mixture of 4-(1-((tert-butyldimethylsilyl)oxy)propan-2-yl)-5-chloro-2-((trifluoromethyl)sulfonyl)pyridazin-3(2H)-one and 4-(1-((tert-butyldimethylsilyl)oxy)propan-2-yl)-5-chloropyridazin-3-yl trifluoromethylsulfonate

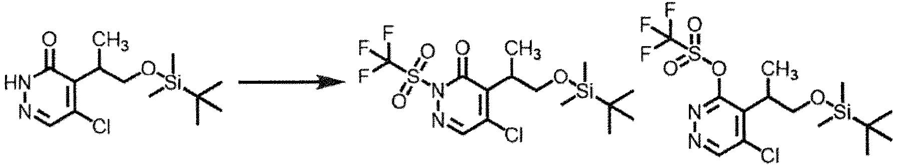

4-(1-((Tert-butyldimethylsilyl)oxy)propan-2-yl)-5-chloropyridazin-3(2H)-one (2.74 g) obtained in the previous step was mixed with dichloromethane (27.4 ml). Under ice-cooling, to the mixture were added pyridine (2.2 ml) and trifluoromethanesulfonic anhydride (2.3 ml). The reaction mixture was stirred overnight while raising the temperature to room temperature. The reaction mixture was purified by silica gel column chromatography (hexane to ethyl acetate:hexane=33:67) to give a crude product (3.37 g) of the title compound.

### Step 9

### 4-(1-((tert-butyldimethylsilyl)oxy)propan-2-yl)-5-chloro-3-(1-ethoxyvinyl)pyridazine

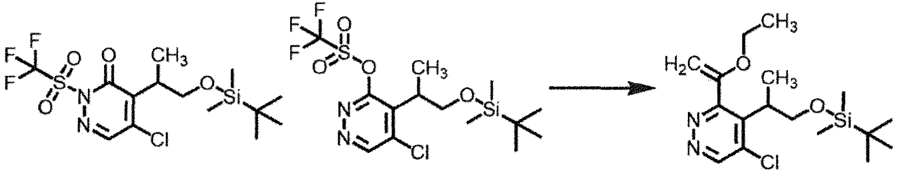

The mixture (3.37 g) of 4-(1-((tert-butyldimethylsilyl)oxy)propan-2-yl)-5-chloro-2-((trifluoromethyl)sulfonyl)pyridazin-3(2H)-one and 4-(1-((tert-butyldimethylsilyl)oxy)propan-2-yl)-5-chloropyridazin-3-yl trifluoromethylsulfonate obtained in the previous step was mixed with 1,4-dioxane (33.7 ml). At room temperature, to the reaction mixture were added tributyl(1-ethoxyvinyl)stannane (2.88 ml), lithium chloride (0.66 g), and copper iodide (0.15 g). The reaction mixture was stirred at 95°C for 2.5 hr, and then stirred at 110°C for 2 hr. The reaction mixture was filtered through celite, and the filtrate was concentrated under reduced pressure. The crude product was filtered through aminosilica, and the filtrate was concentrated under reduced pressure. The obtained residue was purified by aminosilica gel chromatography (hexane to ethyl acetate:hexane=50:50) to give the title compound (1.08 g).
¹H-NMR (400 MHz, DMSO-D₆) - 0.08 (s, 3H), - 0.04 (s, 3H), 0.70 (s, 9H), 1.28 - 1.32 (m, 6H), 3.38 - 3.40 (m, 1H), 3.83 (dd, J=9.54, 7.19Hz, 1H), 3.94 (q, J=7.19Hz, 2H), 4.03 (t, J=9.54Hz, 1H), 4.42 (d, J=2.66Hz, 1H), 4.64 (d, J=2.66Hz, 1H), 9.24 (s, 1H)

### Step 10

### 4-(1-((tert-butyldimethylsilyl)oxy)propan-2-yl)-3-(1-ethoxyvinyl)-5-(3-(trifluoromethyl)-1H-pyrazol-5-yl)pyridazine

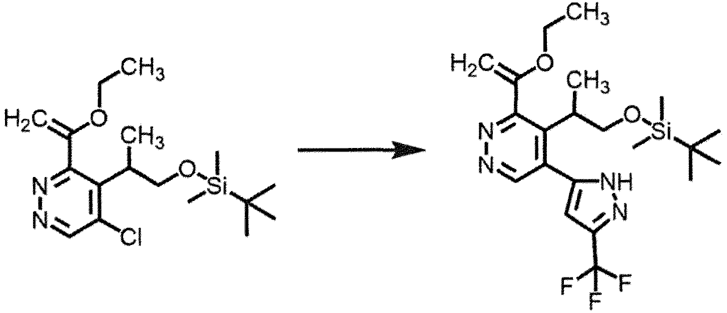

4-(1-((tert-Butyldimethylsilyl)oxy)propan-2-yl)-5-chloro-3-(1-ethoxyvinyl)pyridazine (0.1 g) obtained in the previous step was mixed with 1,4-dioxane (2.0 ml) and water (1.0 ml). To the reaction mixture were added tripotassium phosphate (18.3 g), 5-(4,4,5,5-tetramethyl-1,3,2-dioxaborolan-2-yl)-3-(trifluoromethyl)-1H-pyrazole (18.3 g), and Xphos Pd G4 (0.024 g), and the mixture was stirred at 110°C for 1 hr. At room temperature, to the reaction mixture was added saturated ammonium chloride aqueous solution, and the mixture was extracted with ethyl acetate. The organic layer was washed successively with water and saturated aqueous sodium chloride solution, and filtered through a phase separator. The filtrate was concentrated under reduced pressure. The obtained residue was purified by silica gel column chromatography (ethyl acetate:hexane=25:75 to ethyl acetate) to give a crude product (0.067 g) of the title compound.

### Step 11

### 2-(3-(1-ethoxyvinyl)-5-(3-(trifluoromethyl)-1H-pyrazol-5-yl)pyridazin-4-yl)propan-1-ol

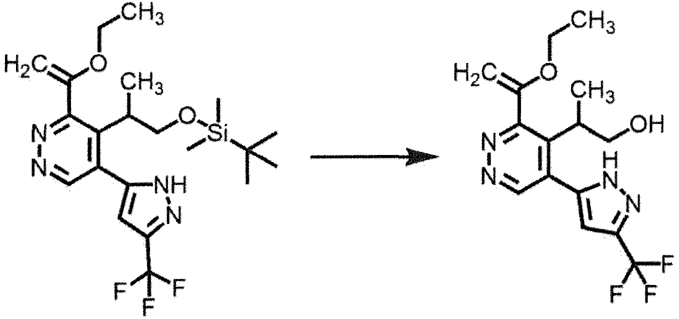

The crude product (0.067 g) of 4-(1-((tert-butyldimethylsilyl)oxy)propan-2-yl)-3-(1-ethoxyvinyl)-5-(3-(trifluoromethyl)-1H-pyrazol-5-yl)pyridazine obtained in the previous step was mixed with tetrahydrofuran (1.0 ml). Under ice-cooling, to the reaction mixture was added 1.0 M tetrabutylammonium fluoride/tetrahydrofuran solution (0.22 ml), and the mixture was stirred at room temperature for 1 hr. To the reaction mixture was added saturated ammonium chloride aqueous solution, and the mixture was extracted with ethyl acetate. The organic layer was washed successively with water and saturated aqueous sodium chloride solution, and filtered through a phase separator. The filtrate was concentrated under reduced pressure. The obtained residue was purified by silica gel column chromatography (ethyl acetate:hexane=50:50 to ethyl acetate, thereafter ethyl acetate:methanol=90:10) to give the title compound (0.026 g).
¹H-NMR (400 MHz, DMSO-D₆) 1.12 (d, J=6.94Hz, 3H), 1.32 (t, J=6.82Hz, 3H), 3.19 - 3.21 (m, 1H), 3.40 - 3.42 (m, 1H), 3.51 (dd, J=10.29, 7.03Hz, 1H), 3.96 (q, J=7.03Hz, 2H), 4.43 (d, J=2.66Hz, 1H), 4.64 (d, J=2.66Hz, 1H), 7.09 (s, 1H), 9.10 (s, 1H)

### Step 12

### 4-(1-ethoxyvinyl)-5-methyl-9-(trifluoromethyl)-5,6-dihydropyrazolo[1',5':1,2]pyrido[3,4-d]pyridazine

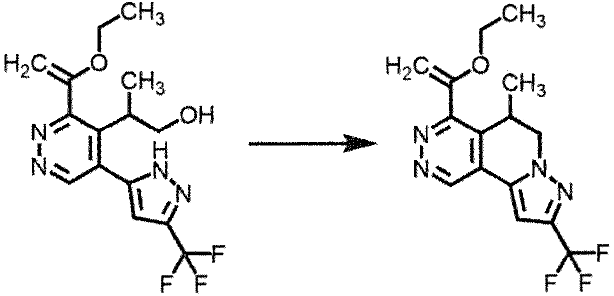

2-(3-(1-Ethoxyvinyl)-5-(3-(trifluoromethyl)-1H-pyrazol-5-yl)pyridazin-4-yl)propan-1-ol (0.026 g) obtained in the previous step was mixed with tetrahydrofuran (0.52 ml). To the reaction mixture were added triphenylphosphine (0.023 g) and diisopropyl azodicarboxylate (0.018 ml), and the mixture was stirred at room temperature for 1 hr. The reaction mixture was concentrated under reduced pressure. The obtained residue was purified by silica gel column chromatography (ethyl acetate:hexane=30:70 to ethyl acetate, thereafter ethyl acetate:methanol=80:20) to give a crude product (0.031 g) of the title compound containing impurity.

### Step 13

### 1-(5-methyl-9-(trifluoromethyl)-5,6-dihydropyrazolo[1',5':1,2]pyrido[3,4-d]pyridazin-4-yl)ethan-1-one

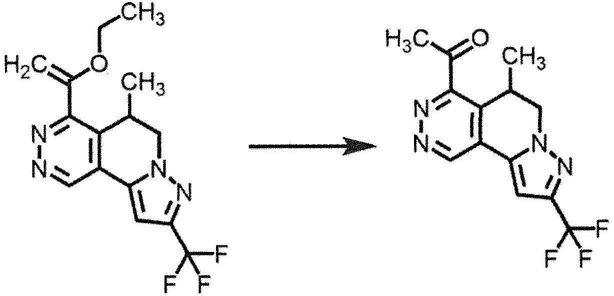

The crude product (0.031 g) of 4-(1-ethoxyvinyl)-5-methyl-9-(trifluoromethyl)-5,6-dihydropyrazolo[1',5':1,2]pyrido[3,4-d]pyridazine obtained in the previous step was mixed with tetrahydrofuran (0.31 ml) and methanol (0.31 ml). To the reaction mixture was added 6 M hydrochloric acid (0.096 ml), and the mixture was stirred at 50°C for 1 hr. At room temperature, to the reaction mixture was added 2 M sodium hydroxide aqueous solution (0.3 ml), and the mixture was extracted with ethyl acetate. The organic layer was washed successively with water and saturated aqueous sodium chloride solution, and filtered through a phase separator. The filtrate was concentrated under reduced pressure. The obtained residue was purified by silica gel column chromatography (ethyl acetate:hexane=30:70 to 90:10) to give a crude product (0.025 g) of the title compound.

### Step 14

### mixture of (R)-1,1,1-trifluoro-2-((R)-5-methyl-9-(trifluoromethyl)-5,6-dihydropyrazolo[1',5':1,2]pyrido[3,4-d]pyridazin-4-yl)propan-2-ol and (S)-1,1,1-trifluoro-2-((S)-5-methyl-9-(trifluoromethyl)-5,6-dihydropyrazolo[1',5':1,2]pyrido[3,4-d]pyridazin-4-yl)propan-2-ol

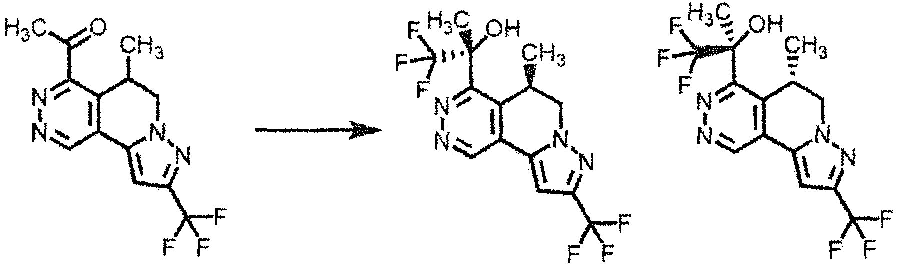

The crude product (0.025 g) of 1-(5-methyl-9-(trifluoromethyl)-5,6-dihydropyrazolo[1',5':1,2]pyrido[3,4-d]pyridazin-4-yl)ethan-1-one obtained in the previous step was mixed with tetrahydrofuran (0.5 ml). Under ice-cooling, to the mixture was added cesium fluoride (0.006 g), and then added dropwise (trifluoromethyl)trimethylsilane (0.025 ml). The reaction mixture was stirred for 1 hr while raising the temperature to room temperature. At room temperature, methanol (0.5 ml) and potassium carbonate (0.035 g) were added, and the mixture was stirred at room temperature for 1 hr. To the reaction mixture was added saturated ammonium chloride aqueous solution, and the mixture was extracted with ethyl acetate. The organic layer was washed with water and saturated aqueous sodium chloride solution, and filtered through a phase separator, and the filtrate was concentrated under reduced pressure. The obtained residue was purified by reversed-phase silica gel chromatography (acetonitrile:water=0:100 to 100:0) to give the title compound (0.009 g) as a racemate.
¹H-NMR (400 MHz, DMSO-D₆) 1.13 (d, J=6.70Hz, 3H), 2.01 (s, 3H), 4.32 - 4.35 (m, 2H), 4.49 - 4.52 (m, 1H), 7.38 (s, 1H), 7.66 (s, 1H), 9.66 (s, 1H)

### Step 15

### (R)-1,1,1-trifluoro-2-((R)-5-methyl-9-(trifluoromethyl)-5,6-dihydropyrazolo[1',5':1,2]pyrido[3,4-d]pyridazin-4-yl)propan-2-ol

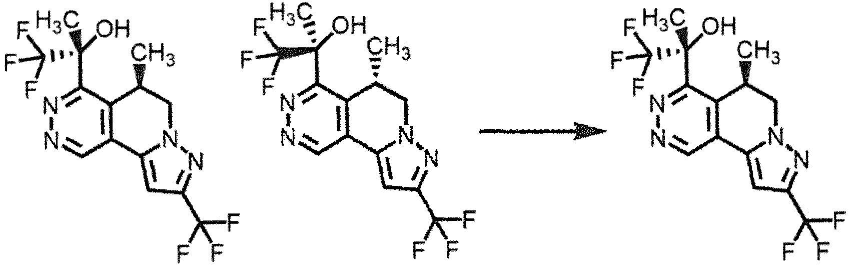

The mixture (0.0067 g) of (R)-1,1,1-trifluoro-2-((R)-5-methyl-9-(trifluoromethyl)-5,6-dihydropyrazolo[1',5':1,2]pyrido[3,4-d]pyridazin-4-yl)propan-2-ol and (S)-1,1,1-trifluoro-2-((S)-5-methyl-9-(trifluoromethyl)-5,6-dihydropyrazolo[1',5':1,2]pyrido[3,4-d]pyridazin-4-yl)propan-2-ol obtained in the previous step was subjected to optical resolution using supercritical fluid chromatography (apparatus name: Waters SFC Prep15 System, column: Daicel CHIRALPAK IC/SFC, 10 mm(I.D.) x 250 mm(L), 5 um, column temperature: 40°C, column back pressure: 120 bar, mobile phase flow rate: 15 ml/min, mobile phase mixing ratio: isocratic, carbon dioxide:methanol=93:7, fraction trigger: UV 214 nm) to give the title compound (0.002 g) as the first peak fraction.
¹H-NMR (400 MHz, CDCl₃) 1.23 (d, J=6.94Hz, 3H), 2.16 (s, 3H), 3.49 (s, 1H), 4.29 - 4.33 (m, 2H), 4.45 (d, J=12.02Hz, 1H), 7.05 (s, 1H), 9.31 (s, 1H)

### Production Example 13

### Synthesis of (R)-1,1,1-trifluoro-2-[(R)-9-(1-fluorocyclopropyl)-5-methyl-5,6-dihydropyrazolo[1',5':1,2]pyrido[3,4-d]pyridazin-4-yl]propan-2-ol and (S)-1,1,1-trifluoro-2-[(S)-9-(1-fluorocyclopropyl)-5-methyl-5,6-dihydropyrazolo[1',5':1,2]pyrido[3,4-d]pyridazin-4-yl]propan-2-ol (Example 101)

### Step 1

### 4-{1-[(tert-butyldimethylsilyl)oxy]propan-2-yl}-3-(1-ethoxyvinyl)-5-[(triisopropylsilyl)ethynyl]pyridazine

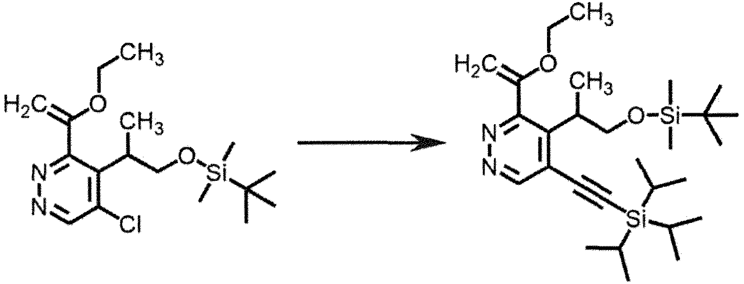

To a suspension of 4-{1-[(tert-butyldimethylsilyl)oxy]propan-2-yl}-5-chloro-3-(1-ethoxyvinyl)pyridazine (300 mg) obtained in the previous step, bis(acetonitrile)palladium(II) dichloride (22 mg), 2-dicyclohexylphosphino-2',4',6'-triisopropylbiphenyl (120 mg), and cesium carbonate (821 mg) in acetonitrile (5 ml) was added ethynyltriisopropylsilane (0.28 ml) under an argon atmosphere, and the mixture was stirred at 85°C for 15 hr. To the reaction mixture was added ethyl acetate, and insoluble material was filtered off through celite. The filtrate was concentrated under reduced pressure and the obtained residue was purified by silica gel column chromatography (ethyl acetate:hexane) to give a mixture (260 mg) containing a crude product of the title compound.

### Step 2

### 2-[3-(1-ethoxyvinyl)-5-ethynylpyridazin-4-yl]propan-1-ol

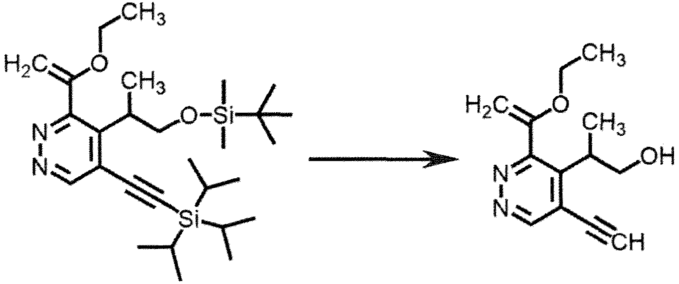

To a solution of 4-{1-[(tert-butyldimethylsilyl)oxy]propan-2-yl}-3-(1-ethoxyvinyl)-5-[(triisopropylsilyl)ethynyl]pyridazine (260 mg) obtained in the previous step in tetrahydrofuran (5 ml) was added under ice-cooling 1 M tetrabutylammonium fluoride/tetrahydrofuran solution (1.2 ml), and the mixture was stirred for 10 min. The mixture was returned to room temperature and stirred for 2 hr. Saturated ammonium chloride aqueous solution was added, and the mixture was extracted with ethyl acetate. The organic layer was dried over anhydrous sodium sulfate, and the solvent was evaporated under reduced pressure. The obtained residue was purified by silica gel column chromatography (ethyl acetate:hexane) to give a crude product (92 mg) of the title compound.

### Step 3

### 4-{1-[(tert-butyldimethylsilyl)oxy]propan-2-yl}-3-(1-ethoxyvinyl)-5-ethynylpyridazine

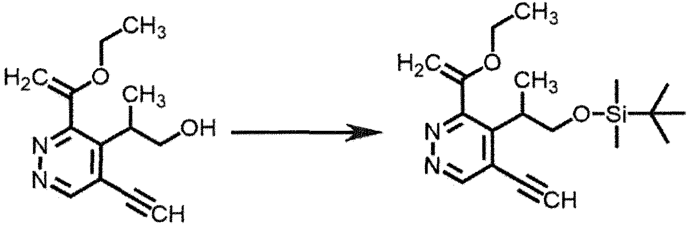

To a solution of 2-[3-(1-ethoxyvinyl)-5-ethynylpyridazin-4-yl]propan-1-ol (35 mg) obtained in the previous step in N,N-dimethylformamide (0.35 ml) were added imidazole (15 mg) and tert-butyldimethylsilyl chloride (27 mg), and the mixture was stirred at room temperature for 1 hr. The reaction mixture was concentrated under reduced pressure and the obtained residue was purified by silica gel column chromatography (ethyl acetate:hexane) to give a crude product (48 mg) of the title compound.

### Step 4

### 3-(5-{1-[(tert-butyldimethylsilyl)oxy]propan-2-yl}-6-(1-ethoxyvinyl)pyridazin-4-yl)-1-(1-fluorocyclopropyl)prop-2-yn-1-one

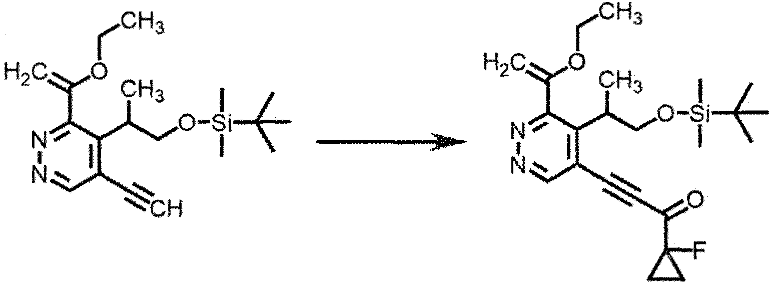

Under an argon atmosphere, to a solution of 4-{1-[(tert-butyldimethylsilyl)oxy]propan-2-yl}-3-(1-ethoxyvinyl)-5-ethynylpyridazine (18 mg) obtained in the previous step in tetrahydrofuran (0.27 ml) was added dropwise at -75°C 2 M lithium diisopropylamide/tetrahydrofuran-heptane-ethylbenzene solution (0.052 ml), and the mixture was stirred for 30 min. At -75°C, a solution of 1-fluoro-N-methoxy-N-methylcyclopropane-1-carboxamide (15 mg) obtained in Auxiliary Step 6 in tetrahydrofuran (0.27 ml) was added dropwise through a cannula. After the dropwise addition, the mixture was stirred at room temperature for 1 hr. Under ice-cooling, 2 M lithium diisopropylamide/tetrahydrofuran-heptane-ethylbenzene solution (0.052 ml) and a solution of 1-fluoro-N-methoxy-N-methylcyclopropane-1-carboxamide (15 mg) in tetrahydrofuran (0.27 ml) were added again, and the mixture was stirred at room temperature for 1 hr. To the reaction mixture was added under ice-cooling saturated ammonium chloride aqueous solution, and the mixture was extracted with ethyl acetate. The organic layer was dried over anhydrous sodium sulfate, and the solvent was evaporated under reduced pressure. The obtained residue was purified by thin layer silica gel column chromatography (ethyl acetate:hexane=1:2) to give a crude product (11 mg) of the title compound.

### Step 5

### 4-{1-[(tert-butyldimethylsilyl)oxy]propan-2-yl}-3-(1-ethoxyvinyl)-5-[3-(1-fluorocyclopropyl)-1H-pyrazol-5-yl]pyridazine

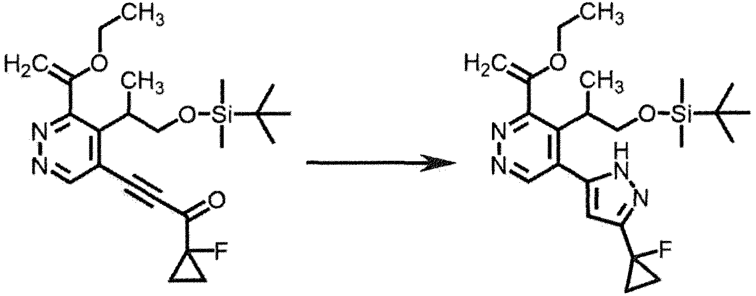

To a solution of 3-(5-{1-[(tert-butyldimethylsilyl)oxy]propan-2-yl}-6-(1-ethoxyvinyl)pyridazin-4-yl)-1-(1-fluorocyclopropyl)prop-2-yn-1-one (11 mg) obtained in the previous step in ethanol (0.33 ml) was added hydrazine monohydrate (0.0015 ml), and the mixture was stirred at 80°C for 1 hr. The reaction mixture was concentrated under reduced pressure, and the obtained residue was purified by thin layer silica gel column chromatography (ethyl acetate:chloroform=3:1) to give a crude product (6 mg) of the title compound.

### Step 6

### 2-{3-(1-ethoxyvinyl)-5-[3-(1-fluorocyclopropyl)-1H-pyrazol-5-yl]pyridazin-4-yl}propan-1-ol

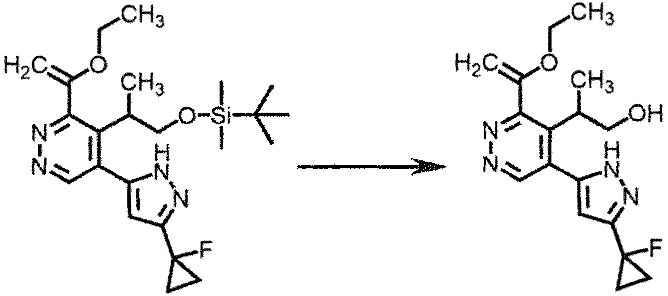

Under an argon atmosphere, to a solution of 4-{1-[(tert-butyldimethylsilyl)oxy]propan-2-yl}-3-(1-ethoxyvinyl)-5-[3-(1-fluorocyclopropyl)-1H-pyrazol-5-yl]pyridazine (6 mg) obtained in the previous step in tetrahydrofuran (0.2 ml) was added under ice-cooling 1 M tetrabutylammonium fluoride/tetrahydrofuran solution (0.02 ml), and the mixture was stirred for 10 min. After stirring at room temperature for 1 hr, saturated ammonium chloride aqueous solution was added, and the mixture was extracted with ethyl acetate. The organic layer was dried over anhydrous sodium sulfate, and the solvent was evaporated under reduced pressure. The obtained residue was purified by thin layer silica gel column chromatography (ethyl acetate) to give a crude product (4 mg) of the title compound.

### Step 7

### 4-(1-ethoxyvinyl)-9-(1-fluorocyclopropyl)-5-methyl-5,6-dihydropyrazolo[1',5':1,2]pyrido[3,4-d]pyridazine

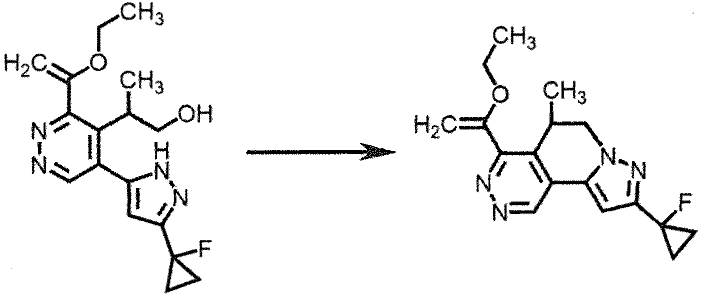

To a solution of 2-{3-(1-ethoxyvinyl)-5-[3-(1-fluorocyclopropyl)-1H-pyrazol-5-yl]pyridazin-4-yl}propan-1-ol (4 mg) obtained in the previous step in tetrahydrofuran (0.15 ml) were added triphenylphosphine (5 mg) and 1,1'-azobis(N,N-dimethylformamide) (3 mg), and the mixture was stirred at room temperature for 2 hr. The reaction mixture was concentrated under reduced pressure and the obtained residue was purified by thin layer silica gel column chromatography (ethyl acetate:hexane=9:1) to give a crude product (3.4 mg) of the title compound.

### Step 8

### 1-[9-(1-fluorocyclopropyl)-5-methyl-5,6-dihydropyrazolo[1',5':1,2]pyrido[3,4-d]pyridazin-4-yl]ethan-1-one

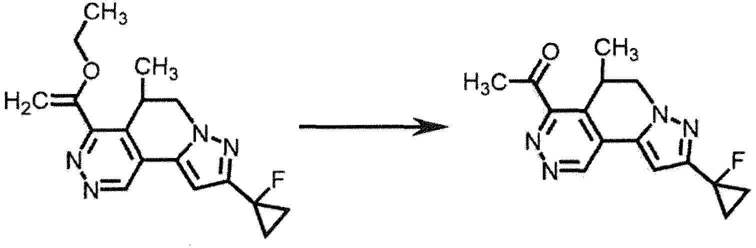

To a mixed solution of 4-(1-ethoxyvinyl)-9-(1-fluorocyclopropyl)-5-methyl-5,6-dihydropyrazolo[1',5':1,2]pyrido[3,4-d]pyridazine (3.4 mg) obtained in the previous step in tetrahydrofuran (0.05 ml) and methanol (0.05 ml) was added 6N hydrochloric acid (0.011 ml), and the mixture was stirred at room temperature for 3 hr. To the reaction mixture was added saturated aqueous sodium hydrogen carbonate solution, and the mixture was extracted with ethyl acetate. The organic layer was dried over anhydrous sodium sulfate, and the solvent was evaporated under reduced pressure. The obtained residue was purified by thin layer silica gel column chromatography (ethyl acetate:hexane=3:1) to give a crude product (3 mg) of the title compound.

### Step 9

### (R)-1,1,1-trifluoro-2-[(R)-9-(1-fluorocyclopropyl)-5-methyl-5,6-dihydropyrazolo[1',5':1,2]pyrido[3,4-d]pyridazin-4-yl]propan-2-ol and (S)-1,1,1-trifluoro-2-[(S)-9-(1-fluorocyclopropyl)-5-methyl-5,6-dihydropyrazolo[1',5':1,2]pyrido[3,4-d]pyridazin-4-yl]propan-2-ol

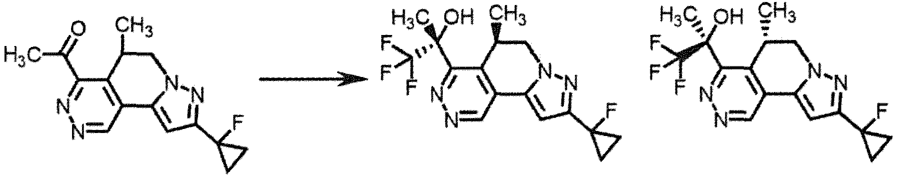

Under an argon atmosphere, a solution of 1-[9-(1-fluorocyclopropyl)-5-methyl-5,6-dihydropyrazolo[1',5':1,2]pyrido[3,4-d]pyridazin-4-yl]ethan-1-one (3 mg) obtained in the previous step in tetrahydrofuran (0.1 ml) were added under ice-cooling trimethyl(trifluoromethyl)silane (0.003 ml) and cesium fluoride (0.3 mg), and the mixture was stirred for 1 hr. Trimethyl(trifluoromethyl)silane (0.003 ml) and cesium fluoride (0.3 mg) were added again and the mixture was stirred under ice-cooling for 1 hr. To the reaction mixture were added methanol (0.1 ml) and potassium carbonate (4 mg), and the mixture was stirred at room temperature for 3 hr. To the reaction mixture was added saturated brine, and the mixture was extracted with ethyl acetate. The organic layer was dried over anhydrous sodium sulfate, and the solvent was evaporated under reduced pressure. The obtained residue was purified by thin layer silica gel column chromatography (ethyl acetate:hexane=3:2) to give the title compound (2 mg).
¹H-NMR (400 MHz, DMSO-D₆) 1.09-1.18 (m, 5H), 1.41-1.50 (m, 2H), 2.00 (s, 3H), 4.12-4.38 (m, 3H), 7.24 (s, 1H), 7.31 (s, 1H), 9.58 (s, 1H).

### Auxiliary Step 6

### 1-fluoro-N-methoxy-N-methylcyclopropane-1-carboxamide

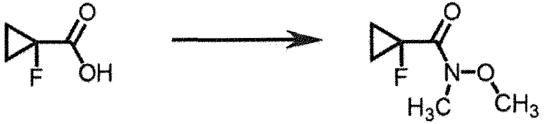

To a solution of 1-fluorocyclopropanecarboxylic acid (500 mg) in acetonitrile (5 ml) were added N,O-dimethylhydroxylamine hydrochloride (515 mg) and 1-[bis(dimethylamino)methylene]-1H-1,2,3-triazolo[4,5-b]pyridinium 3-oxide hexafluorophosphate (2.19 g). Further, N,N-diisopropylethylamine (1.68 ml) was added dropwise, and the mixture was stirred at room temperature overnight. To the reaction mixture were added sodium hydrogen carbonate aqueous solution and ethyl acetate, and the precipitated insoluble material was filtered off. Two layers of the filtrate were separated, and the aqueous layer was extracted with ethyl acetate. The organic layer was washed with 5% potassium hydrogen sulfate aqueous solution, and dried over anhydrous sodium sulfate, and the solvent was evaporated under reduced pressure. The obtained residue was purified by thin layer silica gel column chromatography (ethyl acetate:hexane) to give the title compound (453 mg).

### Production Example 14

### Synthesis of 4-[(R)-5-methyl-4-((R)-1,1,1-trifluoro-2-hydroxypropan-2-yl)-5,6-dihydropyrazolo[1',5':1,2]pyrido[3,4-d]pyridazin-9-yl]bicyclo[2.2.2]octane-1-carbonitrile and 4-[(S)-5-methyl-4-((S)-1,1,1-trifluoro-2-hydroxypropan-2-yl)-5,6-dihydropyrazolo[1',5':1,2]pyrido[3,4-d]pyridazin-9-yl]bicyclo[2.2.2]octane-1-carbonitrile (Example 103)

### Step 1

### methyl 4-{3-[5-{1-[(tert-butyldimethylsilyl)oxy]propan-2-yl}-6-(1-ethoxyvinyl)pyridazin-4-yl]-1-hydroxyprop-2-yn-1-yl}bicyclo[2.2.2]octane-1-carboxylate

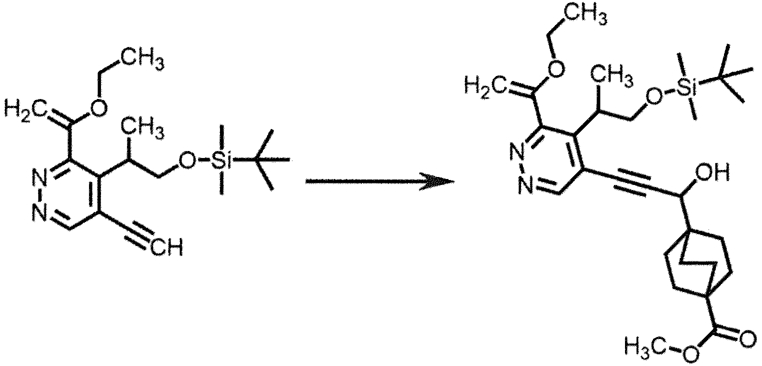

Under an argon atmosphere, to a solution of 4-{1-[(tert-butyldimethylsilyl)oxy]propan-2-yl}-3-(1-ethoxyvinyl)-5-ethynylpyridazine (20 mg) obtained in Production Example 13, Step 3, in tetrahydrofuran (0.2 ml) was added dropwise at -75°C 2 M lithium diisopropylamide/tetrahydrofuran-heptane-ethylbenzene solution (0.04 ml), and the mixture was stirred for 30 min. A solution of methyl 4-formylbicyclo[2.2.2]octane-1-carboxylate (15 mg) in tetrahydrofuran (0.2 ml) was added dropwise through a cannula, and the mixture was stirred under ice-cooling for 1 hr. To the reaction mixture was added saturated aqueous sodium hydrogen carbonate solution, and the mixture was extracted with ethyl acetate. The organic layer was dried over anhydrous sodium sulfate, and the solvent was evaporated under reduced pressure. The obtained residue was purified by thin layer silica gel column chromatography (ethyl acetate:hexane=1:1) to give a crude product (18 mg) of the title compound.

### Step 2

### methyl 4-{3-[5-{1-[(tert-butyldimethylsilyl)oxy]propan-2-yl}-6-(1-ethoxyvinyl)pyridazin-4-yl]propioloyl}bicyclo[2.2.2]octane-1-carboxylate

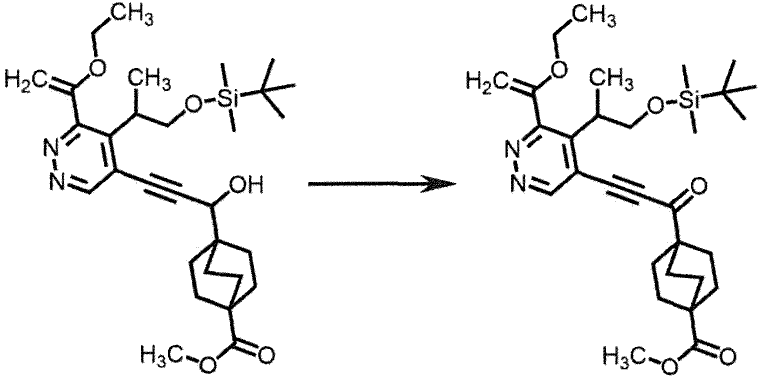

To a solution of methyl 4-{3-[5-{1-[(tert-butyldimethylsilyl)oxy]propan-2-yl}-6-(1-ethoxyvinyl)pyridazin-4-yl]-1-hydroxyprop-2-yn-1-yl}bicyclo[2.2.2]octane-1-carboxylate (9 mg) obtained in the previous step in methylene chloride (0.27 ml) was added manganese dioxide (90 mg), and the mixture was stirred at room temperature overnight. The reaction mixture was diluted with ethyl acetate, and insoluble material was filtered through celite. The filtrate was concentrated under reduced pressure and the obtained residue was purified by thin layer silica gel column chromatography (ethyl acetate:hexane=2:3) to give a crude product (7 mg) of the title compound.

### Step 3

### methyl 4-{5-[5-{1-[(tert-butyldimethylsilyl)oxy]propan-2-yl}-6-(1-ethoxyvinyl)pyridazin-4-yl]-1H-pyrazol-3-yl}bicyclo[2.2.2]octane-1-carboxylate

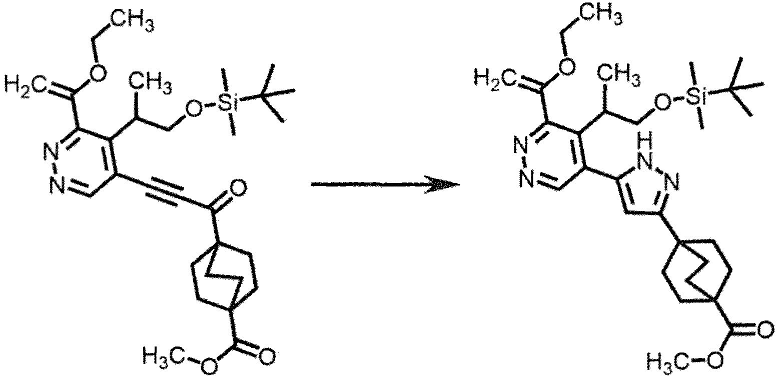

To a solution of methyl 4-{3-[5-{1-[(tert-butyldimethylsilyl)oxy]propan-2-yl}-6-(1-ethoxyvinyl)pyridazin-4-yl]propioloyl}bicyclo[2.2.2]octane-1-carboxylate (15 mg) obtained in the previous step in ethanol (0.45 ml) was added hydrazine monohydrate (0.002 ml), and the mixture was stirred at 85°C for 1 hr. The reaction mixture was concentrated under reduced pressure and the obtained residue was purified by thin layer silica gel column chromatography (ethyl acetate:hexane=4:1) to give a crude product (13 mg) of the title compound.

### Step 4

### methyl 4-{5-[6-(1-ethoxyvinyl)-5-(1-hydroxypropan-2-yl)pyridazin-4-yl]-1H-pyrazol-3-yl}bicyclo[2.2.2]octane-1-carboxylate

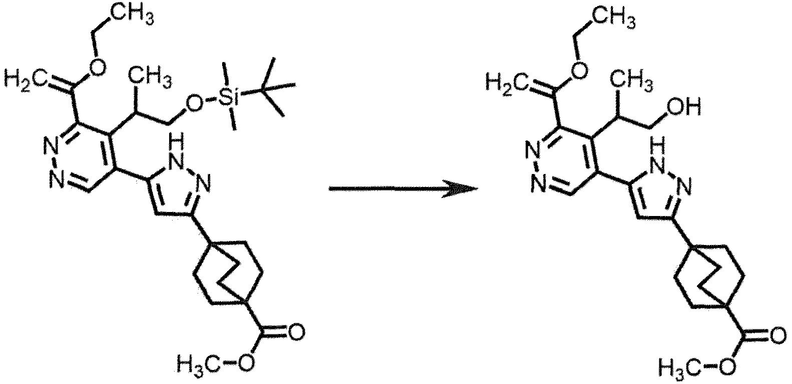

To a solution of methyl 4-{5-[5-{1-[(tert-butyldimethylsilyl)oxy]propan-2-yl}-6-(1-ethoxyvinyl)pyridazin-4-yl]-1H-pyrazol-3-yl}bicyclo[2.2.2]octane-1-carboxylate (13 mg) obtained in the previous step in tetrahydrofuran (0.45 ml) was added under ice-cooling 1 M tetrabutylammonium fluoride/tetrahydrofuran solution (0.035 ml), and the mixture was stirred for 10 min. After stirring at room temperature for 1 hr, saturated ammonium chloride aqueous solution was added, and the mixture was extracted with ethyl acetate. The organic layer was dried over anhydrous sodium sulfate, and the solvent was evaporated under reduced pressure. The obtained residue was purified by thin layer silica gel column chromatography (ethyl acetate) to give a crude product (10 mg) of the title compound.

### Step 5

### methyl 4-[4-(1-ethoxyvinyl)-5-methyl-5,6-dihydropyrazolo[1',5':1,2]pyrido[3,4-d]pyridazin-9-yl]bicyclo[2.2.2]octane-1-carboxylate

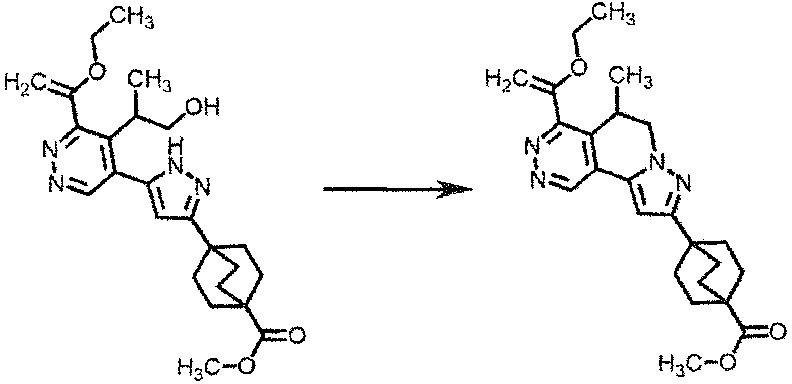

To a solution of methyl 4-{5-[6-(1-ethoxyvinyl)-5-(1-hydroxypropan-2-yl)pyridazin-4-yl]-1H-pyrazol-3-yl}bicyclo[2.2.2]octane-1-carboxylate (10 mg) obtained in the previous step in tetrahydrofuran (0.3 ml) were added triphenylphosphine (9 mg) and 1,1'-azobis(N,N-dimethylformamide) (6 mg), and the mixture was stirred at room temperature overnight. The reaction mixture was concentrated under reduced pressure and the obtained residue was purified by thin layer silica gel column chromatography (ethyl acetate; hexane=4:1) to give a crude product (9 mg) of the title compound.

### Step 6

### methyl 4-(4-acetyl-5-methyl-5,6-dihydropyrazolo[1',5':1,2]pyrido[3,4-d]pyridazin-9-yl)bicyclo[2.2.2]octane-1-carboxylate

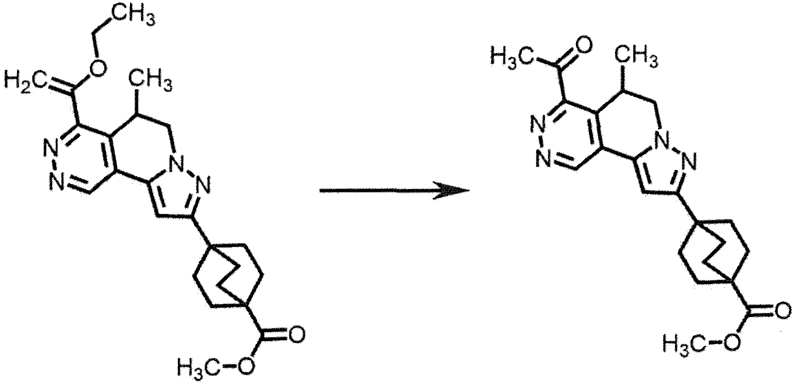

To a mixed solution of methyl 4-[4-(1-ethoxyvinyl)-5-methyl-5,6-dihydropyrazolo[1',5':1,2]pyrido[3,4-d]pyridazin-9-yl]bicyclo[2.2.2]octane-1-carboxylate (9 mg) obtained in the previous step in tetrahydrofuran (0.13 ml) and methanol (0.13 ml) was added 6N hydrochloric acid (0.021 ml), and the mixture was stirred at room temperature for 2 hr. 6N Hydrochloric acid (0.010 ml) was added, and the mixture was stirred at room temperature for 1 hr. To the reaction mixture was added saturated aqueous sodium hydrogen carbonate solution, and the mixture was extracted with ethyl acetate. The organic layer was dried over anhydrous sodium sulfate, and the solvent was evaporated under reduced pressure. The obtained residue was purified by thin layer silica gel column chromatography (ethyl acetate:chloroform=3:2) to give a crude product (7.5 mg) of the title compound.

### Step 7

### Synthesis of methyl 4-[(R)-5-methyl-4-((R)-1,1,1-trifluoro-2-hydroxypropan-2-yl)-5,6-dihydropyrazolo[1',5':1,2]pyrido[3,4-d]pyridazin-9-yl]bicyclo[2.2.2]octane-1-carboxylate and methyl 4-[(S)-5-methyl-4-((S)-1,1,1-trifluoro-2-hydroxypropan-2-yl)-5,6-dihydropyrazolo[1',5':1,2]pyrido[3,4-d]pyridazin-9-yl]bicyclo[2.2.2]octane-1-carboxylate (Example 102)

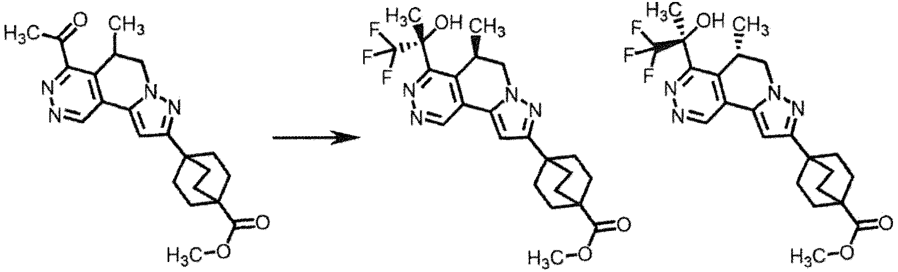

Under an argon atmosphere, to a solution of methyl 4-(4-acetyl-5-methyl-5,6-dihydropyrazolo[1',5':1,2]pyrido[3,4-d]pyridazin-9-yl)bicyclo[2.2.2]octane-1-carboxylate (7.5 mg) obtained in the previous step in tetrahydrofuran (0.23 ml) were added under ice-cooling trimethyl(trifluoromethyl)silane (0.006 ml) and cesium fluoride (0.65 mg), and the mixture was stirred for 1 hr. Trimethyl(trifluoromethyl)silane (0.006 ml) and cesium fluoride (0.65 mg) were added again, and the mixture was stirred under ice-cooling for 1 hr. To the reaction mixture were added methanol (0.23 ml) and potassium carbonate (10 mg), and the mixture was stirred at room temperature for 1 hr. To the reaction mixture was added saturated brine, and the mixture was extracted with ethyl acetate. The organic layer was dried over anhydrous sodium sulfate, and the solvent was evaporated under reduced pressure. The obtained residue was purified by thin layer silica gel column chromatography (ethyl acetate:chloroform=1:1) to give the title compound (7 mg).
¹H-NMR (400 MHz, DMSO-D₆) 1.09 (d, J= 6.73 Hz, 3H), 1.83 (s, 12H), 1.99 (s, 3H), 3.60 (s, 3H), 4.04-4.35 (m, 3H), 6.94 (s, 1H), 7.28 (s, 1H), 9.48 (s, 1H).

### Step 8

### 4-[(R)-5-methyl-4-((R)-1,1,1-trifluoro-2-hydroxypropan-2-yl)-5,6-dihydropyrazolo[1',5':1,2]pyrido[3,4-d]pyridazin-9-yl]bicyclo[2.2.2]octane-1-carboxylic acid and 4-[(S)-5-methyl-4-((S)-1,1,1-trifluoro-2-hydroxypropan-2-yl)-5,6-dihydropyrazolo[1',5':1,2]pyrido[3,4-d]pyridazin-9-yl]bicyclo[2.2.2]octane-1-carboxylic acid

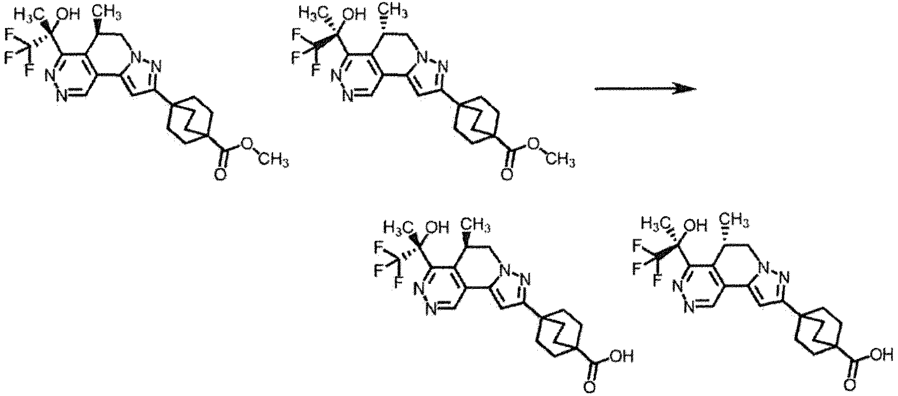

To a mixed solution of the mixture (5.4 mg) of methyl 4-[(R)-5-methyl-4-((R)-1,1,1-trifluoro-2-hydroxypropan-2-yl)-5,6-dihydropyrazolo[1',5':1,2]pyrido[3,4-d]pyridazin-9-yl]bicyclo[2.2.2]octane-1-carboxylate and methyl 4-[(S)-5-methyl-4-((S)-1,1,1-trifluoro-2-hydroxypropan-2-yl)-5,6-dihydropyrazolo[1',5':1,2]pyrido[3,4-d]pyridazin-9-yl]bicyclo[2.2.2]octane-1-carboxylate obtained in the previous step in tetrahydrofuran (0.05 ml) and ethanol (0.2 ml) was added 4N sodium hydroxide aqueous solution (0.029 ml), and the mixture was stirred at 60°C overnight. To the reaction mixture was added 2N hydrochloric acid (0.06 ml), and the mixture was extracted with ethyl acetate. The organic layer was dried over anhydrous sodium sulfate, and the solvent was evaporated under reduced pressure. The obtained residue (5.4 mg) was used as it was in the next step.

### Step 9

### 4-[(R)-5-methyl-4-((R)-1,1,1-trifluoro-2-hydroxypropan-2-yl)-5,6-dihydropyrazolo[1',5':1,2]pyrido[3,4-d]pyridazin-9-yl]bicyclo[2.2.2]octane-1-carboxamide and 4-[(S)-5-methyl-4-((S)-1,1,1-trifluoro-2-hydroxypropan-2-yl)-5,6-dihydropyrazolo[1',5':1,2]pyrido[3,4-d]pyridazin-9-yl]bicyclo[2.2.2]octane-1-carboxamide

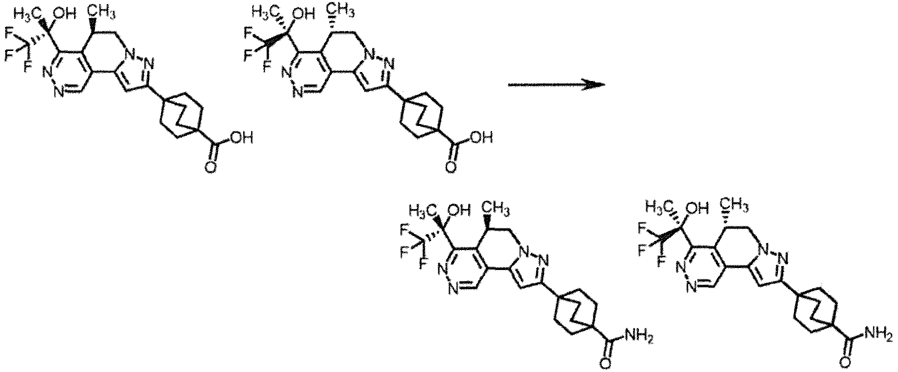

To a solution of the mixture (5.4 mg) of 4-[(R)-5-methyl-4-((R)-1,1,1-trifluoro-2-hydroxypropan-2-yl)-5,6-dihydropyrazolo[1',5':1,2]pyrido[3,4-d]pyridazin-9-yl]bicyclo[2.2.2]octane-1-carboxylic acid and 4-[(S)-5-methyl-4-((S)-1,1,1-trifluoro-2-hydroxypropan-2-yl)-5,6-dihydropyrazolo[1',5':1,2]pyrido[3,4-d]pyridazin-9-yl]bicyclo[2.2.2]octane-1-carboxylic acid obtained in the previous step in N,N-dimethylformamide (0.3 ml) were added 7 M ammonia/methanol solution (0.009 ml) and 1-[bis(dimethylamino)methylene]-1H-1,2,3-triazolo[4,5-b]pyridinium 3-oxide hexafluorophosphate (6.8 mg), and the mixture was stirred at room temperature overnight. The reaction mixture was concentrated under reduced pressure and the obtained residue was purified by thin layer silica gel column chromatography (ethyl acetate:methanol=10:1) to give a crude product (5.4 mg) of the title compound.

### Step 10

### 4-[(R)-5-methyl-4-((R)-1,1,1-trifluoro-2-hydroxypropan-2-yl)-5,6-dihydropyrazolo[1',5':1,2]pyrido[3,4-d]pyridazin-9-yl]bicyclo[2.2.2]octane-1-carbonitrile and 4-[(S)-5-methyl-4-((S)-1,1,1-trifluoro-2-hydroxypropan-2-yl)-5,6-dihydropyrazolo[1',5':1,2]pyrido[3,4-d]pyridazin-9-yl]bicyclo[2.2.2]octane-1-carbonitrile

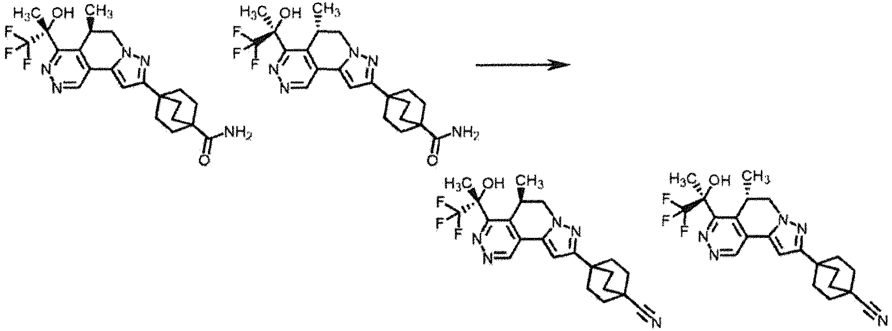

To a solution of the mixture (5.4 mg) of 4-[(R)-5-methyl-4-((R)-1,1,1-trifluoro-2-hydroxypropan-2-yl)-5,6-dihydropyrazolo[1',5':1,2]pyrido[3,4-d]pyridazin-9-yl]bicyclo[2.2.2]octane-1-carboxamide and 4-[(S)-5-methyl-4-((S)-1,1,1-trifluoro-2-hydroxypropan-2-yl)-5,6-dihydropyrazolo[1',5':1,2]pyrido[3,4-d]pyridazin-9-yl]bicyclo[2.2.2]octane-1-carboxamide obtained in the previous step in tetrahydrofuran (0.27 ml) were added under ice-cooling triethylamine (0.008 ml) and trifluoroacetic anhydride (0.003 ml), and the mixture was stirred for 30 min. Triethylamine (0.008 ml) and trifluoroacetic anhydride (0.003 ml) were added, and the mixture was stirred for 30 min. To the reaction mixture was added saturated aqueous sodium hydrogen carbonate solution, and the mixture was extracted with ethyl acetate. The organic layer was dried over anhydrous sodium sulfate, and the solvent was evaporated under reduced pressure. The obtained residue was purified by thin layer silica gel column chromatography (ethyl acetate:hexane=3:2) to give the title compound (4 mg).
¹H-NMR (400 MHz, DMSO-D₆) 1.09 (d, J= 6.70 Hz, 3H), 1.80-1.90 (m, 6H), 1.94-2.04 (m, 9H), 4.04-4.35 (m, 3H), 6.94 (s, 1H), 7.27 (s, 1H), 9.48 (s, 1H).

### Production Example 15

Synthesis of 3-[(R)-5-methyl-4-((R)-1,1,1-trifluoro-2-hydroxypropan-2-yl)-5,6-dihydropyrazolo[1',5':1,2]pyrido[3,4-d]pyridazin-9-yl]bicyclo[1.1.1]pentane-1-carbonitrile and 3-[(S)-5-methyl-4-((S)-1,1,1-trifluoro-2-hydroxypropan-2-yl)-5,6-dihydropyrazolo[1',5':1,2]pyrido[3,4-d]pyridazin-9-yllbicyclo[1.1.1]pentane-1-carbonitrile (Example 104)

### Step 1

### methyl 3-{3-[5-{1-[(tert-butyldimethylsilyl)oxy]propan-2-yl}-6-(1-ethoxyvinyl)pyridazin-4-yl]-1-hydroxyprop-2-yn-1-yl}bicyclo[1.1.1]pentane-1-carboxylate

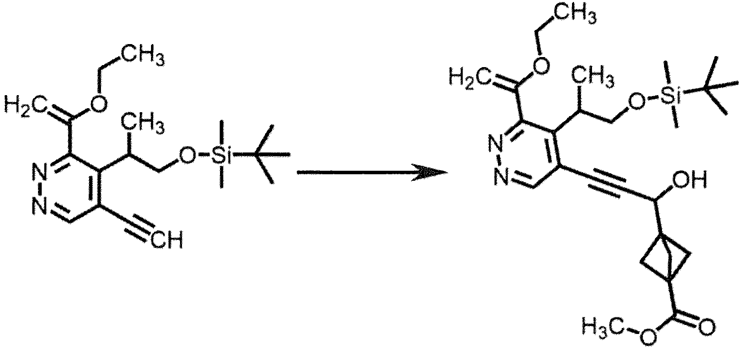

Under an argon atmosphere, to a solution of 4-{1-[(tertbutyldimethylsilyl)oxy]propan-2-yl}-3-(1-ethoxyvinyl)-5-ethynylpyridazine (65 mg) obtained in Production Example 13, Step 3, in tetrahydrofuran (0.65 ml) was added dropwise at - 75°C 2 M lithium diisopropylamide/tetrahydrofuran-heptane-ethylbenzene solution (0.13 ml), and the mixture was stirred for 30 min. A solution of methyl 3-formylbicyclo[1.1.1]pentane-1-carboxylate (38 mg) in tetrahydrofuran (0.65 ml) was added dropwise through a cannula, and the mixture was stirred at -40°C for 1 hr. 2 M lithium diisopropylamide/tetrahydrofuran-heptane-ethylbenzene solution (0.13 ml) and a solution of methyl 3-formylbicyclo[1.1.1]pentane-1-carboxylate (38 mg) in tetrahydrofuran (0.65 ml) were added again, and the mixture was stirred at -30°C for 1 hr. To the reaction mixture was added saturated aqueous sodium hydrogen carbonate solution, and the mixture was extracted with ethyl acetate. The organic layer was dried over anhydrous sodium sulfate, and the solvent was evaporated under reduced pressure. The obtained residue was purified by silica gel column chromatography (ethyl acetate:hexane) to give a crude product (40 mg) of the title compound.

### Step 2

### methyl 3-{3-[5-{1-[(tert-butyldimethylsilyl)oxy]propan-2-yl}-6-(1-ethoxyvinyl)pyridazin-4-yl]propioloyl}bicyclo[1.1.1]pentane-1-carboxylate

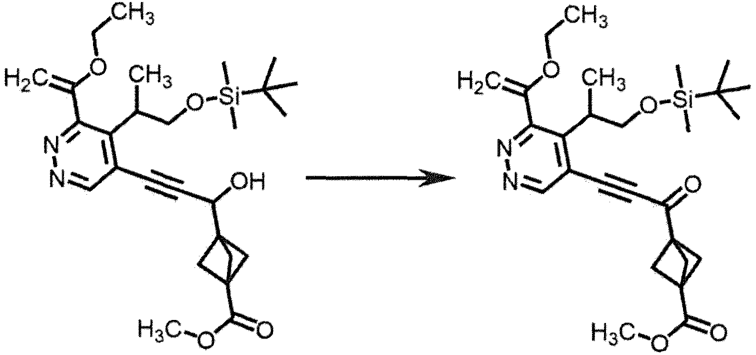

To a solution of methyl 3-{3-[5-{1-[(tertbutyldimethylsilyl)oxy]propan-2-yl}-6-(1-ethoxyvinyl)pyridazin-4-yl]-1-hydroxyprop-2-yn-1-yl}bicyclo[1.1.1]pentane-1-carboxylate (40 mg) obtained in the previous step in methylene chloride (1.2 ml) was added manganese dioxide (400 mg), and the mixture was stirred at room temperature overnight. The reaction mixture was diluted with ethyl acetate, and insoluble material was filtered through celite. The filtrate was concentrated under reduced pressure and the obtained residue was purified by silica gel column chromatography (ethyl acetate:hexane) to give a crude product (25 mg) of the title compound.

### Step 3

### methyl 3-{5-[5-{1-[(tert-butyldimethylsilyl)oxy]propan-2-yl}-6-(1-ethoxyvinyl)pyridazin-4-yl]-1H-pyrazol-3-yl}bicyclo[1.1.1]pentane-1-carboxylate

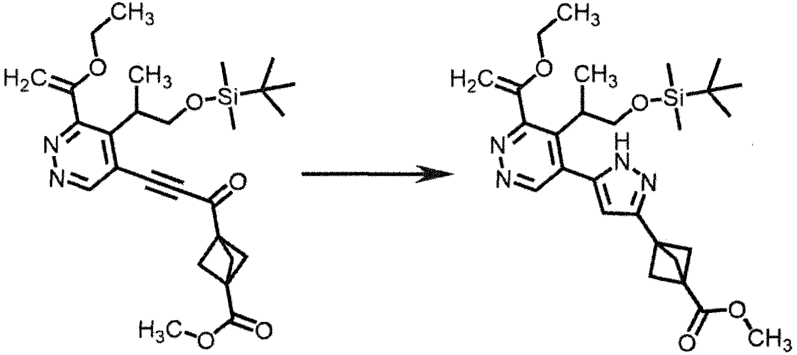

To a solution of methyl 3-{3-[5-{1-[(tertbutyldimethylsilyl) oxy]propan-2-yl}-6-(1-ethoxyvinyl)pyridazin-4-yl]propioloyl}bicyclo[1.1.1]pentane-1-carboxylate (25 mg) obtained in the previous step in ethanol (1.0 ml) was added hydrazine monohydrate (0.003 ml), and the mixture was stirred at 85°C for 1 hr. The reaction mixture was concentrated under reduced pressure and the obtained residue was purified by thin layer silica gel column chromatography (ethyl acetate:chloroform=1:1) to give a crude product (15 mg) of the title compound.

### Step 4

### methyl 3-{5-[6-(1-ethoxyvinyl)-5-(1-hydroxypropan-2-yl)pyridazin-4-yl]-1H-pyrazol-3-yl}bicyclo[1.1.1]pentane-1-carboxylate

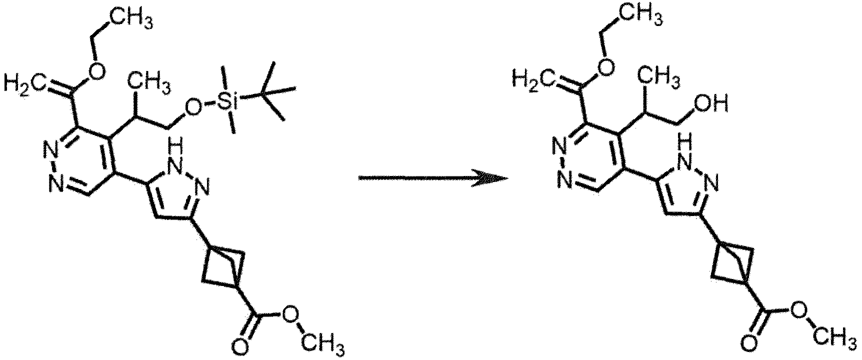

To a solution of methyl 3-{5-[5-{1-[(tertbutyldimethylsilyl)oxy]propan-2-yl}-6-(1-ethoxyvinyl)pyridazin-4-yl]-1H-pyrazol-3-yl}bicyclo[1.1.1]pentane-1-carboxylate (15 mg) obtained in the previous step in tetrahydrofuran (0.5 ml) was added under ice-cooling 1 M tetrabutylammonium fluoride/tetrahydrofuran solution (0.044 ml), and the mixture was stirred for 10 min. The mixture was returned to room temperature and stirred for 1 hr, saturated ammonium chloride aqueous solution was added, and the mixture was extracted with ethyl acetate. The organic layer was dried over anhydrous sodium sulfate, and the solvent was evaporated under reduced pressure. The obtained residue was purified by thin layer silica gel column chromatography (ethyl acetate) to give a crude product (11.5 mg) of the title compound.

### Step 5

### methyl 3-[4-(1-ethoxyvinyl)-5-methyl-5,6-dihydropyrazolo[1',5':1,2]pyrido[3,4-d]pyridazin-9-yl]bicyclo[1.1.1]pentane-1-carboxylate

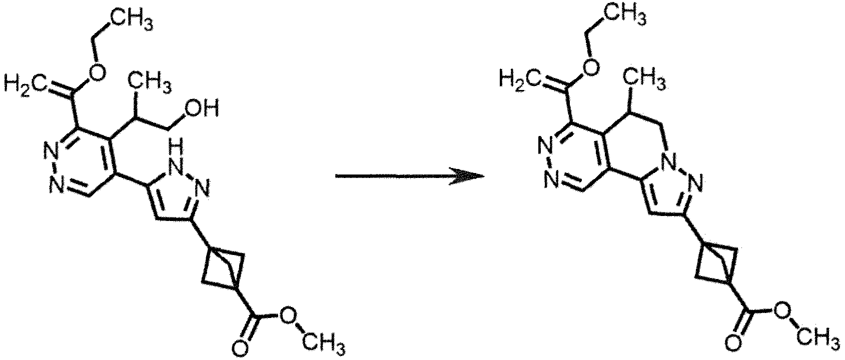

To a solution of methyl 3-{5-[6-(1-ethoxyvinyl)-5-(1-hydroxypropan-2-yl)pyridazin-4-yl]-1H-pyrazol-3-yl}bicyclo[1.1.1]pentane-1-carboxylate (11.5 mg) obtained in the previous step in tetrahydrofuran (0.3 ml) were added triphenylphosphine (11.5 mg) and 1,1'-azobis(N,N-dimethylformamide) (7.5 mg), and the mixture was stirred at room temperature overnight. The reaction mixture was concentrated under reduced pressure and the obtained residue was purified by thin layer silica gel column chromatography (ethyl acetate; hexane=9:1) to give a crude product (9.5 mg) of the title compound.

### Step 6

### methyl 3-(4-acetyl-5-methyl-5,6-dihydropyrazolo[1',5':1,2]pyrido[3,4-d]pyridazin-9-yl)bicyclo[1.1.1]pentane-1-carboxylate

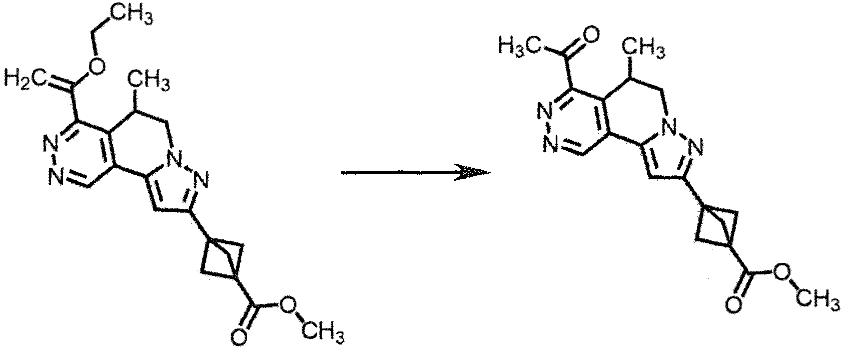

To a mixed solution of methyl 3-[4-(1-ethoxyvinyl)-5-methyl-5,6-dihydropyrazolo[1',5':1,2]pyrido[3,4-d]pyridazin-9-yl]bicyclo[1.1.1]pentane-1-carboxylate (9 mg) obtained in the previous step in tetrahydrofuran (0.13 ml) and methanol (0.13 ml) was added 6N hydrochloric acid (0.025 ml), and the mixture was stirred at room temperature for 2 hr. 6N Hydrochloric acid (0.025 ml) was added, and the mixture was stirred at room temperature for 2 hr. To the reaction mixture was added saturated aqueous sodium hydrogen carbonate solution, and the mixture was extracted with ethyl acetate. The organic layer was dried over anhydrous sodium sulfate, and the solvent was evaporated under reduced pressure. The obtained residue was purified by thin layer silica gel column chromatography (ethyl acetate:chloroform=2:1) to give a crude product (7.5 mg) of the title compound.

### Step 7

### methyl 3-[(R)-5-methyl-4-((R)-1,1,1-trifluoro-2-hydroxypropan-2-yl)-5,6-dihydropyrazolo[1',5':1,2]pyrido[3,4-d]pyridazin-9-yl]bicyclo[1.1.1]pentane-1-carboxylate and methyl 3-[(S)-5-methyl-4-((S)-1,1,1-trifluoro-2-hydroxypropan-2-yl)-5,6-dihydropyrazolo[1',5':1,2]pyrido[3,4-d]pyridazin-9-yl]bicyclo[1.1.1]pentane-1-carboxylate

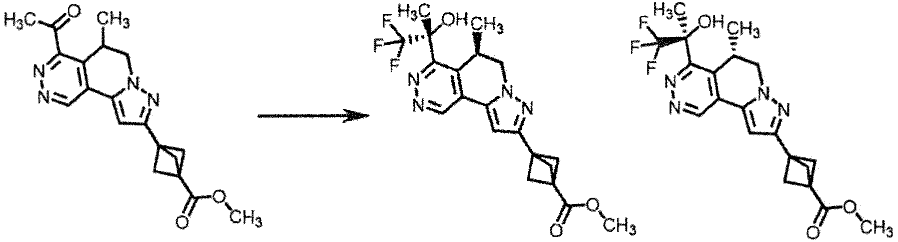

Under an argon atmosphere, to a solution of methyl 3-(4-acetyl-5-methyl-5,6-dihydropyrazolo[1',5':1,2]pyrido[3,4-d]pyridazin-9-yl)bicyclo[1.1.1]pentane-1-carboxylate (7.5 mg) obtained in the previous step in tetrahydrofuran (0.25 ml) were added under ice-cooling trimethyl(trifluoromethyl)silane (0.0065 ml) and cesium fluoride (1.0 mg), and the mixture was stirred for 1 hr. Trimethyl(trifluoromethyl)silane (0.0065 ml) and cesium fluoride (1.0 mg) were added again, and the mixture was stirred under ice-cooling for 1 hr. To the reaction mixture were added methanol (0.25 ml) and potassium carbonate (12 mg), and the mixture was stirred at room temperature for 2 hr. To the reaction mixture was added saturated brine, and the mixture was extracted with ethyl acetate. The organic layer was dried over anhydrous sodium sulfate, and the solvent was evaporated under reduced pressure. The obtained residue was purified by thin layer silica gel column chromatography (ethyl acetate:chloroform=3:2) to give a crude product (6.5 mg) of the title compound.

### Step 8

### 3-[(R)-5-methyl-4-((R)-1,1,1-trifluoro-2-hydroxypropan-2-yl)-5,6-dihydropyrazolo[1',5':1,2]pyrido[3,4-d]pyridazin-9-yl]bicyclo[1.1.1]pentane-1-carboxylic acid and 3-[(S)-5-methyl-4-((S)-1,1,1-trifluoro-2-hydroxypropan-2-yl)-5,6-dihydropyrazolo[1',5':1,2]pyrido[3,4-d]pyridazin-9-yl]bicyclo[1.1.1]pentane-1-carboxylic acid

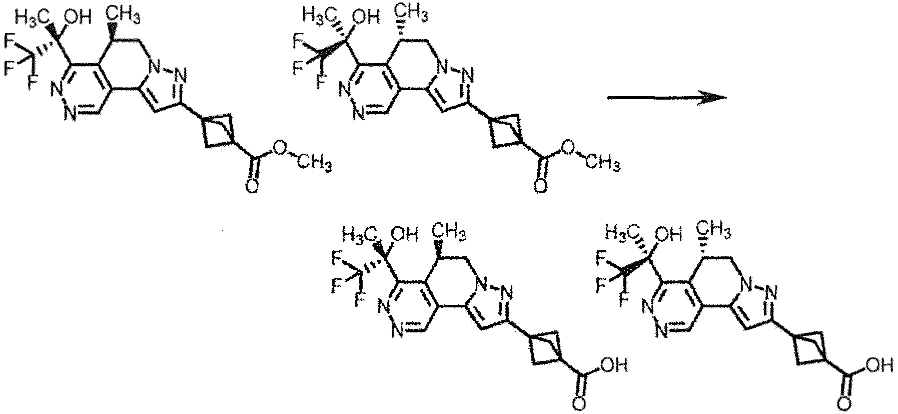

To a mixed solution of the mixture (6.5 mg) of methyl 3-[(R)-5-methyl-4-((R)-1,1,1-trifluoro-2-hydroxypropan-2-yl)-5,6-dihydropyrazolo[1',5':1,2]pyrido[3,4-d]pyridazin-9-yl]bicyclo[1.1.1]pentane-1-carboxylate and methyl 3-[(S)-5-methyl-4-((S)-1,1,1-trifluoro-2-hydroxypropan-2-yl)-5,6-dihydropyrazolo[1',5':1,2]pyrido[3,4-d]pyridazin-9-yl]bicyclo[1.1.1]pentane-1-carboxylate obtained in the previous step in tetrahydrofuran (0.065 ml) and ethanol (0.26 ml) was added 4N sodium hydroxide aqueous solution (0.039 ml), and the mixture was stirred at 60°C for 2 hr. To the reaction mixture was added 2N hydrochloric acid (0.077 ml), and the mixture was extracted with ethyl acetate. The organic layer was dried over anhydrous sodium sulfate, and the solvent was evaporated under reduced pressure. The obtained residue (6 mg) was used as it was in the next step.

### Step 9

### 3-[(R)-5-methyl-4-((R)-1,1,1-trifluoro-2-hydroxypropan-2-yl)-5,6-dihydropyrazolo[1',5':1,2]pyrido[3,4-d]pyridazin-9-yl]bicyclo[1.1.1]pentane-1-carboxamide and 3-[(S)-5-methyl-4-((S)-1,1,1-trifluoro-2-hydroxypropan-2-yl)-5,6-dihydropyrazolo[1',5':1,2]pyrido[3,4-d]pyridazin-9-yl]bicyclo[1.1.1]pentane-1-carboxamide

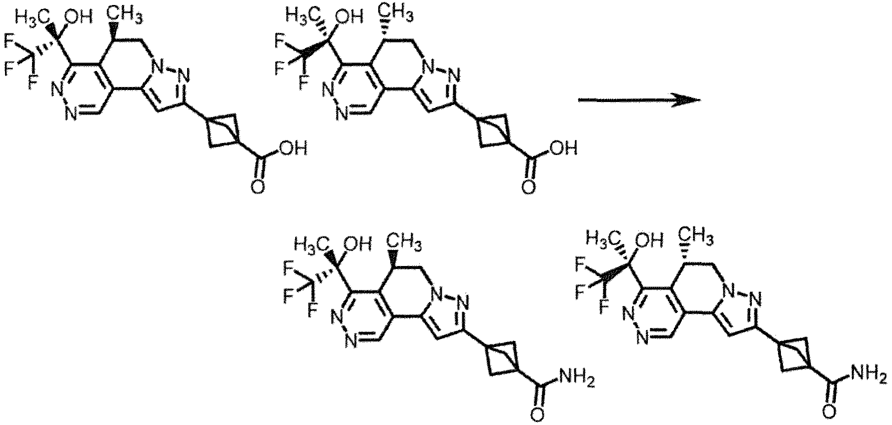

To a solution of 3-[(R)-5-methyl-4-((R)-1,1,1-trifluoro-2-hydroxypropan-2-yl)-5,6-dihydropyrazolo[1',5':1,2]pyrido[3,4-d]pyridazin-9-yl]bicyclo[1.1.1]pentane-1-carboxylic acid and 3-[(S)-5-methyl-4-((S)-1,1,1-trifluoro-2-hydroxypropan-2-yl)-5,6-dihydropyrazolo[1',5':1,2]pyrido[3,4-d]pyridazin-9-yl]bicyclo[1.1.1]pentane-1-carboxylic acid mixture (6 mg) obtained in the previous step in N,N-dimethylformamide (0.32 ml) were added 7 M ammonia/methanol solution (0.011 ml) and 1-[bis(dimethylamino)methylene]-1H-1,2,3-triazolo[4,5-b]pyridinium 3-oxide hexafluorophosphate (9.0 mg), and the mixture was stirred at room temperature overnight. The reaction mixture was concentrated under reduced pressure and the obtained residue was purified by thin layer silica gel column chromatography (ethyl acetate:methanol=10:1) to give a crude product (6.0 mg) of the title compound.

### Step 10

### 3-[(R)-5-methyl-4-((R)-1,1,1-trifluoro-2-hydroxypropan-2-yl)-5,6-dihydropyrazolo[1',5':1,2]pyrido[3,4-d]pyridazin-9-yl]bicyclo[1.1.1]pentane-1-carbonitrile and 3-[(S)-5-methyl-4-((S)-1,1,1-trifluoro-2-hydroxypropan-2-yl)-5,6-dihydropyrazolo[1',5':1,2]pyrido[3,4-d]pyridazin-9-yl]bicyclo[1.1.1]pentane-1-carbonitrile

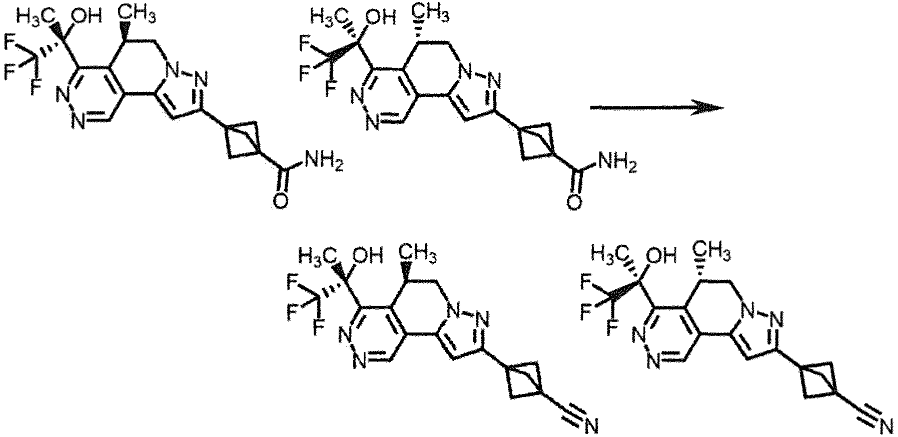

To a solution of the mixture (6 mg) of 3-[(R)-5-methyl-4-((R)-1,1,1-trifluoro-2-hydroxypropan-2-yl)-5,6-dihydropyrazolo[1',5':1,2]pyrido[3,4-d]pyridazin-9-yl]bicyclo[1.1.1]pentane-1-carboxamide and 3-[(S)-5-methyl-4-((S)-1,1,1-trifluoro-2-hydroxypropan-2-yl)-5,6-dihydropyrazolo[1',5':1,2]pyrido[3,4-d]pyridazin-9-yl]bicyclo[1.1.1]pentane-1-carboxamide obtained in the previous step in tetrahydrofuran (0.3 ml) were added under ice-cooling triethylamine (0.010 ml) and trifluoroacetic anhydride (0.004 ml), and the mixture was stirred for 30 min. To the reaction mixture was added saturated aqueous sodium hydrogen carbonate solution, and insoluble material was filtered off. The filtrate was concentrated under reduced pressure and the obtained residue was purified by thin layer silica gel column chromatography (ethyl acetate:hexane=3:2) to give the title compound (3.5 mg).
¹H-NMR (400 MHz, DMSO-D₆) 1.09 (d, J= 6.94 Hz, 3H), 1.98 (s, 3H), 2.57 (s, 6H), 4.08-4.37 (m, 3H), 6.99 (s, 1H), 7.29 (s, 1H), 9.49 (s, 1H).

### Production Example 16

Synthesis of (1S,4r)-1-methyl-4-((S)-4-methyl-3-((R)-1,1,1-trifluoro-2-hydroxypropan-2-yl)-4,5-dihydro-7H-isoxazolo[5,4-e]indazol-7-yl)cyclohexan-1-ol (Example 105)

### Step 1

### (1R,2S,5R)-2-isopropyl-5-methylcyclohexyl 3-oxobutanoate

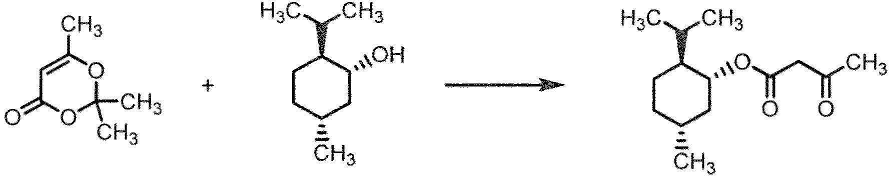

(-)-Menthol (19.9 g) was mixed with toluene (100 ml). At room temperature, 2,2,6-trimethyl-1,3-dioxin-4-one (20 g) was added, and the mixture was stirred using a Dean-Stark apparatus at 140°C for 1.5 hr. The reaction mixture was concentrated under reduced pressure. The obtained residue was distilled under reduced pressure to give the title compound (28.18 g).
¹H-NMR (400 MHz, CDCl₃) 0.77 (d, J=6.73Hz, 3H), 0.79 - 2.09 (m, 15H), 2.26 (s, 3H), 3.43 (s, 2H), 4.74 (td, J=11.03, 4.24Hz, 1H)

### Step 2

### (1R,2S,5R)-2-isopropyl-5-methylcyclohexyl (1R,2S)-2-methyl-4,6-dioxocyclohexane-1-carboxylate

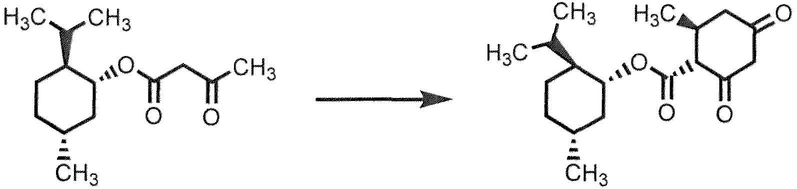

(1R,2S,5R)-2-Isopropyl-5-methylcyclohexyl 3-oxobutanoate (28.18 g) obtained in the previous step was mixed with tert-butanol (127 ml). Potassium tert-butoxide (12.91 g) was added, and the mixture was stirred at 125°C for 45 min. To the reaction mixture was added dropwise ethyl crotonate (13.72 ml), and the mixture was heated under reflux for 1.5 hr. After cooling the reaction mixture to room temperature, 5 v/v% sulfuric acid aqueous solution (100 ml) and dichloromethane (120 ml) were added and the layers were separated. The aqueous layer was extracted twice with dichloromethane (120 ml). The combined organic layer was dried over sodium sulfate and concentrated under reduced pressure. The obtained residue was mixed with toluene (120 ml), and the mixture was stirred at 120°C for 30 min. The mixture was stirred overnight while allowing to cool to room temperature. The obtained solid was collected by filtration to give the title compound (7.76 g). ¹H-NMR (400 MHz, CDCl₃) 0.78 (d, J=6.73Hz, 3H), 0.91 (t, J=7.11Hz, 6H), 0.98 - 1.07 (m, 1H), 1.10 (d, J=6.73Hz, 3H), 1.36 - 1.46 (m, 1H), 1.46 - 1.78 (m, 5H), 1.82 - 1.96 (m, 1H), 1.97 - 2.09 (m, 1H), 2.39 (dd, J=15.71, 8.98Hz, 1H), 2.56 - 2.68 (m, 1H), 2.81 (dd, J=15.71, 4.49Hz, 1H), 3.30 (d, J=8.98Hz, 1H), 3.40 (d, J=17.20Hz, 1H), 3.63 (d, J=17.20Hz, 1H), 4.79 (td, J=10.85, 4.49Hz, 1H)

### Step 3

### (1R,2S,5R)-2-isopropyl-5-methylcyclohexyl (1R,2S,E)-3-((dimethylamino)methylene)-6-methyl-2,4-dioxocyclohexane-1-carboxylate

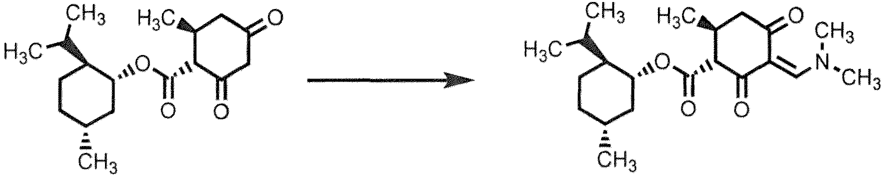

(1R,2S,5R)-2-Isopropyl-5-methylcyclohexyl (1R,2S)-2-methyl-4,6-dioxocyclohexane-1-carboxylate (7.76 g) obtained in the previous step was mixed with N,N-dimethylformamide dimethyl acetal (6.74 ml). The reaction mixture was stirred at 120°C for 1 hr. The reaction mixture was concentrated under reduced pressure and azeotroped twice with toluene. The obtained residue was dried under reduced pressure to give a crude product (8.7 g) of the title compound.

### Step 4

### mixture of (1R,2S,5R)-2-isopropyl-5-methylcyclohexyl (5R,6S)-1-(4-methoxybenzyl)-6-methyl-4-oxo-4,5,6,7-tetrahydro-1H-indazole-5-carboxylate and (1R,2S,5R)-2-isopropyl-5-methylcyclohexyl (5R,6S)-2-(4-methoxybenzyl)-6-methyl-4-oxo-4,5,6,7-tetrahydro-2H-indazole-5-carboxylate

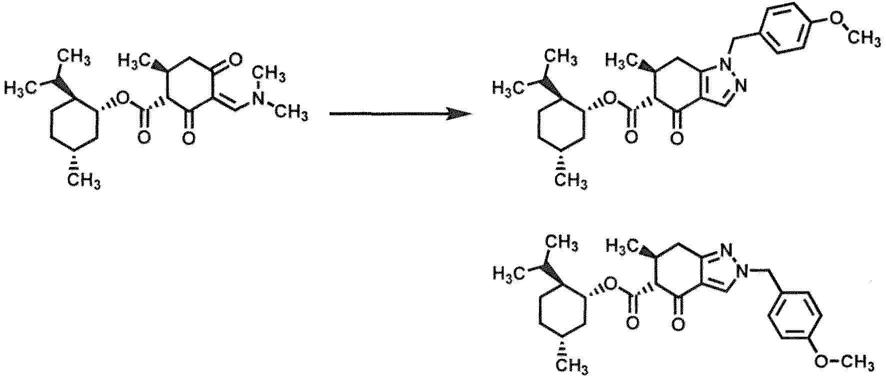

To the crude product (8 g) of (1R,2S,5R)-2-isopropyl-5-methylcyclohexyl (1R,2S,E)-3-((dimethylamino)methylene)-6-methyl-2,4-dioxocyclohexane-1-carboxylate obtained in the previous step and (4-methoxybenzyl)hydrazine hydrochloride (4.34 g) was added isopropanol (120 ml), and the mixture was stirred at 50°C for 30 min. To the reaction mixture was added acetic acid (6.27 ml), and the mixture was stirred at 140°C for 6 hr. The reaction mixture was allowed to cool to room temperature and concentrated under reduced pressure. To the obtained residue were added ethyl acetate and 1N sodium hydroxide aqueous solution, and the mixture was extracted with ethyl acetate. The organic layer was washed successively with water and saturated aqueous sodium chloride solution. After filtration through a phase separator, the filtrate was concentrated under reduced pressure to give a crude product (9.85 g) of the title compound.

### Step 5

### mixture of (S)-1-(4-methoxybenzyl)-6-methyl-1,5,6,7-tetrahydro-4H-indazol-4-one and (S)-2-(4-methoxybenzyl)-6-methyl-2,5,6,7-tetrahydro-4H-indazol-4-one

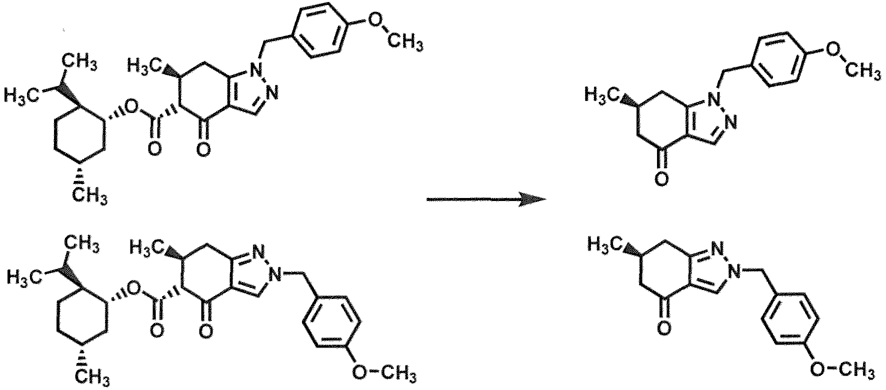

The crude product (9.85 g) of the mixture of (1R,2S,5R)-2-isopropyl-5-methylcyclohexyl (5R,6S)-1-(4-methoxybenzyl)-6-methyl-4-oxo-4,5,6,7-tetrahydro-1H-indazole-5-carboxylate and (1R,2S,5R)-2-isopropyl-5-methylcyclohexyl (5R,6S)-2-(4-methoxybenzyl)-6-methyl-4-oxo-4,5,6,7-tetrahydro-2H-indazole-5-carboxylate obtained in the previous step was mixed with DMSO (148 ml)-water (4.93 ml). To the mixture was added 4-dimethylaminopyridine (4.25 g), and the mixture was stirred at 160°C for 4 hr. At room temperature, water was added to the reaction mixture, and the mixture was extracted with toluene. The organic layer was washed successively with water and saturated aqueous sodium chloride solution, and filtered through a phase separator, and the filtrate was concentrated under reduced pressure. The obtained residue was purified by silica gel column chromatography (hexane:ethyl acetate=80:20 to 24:76) to give a mixture (3.7 g) of the title compound.

### Step 6

### mixture of ethyl (4S)-8b-hydroxy-6-(4-methoxybenzyl)-4-methyl-3a,5,6,8b-tetrahydro-4H-isoxazolo[5,4-e]indazole-3-carboxylate and ethyl (4S)-8b-hydroxy-7-(4-methoxybenzyl)-4-methyl-3a,5,7,8b-tetrahydro-4H-isoxazolo[5,4-e]indazole-3-carboxylate

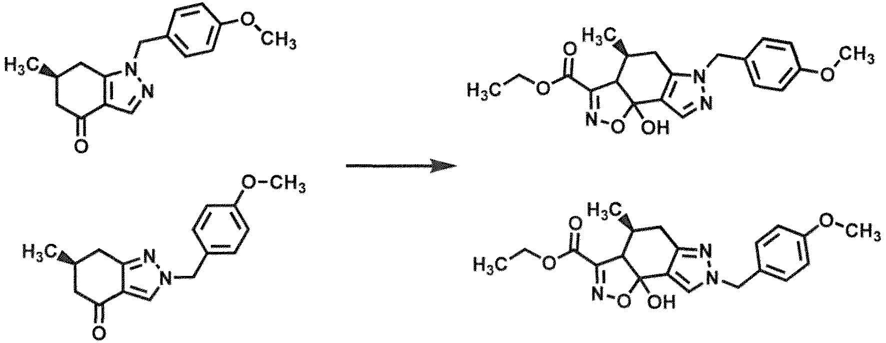

ethyl 2-chloro-2-(hydroxyimino)acetate (2.489 g) was mixed with tetrahydrofuran (37 ml). To this solution was added dropwise at -78°C 1.3 M lithium bis(trimethylsilyl)amide/tetrahydrofuran solution (13.69 ml) to prepare a nitrile oxide solution. In a separate reaction vessel, the mixture (3.7 g) of (S)-1-(4-methoxybenzyl)-6-methyl-1,5,6,7-tetrahydro-4H-indazol-4-one and (S)-2-(4-methoxybenzyl)-6-methyl-2,5,6,7-tetrahydro-4H-indazol-4-one obtained in the previous step was mixed with tetrahydrofuran (37 ml). To this solution was added dropwise at -78°C 1.3 M lithium bis(trimethylsilyl)amide/tetrahydrofuran solution (12.63 ml). The reaction mixture was added dropwise through a cannula to the aforementioned nitrile oxide solution cooled to -78°C, and the mixture was stirred for 1 hr. The reaction mixture was stirred for 1 hr while raising the temperature to room temperature. To the reaction mixture was added dropwise at room temperature 2N hydrochloric acid (41.1 ml), saturated aqueous sodium hydrogen carbonate solution was added, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=40:60 to 85:15) to give a crude product (3.4 g) of the title compound.

### Step 7

### mixture of ethyl (S)-6-(4-methoxybenzyl)-4-methyl-5,6-dihydro-4H-isoxazolo[5,4-e]indazole-3-carboxylate and ethyl (S)-7-(4-methoxybenzyl)-4-methyl-5,7-dihydro-4H-isoxazolo[5,4-e]indazole-3-carboxylate

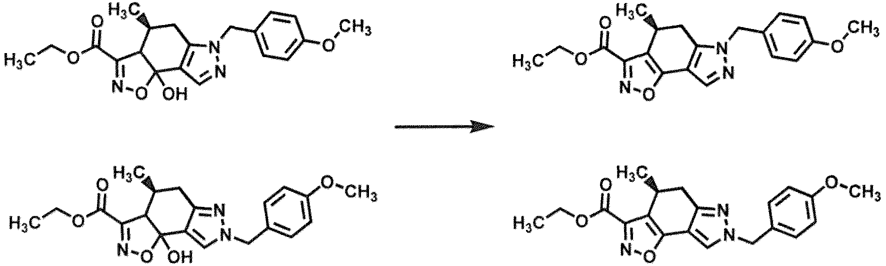

The mixture (3.4 g) of ethyl (4S)-8b-hydroxy-6-(4-methoxybenzyl)-4-methyl-3a,5,6,8b-tetrahydro-4H-isoxazolo[5,4-e]indazole-3-carboxylate and ethyl (4S)-8b-hydroxy-7-(4-methoxybenzyl)-4-methyl-3a,5,7,8b-tetrahydro-4H-isoxazolo[5,4-e]indazole-3-carboxylate obtained in the previous step was dissolved in tetrahydrofuran (77.16 ml). Under ice-cooling, to the reaction mixture were successively added triethylamine (3.69 ml) and methanesulfonic anhydride (1.998 g). The reaction mixture was stirred at 0°C for 30 min. At room temperature, to the reaction mixture was added triethylamine (3.69 ml), and the mixture was heated under reflux at 70°C overnight. The reaction mixture was concentrated under reduced pressure. To the obtained residue was added at room temperature saturated ammonium chloride aqueous solution, and the mixture was extracted with ethyl acetate. The organic layer was washed with saturated aqueous sodium chloride solution and dried over magnesium sulfate. Magnesium sulfate was filtered off, and the filtrate was concentrated under reduced pressure. The obtained residue was purified by silica gel column chromatography (ethyl acetate:hexane=25:75 to 80:20) to give a mixture (2.4 g) of the title compound.

### Step 8

### mixture of (S)-1-(6-(4-methoxybenzyl)-4-methyl-5,6-dihydro-4H-isoxazolo[5,4-e]indazol-3-yl)ethan-1-one and (S)-1-(7-(4-methoxybenzyl)-4-methyl-5,7-dihydro-4H-isoxazolo[5,4-e]indazol-3-yl)ethan-1-one

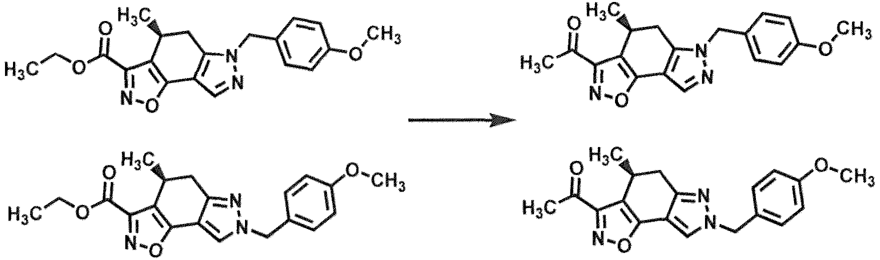

The mixture (2.4 g) of ethyl (S)-6-(4-methoxybenzyl)-4-methyl-5,6-dihydro-4H-isoxazolo[5,4-e]indazole-3-carboxylate and ethyl (S)-7-(4-methoxybenzyl)-4-methyl-5,7-dihydro-4H-isoxazolo[5,4-e]indazole-3-carboxylate obtained in the previous step was dissolved in toluene (24 ml). Under cooling at -20°C, a mixture of triethylamine (4.1 ml) and 1.04 M methylmagnesium bromide/tetrahydrofuran solution (7.85 ml) was added dropwise, and the mixture was stirred at 0°C for 1 hr. Under ice-cooling, a mixture of triethylamine (1.27 ml) and 1.08 M methylmagnesium bromide/tetrahydrofuran solution (2.4 ml) was further added dropwise, and the mixture was stirred at 0°C for 1 hr. To the reaction mixture was added 2N hydrochloric acid, and the mixture was extracted twice with ethyl acetate. The organic layer was washed successively with saturated aqueous sodium hydrogen carbonate solution and saturated aqueous sodium chloride solution, and dried over magnesium sulfate. Magnesium sulfate was filtered off, and the filtrate was concentrated under reduced pressure. The obtained residue was purified by silica gel column chromatography (ethyl acetate:hexane=30:70 to 53:47) to give a mixture (1.54 g) of the title compound.

### Step 9

### (S)-1-(4-methyl-5,6-dihydro-4H-isoxazolo[5,4-e]indazol-3-yl)ethan-1-one

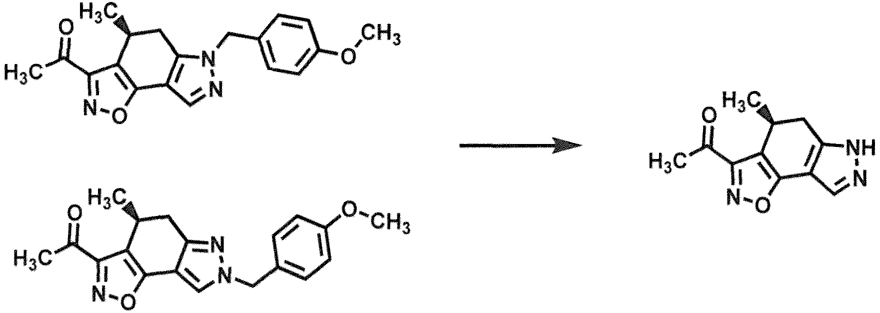

The mixture (1.2 g) of (S)-1-(6-(4-methoxybenzyl)-4-methyl-5,6-dihydro-4H-isoxazolo[5,4-e]indazol-3-yl)ethan-1-one and (S)-1-(7-(4-methoxybenzyl)-4-methyl-5,7-dihydro-4H-isoxazolo[5,4-e]indazol-3-yl)ethan-1-one obtained in the previous step was mixed with trifluoroacetic acid (11.6 ml). To the reaction mixture was added anisole (0.75 ml), and the mixture was stirred at 130°C for 1 hr under microwave (Biotage (registered trademark) Initiator+) radiation. To the reaction mixture was added toluene, and the mixture was concentrated under reduced pressure. The obtained residue was purified by silica gel column chromatography (ethyl acetate:hexane=20:80 to 85:15) to give the title compound (0.71 g).
¹H-NMR (400 MHz, CDCl₃) 0.99 (d, J=6.94Hz, 3H), 2.58 (s, 3H), 2.70 - 2.81 (m, 1H), 2.88 - 3.00 (m, 1H), 3.41 - 3.52 (m, 1H), 8.20 (s, 1H), 13.07 (s, 1H)

### Step 10

### mixture of 1-((S)-7-((1r,4r)-4-(methoxymethoxy)-4-methylcyclohexyl)-4-methyl-5,7-dihydro-4H-isoxazolo[5,4-e]indazol-3-yl)ethan-1-one and 1-((S)-6-((1r,4r)-4-(methoxymethoxy)-4-methylcyclohexyl)-4-methyl-5,6-dihydro-4H-isoxazolo[5,4-e]indazol-3-yl)ethan-1-one

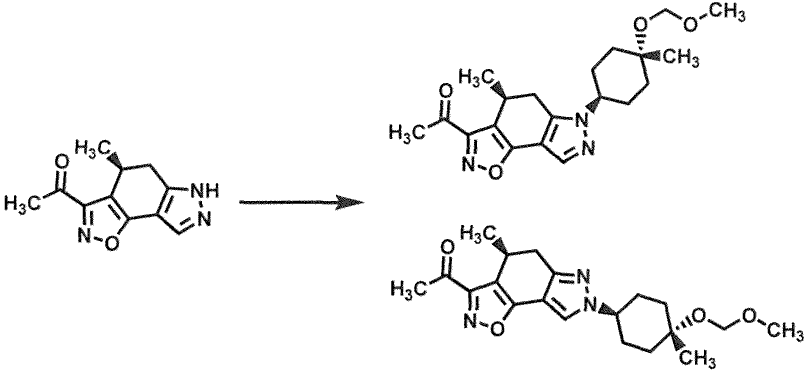

(S)-1-(4-Methyl-5,6-dihydro-4H-isoxazolo[5,4-e]indazol-3-yl)ethan-1-one (0.8 g) obtained in the previous step was mixed with dimethylformamide (0.728 ml). To the reaction mixture were added cesium carbonate (2.184 g) and cis-4-(methoxymethoxy)-4-methylcyclohexyl methanesulfonate (0.1314 g), and the mixture was stirred at 80°C for 3 hr. At room temperature, to the reaction mixture was added saturated ammonium chloride aqueous solution, and the mixture was extracted with ethyl acetate. The organic layer was washed successively with water and saturated aqueous sodium chloride solution, and dried over sodium sulfate. Sodium sulfate was filtered off, and the filtrate was concentrated under reduced pressure. The obtained residue was purified by silica gel column chromatography (ethyl acetate:hexane=12:88 to 100:0) to give a mixture (897.9 mg) of the title compound.

### Step 11

### mixture of (R)-1,1,1-trifluoro-2-((S)-7-((1r,4S)-4-(methoxymethoxy)-4-methylcyclohexyl)-4-methyl-5,7-dihydro-4H-isoxazolo[5,4-e]indazol-3-yl)propan-2-ol and (R)-1,1,1-trifluoro-2-((S)-6-((1r,4S)-4-(methoxymethoxy)-4-methylcyclohexyl)-4-methyl-5,6-dihydro-4H-isoxazolo[5,4-e]indazol-3-yl)propan-2-ol

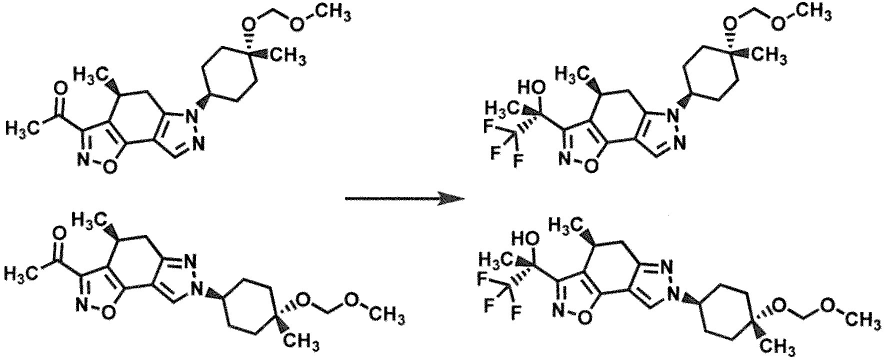

The mixture (897.9 mg) of 1-((S)-7-((1r,4r)-4-(methoxymethoxy)-4-methylcyclohexyl)-4-methyl-5,7-dihydro-4H-isoxazolo[5,4-e]indazol-3-yl)ethan-1-one and 1-((S)-6-((1r,4r)-4-(methoxymethoxy)-4-methylcyclohexyl)-4-methyl-5,6-dihydro-4H-isoxazolo[5,4-eJindazol-3-yl)ethan-1-one obtained in the previous step was mixed with N,N-dimethylacetamide (4.041 ml). Under ice-cooling, to the mixture was added lithium acetate (42.8 mg), and then (trifluoromethyl)trimethylsilane (0.481 ml) was added dropwise. The reaction mixture was stirred under ice-cooling for 1 hr. Under ice-cooling, methanol (0.404 ml) and potassium carbonate (0.359 g) were added, and the mixture was stirred at room temperature overnight. To the reaction mixture was added water, and the mixture was extracted twice with ethyl acetate. The organic layer was washed successively with water and saturated aqueous sodium chloride solution, and dried over sodium sulfate. Sodium sulfate was filtered off, and the filtrate was concentrated under reduced pressure. The obtained residue was purified by silica gel chromatography (ethyl acetate:hexane=8:92 to 66:34) to give a mixture (774 mg) of the title compound.

### Step 12

### (1S,4r)-1-methyl-4-((S)-4-methyl-3-((R)-1,1,1-trifluoro-2-hydroxypropan-2-yl)-4,5-dihydro-7H-isoxazolo[5,4-e]indazol-7-yl)cyclohexan-1-ol

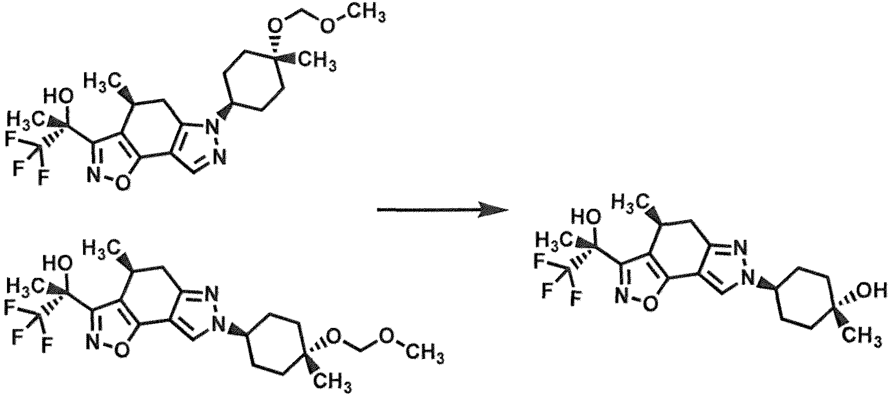

The mixture (774 mg) of (R)-1,1,1-trifluoro-2-((S)-7-((1r,4S)-4-(methoxymethoxy)-4-methylcyclohexyl)-4-methyl-5,7-dihydro-4H-isoxazolo[5,4-e]indazol-3-yl)propan-2-ol and (R)-1,1,1-trifluoro-2-((S)-6-((1r,4S)-4-(methoxymethoxy)-4-methylcyclohexyl)-4-methyl-5,6-dihydro-4H-isoxazolo[5,4-e]indazol-3-yl)propan-2-ol obtained in the previous step was mixed with tetrahydrofuran (6.656 ml). At room temperature, to the reaction mixture was added 6N hydrochloric acid (1.251 ml), and the mixture was stirred at 60°C for 30 min. Under ice-cooling, to the reaction mixture was added saturated aqueous sodium hydrogen carbonate solution, and the mixture was extracted twice with ethyl acetate. The organic layer was washed with saturated aqueous sodium chloride solution, and dried over sodium sulfate. Sodium sulfate was filtered off, and the filtrate was concentrated under reduced pressure. The obtained residue was purified by silica gel chromatography (ethyl acetate:hexane=80:20 to 100:0). A fraction containing a more-polar isomer was concentrated under reduced pressure, ethyl acetate (1 ml) was added, and the mixture was stirred at room temperature. The precipitated solid was collected by filtration to give the title compound (192.8 mg). The steric configuration of the title compound was determined by X-ray crystal structure analysis.
¹H-NMR (400 MHz, DMSO-D₆) 1.03 (d, J=6.73Hz, 3H), 1.16 (s, 3H), 1.50 - 1.62 (m, 4H), 1.74 (s, 3H), 1.87 - 1.99 (m, 4H), 2.71 (d, J=16.09Hz, 1H), 2.84 (dd, J=16.09, 6.73Hz, 1H), 3.35 - 3.37 (m, 1H), 4.14 - 4.19 (m, 1H), 4.40 (s, 1H), 7.03 (s, 1H), 8.22 (s, 1H)

The compounds of Examples 1 to 105 were obtained by methods similar to those of the above-mentioned Production Method 1 to Production Method 10, and Production Examples 1 to 16, and by using other known methods as necessary. The structural formulas and property data of the Example compounds are shown in the following Tables. Remarks in the Tables show the following contents.

### Remarks

1 (Examples 14 to 24, 26, 27, 29, 30, 32, 34 to 39, 42 to 58, 99 and 101 to 104)
racemate
2 (Example 26)
cis-trans of cyclobutane moiety undetermined, cis-trans isomer of Example 29
3 (Example 27)
cis-trans of cyclobutane moiety undetermined, cis-trans isomer of Example 30
4 (Example 29)
cis-trans of cyclobutane moiety undetermined, cis-trans isomer of Example 26
5 (Example 30)
cis-trans of cyclobutane moiety undetermined, cis-trans isomer of Example 27

**[Table 1-1]**

| Example | Structure | ¹H-NMR | Mass M+1 | Mass M-1 | Remarks |
|---|---|---|---|---|---|
| 1 | 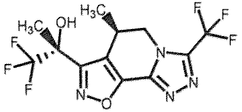 | (400 MHz, DMSO-D6) 1.21 (d, J=6.94Hz, 3H), 1.82 (s, 3H), 3.56 - 3.60 (m, 1H), 4.40 (d, J=3.24Hz, 2H), 7.49 (s, 1H) | 357 | 401 (M-1 +46) | |
| 2 | 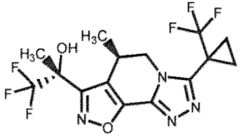 | (400 MHz, DMSO-D6) 1.15 (d, J=6.94Hz, 3H), 1.32 - 1.34 (m, 1H), 1.47 - 1.49 (m, 1H), 1.53 - 1.62 (m, 2H), 1.81 (s, 3H), 3.55 (t, J=5.90Hz, 1H), 4.22 (dd, J=12.77, 4.74Hz, 1H), 4.37 (d, J=12.77Hz, 1H), 7.42 (s, 1H) | 397 | 441 (M-1+46) | |
| 3 | 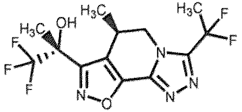 | (400 MHz, DMSO-D6) 1.15 (d, J=7.17Hz, 3H), 1.80 (s, 3H), 2.22 (t, J=19.54Hz, 3H), 3.51 - 3.57 (m, 1H), 4.33 (dd, J=13.41, 5.09Hz, 1H), 4.44 (d, J=13.41Hz, 1H), 7.43 (s, 1H) | 353 | 397 (M-1 +46) | |
| 4 | 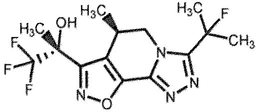 | (400 MHz, DMSO-D6) 1.16 (d, J=6.47Hz, 3H), 1.81 (t, J=7.86Hz, 6H), 1.87 (d, J=15.95Hz, 3H), 3.50 - 3.53 (m, 1H), 4.29 (dd, J=13.00, 4.97Hz, 1H), 4.51 (d, J=13.00Hz, 1H), 7.41 (s, 1H) | 349 | 393 (M-1 +46) | |
| 5 | 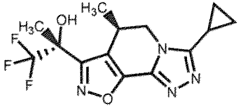 | (400 MHz, DMSO-D6) 0.95 - 1.05 (m, 4H), 1.16 (d, J=6.94Hz, 3H), 1.79 (s, 3H), 2.06 - 2.08 (m, 1H), 3.51 - 3.52 (m, 1H), 4.13 (dd, J=13.53, 5.55Hz, 1H), 4.38 (d, J=13.53Hz, 1H), 7.36 (s, 1H) | 329 | 373 (M-1 +46) | |

**[Table 1-2]**

| Example | Structure | ¹H-NMR | Mass M+1 | Mass M-1 | Remarks |
|---|---|---|---|---|---|
| 6 | 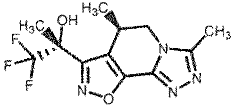 | (400 MHz, DMSO-D6) 1.15 (d, J=6.70Hz, 3H), 1.80 (s, 3H), 2.44 (s, 3H), 3.50 - 3.52 (m, 1H), 4.06 (dd, J=12.89, 4.74Hz, 1H), 4.23 (d, J=12.89Hz, 1H), 7.37 (s, 1H) | 303 | 347 (M-1 +46) | |
| 7 | 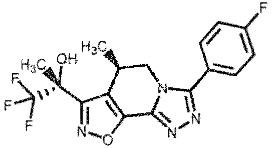 | (400 MHz, DMSO-D6) 1.14 (d, J=6.94Hz, 3H), 1.82 (s, 3H), 3.51 - 3.57 (m, 1H), 4.27 (d, J=12.72Hz, 1H), 4.41 (dd, J=13.29, 4.97Hz, 1H), 7.42 (s, 1H), 7.47 (t, J=8.79Hz, 2H), 7.81 - 7.83 (m, 2H) | 383 | 427 (M-1+46) | |
| 8 | 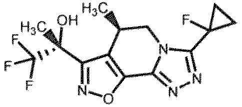 | (400 MHz, DMSO-D6) 1.17 (d, J=6.94Hz, 3H), 1.27 - 1.37 (m, 2H), 1.52 - 1.65 (m, 2H), 1.80 (s, 3H), 3.53 - 3.59 (m, 1H), 4.45 - 4.27 (m, 2H), 7.41 (s, 1H) | 347 | 391 (M-1+46) | |
| 9 | 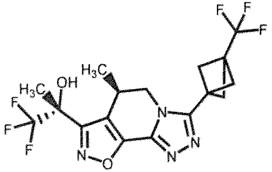 | (400 MHz, DMSO-D6) 1.16 (t, J=4.05Hz, 3H), 1.81 (s, 3H), 2.49 - 2.60 (m, 6H), 3.43 - 3.49 (m, 1H), 4.17 (dd, J=12.77, 5.20Hz, 1H), 4.43 (d, J=12.77Hz, 1H), 7.40 (s, 1H) | 423 | 467 (M-1+46) | |
| 10 | 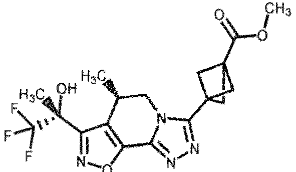 | (400 MHz, DMSO-D6) 1.15 (d, J=7.17Hz, 3H), 1.80 (s, 3H), 2.49 - 2.58 (m, 6H), 3.43 - 3.48 (m, 1H), 3.66 (s, 3H), 4.16 (dd, J=13.12, 5.20Hz, 1H), 4.40 (d, J=13.12Hz, 1H), 7.39 (s, 1H) | 413 | 457 (M-1+46) | |

**[Table 1-3]**

| Example | Structure | ¹H-NMR | Mass M+1 | Mass M-1 | Remarks |
|---|---|---|---|---|---|
| 11 | 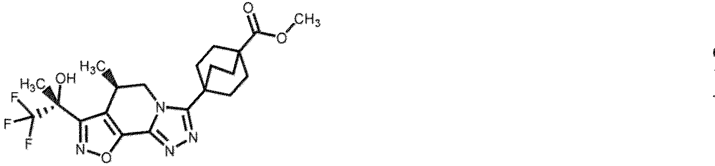 | (400 MHz, DMSO-D6) 1.13 (d, J=7.17Hz, 3H), 1.77 - 1.86 (m, 9H), 1.90 - 2.04 (m, 6H), 3.39 - 3.45 (m, 1H), 3.59 (s, 3H), 4.11 - 4.18 (m, 1H), 4.66 (d, J=12.48Hz, 1H), 7.35 (s, 1H) | 455 | 499 (M-1+46) | |
| 12 | 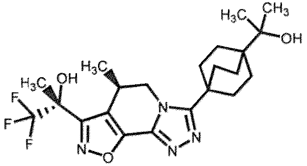 | (400 MHz, DMSO-D6) 1.01 (s, 6H), 1.12 (d, J=6.70Hz, 3H), 1.53 - 1.59 (m, 6H), 1.78 (s, 3H), 1.86 - 1.97 (m, 6H), 3.38 - 3.45 (m, 1H), 3.92 (s, 1H), 4.08 - 4.15 (m, 1H), 4.66 - 4.60 (m, 1H), 7.36 (s, 1H) | 455 | 499 (M-1+46) | |
| 13 | 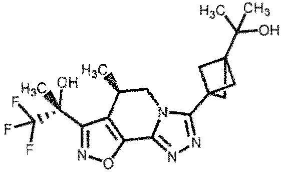 | (400 MHz, DMSO-D6) 1.09 (s, 6H), 1.14 (d, J=6.94Hz, 3H), 1.80 (s, 3H), 2.04 - 2.13 (m, 6H), 3.46 - 3.48 (m, 1H), 4.14 (dd, J=13.46, 4.97Hz, 1H), 4.30 (s, 1H), 4.36 (d, J=13.46Hz, 1H), 7.38 (s, 1H) | 413 | 457 (M-1 +46) | |
| 14 | 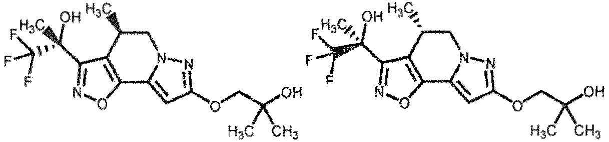 | (400 MHz, DMSO-D6) 1.18 - 1.21 (m, 9H), 1.78 (s, 3H), 3.39 - 3.41 (m, 1H), 3.87 (s, 2H), 4.08 - 4.10 (m, 2H), 4.62 (s, 1H), 6.25 (s, 1H), 7.29 (s, 1H) | 376 | 420 (M-1 +46) | 1 |

**[Table 1-4]**

| Example | Structure | ¹H-NMR | Mass M+1 | Mass M-1 | Remarks |
|---|---|---|---|---|---|
| 15 | 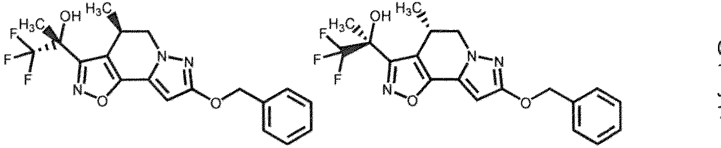 | 400 MHz, DMSO-D6) 1.20 (d, J=6.94Hz, 3H), 1.78 (s, 3H), 3.41 - 3.42 (m, 1H), 4.12 (d, J=3.24Hz, 2H), 5.20 (s, 2H), 6.32 (s, 1H), 7.30 - 7.48 (m, 6H) | 394 | 438 (M-1+46) | 1 |
| 16 | 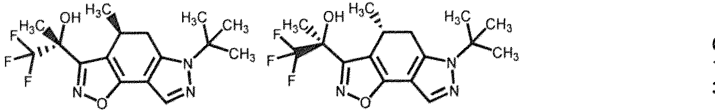 | (400 MHz, DMSO-D6) 1.04 (d, J=6.94Hz, 3H), 1.58 (s, 9H), 1.73 (s, 3H), 3.01 - 3.11 (m, 1H), 3.23 - 3.42 (m, 2H), 7.02 (s, 1H), 7.71 (s, 1H) | 344 | no peak | 1 |
| 17 | 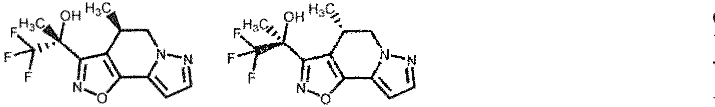 | (400 MHz, DMSO-D6) 1.16 (d, J=6.94Hz, 3H), 1.78 (s, 3H), 3.44 - 3.46 (m, 1H), 4.25 (dd, J=13.00, 5.20Hz, 1H), 4.34 (d, J=13.00Hz, 1H), 6.79 (d, J=1.85Hz, 1H), 7.29 (s, 1H), 7.64 (d, J=2.08Hz, 1H) | 288 | 332 (M-1+46) | 1 |
| 18 | 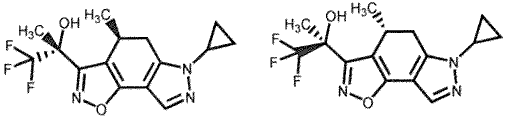 | (400 MHz, DMSO-D6) 0.93 - 1.14 (m, 7H), 1.73 (s, 3H), 2.98 - 3.06 (m, 2H), 3.35 - 3.46 (m, 1H), 3.58 - 3.68 (m, 1H), 7.04 (s, 1H), 7.71 (s, 1H) | 328 | 372 (M-1+46) | 1 |
| 19 | 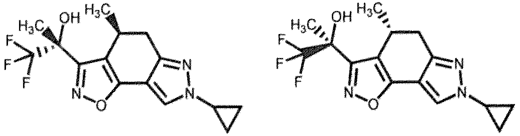 | (400 MHz, DMSO-D6) 0.92 - 0.99 (m, 2H), 1.01 (d, J=6.94Hz, 3H), 1.04 - 1.10 (m, 2H), 1.73 (s, 3H), 2.67 (d, J=15.95Hz, 1H), 2.81 (dd, J=15.95, 6.70Hz, 1H), 3.27 - 3.39 (m, 1H), 3.67 - 3.77 (m, 1H), 7.03 (s, 1H), 8.19 (s, 1H) | 328 | no peak | 1 |

**[Table 1-5]**

| Example | Structure | ¹H-NMR | Mass M+1 | Mass M-1 | Remarks |
|---|---|---|---|---|---|
| 20 | 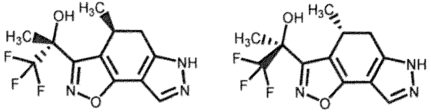 | (400 MHz, DMSO-D6) 1.01 (d, J=6.94Hz, 3H), 1.73 (s, 3H), 2.62 - 2.92 (m, 2H), 3.32 - 3.41 (m, 1H), 7.02 (s, 1H), 8.13 (s, 1H), 12.98 (s, 1H) | 288 | 286 | 1 |
| 21 | 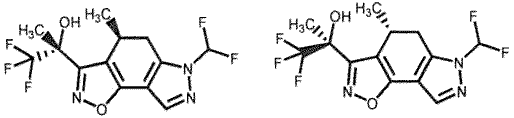 | (400 MHz, DMSO-D6) 1.06 (d, J=6.94Hz, 3H), 1.74 (s, 3H), 3.11 - 3.17 (m, 2H), 3.38 - 3.51 (m, 1H), 7.13 (s, 1H), 7.94 (t, J=57.68Hz, 1H), 8.15 (s, 1H) | 338 | 382 (M-1+46) | 1 |
| 22 | 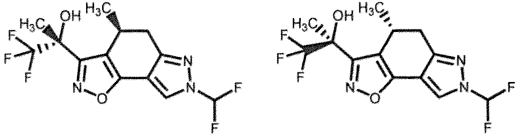 | (400 MHz, DMSO-D6) 1.05 (t, J=7.51Hz, 3H), 1.75 (s, 3H), 2.81 (d, J=15.95Hz, 1H), 2.93 (dd, J=16.41, 6.70Hz, 1H), 3.37 - 3.48 (m, 1H), 7.15 (s, 1H), 7.80 (t, J=58.84Hz, 1H), 8.67 (s, 1H) | 338 | 382 (M-1 +46) | 1 |
| 23 | 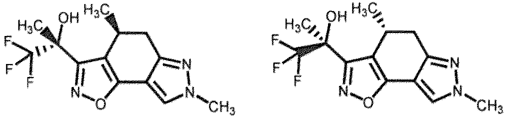 | (400 MHz, DMSO-D6) 1.01 (d, J=6.94Hz, 3H), 1.73 (s, 3H), 2.68 (d, J=15.84Hz, 1H), 2.83 (dd, J=15.84, 6.94Hz, 1H), 3.28 - 3.39 (m, 1H), 3.83 (s, 3H), 7.03 (s, 1H), 8.09 (s, 1H) | 302 | no peak | 1 |
| 24 | 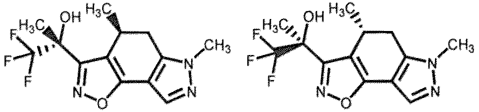 | (400 MHz, DMSO-D6) 1.05 (d, J=6.94Hz, 3H), 1.73 (s, 3H), 2.90 - 2.96 (m, 2H), 3.29 - 3.44 (m, 1H), 3.80 (s, 3H), 7.03 (s, 1H), 7.74 (s, 1H) | 302 | no peak | 1 |

**[Table 1-6]**

| Example | Structure | ¹H-NMR | Mass M+1 | Mass M-1 | Remarks |
|---|---|---|---|---|---|
| 25 | 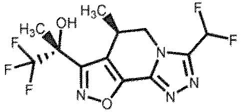 | (400 MHz, DMSO-D6) 1.16 (t, J=3.47Hz, 3H), 1.80 (s, 3H), 3.55 (t, J=6.01Hz, 1H), 4.30 (dd, J=13.35, 4.97Hz, 1H), 4.42 (d, J=13.35Hz, 1H), 7.44 (s, 1H), 7.51 (t, J=51.44Hz, 1H) | 339 | 383 (M-1+46) | |
| 26 | 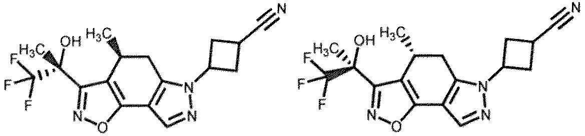 | (400 MHz, DMSO-D6) 1.04 (d, J=6.94Hz, 3H), 1.74 (s, 3H), 2.70 - 3.05 (m, 6H), 3.38 - 3.40 (m, 1H), 3.50 - 3.53 (m, 1H), 5.24 - 5.26 (m, 1H), 7.05 (s, 1H), 7.89 (s, 1H) | 367 | 411 (M-1+46) | 1,2 |
| 27 | 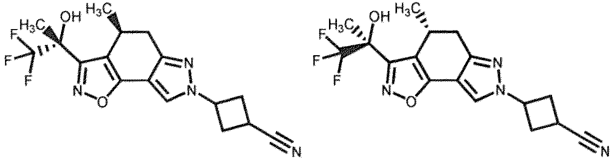 | (400 MHz, DMSO-D6) 1.04 (d, J=6.94Hz, 3H), 1.74 (s, 3H), 2.73 - 2.93 (m, 6H), 3.35 - 3.38 (m, 1H), 3.45 - 3.51 (m, 1H), 5.18 - 5.20 (m, 1H), 7.06 (s, 1H), 8.25 (s, 1H) | 367 | 411 (M-1+46) | 1, 3 |
| 28 | 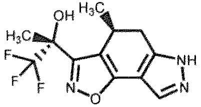 | (400 MHz, DMSO-D6) 1.01 (d, J=6.94Hz, 3H), 1.73 (s, 3H), 2.62 - 2.92 (m, 2H), 3.32 - 3.41 (m, 1H), 7.02 (s, 1H), 8.13 (s, 1H), 12.98 (s, 1H) | 288 | 286 | |
| 29 | 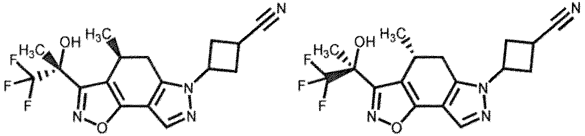 | (400 MHz, DMSO-D6) 1.04 (d, J=6.70Hz, 3H), 1.74 (s, 3H), 2.76 - 2.94 (m, 6H), 3.24 - 3.27 (m, 1H), 3.37 - 3.41 (m, 1H), 4.95 - 4.97 (m, 1H), 7.05 (s, 1H), 7.92 (s, 1H) | 367 | 411 (M-1+46) | 1,4 |

**[Table 1-7]**

| Example | Structure | ¹H-NMR | Mass M+1 | Mass M-1 | Remarks |
|---|---|---|---|---|---|
| 30 | 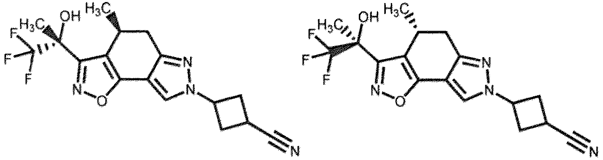 | (400 MHz, DMSO-D6) 1.02 (d, J=6.94Hz, 3H), 1.73 (s, 3H), 2.72 - 2.91 (m, 6H), 3.19 - 3.24 (m, 1H), 3.34 - 3.38 (m, 1H), 4.83 - 4.90 (m, 1H), 7.04 (s, 1H), 8.29 (s, 1H) | 367 | 411 (M-1+46) | 1,5 |
| 31 | 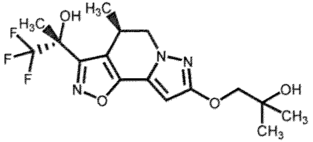 | (400 MHz, DMSO-D6) 1.16 -1.20 (m, 9H), 1.77 (s, 3H), 3.38 - 3.41 (m, 1H), 3.87 (s, 2H), 4.08 - 4.09 (m, 2H), 4.61 (s, 1H), 6.24 (s, 1H), 7.28 (s, 1H) | 376 | 420 (M-1+46) | |
| 32 | 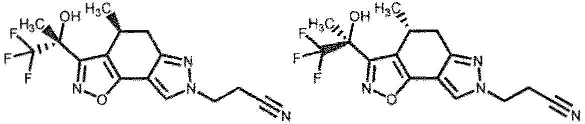 | (400 MHz, DMSO-D6) 1.04 (d, J=6.94Hz, 3H), 1.75 (s, 3H), 2.74 (d, J=14.85Hz, 1H), 2.87 (dd, J=14.85, 5.66Hz, 1H), 3.10 (t, J=6.47Hz, 2H), 3.37 - 3.40 (m, 1H), 4.41 (t, J=6.47Hz, 2H), 7.06 (s, 1H), 8.24 (s, 1H) | 341 | 385 (M-1+46) | 1 |
| 33 | 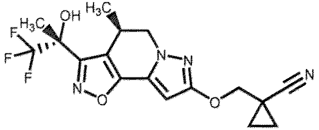 | (400 MHz, DMSO-D6) 1.14 - 1.19 (m, 7H), 1.76 (s, 3H), 3.37 - 3.42 (m, 1H), 4.08 - 4.09 (m, 2H), 4.15 (s, 2H), 6.31 (s, 1H), 7.27 (s, 1H) | 383 | 427 (M-1+46) | |
| 34 | 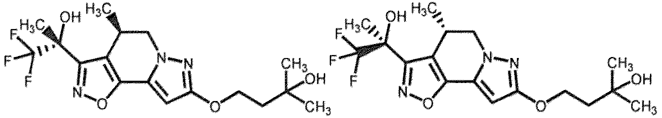 | (400 MHz, DMSO-D6) 1.15 (s, 6H), 1.19 (d, J=7.17Hz, 3H), 1.77 (s, 3H), 1.83 (t, J=7.23Hz, 2H), 3.38 - 3.40 (m, 1H), 4.09 (d, J=3.01Hz, 2H), 4.21 (t, J=7.23Hz, 2H), 4.35 (s, 1H), 6.23 (s, 1H), 7.29 (s, 1H) | 390 | 434 (M-1+46) | 1 |

**[Table 1-8]**

| Example | Structure | ¹H-NMR | Mass M+1 | Mass M-1 | Remarks |
|---|---|---|---|---|---|
| 35 | 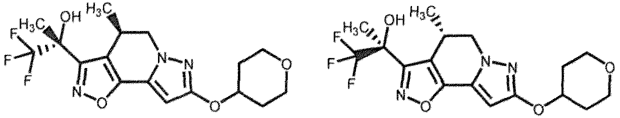 | (400 MHz, DMSO-D6) 1.19 (d, J=6.94Hz, 3H), 1.60 - 1.63 (m, 2H), 1.77 (s, 3H), 2.00 - 2.02 (m, 2H), 3.41 - 3.47 (m, 3H), 3.84 - 3.86 (m, 2H), 4.09 (d, J=3.24Hz, 2H), 4.66 - 4.67 (m, 1H), 6.28 (s, 1H), 7.27 (s, 1H) | 388 | 432 (M-1 +46) | 1 |
| 36 | 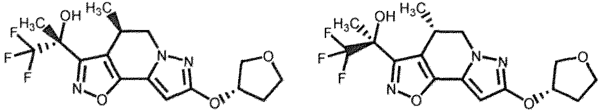 | (400 MHz, DMSO-D6) 1.20 (d, J=6.94Hz, 3H), 1.77 (s, 3H), 2.02 - 2.04 (m, 1H), 2.17 - 2.21 (m, 1H), 3.39 - 3.41 (m, 1H), 3.72 - 3.80 (m, 1H), 3.83 - 3.86 (m, 3H), 4.10 (d, J=3.24Hz, 2H), 5.14 (s, 1H), 6.27 (s, 1H), 7.28 (s, 1H) | 374 | 418 (M-1+46) | 1 |
| 37 | 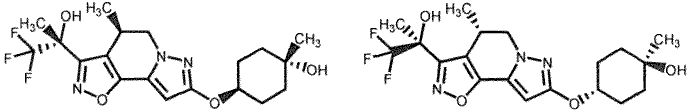 | (400 MHz, DMSO-D6) 1.16 (d, J=17.11Hz, 3H), 1.19 (q, J=6.94Hz, 3H), 1.38 - 1.41 (m, 2H), 1.57 - 1.66 (m, 4H), 1.77 (s, 3H), 1.89 - 1.93 (m, 2H), 3.37 - 3.41 (m, 1H), 4.08 (d, J=3.24Hz, 2H), 4.16 (s, 1H), 4.56 - 4.61 (m, 1H), 6.24 (s, 1H), 7.27 (s, 1H) | 416 | 460 (M-1+46) | 1 |
| 38 | 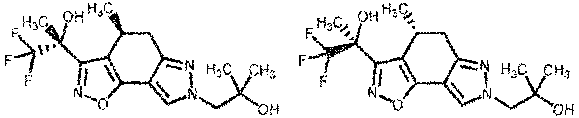 | (400 MHz, DMSO-D6) 1.02 (d, J=6.94Hz, 3H), 1.06 (s, 3H), 1.07 (s, 3H), 1.73 (s, 3H), 2.68 - 2.74 (m, 1H), 2.80 - 2.87 (m, 1H), 3.33 - 3.40 (m, 1H), 4.01 (s, 2H), 4.71 (s, 1H), 7.03 (s, 1H), 8.00 (s, 1H) | 360 | 404 (M-1+46) | 1 |
| 39 | 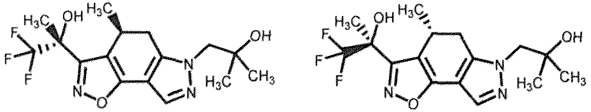 | (400 MHz, DMSO-D6) 1.02 (d, J=6.94Hz, 3H), 1.07 (s, 3H), 1.12 (s, 3H), 1.73 (s, 3H), 2.88 - 2.95 (m, 1H), 3.00 - 3.07 (m, 1H), 3.33 - 3.40 (m, 1H), 4.02 (s, 2H), 4.62 (s, 1H), 7.01 (s, 1H), 7.77 (s, 1H) | 360 | 404 (M-1+46) | 1 |

**[Table 1-9]**

| Example | Structure | ¹H-NMR | Mass M+1 | Mass M-1 | Remarks |
|---|---|---|---|---|---|
| 40 | 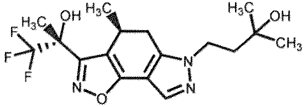 | (400 MHz, DMSO-D6) 1.06 (d, J=6.73Hz, 3H), 1.12 (s, 6H), 1.74 (s, 3H), 1.81 - 1.83 (m, 2H), 2.92 - 2.99 (m, 2H), 3.40 - 3.41 (m, 1H), 4.18 - 4.20 (m, 2H), 4.45 (s, 1H), 7.04 (s, 1H), 7.76 (s,1H) | 374 | 418 (M-1+46) | |
| 41 | 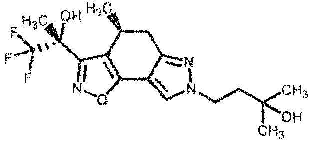 | (400 MHz, DMSO-D6) 1.03 (d, J=7.48Hz, 3H), 1.12 (s, 6H), 1.74 (s, 3H), 1.91 - 1.92 (m, 2H), 2.70 (d, J=16.39Hz, 1H), 2.84 (dd, J=16.39, 7.11Hz, 1H), 3.28 - 3.38 (m, 1H), 4.19 (t, J=7.85Hz, 2H), 4.43 (s, 1H), 7.03 (s, 1H), 8.15 (s, 1H) | 374 | 418 (M-1+46) | |
| 42 | 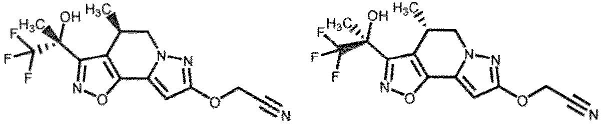 | (400 MHz, DMSO-D6) 1.20 (d, J=6.94Hz, 3H), 1.78 (s, 3H), 3.43 - 3.44 (m, 1H), 4.16 - 4.17 (m, 2H), 5.17 (s, 2H), 6.42 (s, 1H), 7.31 (s, 1H) | 343 | 387 (M-1 +46) | 1 |
| 43 | 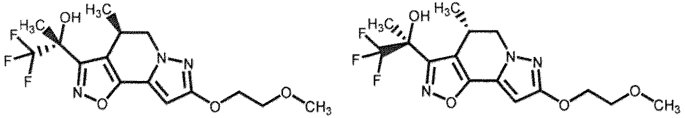 | (400 MHz, DMSO-D6) 1.19 (d, J=6.94Hz, 3H), 1.78 (s, 3H), 3.30 (s, 3H), 3.39 - 3.42 (m, 1H), 3.63 (t, J=4.51Hz, 2H), 4.09 (d, J=3.24Hz, 2H), 4.22 - 4.24 (m, 2H), 6.26 (s, 1H), 7.29 (s, 1 H) | 362 | 406 (M-1+46) | 1 |
| 44 | 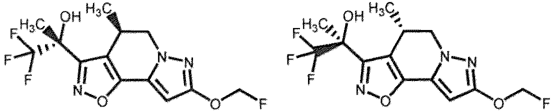 | (400 MHz, DMSO-D6) 1.20 (d, J=6.94Hz, 3H), 1.78 (s, 3H), 3.42 - 3.44 (m, 1H), 4.16 (s, 2H), 5.81 (q, J=2.39Hz, 1H), 5.95 (q, J=2.39Hz, 1H), 6.47 (s, 1H), 7.30 (s, 1H) | 336 | 380 (M-1 +46) | 1 |

**[Table 1-10]**

| Example | Structure | ¹H-NMR | Mass M+1 | Mass M-1 | Remarks |
|---|---|---|---|---|---|
| 45 | 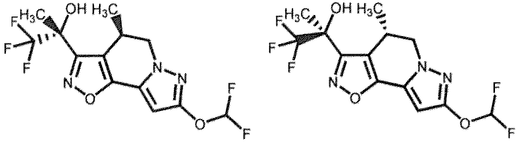 | (400 MHz, DMSO-D6) 1.18 (d, J=6.94Hz, 3H), 1.21 - 1.24 (m, 1H), 1.77 (s, 3H), 3.39 - 3.48 (m, 1H), 4.14 - 4.25 (m, 2H), 6.58 (s, 1H), 7.51 - 7.14 (m, 1H) | 354 | 398 (M-1 +46) | 1 |
| 46 | 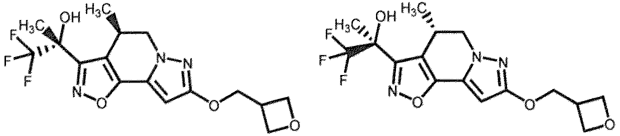 | (400 MHz, DMSO-D6) 1.20 (d, J=6.94Hz, 3H), 1.78 (s, 3H), 3.34 - 3.42 (m, 2H), 4.10 - 4.10 (m, 2H), 4.35 (d, J=6.70Hz, 2H), 4.41 (t, J=6.01Hz, 2H), 4.69 (dd, J=7.98, 6.13Hz, 2H), 6.28 (s, 1H), 7.30 (s, 1H) | 374 | 418 (M-1+46) | 1 |
| 47 | 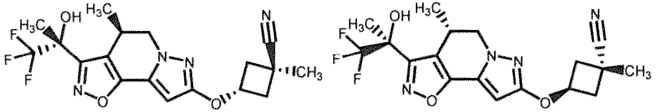 | (400 MHz, DMSO-D6) 1.19 (d, J=7.17Hz, 3H), 1.54 (s, 3H), 1.77 (s, 3H), 2.26 - 2.27 (m, 2H), 2.95 - 3.01 (m, 2H), 3.38 - 3.41 (m, 1H), 4.10 - 4.10 (m, 2H), 4.97 (t, J=7.28Hz, 1H), 6.28 (s, 1H), 7.28 (s, 1H) | 397 | 441 (M-1+46) | 1 |
| 48 | 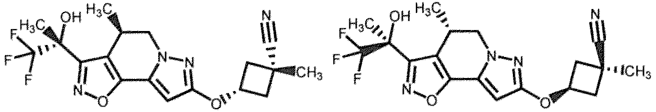 | (400 MHz, DMSO-D6) 1.19 (d, J=6.94Hz, 3H), 1.52 (s, 3H), 1.77 (s, 3H), 2.56 - 2.59 (m, 2H), 2.64 - 2.67 (m, 2H), 3.40 - 3.41 (m, 1H), 4.09 - 4.09 (m, 2H), 4.97 - 5.03 (m, 1H), 6.27 (s, 1H), 7.28 (s, 1H) | 397 | 441 (M-1+46) | 1 |
| 49 | 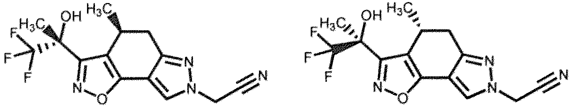 | (400 MHz, DMSO-D6) 1.05 (d, J=6.73Hz, 3H), 1.75 (s, 3H), 2.77 (d, J=16.09Hz, 1H), 2.90 (dd, J=16.09, 6.73Hz, 1H), 3.38 - 3.41 (m, 1H), 5.50 (s, 2H), 7.10 (s, 1H), 8.25 (s, 1H) | 327 | 371 (M-1 +46) | 1 |

**[Table 1-11]**

| Example | Structure | ¹H-NMR | Mass M+1 | Mass M-1 | Remarks |
|---|---|---|---|---|---|
| 50 | 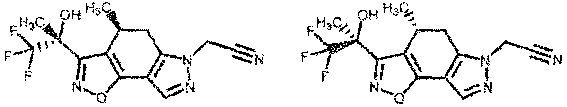 | (400 MHz, DMSO-D6) 1.06 (d, J=6.73Hz, 3H), 1.75 (s, 3H), 2.98 (dd, J=16.27, 7.11Hz, 1H), 3.09 (d, J=16.27Hz, 1H), 3.41 - 3.48 (m, 1H), 5.58 (s, 2H), 7.09 (s, 1H), 7.95 (s, 1H) | 327 | 371 (M-1+46) | 1 |
| 51 | 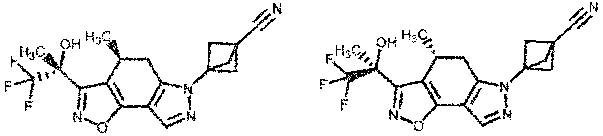 | (400 MHz, DMSO-D6) 1.07 (d, J=6.73Hz, 3H), 1.74 (s, 3H), 2.84 - 2.90 (m, 6H), 3.04 (d, J=4.49Hz, 2H), 3.37 - 3.41 (m, 1H), 7.07 (s, 1H), 7.86 (s, 1H) | 379 | 423 (M-1 +46) | 1 |
| 52 | 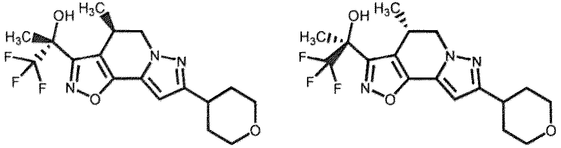 | (400 MHz, DMSO-D6) 1.15 (d, J=6.94Hz, 3H), 1.58 - 1.71 (m, 2H), 1.76 (s, 3H), 1.79 - 1.88 (m, 2H), 2.80 - 2.93 (m, 1H), 3.36 - 3.49 (m, 3H), 3.83 - 3.93 (m, 2H), 4.17 (dd, J=13.41, 5.09Hz, 1H), 4.24 (d, J=12.02Hz, 1H), 6.65 (s, 1H), 7.25 (s, 1H) | 372 | 416 (M-1+46) | 1 |
| 53 | 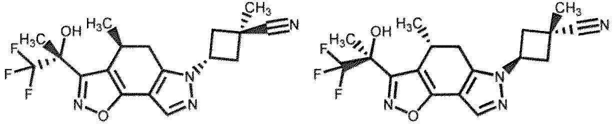 | (400 MHz, DMSO-D6) 1.03 (d, J=6.94Hz, 3H), 1.56 (s, 3H), 1.73 (s, 3H), 2.59 (dd, J=11.97, 9.02Hz, 1H), 2.73 (dd, J=11.97, 8.67Hz, 1H), 2.91 - 3.03 (m, 4H), 3.37 - 3.39 (m, 1H), 5.13 - 5.15 (m, 1H), 7.03 (s, 1H), 7.88 (s, 1H) | 381 | 425 (M-1 +46) | 1 |

**[Table 1-12]**

| Example | Structure | ¹H-NMR | Mass M+1 | Mass M-1 | Remarks |
|---|---|---|---|---|---|
| 54 | 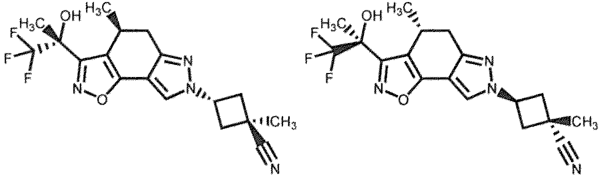 | (400 MHz, DMSO-D6) 1.03 (d, J=6.94Hz, 3H), 1.57 (s, 3H), 1.74 (s, 3H), 2.64 - 2.67 (m, 2H), 2.75 (d, J=15.95Hz, 1H), 2.87 (dd, J=15.95, 6.47Hz, 1H), 2.96 - 2.98 (m, 2H), 3.37 - 3.38 (m, 1H), 5.06 - 5.11 (m, 1H), 7.06 (s, 1H), 8.27 (s, 1H) | 381 | 425 (M-1+46) | 1 |
| 55 | 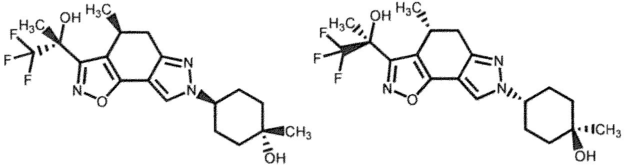 | (400 MHz, DMSO-D6) 1.03 (d, J=6.73Hz, 3H), 1.17 (s, 3H), 1.50 - 1.62 (m, 4H), 1.74 (s, 3H), 1.87 - 1.99 (m, 4H), 2.71 (d, J=16.09Hz, 1H), 2.84 (dd, J=16.09, 6.73Hz, 1H), 3.35 - 3.37 (m, 1H), 4.14 - 4.19 (m, 1H), 4.40 (s, 1H), 7.03 (s, 1H), 8.22 (s, 1H) | 400 | 444 (M-1 +46) | 1 |
| 56 | 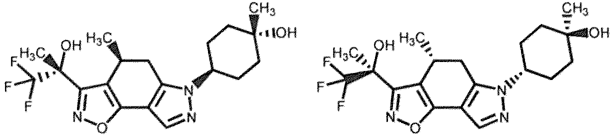 | (400 MHz, DMSO-D6) 1.05 (d, J=6.73Hz, 3H), 1.20 (s, 3H), 1.56 - 1.64 (m, 4H), 1.74 (s, 3H), 1.83 - 1.96 (m, 4H), 2.94 - 3.01 (m, 2H), 3.39 - 3.40 (m, 1H), 4.25 - 4.26 (m, 1H), 4.43 (s, 1H), 7.03 (s, 1H), 7.77 (s, 1H) | 400 | 444 (M-1+46) | 1 |
| 57 | 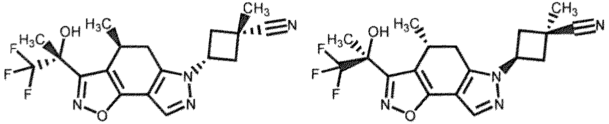 | (400 MHz, DMSO-D6) 1.04 (d, J=6.73Hz, 3H), 1.62 (s, 3H), 1.74 (s, 3H), 2.58 - 2.64 (m, 2H), 2.88 - 3.14 (m, 4H), 3.39 - 3.41 (m, 1H), 5.20 - 5.22 (m, 1H), 7.05 (s, 1H), 7.92 (s, 1H) | 381 | 425 (M-1+46) | 1 |

**[Table 1-13]**

| Example | Structure | ¹H-NMR | Mass M+1 | Mass M-1 | Remarks |
|---|---|---|---|---|---|
| 58 | 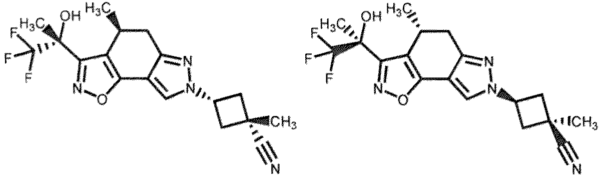 | (400 MHz, DMSO-D6) 1.04 (d, J=6.73Hz, 3H), 1.59 (s, 3H), 1.74 (s, 3H), 2.61 - 2.63 (m, 2H), 2.77 (d, J=15.71Hz, 1H), 2.88 (dd, J=15.71, 6.73Hz, 1H), 2.96 - 3.02 (m, 2H), 3.36 - 3.39 (m, 1H), 5.11 - 5.13 (m, 1H), 7.05 (d, J=3.74Hz, 1H), 8.24 (s, 1H) | 381 | 425 (M-1+46) | 1 |
| 59 | 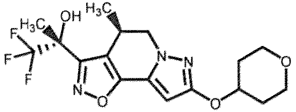 | (400 MHz, DMSO-D6) 1.19 (d, J=6.94Hz, 3H), 1.60 - 1.63 (m, 2H), 1.77 (s, 3H), 2.00 - 2.02 (m, 2H), 3.41 - 3.47 (m, 3H), 3.84 - 3.86 (m, 2H), 4.09 (d, J=3.24Hz, 2H), 4.66 - 4.67 (m, 1H), 6.28 (s, 1H), 7.27 (s, 1H) | 388 | 432 (M-1+46) | |
| 60 | 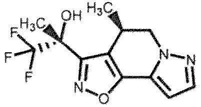 | (400 MHz, DMSO-D6) 1.16 (d, J=6.94Hz, 3H), 1.78 (s, 3H), 3.44 - 3.46 (m, 1H), 4.25 (dd, J=13.00, 5.20Hz, 1H), 4.34 (d, J=13.00Hz, 1H), 6.79 (d, J=1.85Hz, 1H), 7.29 (s, 1H), 7.64 (d, J=2.08Hz, 1H) | 288 | 332 (M-1+46) | |
| 61 | 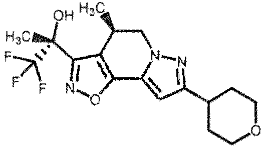 | (400 MHz, DMSO-D6) 1.15 (d, J=6.94Hz, 3H), 1.58 - 1.71 (m, 2H), 1.76 (s, 3H), 1.79 - 1.88 (m, 2H), 2.80 - 2.93 (m, 1H), 3.36 - 3.49 (m, 3H), 3.83 - 3.93 (m, 2H), 4.17 (dd, J=13.41, 5.09Hz, 1H), 4.24 (d, J=12.02Hz, 1H), 6.65 (s, 1H), 7.25 (s, 1H) | 372 | 416 (M-1+46) | |
| 62 | 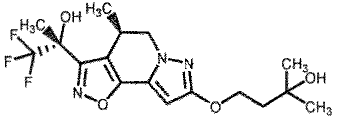 | (400 MHz, DMSO-D6) 1.15 (s, 6H), 1.19 (d, J=7.17Hz, 3H), 1.77 (s, 3H), 1.83 (t, J=7.23Hz, 2H), 3.38 - 3.40 (m, 1H), 4.09 (d, J=3.01Hz, 2H), 4.21 (t, J=7.23Hz, 2H), 4.35 (s, 1H), 6.23 (s, 1H), 7.29 (s, 1H) | 390 | 434 (M-1 +46) | |

**[Table 1-14]**

| Example | Structure | ¹H-NMR | Mass M+1 | Mass M-1 | Remarks |
|---|---|---|---|---|---|
| 63 | 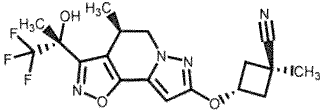 | (400 MHz, DMSO-D6) 1.19 (d, J=7.17Hz, 3H), 1.54 (s, 3H), 1.77 (s, 3H), 2.26 - 2.27 (m, 2H), 2.95 - 3.01 (m, 2H), 3.38 - 3.41 (m, 1H), 4.10 - 4.10 (m, 2H), 4.97 (t, J=7.28Hz, 1H), 6.28 (s, 1H), 7.28 (s, 1H) | 397 | 441 (M-1+46) | |
| 64 | 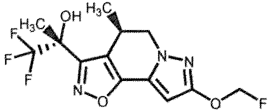 | (400 MHz, DMSO-D6) 1.20 (d, J=6.94Hz, 3H), 1.78 (s, 3H), 3.42 - 3.44 (m, 1H), 4.16 (s, 2H), 5.81 (q, J=2.39Hz, 1H), 5.95 (q, J=2.39Hz, 1H), 6.47 (s, 1H), 7.30 (s, 1H) | 336 | 380 (M-1+46) | |
| 65 | 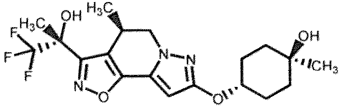 | (400 MHz, DMSO-D6) 1.16 (d, J=17.11Hz, 3H), 1.19 (q, J=6.94Hz, 3H), 1.38 - 1.41 (m, 2H), 1.57 - 1.66 (m, 4H), 1.77 (s, 3H), 1.89 - 1.93 (m, 2H), 3.37 - 3.41 (m, 1H), 4.08 (d, J=3.24Hz, 2H), 4.16 (s, 1H), 4.56 - 4.61 (m, 1H), 6.24 (s, 1H), 7.27 (s, 1H) | 416 | 460 (M-1+46) | |
| 66 | 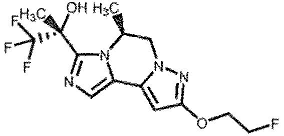 | (400 MHz, DMSO-D6) 1.27 (d, J=6.70Hz, 3H), 1.80 (s, 3H), 4.10 - 4.22 (m, 2H), 4.28 (t, J=3.81Hz, 1H), 4.36 (t, J=3.81Hz, 1H), 4.64 (t, J=3.81Hz, 1H), 4.76 (t, J=3.81Hz, 1H), 5.26 - 5.36 (m, 1H), 6.02 (s, 1H), 7.24 (s, 1H), 7.28 (s, 1H) | 349 | 347 | |
| 67 | 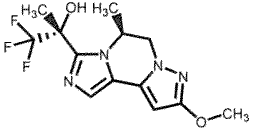 | (400 MHz, DMSO-D6) 1.27 (d, J=6.47Hz, 3H), 1.80 (s, 3H), 3.79 (s, 3H), 4.10 - 4.21 (m, 2H), 5.26 - 5.35 (m, 1H), 5.98 (s, 1H), 7.23 (s, 1H), 7.27 (s, 1H) | 317 | 315 | |

**[Table 1-15]**

| Example | Structure | ¹H-NMR | Mass M+1 | Mass M-1 | Remarks |
|---|---|---|---|---|---|
| 68 | 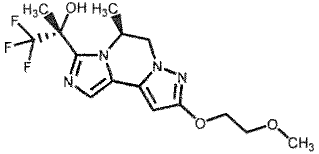 | (400 MHz, DMSO-D6) 1.27 (d, J=6.24Hz, 3H), 1.80 (s, 3H), 3.28 (s, 3H), 3.58 - 3.65 (m, 2H), 4.09 - 4.22 (m, 4H), 5.26 - 5.35 (m, 1H), 5.98 (d, J=1.16Hz, 1H), 7.23 (s, 1H), 7.27 (s, 1H) | 361 | 359 | |
| 69 | 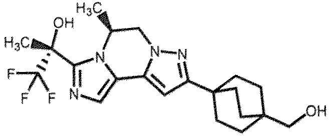 | (400 MHz, DMSO-D6) 1.21 (d, J=6.47Hz, 3H), 1.37 - 1.44 (m, 6H), 1.71 - 1.77 (m, 6H), 1.80 (s, 3H), 3.05 (d, J=5.55Hz, 2H), 4.17 - 4.23 (m, 1H), 4.28 - 4.33 (m, 2H), 5.34 - 5.27 (m, 1H), 6.33 (s, 1H), 7.20 (s, 1H), 7.24 (s, 1H) | 425 | 469 (M-1+46) | |
| 70 | 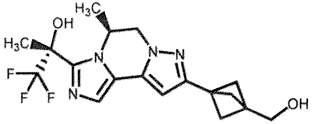 | (400 MHz, DMSO-D6) 1.22 (d, J=6.70Hz, 3H), 1.80 (s, 3H), 1.87 (s, 6H), 3.42 (d, J=5.55Hz, 2H), 4.18 - 4.25 (m, 1H), 4.30 - 4.36 (m, 1H), 4.49 - 4.53 (m, 1H), 5.36 - 5.28 (m, 1H), 6.34 (s, 1H), 7.22 (s, 1H), 7.25 (s, 1H) | 383 | 427 (M-1+46) | |
| 71 | 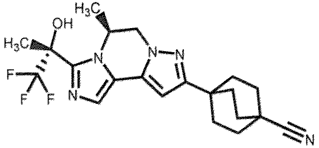 | (400 MHz, DMSO-D6) 1.22 (d, J=6.73Hz, 3H), 1.80 - 1.86 (m, 9H), 1.94 - 1.99 (m, 6H), 4.19 - 4.25 (m, 1H), 4.30 - 4.36 (m, 1H), 5.36 - 5.28 (m, 1H), 6.37 (s, 1H), 7.23 (s, 1H), 7.27 (s, 1H) | 420 | 464 (M-1+46) | |
| 72 | 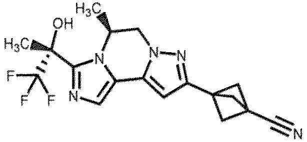 | (400 MHz, DMSO-D6) 1.22 (d, J=6.73Hz, 3H), 1.81 (s, 3H), 2.50 (s, 6H), 4.22 - 4.28 (m, 1H), 4.33 - 4.39 (m, 1H), 5.38 - 5.30 (m, 1H), 6.44 (s, 1H), 7.26 (s, 1H), 7.29 (s, 1H) | 378 | 422 (M-1 +46) | |

**[Table 1-16]**

| Example | Structure | ¹H-NMR | Mass M+1 | Mass M-1 | Remarks |
|---|---|---|---|---|---|
| 73 | 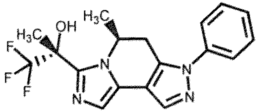 | (400 MHz, DMSO-D6) 1.09 (d, J=6.24Hz, 3H), 1.81 (s, 3H), 2.99 (d, J=16.93Hz, 1H), 3.46 (dd, J=16.93, 7.28Hz, 1H), 5.33 - 5.35 (m, 1H), 7.05 (s, 1H), 7.08 (s, 1H), 7.42 - 7.43 (m, 1H), 7.53 - 7.59 (m, 4H), 7.97 (s, 1H) | 363 | 407 (M-1+46) | |
| 74 | 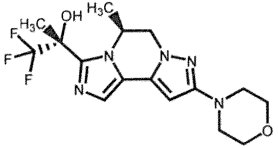 | (400 MHz, DMSO-D6) 1.26 (d, J=6.47Hz, 3H), 1.80 (s, 3H), 3.06 - 3.10 (m, 4H), 3.66 - 3.70 (m, 4H), 4.08 - 4.21 (m, 2H), 5.25 - 5.33 (m, 1H), 6.03 (s, 1H), 7.19 (s, 1H), 7.25 (s, 1H) | 372 | 370 | |
| 75 | 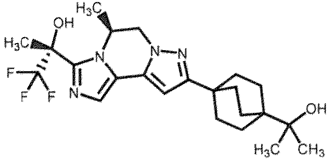 | (400 MHz, DMSO-D6) 0.99 (s, 6H), 1.21 (d, J=6.70Hz, 3H), 1.38 - 1.43 (m, 1H), 1.56 - 1.47 (m, 5H), 1.69 - 1.76 (m, 6H), 1.80 (s, 3H), 3.82 (s, 1H), 4.16 - 4.23 (m, 1H), 4.28 - 4.34 (m, 1H), 5.26 - 5.34 (m, 1H), 6.32 (s, 1H), 7.20 (s, 1H), 7.24 (s, 1H) | 453 | 497 (M-1+46) | |
| 76 | 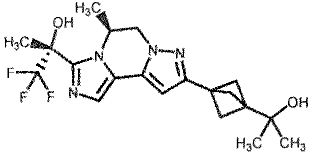 | (400 MHz, DMSO-D6) 1.11 (s, 6H), 1.28 (d, J=6.47Hz, 3H), 1.86 (s, 3H), 1.89 (s, 6H), 4.20 (s, 1H), 4.25 - 4.31 (m, 1H), 4.37 - 4.42 (m, 1H), 5.42 - 5.34 (m, 1H), 6.40 (s, 1H), 7.29 (s, 1H), 7.32 (s, 1H) | 411 | 455 (M-1+46) | |
| 77 | 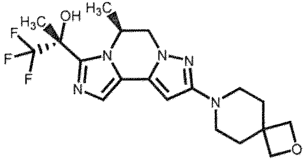 | (400 MHz, DMSO-D6) 1.26 (d, J=6.70Hz, 3H), 1.81 (s, 3H), 1.82 - 1.85 (m, 4H), 3.05 - 3.09 (m, 4H), 4.07 - 4.18 (m, 2H), 4.32 (s, 4H), 5.26 - 5.32 (m, 1H), 6.03 (s, 1H), 7.18 (s, 1H), 7.25 (s, 1H) | 412 | 410 | |

**[Table 1-17]**

| Example | Structure | ¹H-NMR | Mass M+1 | Mass M-1 | Remarks |
|---|---|---|---|---|---|
| 78 | 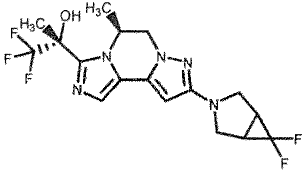 | (400 MHz, DMSO-D6) 1.27 (d, J=6.47Hz, 3H), 1.81 (s, 3H), 2.57 - 2.61 (m, 2H), 3.49 - 3.53 (m, 2H), 3.57 - 3.60 (m, 2H), 4.07 - 4.20 (m, 2H), 5.25 - 5.32 (m, 1H), 5.81 (s, 1H), 7.20 (s, 1H), 7.26 (s, 1H) | 404 | 402 | |
| 79 | 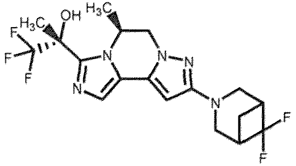 | (400 MHz, DMSO-D6) 1.29 (d, J=6.47Hz, 3H), 1.69 (dd, J=17.22, 9.83Hz, 1H), 1.82 (s, 3H), 1.94 - 1.99 (m, 1H), 2.94 - 2.99 (m, 2H), 3.45 (dd, J=10.17, 2.54Hz, 2H), 3.72 - 3.75 (m, 2H), 4.11 (dd, J=13.29, 3.81Hz, 1H), 4.17 - 4.21 (m, 1H), 5.25 - 5.33 (m, 1H), 5.87 (s, 1H), 7.22 (s, 1H), 7.26 (s, 1H) | 418 | 416 | |
| 80 | 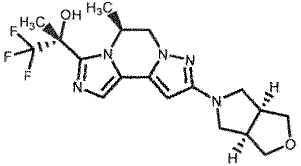 | (400 MHz, DMSO-D6) 1.27 (d, J=6.47Hz, 3H), 1.81 (s, 3H), 2.88 - 2.92 (m, 2H), 3.07 - 3.10 (m, 2H), 3.21 - 3.25 (m, 2H), 3.46 (dd, J=8.90, 4.05Hz, 2H), 3.83 - 3.87 (m, 2H), 4.10 (dd, J=13.52, 4.05Hz, 1H), 4.17 - 4.20 (m, 1H), 5.27 - 5.31 (m, 1H), 5.88 (s, 1H), 7.20 (s, 1H), 7.25 (s, 1H) | 398 | 396 | |
| 81 | 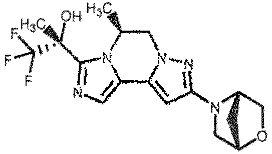 | (400 MHz, DMSO-D6) 1.28 (d, J=6.47Hz, 3H), 1.75 -1.78 (m, 1H), 1.81 (s, 3H), 1.86 (dd, J=9.94, 1.85Hz, 1H), 3.08 (d, J=9.48Hz, 1H), 3.34 - 3.37 (m, 1H), 3.66 - 3.68 (m, 1H), 3.71 (d, J=7.40Hz, 1H), 4.11 (dd, J=13.41, 3.70Hz, 1H), 4.17 - 4.20 (m, 1H), 4.28 (s, 1H), 4.54 (s, 1H), 5.29 - 5.31 (m, 1H), 5.90 (s, 1H), 7.19 (s, 1H), 7.26 (s, 1H) | 384 | 382 | |

**[Table 1-18]**

| Example | Structure | ¹H-NMR | Mass M+1 | Mass M-1 | Remarks |
|---|---|---|---|---|---|
| 82 | 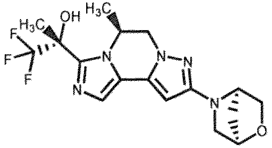 | (400 MHz, DMSO-D6) 1.27 (d, J=6.47Hz, 3H), 1.75 - 1.79 (m, 1H), 1.81 (s, 3H), 1.85 - 1.88 (m, 1H), 3.10 (d, J=9.71Hz, 1H), 3.34 - 3.35 (m, 1H), 3.66 - 3.68 (m, 1H), 3.70 (d, J=7.63Hz, 1H), 4.11 (dd, J=13.64, 3.47Hz, 1H), 4.17 - 4.20 (m, 1H), 4.28 (s, 1H), 4.54 (s, 1H), 5.28 - 5.31 (m, 1H), 5.91 (s, 1H), 7.20 (s, 1H), 7.26 (s, 1H) | 384 | 382 | |
| 83 | 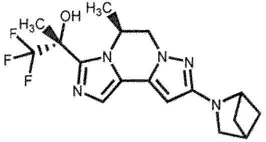 | (400 MHz, DMSO-D6) 1.24 - 1.31 (m, 5H), 1.79 - 1.84 (m, 5H), 2.82 - 2.86 (m, 1H), 3.21 (dd, J=11.44, 7.74Hz, 2H), 4.08 - 4.19 (m, 3H), 5.27 - 5.32 (m, 1H), 5.95 (s, 1H), 7.19 (s, 1H), 7.25 (s, 1H) | 368 | 366 | |
| 84 | 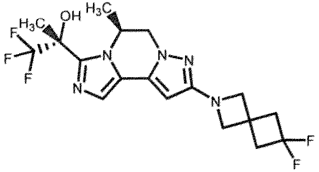 | (400 MHz, DMSO-D6) 1.26 (d, J=6.47Hz, 3H), 1.81 (s, 3H), 2.83 (t, J=12.60Hz, 4H), 3.87 (s, 4H), 4.10 - 4.16 (m, 2H), 5.28 - 5.30 (m, 1H), 5.80 (s, 1H), 7.20 (s, 1H), 7.26 (s, 1H) | 418 | 462 (M-1+46) | |
| 85 | 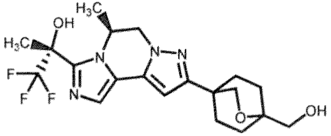 | (400 MHz, DMSO-D6) 1.20 (d, J=6.47Hz, 3H), 1.60 - 1.97 (m, 11H), 3.19 (d, J=6.01Hz, 2H), 3.84 (s, 2H), 4.18 - 4.25 (m, 1H), 4.32 - 4.37 (m, 1H), 4.49 - 4.53 (m, 1H), 5.35 - 5.28 (m, 1H), 6.41 (s, 1H), 7.22 (s, 1H), 7.25 (s, 1H) | 427 | 471 (M-1+46) | |

**[Table 1-19]**

| Example | Structure | ¹H-NMR | Mass M+1 | Mass M-1 | Remarks |
|---|---|---|---|---|---|
| 86 | 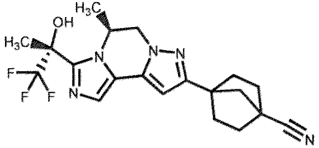 | (400 MHz, DMSO-D6) 1.22 (d, J=6.47Hz, 3H), 1.64 - 1.74 (m, 2H), 1.80 (s, 3H), 1.83 - 1.92 (m, 2H), 1.92 - 2.10 (m, 6H), 4.23 (dd, J=13.41, 3.93Hz, 1H), 4.35 (d, J=13.41Hz, 1H), 5.27 - 5.39 (m, 1H), 6.45 (s, 1H), 7.23 (s, 1H), 7.25 (s, 1H) | 406 | 404 | |
| 87 | 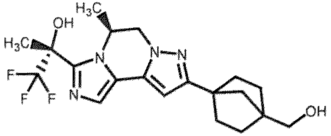 | (400 MHz, DMSO-D6) 1.23 (d, J=6.47Hz, 3H), 1.25 - 1.35 (m, 2H), 1.46 (s, 2H), 1.58 - 1.71 (m, 4H), 1.80 (s, 3H), 1.84 - 1.95 (m, 2H), 3.47 (d, J=5.32Hz, 2H), 4.21 (dd, J=13.52, 4.05Hz, 1H), 4.33 (d, J=13.52Hz, 1H), 4.42 (t, J=5.32Hz, 1H), 5.26 - 5.36 (m, 1H), 6.37 (s, 1H), 7.21 (s, 1H), 7.23 (s, 1H) | 411 | 409 | |
| 88 | 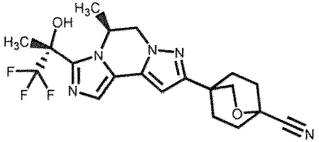 | (400 MHz, DMSO-D6) 1.20 (d, J=6.47Hz, 3H), 1.80 (s, 3H), 1.92 - 2.18 (m, 6H), 2.25 - 2.35 (m, 2H), 3.94 (s, 2H), 4.20 - 4.25 (m, 1H), 4.32 - 4.37 (m, 1H), 5.36 - 5.28 (m, 1H), 6.44 (s, 1H), 7.23 (s, 1H), 7.26 (s, 1H) | 422 | 466 (M-1 +46) | |
| 89 | 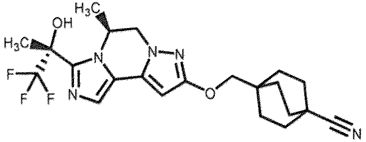 | (400 MHz, DMSO-D6) 1.26 (d, J=6.70Hz, 3H), 1.48 - 1.54 (m, 6H), 1.80 (s, 3H), 1.84 - 1.90 (m, 6H), 3.71 (s, 2H), 4.10 - 4.14 (m, 2H), 5.34 - 5.26 (m, 1H), 5.95 (s, 1H), 7.20 (s, 1H), 7.26 (s, 1H) | 450 | 494 (M-1+46) | |
| 90 | 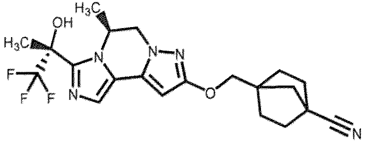 | (400 MHz, DMSO-D6) 1.27 (d, J=6.47Hz, 3H), 1.38 - 1.44 (m, 2H), 1.68 - 1.75 (m, 4H), 1.78 - 1.86 (m, 5H), 1.91 - 2.00 (m, 2H), 4.13 - 4.18 (m, 4H), 5.35 - 5.28 (m, 1H), 5.99 (s, 1H), 7.22 (s, 1H), 7.27 (s, 1H) | 436 | 480 (M-1+46) | |

**[Table 1-20]**

| Example | Structure | ¹H-NMR | Mass M+1 | Mass M-1 | Remarks |
|---|---|---|---|---|---|
| 91 | 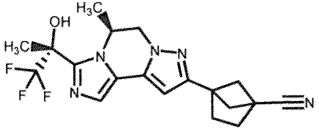 | (400 MHz, DMSO-D6) 1.22 (d, J=6.47Hz, 3H), 1.76 - 1.84 (m, 5H), 1.95 - 2.02 (m, 2H), 2.07 - 2.13 (m, 2H), 2.20 - 2.27 (m, 2H), 4.24 (dd, J=13.64, 3.93Hz, 1H), 4.36 (d, J=12.72Hz, 1H), 5.27 - 5.38 (m, 1H), 6.49 (s, 1H), 7.24 (s, 1H), 7.26 (s, 1H) | 392 | 390 | |
| 92 | 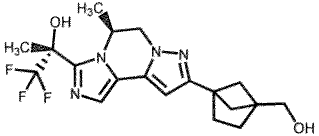 | (400 MHz, DMSO-D6) 1.23 (d, J=6.47Hz, 3H), 1.29 - 1.33 (m, 2H), 1.55 - 1.62 (m, 2H), 1.65 - 1.70 (m, 2H), 1.80 (s, 3H), 1.87 - 1.94 (m, 2H), 3.51 (d, J=5.32Hz, 2H), 4.22 (dd, J=13.52, 4.28Hz, 1H), 4.34 (d, J=12.48Hz, 1H), 4.43 (t, J=5.32Hz, 1H), 5.27 - 5.37 (m, 1H), 6.38 (s, 1H), 7.22 (s, 1H), 7.24 (s, 1H) | 397 | 395 | |
| 93 | 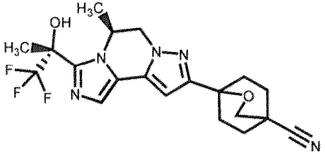 | (400 MHz, DMSO-D6) 1.20 (d, J=6.70Hz, 3H), 1.80 (s, 3H), 1.91 - 2.04 (m, 2H), 2.06 - 2.29 (m, 6H), 4.05 (s, 2H), 4.23 (dd, J=13.52, 4.05Hz, 1H), 4.34 (d, J=13.52Hz, 1H), 5.28 - 5.38 (m, 1H), 6.46 (s, 1H), 7.24 (s, 1H), 7.25 (s,1H) | 422 | 420 | |
| 94 | 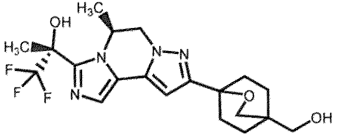 | (400 MHz, DMSO-D6) 1.21 (d, J=6.47Hz, 3H), 1.43 - 1.55 (m, 2H), 1.58 - 1.68 (m, 2H), 1.80 (s, 3H), 1.84 - 1.98 (m, 2H), 2.00 - 2.14 (m, 2H), 3.12 (d, J=5.32Hz, 2H), 3.69 (s, 2H), 4.22 (dd, J=13.52, 4.05Hz, 1H), 4.33 (d, J=12.72Hz, 1H), 4.48 (t, J=5.32Hz, 1H), 5.26 - 5.37 (m, 1H), 6.43 (s, 1H), 7.23 (s, 1H), 7.24 (s, 1H) | 427 | 425 | |
| 95 | 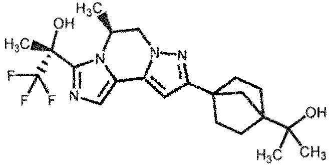 | (400 MHz, DMSO-D6) 1.24 (s, 1H), 1.26 (s, 6H), 1.35 (d, J=6.70Hz, 3H), 1.39 - 1.48 (m, 3H), 1.69 (s, 2H), 1.76 - 1.85 (m, 2H), 1.87 - 2.00 (m, 7H), 3.07 (s, 1H), 4.25 - 4.37 (m, 2H), 5.25 - 5.36 (m, 1H), 6.28 (s, 1H), 7.21 (s, 1H) | 439 | 437 | |

**[Table 1-21]**

| Example | Structure | ¹H-NMR | Mass M+1 | Mass M-1 | Remarks |
|---|---|---|---|---|---|
| 96 | 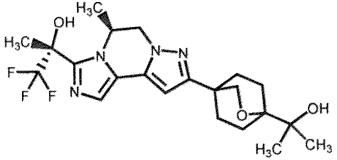 | (400 MHz, DMSO-D6) 1.04 (s, 6H), 1.21 (d, J=6.47Hz, 3H), 1.64 - 1.72 (m, 2H), 1.78 - 1.96 (m, 9H), 3.84 (s, 2H), 3.93 (s, 1H), 4.19 - 4.25 (m, 1H), 4.32 - 4.37 (m, 1H), 5.36 - 5.27 (m, 1H), 6.40 (s, 1H), 7.22 (s, 1H), 7.26 (s, 1H) | 455 | 499 (M-1 +46) | |
| 97 | 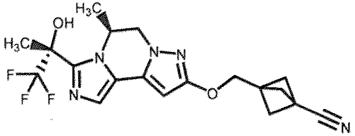 | (400 MHz, DMSO-D6) 1.25 (d, J=6.47Hz, 3H), 1.80 (s, 3H), 2.23 (s, 6H), 4.09 (s, 2H), 4.12 - 4.15 (m, 2H), 5.34 - 5.27 (m, 1H), 5.97 (s, 1H), 7.21 (s, 1H), 7.28 (s, 1H) | 408 | 452 (M-1+46) | |
| 98 | 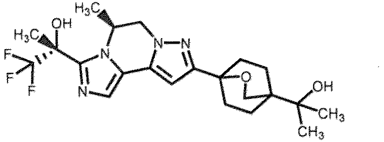 | (400 MHz, DMSO-D6) 1.01 (s, 6H), 1.21 (d, J=6.47Hz, 3H), 1.53 - 1.65 (m, 2H), 1.67 - 1.94 (m, 7H), 1.98 - 2.12 (m, 2H), 3.80 (d, J=6.24Hz, 2H), 4.08 (s, 1H), 4.22 (dd, J=13.76, 4.05Hz, 1H), 4.33 (d, J=13.76Hz, 1H), 5.27 - 5.37 (m, 1H), 6.43 (s, 1H), 7.23 (s, 1H), 7.24 (s, 1H) | 455 | 499 (M-1 +46) | |
| 99 | 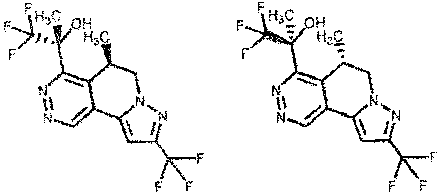 | (400 MHz, DMSO-D6) 1.13 (d, J=6.70Hz, 3H), 2.01 (s, 3H), 4.32 - 4.35 (m, 2H), 4.49 - 4.52 (m, 1H), 7.38 (s, 1H), 7.66 (s, 1H), 9.66 (s, 1H) | 367 | 411 (M-1+46) | 1 |

**[Table 1-22]**

| Example | Structure | ¹H-NMR | Mass M+1 | Mass M-1 | Remarks |
|---|---|---|---|---|---|
| 100 | 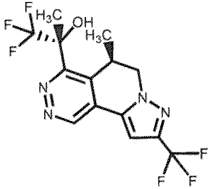 | (400 MHz, CDCI3) 1.23 (d, J=6.94Hz, 3H), 2.16 (s, 3H), 3.49 (s, 1H), 4.29 - 4.33 (m, 2H), 4.45 (d, J=12.02Hz, 1H), 7.05 (s, 1H), 9.31 (s, 1H) | 367 | 411 (M-1+46) | |
| 101 | 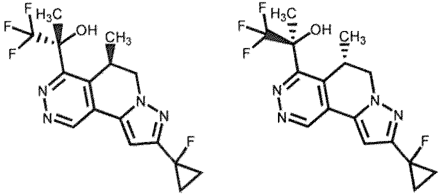 | (400 MHz, DMSO-D6) 1.09-1.18 (m, 5H), 1.41-1.50 (m, 2H), 2.00 (s, 3H), 4.12-4.38 (m, 3H), 7.24 (s, 1H), 7.31 (s, 1H), 9.58 (s, 1H) | 357 | 355 | 1 |
| 102 | 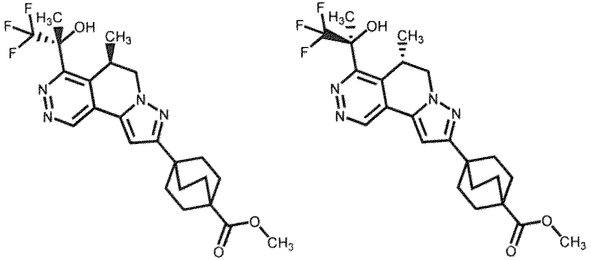 | (400 MHz, DMSO-D6) 1.03 (d, J=6.94Hz, 3H), 1.57 (s, 3H), 1.74 (s, 3H), 2.64 - 2.67 (m, 2H), 2.75 (d, J=15.95Hz, 1H), 2.87 (dd, J=15.95, 6.47Hz, 1H), 2.96 - 2.98 (m, 2H), 3.37 - 3.38 (m, 1H), 5.06 - 5.11 (m, 1H), 7.06 (s, 1H), 8.27 (s, 1H) | 381 | 425 (M-1+46) | 1 |

**[Table 1-23]**

| Example | Structure | ¹H-NMR | Mass M+1 | Mass M-1 | Remarks |
|---|---|---|---|---|---|
| 103 | 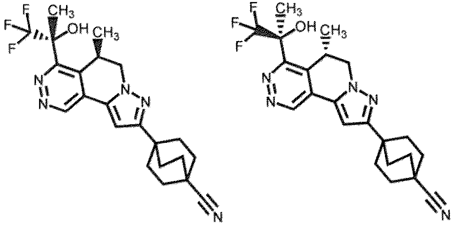 | (400 MHz, DMSO-D6) 1.09 (d, J = 6.70 Hz, 3H), 1.80-1.90 (m, 6H), 1.94-2.04 (m, 9H), 4.04-4.35 (m, 3H), 6.94 (s, 1H), 7.27 (s, 1H), 9.48 (s, 1H) | 432 | 430 | 1 |
| 104 | 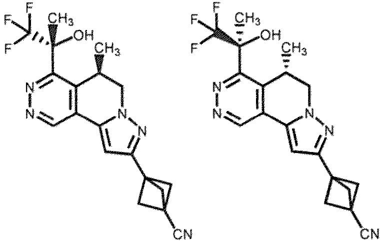 | (400 MHz, DMSO-D6) 1.09 (d, J = 6.94 Hz, 3H), 1.98 (s, 3H), 2.57 (s, 6H), 4.08-4.37 (m, 3H), 6.99 (s, 1H), 7.29 (s, 1H), 9.49 (s, 1H) | 390 | 388 | 1 |
| 105 | 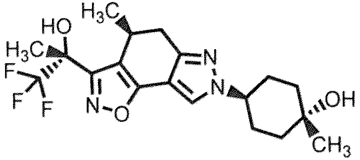 | (400 MHz, DMSO-D6) 1.03 (d, J=6.73Hz, 3H), 1.16 (s, 3H), 1.50 - 1.62 (m, 4H), 1.74 (s, 3H), 1.87 - 1.99 (m, 4H), 2.71 (d, J=16.09Hz, 1H), 2.84 (dd, J=16.09, 6.73Hz, 1H), 3.35 - 3.37 (m, 1H), 4.14 - 4.19 (m, 1H), 4.40 (s, 1H), 7.03 (s, 1H), 8.22 (s, 1H) | 400 | 444 (M-1+46) | |

### Experimental Example 1

### (Inhibitory action of PDHK2 activity)

In the case of human PDHK2 (hPDHK2, NCBI Reference Database Accession number NM_002611.4), modified hPDHK2 cDNA wherein FLAG-Tag sequence was added to the N terminal of hPDHK2 cDNA clone (pReceiver-M01/PDK2-GeneCopoeia) as the base was prepared by polymerase chain reaction (PCR) and ligated to the NdeI/EcoRI site of pET-17b vector (Merck KGaA, Catalog Number 69663-3). The recombinant construct was transformed into Escherichia coli DH5α. The recombinant clones were identified, and plasmid DNA was isolated and subjected to the DNA sequence analysis. One clone which had the expected nucleic acid sequence was selected for expression work.

For expression of hPDHK2 activity, Escherichia coli strain BL21(DE3) cells (Merck KGaA, Catalog Number 69450-4) were transformed with the pET17b vector containing modified hPDHK2 cDNA. The Escherichia coli were grown to an optical density 0.6 (600 nmol/L) at 30°C. Protein expression was induced by the addition of 500 µmol/L isopropyl-β-thiogalactopyranoside. The Escherichia coli were cultured at 20°C for 17 to for 18 hr and harvested by centrifugation. The harvested Escherichia coli was resuspended in a suspension buffer (20 mmol/L HEPES-NaOH, 500 mmol/L sodium chloride, 1% ethylene glycol, 0.1% Pluronic (registered trademark) F-68 (pH 8.0), complete, EDTA-free (Roche) (pH 8.0)), and disrupted by a microfluidizer M-110H (MIZUHO INDUSTRIAL CO., LTD.). The precipitate was removed by centrifugation and the supernatant was added to DDDDK-tagged Protein PURIFICATION GEL (MEDICAL & BIOLOGICAL LABORATORIES CO., LTD., Code No. 3329). DDDDK-tagged Protein PURIFICATION GEL was washed with a washing buffer (20 mmol/L HEPES-NaOH, 500 mmol/L sodium chloride, 1% ethylene glycol, 0.1% Pluronic F-68 (pH 8.0)) and the bound protein was eluted with elution buffer 1 (20 mmol/L HEPES-NaOH, 100 µg/mL peptide (amino acid sequence DYKDDDDK) (SEQ ID NO: 1), 500 mmol/L sodium chloride, 1% ethylene glycol, 0.1% Pluronic F-68 (pH 8.0)). The eluted fractions containing FLAG-Tagged protein were pooled, concentrated by an ultrafiltration method, added to a gel filtration column (HiLoad 26/60 Superdex 200 (GE Healthcare Life Sciences, Code No. 17-1070-01)), and eluted with elution buffer 2 (20 mmol/L HEPES-NaOH, 150 mmol/L sodium chloride, 0.5 mmol/L ethylenediaminetetraacetic acid (EDTA), 1% ethylene glycol, 0.1% Pluronic F-68 (pH 8.0)). The eluted fractions were pooled and preserved at -80°C.

PDH (porcine heart PDH complex, Sigma P7032) and 0.5 µg/mL hPDHK2 were mixed in an assay buffer (50 mmol/L 3-morpholinopropanesulfonic acid (pH 7.0), 20 mmol/L dipotassium hydrogen phosphate, 60 mmol/L potassium chloride, 2 mmol/L magnesium chloride, 0.4 mmol/L EDTA, 0.2% poloxamer, 2 mmol/L dithiothreitol) so that the final concentration of PDH was 0.025 U/mL, and the mixture was incubated at 4°C overnight to obtain a PDH/hPDHK2 complex solution.

PDH was mixed with the assay buffer so that the final concentration was 0.025 U/mL, and the mixture was incubated at 4°C overnight to prepare a PDH solution.

The test compounds were diluted with DMSO. To measure an inhibitory action of the test compound on the PDHK activity in the PDH/hPDHK2 complex solution, PDH/hPDHK2 complex solution (20 µL), test compound (1.5 µL) and 1.06 µmol/L ATP (diluted with assay buffer) (8.5 µL) were added to a 384 well microplate (Greiner Bio-One 781801) and PDHK reaction was performed at room temperature for 45 min (test compound well). DMSO (1.5 pL) was added to control wells instead of test compound. In addition, DMSO (1.5 µL) was added to blank wells instead of the test compound, and PDH solution was added instead of the PDH/hPDHK2 complex solution. To measure an inhibitory action of the test compound on the PDHK activity inherent in the PDH solution, a test compound was added and the PDH solution instead of the PDH/hPDHK2 complex solution was added to a blank + test compound well.

Then, 10 µL of substrates (5 mmol/L sodium pyruvate, 5 mmol/L Coenzyme A, 12 mmol/L NAD, 5 mmol/L thiamine pyrophosphate, diluted with assay buffer) were added. The mixture was incubated at room temperature for 90 min, and the residual PDH activity was measured.

The absorbance of each well at 340 nm was measured using a microplate reader to detect NADH produced by the PDH reaction. The PDH activity of each well was calculated from the changes in the absorbance before and after the PDH reaction. The PDH activity of the test compound-treated sample was calculated from the formula {PDH activity of test compound well - (PDH activity of blank + test compound well - PDH activity of blank well)}. The hPDHK2 inhibition rate (%) of the test compound was calculated from the formula [{(PDH activity of the test compound-treated sample - PDH activity of control well)/PDH activity of blank well - PDH activity of control well)} × 100]. IC₅₀ value was calculated according to a logistic regression method based on a test compound concentration and hPDHK2 inhibitory rate (%).

The results are shown in the following Tables. When IC₅₀ value could not be calculated, the inhibitory rate at the lowest or highest concentration of the test compound in the assay is shown. For example, the compound of Example 102 showed 3% hPDHK2 inhibitory rate at 100.0 nM.

**[Table 2-1]**

| Example No. | hPDHK2 IC₅₀ (nM) |
|---|---|
| 1 | 24.3 |
| 2 | 19.2 |
| 3 | 20.4 |
| 4 | 10.9 |
| 5 | 10.6 |
| 6 | 11.7 |
| 7 | 9.2 |
| 8 | 11.7 |
| 9 | 9.5 |
| 10 | 9.4 |
| 11 | 6.6 |
| 12 | 6.0 |
| 13 | 7.5 |
| 14 | 14.4 |
| is | 10.9 |
| 16 | 12.2 |
| 17 | 21.3 |
| 18 | 12.5 |
| 19 | 13.8 |
| 20 | 22.6 |
| 21 | 19.8 |
| 22 | 22.4 |
| 23 | 18.8 |
| 24 | 25.4 |
| 25 | 15.7 |
| 26 | 18.2 |
| 27 | 31.5 |
| 28 | 8.5 |
| 29 | 23.6 |
| 30 | 21.2 |

**[Table 2-2]**

| Example No. | hPDHK2 IC₅₀ (nM) |
|---|---|
| 31 | 13.7 |
| 32 | 20.1 |
| 33 | 8.7 |
| 34 | 14.7 |
| 35 | 17.8 |
| 36 | 14.0 |
| 37 | 17.2 |
| 38 | 25.2 |
| 39 | 14.4 |
| 40 | 5.8 |
| 41 | 9.1 |
| 42 | 19.2 |
| 43 | 28.2 |
| 44 | 18.3 |
| 45 | 26.4 |
| 46 | 15.9 |
| 47 | 25.8 |
| 48 | 18.1 |
| 49 | 19.5 |
| 50 | 14.5 |
| 51 | 13.7 |
| 52 | 15.0 |
| 53 | 8.8 |
| 54 | 7.2 |
| 55 | 14.3 |
| 56 | 10.7 |
| 57 | 28.5 |
| 58 | 20.4 |
| 59 | 7.1 |
| 60 | 6.7 |

**[Table 2-3]**

| Example No. | hPDHK2 IC₅₀ (nM) |
|---|---|
| 61 | 9.8 |
| 62 | 8 |
| 63 | 9.1 |
| 64 | 7.5 |
| 65 | 6.6 |
| 66 | 13.7 |
| 67 | 11.5 |
| 68 | 15.2 |
| 69 | 18.0 |
| 70 | 21.2 |
| 71 | 6.6 |
| 72 | 12.3 |
| 73 | 15.7 |
| 74 | 18.4 |
| 75 | 10.0 |
| 76 | 14.5 |
| 77 | 14.4 |
| 78 | 15.1 |
| 79 | 35.2 |
| 80 | 43.4 |
| 81 | 29.8 |
| 82 | 34.0 |
| 83 | 18.6 |
| 84 | 36.5 |
| 85 | 16.6 |
| 86 | 14.1 |
| 87 | 11.0 |
| 88 | 23.8 |
| 89 | 37.7 |
| 90 | 30.5 |

**[Table 2-4]**

| Example No. | hPDHK2 IC₅₀ (nM) |
|---|---|
| 91 | 23.3 |
| 92 | 31.9 |
| 93 | 33.4 |
| 94 | 49.5 |
| 95 | 35.0 |
| 96 | 32.5 |
| 97 | 8.8 |
| 98 | 9.1 |
| 99 | 23.4 |
| 100 | 11.1 |
| 101 | 29.2 |
| 102 | >100.0 (3%) |
| 103 | 24.3 |
| 104 | 36.9 |
| 105 | 10.0 |

As Formulation Examples of the present invention, the following preparations can be mentioned. However, the present invention is not limited by these Formulation Examples.

### Formulation Example 1: Production of capsule

| | |
|---|---|
| 1) compound of Example 1 | 30 mg |
| 2) crystalline cellulose | 10 mg |
| 3) lactose | 19 mg |
| 4) magnesium stearate | 1 mg |

1), 2), 3) and 4) are mixed and filled in a gelatin capsule.

### Formulation Example 2: Production of tablet

| | |
|---|---|
| 1) compound of Example 1 | 10 g |
| 2) lactose | 50 g |
| 3) cornstarch | 15 g |
| 4) carmellose calcium | 44 g |
| 5) magnesium stearate | 1 g |

The total amount of 1), 2), 3) and 30 g of 4) are kneaded with water, vacuum dried, and sieved. The sieved powder is mixed with 14 g of 4) and 1 g of 5), and the mixture is punched by a tableting machine. In this way, 1000 tablets each containing 10 mg of the compound of Example 1 per tablet are obtained.

### Formulation Example 3: Production of injection

| | |
|---|---|
| 1) compound of Example 1 | 5 mg |
| 2) D-mannitol | 5 g |
| 3) distilled water | 100 mL |

| | |
|---|---|
| 1) and 2) are dissolved in 3) and the solution is filled in a container for injection, sealed and sterilized. | |

### [Industrial Applicability]

Since the compound of the formula [I], the formula [II] or the formula [III] or a pharmaceutically acceptable salt thereof of the present invention has a PDHK inhibitory activity, it is useful as an active ingredient of a medicament for the treatment or prophylaxis of diabetes (type 1 diabetes, type 2 diabetes etc.), insulin resistance syndrome, metabolic syndrome, hyperglycemia, hyperlactacidemia, diabetic complications (diabetic neuropathy, diabetic retinopathy, diabetic nephropathy, cataract etc.), cardiac failure (acute cardiac failure, chronic cardiac failure), cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia (large-vessel type or small-vessel type vascular dementia), glaucoma, diabetic retinopathy, retinopathy of prematurity, retinal vein occlusion, ischemic optic neuropathy, or chronic kidney disease.

## Claims

1. A compound of the formula [I], the formula [II], or the formula [III], or a pharmaceutically acceptable salt thereof: 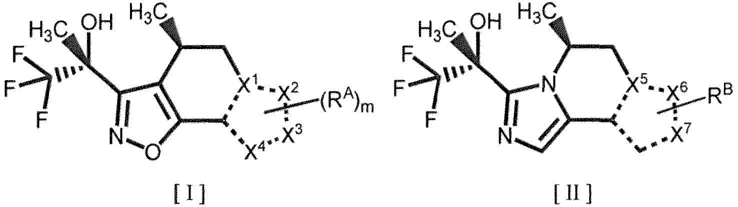 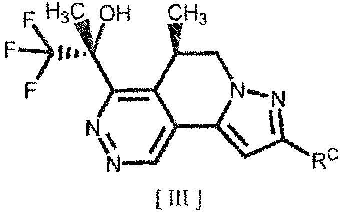 wherein
-- is a single bond or a double bond,
X¹, X², X³, X⁴, X⁵, X⁶ and X⁷ are each independently C or N,
R^{A} is
(1) C₁₋₆ alkyl wherein the C₁₋₆ alkyl is optionally substituted by one substituent selected from the group consisting of hydroxy and cyano,
(2) halo C₁₋₄ alkyl,
(3) -OR^{a} wherein R^{a} is
(i) C₁₋₆ alkyl wherein the C₁₋₆ alkyl is optionally substituted by one substituent selected from the group consisting of
(a) hydroxy,
(b) cyano,
(c) C₁₋₄ alkoxy,
(d) C₃₋₆ cycloalkyl optionally substituted by one cyano,
(e) phenyl, and
(f) 4- to 6-membered saturated heterocyclyl having one oxygen atom,
(ii) halo C₁₋₄ alkyl,
(iii) 4- to 6-membered saturated heterocyclyl having one oxygen atom, or
(iv) C₃₋₆ cycloalkyl wherein the C₃₋₆ cycloalkyl is optionally substituted by 1 or 2 substituents independently selected from the group consisting of C₁₋₆ alkyl, hydroxy, and cyano,
(4) phenyl optionally substituted by one halogen,
(5) C₃₋₆ cycloalkyl wherein the C₃₋₆ cycloalkyl is optionally substituted by 1 or 2 substituents independently selected from the group consisting of
(i) halogen,
(ii) C₁₋₉ alkyl,
(iii) halo C₁₋₄ alkyl,
(iv) hydroxy, and
(v) cyano,
(6) 4- to 6-membered saturated heterocyclyl having one oxygen atom, or
(7) bridged C₅₋₁₀ cycloalkyl wherein the bridged C₅₋₁₀ cycloalkyl is optionally substituted by one substituent selected from the group consisting of
(i) C₁₋₄ alkoxycarbonyl,
(ii) hydroxy C₁₋₄ alkyl,
(iii) halo C₁₋₄ alkyl, and
(iv) cyano,
m is 0 or 1,
R^{B} is
(1) phenyl,
(2) 4- to 6-membered saturated heterocyclyl having 1 or 2 hetero atoms independently selected from a nitrogen atom and an oxygen atom,
(3) 6- to 10-membered saturated fused heterocyclyl having 1 or 2 hetero atoms independently selected from a nitrogen atom and an oxygen atom wherein the saturated fused heterocyclyl is optionally substituted by 1 or 2 halogens,
(4) 6- to 10-membered spiro heterocyclyl having 1 or 2 hetero atoms independently selected from a nitrogen atom and an oxygen atom wherein the spiro heterocyclyl is optionally substituted by 1 or 2 halogens,
(5) 5- to 10-membered bridged heterocyclyl having 1 or 2 hetero atoms independently selected from a nitrogen atom and an oxygen atom wherein the bridged heterocyclyl is optionally substituted by 1 or 2 substituents independently selected from the group consisting of halogen, cyano, and hydroxy C₁₋₄ alkyl,
(6) bridged C₅₋₁₀ cycloalkyl wherein the bridged C₅₋₁₀ cycloalkyl is optionally substituted by one substituent selected from the group consisting of hydroxy C₁₋₉ alkyl and cyano, or
(7) -OCH₂Cy¹ wherein Cy¹ is bridged C₅₋₁₀ cycloalkyl optionally substituted by one cyano, and
R^{C} is
(1) halo C₁₋₄ alkyl,
(2) C₃₋₆ cycloalkyl optionally substituted by one halogen, or
(3) bridged C₅₋₁₀ cycloalkyl wherein the bridged C₅₋₁₀ cycloalkyl is optionally substituted by one substituent selected from the group consisting of cyano and C₁₋₄ alkoxycarbonyl.

2. The compound according to claim 1 or a pharmaceutically acceptable salt thereof, which is a compound of the formula [I] or a pharmaceutically acceptable salt thereof.

3. The compound according to claim 1 or 2, wherein R^{A} is
(1) halo C₁₋₉ alkyl,
(2) -OR^{a} wherein R^{a} is
(i) C₁₋₆ alkyl wherein the C₁₋₆ alkyl is optionally substituted by one substituent selected from the group consisting of
(a) hydroxy,
(b) C₃₋₆ cycloalkyl optionally substituted by one cyano, and
(c) 4- to 6-membered saturated heterocyclyl having one oxygen atom,
(ii) halo C₁₋₄ alkyl,
(iii) 4- to 6-membered saturated heterocyclyl having one oxygen atom, or
(iv) C₃₋₆ cycloalkyl wherein the C₃₋₆ cycloalkyl is optionally substituted by 1 or 2 substituents independently selected from the group consisting of C₁₋₆ alkyl, hydroxy, and cyano,
(3) C₃₋₆ cycloalkyl wherein the C₃₋₆ cycloalkyl is optionally substituted by 1 or 2 substituents independently selected from the group consisting of
(i) C₁₋₄ alkyl,
(ii) hydroxy, and
(iii) cyano, or
(4) 4- to 6-membered saturated heterocyclyl having one oxygen atom, or a pharmaceutically acceptable salt thereof.

4. The compound according to claim 1 or 2, wherein m is 0, or a pharmaceutically acceptable salt thereof.

5. The compound according to claim 1, or a pharmaceutically acceptable salt thereof, which is a compound of the formula [II-a]: 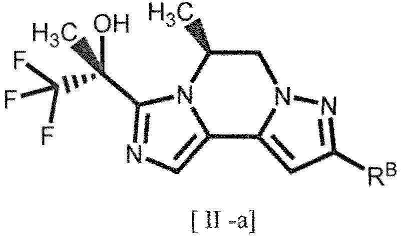 wherein R^{B} is
(1) 6- to 10-membered spiro heterocyclyl having 1 or 2 hetero atoms independently selected from a nitrogen atom and an oxygen atom wherein the spiro heterocyclyl is optionally substituted by 1 or 2 halogens,
(2) 5- to 10-membered bridged heterocyclyl having 1 or 2 hetero atoms independently selected from a nitrogen atom and an oxygen atom wherein the bridged heterocyclyl is optionally substituted by 1 or 2 substituents independently selected from the group consisting of halogen, cyano, and hydroxy C₁₋₄ alkyl,
(3) bridged C₅₋₁₀ cycloalkyl wherein the bridged C₅₋₁₀ cycloalkyl is optionally substituted by one substituent selected from the group consisting of hydroxy C₁₋₄ alkyl and cyano, or
(4) -OCH₂Cy¹ wherein Cy¹ is bridged C₅₋₁₀ cycloalkyl optionally substituted by one cyano,
or a pharmaceutically acceptable salt thereof.

6. A compound selected from the following formulas: 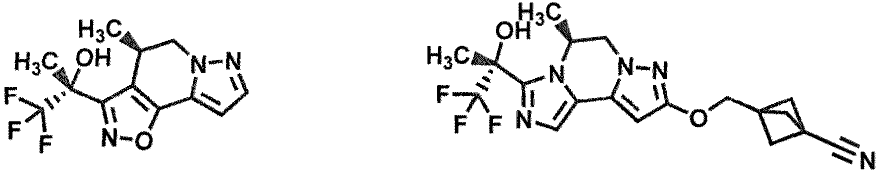 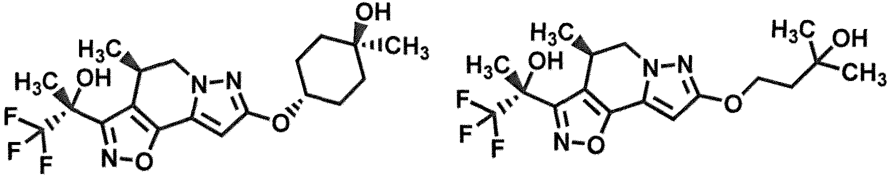 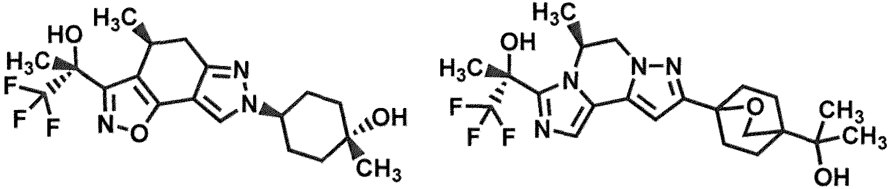 or a pharmaceutically acceptable salt thereof.

7. A pharmaceutical composition comprising the compound according to any one of claims 1 to 6 or a pharmaceutically acceptable salt thereof, and a pharmaceutically acceptable carrier.

8. A PDHK inhibitor comprising the compound according to any one of claims 1 to 6 or a pharmaceutically acceptable salt thereof.

9. A PDHK2 inhibitor comprising the compound according to any one of claims 1 to 6 or a pharmaceutically acceptable salt thereof.

10. An agent for the treatment or prophylaxis of diabetes, insulin resistance syndrome, metabolic syndrome, hyperglycemia, hyperlactacidemia, diabetic complication, cardiac failure, cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy or chronic kidney disease, the agent comprising the compound according to any one of claims 1 to 6 or a pharmaceutically acceptable salt thereof.

11. The agent according to claim 10, wherein the diabetes is type 1 diabetes or type 2 diabetes.

12. The agent according to claim 10, wherein the vascular dementia is a large-vessel type of vascular dementia or a small-vessel type of vascular dementia.

13. The agent according to claim 10, wherein the cardiac failure is acute cardiac failure or chronic cardiac failure.

14. The agent according to claim 10, wherein the pulmonary hypertension is pulmonary arterial hypertension.

15. A method for inhibiting PDHK, comprising administering a therapeutically effective amount of the compound according to any one of claims 1 to 6 or a pharmaceutically acceptable salt thereof to a mammal.

16. A method for treating or preventing a disease selected from the group consisting of diabetes, insulin resistance syndrome, metabolic syndrome, hyperglycemia, hyperlactacidemia, diabetic complication, cardiac failure, cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy and chronic kidney disease, the method comprising administering a therapeutically effective amount of the compound according to any one of claims 1 to 6 or a pharmaceutically acceptable salt thereof to a mammal.

17. The method according to claim 16, wherein the diabetes is type 1 diabetes or type 2 diabetes.

18. The method according to claim 16, wherein the vascular dementia is a large-vessel type of vascular dementia or a small-vessel type of vascular dementia.

19. The method according to claim 16, wherein the cardiac failure is acute cardiac failure or chronic cardiac failure.

20. The method according to claim 16, wherein the pulmonary hypertension is pulmonary arterial hypertension.

21. Use of the compound according to any one of claims 1 to 6 or a pharmaceutically acceptable salt thereof in the manufacture of a PDHK inhibitor.

22. Use of the compound according to any one of claims 1 to 6 or a pharmaceutically acceptable salt thereof in the manufacture of an agent for the treatment or prophylaxis of a disease selected from the group consisting of diabetes, insulin resistance syndrome, metabolic syndrome, hyperglycemia, hyperlactacidemia, diabetic complication, cardiac failure, cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy and chronic kidney disease.

23. The use according to claim 22, wherein the diabetes is type 1 diabetes or type 2 diabetes.

24. The use according to claim 22, wherein the vascular dementia is a large-vessel type of vascular dementia or a small-vessel type of vascular dementia.

25. The use according to claim 22 wherein the cardiac failure is acute cardiac failure or chronic cardiac failure.

26. The use according to claim 22 wherein the pulmonary hypertension is pulmonary arterial hypertension.

27. The compound according to any one of claims 1 to 6 or a pharmaceutically acceptable salt thereof for use in the treatment or prophylaxis of a disease selected from the group consisting of diabetes, insulin resistance syndrome, metabolic syndrome, hyperglycemia, hyperlactacidemia, diabetic complication, cardiac failure, cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy and chronic kidney disease.

28. The compound according to claim 27 or a pharmaceutically acceptable salt thereof, wherein the diabetes is type 1 diabetes or type 2 diabetes.

29. The compound according to claim 27 or a pharmaceutically acceptable salt thereof, wherein the vascular dementia is a large-vessel type of vascular dementia or a small-vessel type of vascular dementia.

30. The compound according to claim 27 or a pharmaceutically acceptable salt thereof, wherein the cardiac failure is acute cardiac failure or chronic cardiac failure.

31. The compound according to claim 27 or a pharmaceutically acceptable salt thereof, wherein the pulmonary hypertension is pulmonary arterial hypertension.
